# EUROPÄISCHE PATENTANMELDUNG

(11) **EP 2 112 149 A1**
(43) Veröffentlichungstag der Anmeldung: **28.10.2009**
(21) Anmeldenummer: 08007742.3
(22) Anmeldetag: 22.04.2008
(51) Int. Cl.: C07D 413/12, C07D 413/14, A01N 43/76

(54) **2-[(1h-Pyrazol-4-ylmethyl)-sulfonyl]-Oxazol-Derivate, 2-[(1H-Pyrazol-4-ylmethyl)-sulfanyl]-Oxazol-Derivate und chirale 2-[(1H-Pyrazol-4-ylmethyl)-sulfinyl]-Oxazol-Derivate, Verfahren zu deren Herstellung sowie deren Verwendung als Herbizide und Pflanzenwachstumsregulatoren**

(71) Anmelder: Bayer CropScience Aktiengesellschaft, 40789 Monheim (DE)
(72) Erfinder: Martelletti, Dr. Arianna, 65843 Sulzbach (DE); Dietrich, Dr. Hansjörg, 65835 Liederbach (DE); Rosinger, Dr, Christopher, 65719 Hofheim (DE); Dittgen, Dr. Jan, 60316 Frankfurt (DE); Feucht, Dr. Dieter, 65760 Eschborn (DE); Haeuser-Hahn, Dr. Isolde, 51375 Leverkusen (DE); Schmutzler, Dirk, 65795 Hattersheim (DE); Paulitz, Dr. Christian, 65835 Liederbach (DE); Kehne, Dr. Heinz, 65719 Hofheim (DE)

(57) **Zusammenfassung**

2-[(1H-Pyrazol-4-ylmethyl)-sulfonyl]-Oxazol-Derivate, 2-[(1H-Pyrazol-4-ylmethyl)-sulfanyl]-Oxazol-Derivate und chirale 2-[(1H-Pyrazol-4-ylmethyl)-sulfinyl]-Oxazol-Derivate, Verfahren zu deren Herstellung sowie deren Verwendung als Herbizide und Pflanzenwachstumsregulatoren

Gegenstand der Erfindung sind 2-[(1H-Pyrazol-4-ylmethyl)-sulfonyl]-Oxazol-Derivate, chirale 2-[(1H-Pyrazol-4-ylmethyl)-sulfinyl]-Oxazol-Derivate und 2-[(1H-Pyrazol-4-ylmethyl)-sulfanyl]-Oxazol-Derivate der allgemeinen Formel (I) und deren Salze

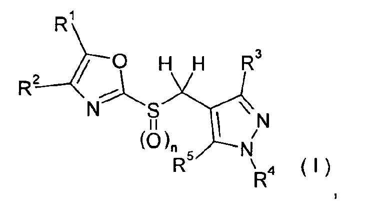

Verfahren zu deren Herstellung, sowie deren Verwendung als Herbizide und Pflanzenwachstumsregulatoren, insbesondere als Herbizide zur selektiven Bekämpfung von Schadpflanzen in Nutzpflanzenkulturen.

## Beschreibung

Die vorliegende Erfindung betrifft 2-[(1H-Pyrazol-4-yl-methyl)-sulfonyl]-oxazol-Derivate, chirale 2-[(1H-Pyrazol-4-yl-methyl)-sulfinyl]-oxazol-Derivate und 2-[(1H-Pyrazol-4-yl-methyl)-sulfanyl]-oxazol-Derivate sowie Verfahren zu deren Herstellung. Weiterer Gegenstand der vorliegenden Erfindung ist deren Verwendung als Herbizid, insbesondere als Herbizid zur selektiven Bekämpfung von Schadpflanzen in Nutzpflanzenkulturen, und als Pflanzenwachstumsregulator allein oder in Kombination mit Safenern und/oder in Mischung mit anderen Herbiziden.

Aus verschiedenen Schriften ist bereits bekannt, dass bestimmte Oxazol-Derivate herbizide bzw. schädlingsbekämpfende Eigenschaften besitzen.

So sind in der WO 2004/013112 A herbizid wirksame Oxazol-Derivate beschrieben, welche an der 2-Position des Oxazol-Ringes eine Thioethergruppe tragen, die wiederum eine Fluoroalken-Einheit umfasst.

US 4,022,607 beschreibt 2-(Alkylsulfinyl)-oxazol-Derivate, deren Herstellung, sowie deren Verwendung als Herbizid.

DE 10 254 876 A beschreibt 2-(Fluoralkenylthio)-oxazole und ihre Verwendung als Herbizide.

EP 0 435 794 A beschreibt 1-Heterocyclylsulfonyl-2-phenyl-2-propene und ihre Verwendung als Herbizide.

Schädlingsbekämpfende Eigenschaften von 2-Trifluorobutenthiooxazol-Derivaten sind beispielsweise in WO 2001/066529 A, WO 99/52874 A und WO 95/24403 A beschrieben.

1-Alkyl-5-nitro-imidazolyl-2-alkyl-heteroarylsulfide, in welchen das Heteroaryl ein substituiertes Oxazol ist, sind in DE 23 59 922 A beschrieben.

Des weiteren sind auch 2-[(1H-Pyrazol-4-ylmethyl)-sulfinyl] und 2-[(1H-Pyrazol-4-ylmethyl)-sulfonyl]-Derivate beschrieben, die herbizide Eigenschaften besitzen. So sind in WO 2007/071900 A, WO 02/62770 A und WO 2006/123088 A einige 2-[(1H-Pyrazol-4-ylmethyl)-sulfinyl] und 2-[(1H-Pyrazol-4-ylmethyl)-sulfonyl]-Derivate beschrieben, die eine geeignete substituierte (1 H-Pyrazol-4-ylmethyl)-Gruppe als Substituent an der 2-Sulfonyl-bzw 2-Sulfinyl-Gruppe tragen. Ein Verfahren zu deren Herstellung ist ebenfalls in den obengenannten Schriften beschrieben.

Des weiteren sind auch 2-(Arylmethyl-sulfonyl)-substituierte-Derivate beschrieben, die herbizide Eigenschaften besitzen. So sind in JP 2003/096059, WO 2001/112613 A und US 3,960,542 einige 2-(Arylmethyl-sulfonyl)-Derivate beschrieben, die eine geeignete substituierte Phenylmethyl-Gruppe als Substituent an der 2-SulfonylGruppe tragen. Ein Verfahren zu deren Herstellung ist ebenfalls in den obengenannten Schriften beschrieben

Die aus den oben genannten Schriften bereits bekannten Wirkstoffe weisen bei ihrer Anwendung jedoch Nachteile auf, sei es, (a) dass sie keine oder aber eine nur unzureichende herbizide Wirkung gegen Schadpflanzen, (b) ein zu geringes Spektrum der Schadpflanzen, welches mit einem Wirkstoff bekämpft werden kann, oder (c) eine zu geringe Selektivität in Nutzpflanzenkulturen besitzen.

Es ist deshalb wünschenswert, alternative chemische Wirkstoffe bereitzustellen, die gegebenenfalls mit Vorteilen als Herbizide oder Pflanzenwachstumsregulatoren eingesetzt werden können.

Erfindungsgemäß wurden nun 2-[(1H-Pyrazol-4-yl-methyl)-sulfonyl]-oxazol-Derivate, chirale 2-[(1H-Pyrazol-4-yl-methyl)-sulfinyl]-oxazol-Derivate und 2-[(1H-Pyrazol-4-yl-methyl)-sulfanyl]-oxazol-Derivate gefunden, welche Vorteile gegenüber den vorbeschriebenen Verbindungen aufweisen.

Gegenstand der vorliegenden Erfindung sind erfindungsgemäße Verbindungen der allgemeinen Formel (I) und deren agrochemisch verträglichen Salze 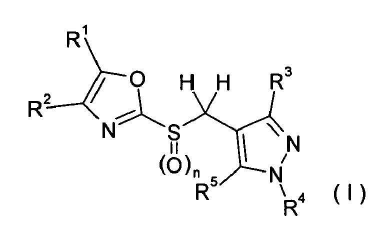 worin die Reste und Indizes die folgende Bedeutung aufweisen:
- n ist gleich 0, 1 oder 2;
- die Substituenten R¹ und R² sind, jeweils unabhängig voneinander, ausgewählt aus der Gruppe, bestehend aus
   - Wasserstoff, Halogen, Nitro, Cyano, Formyl, C(O)OH, Hydroxy, Amino,
   - (C₁-C₆)-Alkyl, (C₁-C₆)-Alkylcarbonyl, (C₁-C₆)-Alkylcarbonyl-(C₁-C₄)-alkyl, (C₁-C₆)-Alkylcarbonyloxy,
   - (C₁-C₆)-Alkoxy, (C₁-C₆)-Alkoxycarbonyl, (C₁-C₆)-Alkoxycarbonyl-(C₁-C₆)-alkyl, (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy,
   - (C₂-C₆)-Alkenyl, (C₂-C₆)-Alkenyloxy, (C₂-C₆)-Alkinyl, (C₂-C₆)-Alkinyloxy,
   - (C₁-C₆)-Alkylthio, (C₁-C₆)-Alkylsulfinyl, (C₁-C₆)-Alkylsulfonyl, (C₁-C₆)-Al-kylsulfonyloxy, (C₁-C₆)-Alkylsulfonyl-(C₁-C₆)-alkyl, (C₁-C₆)-Alkylsulfinyl-(C₁-C₆)-alkyl, C₁-C₆)-Alkylthio-(C₁-C₆)-alkyl, (C₁-C₆)-Alkylthio-(C₁-C₆)-alkoxy,
   - Mono-((C₁-C₆)-alkyl)-amino, Di-((C₁-C₆)-alkyl)-amino, N-((C₁-C₆)-Alkanoyl)-amino, Aminocarbonyl-(C₁-C₆)-alkyl, Mono-((C₁-C₆)-alkyl)-aminocarbonyl, Di-((C₁-C₆)-alkyl)-aminocarbonyl, Mono-((C₁-C₆)-alkyl)-aminosulfonyl, Di-((C₁-C₆)-alkyl)-aminosulfonyl,
   - (C₃-C₈)-Cycloalkyl, (C₃-C₈)-Cycloalkoxy, (C₃-C₈)-Cycloalkyl-(C₁-C₆)-alkyl, (C₃-C₈)-Cycloalkyl-(C₁-C₆)-alkoxy, (C₃-C₈)-Cycloalkylcarbonyl, (C₃-C₈)-Cycloalkoxycarbonyl,
   - (C₃-C₈)-Cycloalkenyl, (C₃-C₈)-Cycloalkenyloxy, (C₃-C₈)-Cycloalkylthio, (C₃-C₈)-Cycloalkylsulfinyl, (C₃-C₈)-Cycloalkylsulfonyl, (C₃-C₈)-Cycloalkylsulfonyloxy,
   - Cyano-(C₁-C₆)-alkoxy, Cyano-(C₁-C₆)-alkyl,
   - -CONH-SO₂-(C₁-C₆)-Alkyl, -NHCHO, -NHCO-(C₁-C₆)-Alkyl, -NHCO₂-(C₁-C₆)-Alkyl, -NHCONH-(C₁-C₆)-Alkyl, -NHSO₂-(C₁-C₆)-Alkyl, -OCONH-(C₁-C₆)-Alkyl, (C₁-C₆)-Alkylaminosulfonyl-(C₁-C₂)-alkyl, Di-(C₁-C₆)-alkylaminosulfonyl-(C₁-C₂)-alkyl,
   - -C(O)NHR⁹, -C(O)NR⁹R¹⁰,
      - wobei R⁹ und R¹⁰ unabhängig voneinander Wasserstoff, (C₁-C₆)-Alkyl, (C₃-C₆)-Cycloalkyl, (C₁-C₆)-Haloalkyl sind, oder
      - wobei R⁹ und R¹⁰ zusammen eine (C₁-C₆)-AlkylenGruppe bilden, die ein Sauerstoff-, oder Schwefel-Atom oder ein oder zwei Amino oder (C₁-C₆)-AlkylaminoGruppen enthalten kann,
   wobei die zuvor genannten Reste R¹ und R² einfach oder mehrfach und unabhängig voneinander substituiert sein können;
- die Substituenten R³ bis R⁵ sind, jeweils unabhängig voneinander, ausgewählt aus der Gruppe, bestehend aus
   - Wasserstoff, Halogen, Hydroxy, Cyano, Nitro, Amino, C(O)OH, Formyl,
   - (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl, (C₁-C₆)-Haloalkylcarbonyl-(C₁-C₄)-alkyl, (C₁-C₆)-Alkylcarbonyl-(C₁-C₄)-haloalkyl, (C₁-C₆)-Haloalkylcarbonyl-(C₁-C₄)-haloalkyl,
   - (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl,
   - (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl,
   - (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl,
   - (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy,
   - (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy,
   - (C₆-C₁₄)-Aryl, (C₆-C₁₄)-Aryloxy, (C₆-C₁₄)-Arylcarbonyl, (C₆-C₁₄)-Aryloxycarbonyl,
   - (C₆-C₁₄)-Aryl-(C₁-C₆)-alkyl, (C₆-C₁₄)-Aryl-(C₁-C₆)-alkoxy, (C₆-C₁₄)-Aryloxy-(C₁-C₆)-alkyl, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy,
   - (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy,
   - (C₄-C₁₄)-Arylsulfonyl, (C₆-C₁₄)-Arylthio, (C₆-C₁₄)-Arylsulfinyl,
   - Mono-((C₁-C₆)-alkyl)-amino, Mono-((C₁-C₆)-haloalkyl)-amino, Di-((C₁-C₆)-alkyl)-amino, Di-((C₁-C₆)-haloalkyl)-amino, ((C₁-C₆)-Alkyl-(C₁-C₆)-haloalkyl)-amino, N-((C₁-C₆)-Alkanoyl)-amino, N-((C₁-C₆)-Haloalkanoyl)-amino, Aminocarbonyl-(C₁-C₆)-alkyl, Mono-(C₁-C₆)-Alkylaminocarbonyl-(C₁-C₆)-alkyl, Di-(C₁-C₆)-Alkylaminocarbonyl-(C₁-C₆)-alkyl, Mono-((C₁-C₆)-alkyl)-aminocarbonyl,
   - (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy,
   - (C₃-C₈)-Cycloalkyl, (C₃-C₈)-Cycloalkoxy, (C₃-C₈)-Cycloalkyl-(C₁-C₆)-alkyl, (C₃-C₈)-Cycloalkyl-(C1-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cyclloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cyclloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy,
   - (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycyoalkenyl-(C₁-C₆)-alkoxycarbonyloxy, (C₃-C₈)-Cycyoalkenyl-(C₁-C₆)-haloalkoxycarbonyloxy,
   - (C₃-C₈)-Cycloalkylthio, (C₃-C₈)-Alkenylthio, (C₃-C₈)-Cycloalkenylthio, (C₃-C₆)-Alkinylthio,
   - Hydroxy-(C₁-C₆)-alkyl, Hydroxy-(C₁-C₆)-alkoxy, Cyano-(C₁-C₆)-alkoxy, Cyano-(C₁-C₆)-alkyl,
   - 3-Oxetanyloxy-,
   - C(O)NR⁹R¹⁰
      - wobei R⁹ und R¹⁰ unabhängig voneinander Wasserstoff, (C₁-C₆)-Alkyl, (C₃-C₆)-Cycloalkyl oder (C₁-C₆)-Haloalkyl bedeuten, oder
      - wobei R⁹ und R¹⁰ zusammen eine (C₁-C₆)-Alkylen-Gruppe bilden, die ein Sauerstoff-, oder Schwefel-Atom oder ein oder zwei Amino oder (C₁-C₆)-Alkylamino-Gruppen enthalten kann,
   wobei die zuvor genannten Reste R³ bis R⁵ einfach oder mehrfach und unabhängig voneinander substituiert sein können, und/oder
   zueinander benachbarte Reste R⁴ und R⁵ untereinander zyklisch verknüpft sein können und/oder eine (C₁-C₆)-Alkylengruppe bilden, die ein oder mehrere Sauerstoff- und/oder Schwefelatome enthalten können, wobei die (C₁-C₆)-Alkylengruppe durch Halogen einfach oder mehrfach substituiert sein kann und die jeweilgen Halogensubstituenten gleich oder verschieden sein können.

Falls die Reste, insbesondere Reste, umfassend eine Arylgruppe, eine Cycloalkylgruppe, eine Alkylgruppe, eine Alkenylgruppe, eine Alkinylgruppe und eine Cycloalkyenylgruppe, einen oder mehere Substituenten umfassen, wie beispielsweise Cycloalkyl oder Aryl, so sind die Substituenten vorzugsweise ausgewählt aus der Gruppe, bestehend aus (C₁-C₆)-Alkyl, (C₁-C₆)-Haloalkyl, (C₁-C₆)-Alkoxy, Nitro, Cyano, (C₁-C₃)-Cycloalkyl, (C₁-C₆)-Haloalkoxy, (C₁-C₆)-Alkylthio, (C₁-C₆)-Alkylcarbonyl, (C₁-C₆)-Alkoxycarbonyl oder Halogen, wobei die genannten Reste gegebenenfalls untereinander zyklisch verknüpft sein können, unter der Voraussetzung, dass sie ortho-ständig sind.

In einer ersten Ausführungsform der vorliegenden Erfindung sind Verbindungen der allgemeinen Formel (I) umfasst, in denen
- R¹ bevorzugt: ausgewählt wird aus der Gruppe, bestehend aus H, Halogen, Nitro, Cyano, Carboxyl, (C₁-C₆)-Alkyl, (C₃-C₆)-Cycloalkyl, (C₃-C₆)-Cycloalkoxy, (C₁-C₆)-Alkoxy, (C₁-C₆)-Alkylcarbonyl, (C₃-C₆)-Cycloalkylcarbonyl, (C₁-C₆)-Alkoxycarbonyl, (C₃-C₆)-Cycloalkoxycarbonyl, Mono-((C₁-C₄)-alkyl)-aminocarbonyl, Di-((C₁-C₄)-alkyl)-aminocarbonyl, Mono-((C₁-C₄)-alkyl)-aminosulfonyl, Di-((C₁-C₄)-alkyl)-aminosulfonyl, (C₁-C₄)-Alkylthio, (C₃-C₆)-Cycloalkylthio, (C₁-C₄)-Alkylsulfinyl, (C₃-C₆)-Cycloalkylsulfinyl, (C₁-C₄)-Alkylsulfonyl, (C₃-C₆)-Cycloalkylsulfonyl, (C₁-C₄)-Alkylsulfonyloxy, (C₃-C₆)-Cycloalkylsulfonyloxy, (C₂-C₃)-Alkenyl, (C₂-C₃)-Alkinyl, (C₂-C₃)-Alkenyloxy, (C₂-C₃)-Alkinyloxy, -NHCO-(C₁-C₃)-Alkyl, -NHCO₂-(C₁-C₃)-Alkyl, -NHCONH-(C₁-C₃)-Alkyl,-NHSO₂-(C₁-C₃)-Alkyl, -OCONH-(C₁-C₃)-Alkyl, -CONHR⁹, -CONR⁹R¹⁰, wobei R⁹ und R¹⁰ unabhängig voneinander Wasserstoff, (C₁-C₆)-Alkyl, (C₃-C₆)-Cycloalkyl, (C₁-C₆)-Haloalkyl ist,
wobei der zuvor genannte Rest R¹ einfach oder mehrfach und unabhängig voneinander durch Reste substituiert sein kann, welche ausgewählt sind aus der Gruppe, bestehend aus Halogen und (C₁-C₆)-Alkyl;
- R¹ besonders bevorzugt: ausgewählt ist aus der Gruppe, bestehend aus H, F, Cl, Br, I, Me, Et, NO₂, CHF₂ und CF₃; und
- R¹ ganz besonders bevorzugt: ausgewählt ist aus der Gruppe, bestehend aus H, F, Cl, Br, I und Me.

In einer zweiten Ausführungsform der vorliegenden Erfindung sind Verbindungen der allgemeinen Formel (I) umfasst, in denen
- R² bevorzugt: ausgewählt wird aus der Gruppe, bestehend aus H, Halogen, Nitro, Cyano, Carboxyl, (C₁-C₆)-Alkyl, (C₃-C₆)-Cycloalkyl, (C₃-C₆)-Cycloalkoxy, (C₁-C₆)-Alkoxy, (C₁-C₆)-Alkylcarbonyl, (C₃-C₆)-Cycloalkylcarbonyl, (C₁-C₆)-Alkoxycarbonyl, (C₃-C₆)-Cycloalkoxycarbonyl, Mono-((C₁-C₄)-alkyl)-aminocarbonyl, Di-((C₁-C₄)-alkyl)-aminocarbonyl, Mono-((C₁-C₄)-alkyl)-aminosulfonyl, Di-((C₁-C₄)-alkyl)-aminosulfonyl, (C₁-C₄)-Alkylthio, (C₃-C₆)-Cycloalkylthio, (C₁-C₄)-Alkylsulfinyl, (C₃-C₆)-Cycloalkylsulfinyl, (C₁-C₄)-Alkylsulfonyl, (C₃-C₆)-Cycloalkylsulfonyl, (C₁-C₄)-Alkylsulfonyloxy, (C₃-C₆)-Cycloalkylsulfonyloxy, (C₂-C₃)-Alkenyl, (C₂-C₃)-Alkinyl, (C₂-C₃)-Alkenyloxy, (C₂-C₃)-Alkinyloxy, -NHCO-(C₁-C₃)-Alkyl, -NHCO₂-(C₁-C₃)-Alkyl, -NHCONH-(C₁-C₃)-Alkyl, -NHSO₂-(C₁-C₃)-Alkyl, -OCONH-(C₁-C₃)-Alkyl, -CONHR⁹, -CONR⁹R¹⁰, wobei R⁹ und R¹⁰ unabhängig voneinander Wasserstoff, (C₁-C₆)-Alkyl, (C₃-C₆)-Cycloalkyl, (C₁-C₆)-Haloalkyl ist,
wobei der zuvor genannte Reste R² einfach oder mehrfach und unabhängig voneinander durch Reste substituiert sein können, welche ausgewählt sind aus der Gruppe, bestehend aus Halogen und (C₁-C₆)-Alkyl;
- R² besonders bevorzugt: ausgewählt ist aus der Gruppe, bestehend aus H, F, Cl, Br, I, Me, Et, NO₂, CHF₂ und CF₃; und
- R² ganz besonders bevorzugt: ausgewählt ist aus der Gruppe, bestehend aus H, F, Cl und Br.

Eine dritte Ausführungsform der vorliegenden Erfindung umfasst Verbindungen der allgemeinen Formel (I), in denen
- R³ bevorzugt: ausgewählt ist aus der Gruppe, bestehend aus Wasserstoff, Halogen, Hydroxy, Cyano, Nitro, Amino, (C₁-C₄)-Alkyl, (C₃-C₆)-Cycloalkyl, (C₁-C₄)-Haloalkyl, (C₁-C₄)-Alkoxy, (C₁-C₄)-Alkoxy-(C₁-C₂)-alkyl, (C₃-C₆)-Cycloalkoxy, (C₁-C₄)-Haloalkoxy, (C₁-C₄)-Alkylthio, (C₁-C₄)-Alkylthio-(C₁-C₂)-alkyl, (C₁-C₄)-Alkylsulfinyl, (C₁-C₄)-Alkylsulfinyl-(C₁-C₂)-alkyl, (C₁-C₄)-Alkylsulfonyl, (C₁-C₄)-Alkylsulfonyl-(C₁-C₂)-alkyl, Di-(C₁-C₄)-Alkylamino, (C₂-C₄)-Alkenyl, (C₂-C₄)-Alkinyl, (C₃-C₄)-Alkenyloxy, (C₃-C₄)-Alkinyloxy, (C₃-C₆)-Cycloalkyl-(C₁-C₂)-alkoxy, Hydroxy-(C₁-C₂)-alkyl, Hydroxy-(C₁-C₂)-alkoxy, Cyano-(C₁-C₂)-alkoxy, Cyano-(C₁-C₂)-alkyl; Phenyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; Phenyl-(C₁-C₂)-alkyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; Phenyl-(C₁-C₂)-alkoxy, Phenoxy, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; (C₁-C₄)-Alkylcarbonyloxy, (C₃-C₆)-Cyclloalkyl-(C₁-C₂)-alkyl, (C₁-C₄)-Alkylcarbonyl-(C₁-C₂)-alkyl, (C₁-C₄)-Alkoxycarbonyl-(C₁-C₂)-alkyl, Aminocarbonyl-(C₁-C₂)-alkyl und 3-Oxetanyloxy-, -C(O)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann; und
- R³ besonders bevorzugt: ausgewählt ist aus der Gruppe, bestehend aus H, F, Cl, Br, I, CN, Me, Et, Pr, iPr, tBu, CHF₂, CF₃, OMe, OEt, OCHF₂ und OCH₂CF₃; und
- R³ ganz besonders bevorzugt: ausgewählt ist aus der Gruppe, bestehend aus F, Cl, Br, CHF₂, CF₃, OCHF₂, OCF₃, OCH₂CF₃, Me, OMe, Pr, iPr und Et.

Eine vierte Ausführungsform der vorliegenden Erfindung umfasst Verbindungen der allgemeinen Formel (I), in denen
- R⁴ bevorzugt: ausgewählt ist aus der Gruppe, bestehend aus Wasserstoff; (C₁-C₄)-Alkyl; (C₁-C₄)-Haloalkyl; Phenyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cy-cloalkyl, (C₁-C₆)-Alkoxy, (C₁-C₆)-Haloalkoxy oder (C₁-C₆)-Alkylthio substituiert ist; Phenyl-(C₁-C₂)-alkyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; (C₃-C₆)-Cycloalkyl; (C₃-C₆)-Cycloalkyl-(C₁-C₂)-alkyl, wobei der Cycloalkylrest gegebenenfalls mit (C₁-C₄)-Alkyl substituiert ist; (C₂-C₄)-Alkenyl, (C₂-C₄)-Alkinyl, (C₁-C₄)-Alkoxy-(C₁-C₂)-alkyl, (C₁-C₄)-Alkylthio-(C₁-C₂)-alkyl, (C₁-C₄)-Alkylsulfinyl-(C₁-C₂)-alkyl, Cyano-(C₁-C₂)-alkyl, (C₁-C₄)-Alkylsulfonyl-(C₁-C₂)-alkyl, (C₁-C₄)-Alkoxycarbonyl-(C₁-C₂)-alkyl, Aminocarbonyl-(C₁-C₂)-alkyl, Mono-(C₁-C₄)-Alkylaminocarbonyl-(C₁-C₂)-alkyl, Di-(C₁-C₄)-Alkylaminocarbonyl-(C₁-C₂)-alkyl, Hydroxy-(C₁-C₄)-alkyl, (C₁-C₄)-Alkylcarbonyl-(C₁-C₄)-alkyl, (C₁-C₄)-Alkoxycarbonyl-(C₁-C₂)-alkyl, (C₁-C₄)-Alkylsulfonyl; Phenylsulfonyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; (C₁-C₄)-Alkylcarbonyl; Phenylcarbonyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; und (C₁-C₄)-Alkoxycarbonyl;
- R⁴ besonders bevorzugt: ausgewählt ist aus der Gruppe, bestehend aus H, Me, Et, Pr, cPr, iPr, Bu, iBu, sBu, tBu, cPen, cHex, CHF₂, CH₂CF₃, Ph, Ph(4-Cl), CH₂cPr, CH₂cPr(2-Me), CHMecPr, CH₂cBu, CH₂cPen, CH₂cHex, CH₂Ph, CH₂CH=CH₂, CH₂C≡CH, CHMeC≡CH, CH₂C≡CMe, CH₂OMe, CH₂OEt, CH₂CH₂OH, CH₂CH₂OMe, CH₂CH₂OEt, CH₂CH₂C(O)Me, CH₂SMe, CH₂SO₂Me, CH₂CN, CH₂C(O)OMe, CH₂C(O)OEt, CH₂C(O)NH₂, CH₂C(O)NMe₂, CH₂C(O)Me, SO₂Me, SO₂Ph, C(O)Me, C(O)Ph und C(O)OMe; und
- R⁴ ganz besonders bevorzugt: ausgewählt ist aus der Gruppe, bestehend aus Me, Et und CHF₂.

Eine fünfte Ausführungsform der vorliegenden Erfindung umfasst Verbindungen der allgemeinen Formel (I), in denen
- R⁵ bevorzugt: ausgewählt ist aus der Gruppe, bestehend aus Wasserstoff, Halogen, Hydroxy, Cyano, Nitro, Amino, (C₁-C₄)-Alkyl, (C₁-C₄)-Haloalkyl, (C₃-C₆)-Cycloalkyl, (C₁-C₄)-Alkoxy, (C₁-C₄)-Alkoxy-(C₁-C₄)-alkoxy, (C₁-C₄)-Alkoxy-(C₁-C₂)-alkyl, (C₃-C₆)-Cycloalkoxy, (C₁-C₄)-Haloalkoxy, (C₁-C₄)-Alkylthio, (C₁-C₄)-Alkylthio-(C₁-C₂)-alkyl, (C₁-₄)-Alkylsulfinyl, (C₁-C₄)-Alkyisulfinyl-(C₁-C₂)-alkyl, (C₁-C₄)-Alkylsulfonyl, (C₁-C₄)-Alkylsulfonyl-(C₁-C₂)-alkyl, Di-(C₁-C₄)-Alkylamino, (C₂-C₄)-Alkenyl, (C₂-C₄)-Haloalkenyl, Cyano-(C₁-C₄)-alkyl, (C₂-C₄)-Alkinyl, (C₃-C₄)-Alkenyloxy, (C₃-C₄)-Alkinyloxy, (C₃-C₆)-Cycloalkyl-(C₁-C₂)-alkoxy, Hydroxy-(C₁-C₂)-alkyl, Hydroxy-(C₁-C₂)-alkoxy, Cyano-(C₁-C₂)-alkoxy, Cyano-(C₁-C₂)-alkyl; Phenyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; Phenyl-(C₁-C₂)-alkyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; Phenyl-(C₁-C₂)-alkoxy; Phenoxy, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; (C₁-C₄)-Alkylcarbonyloxy, (C₃-C₆)-Cycloalkyl-(C₁-C₂)-alkyl, (C₁-C₄)-Alkylcarbonyl-(C₁-C₂)-alkyl, (C₁-C₄)-Alkoxycarbonyl-(C₁-C₂)-alkyl, Aminocarbonyl-(C₁-C₂)-alkyl und 3-Oxetanyloxy-,-C(O)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann; und
- R⁵ besonders bevorzugt: ausgewählt ist aus der Gruppe, bestehend aus H, F, Cl, Br, I, CN, Me, Et, CHF₂, CF₃, OCHF₂, OCH₂CF₃, OMe, OEt, OPr, OiPr, OtBu, SO₂Me, SO₂iPr, 3-Oxetanyloxy-, OPh, OCH₂CH=CH₂, OCH₂C≡CH OCH₂CHF₂, SEt, OCH₂CH₂OCH₃, SMe, OCH₂CH₂CH₂F, OCH(CH₂F)₂, OCH₂CF=CH₂, OCH(CH₃)CF₃, OCH₂CN, OCH(CH₃)CH₂F, OCH₂CF₂CHF₂ und OCH(CH₃)₂; und
- R⁵ ganz besonders bevorzugt: ausgewählt ist aus der Gruppe, bestehend aus H, F, Cl, Br, CHF₂, CF₃, OCHF₂ und OCH₂CF₃.

Im Rahmen der vorliegenden Erfindung ist es möglich, die einzelnen bevorzugten, besonders bevorzugten und ganz besonders bevorzugten Bedeutungen für die Substituenten R¹ bis R⁵ wie in den fünf vorstehenden Ausführungsformen definiert beliebig miteinander zu kombinieren. Das heißt, dass Verbindungen der allgemeinen Formel (I) von der vorliegenden Erfindung umfasst sind, in welchen beispielsweise der Substituent R³ eine bevorzugte Bedeutung aufweist und die Substituenten R¹, R², R⁴ und R⁵ die allgemeine Bedeutung aufweisen oder aber der Substituent R⁴ eine bevorzugte Bedeutung aufweist, der Substituent R⁵ eine besonders bevorzugte Bedeutung aufweist und die Substituenten R¹ bis R³ eine ganz besondere Bedeutung aufweisen.

Im Rahmen der vorliegenden Erfindung sind von der Verbindung der allgemeinen Formel (I) auch Verbindungen umfasst, die durch a) Protonierung, b) Alkylierung oder c) Oxidation an einem Stickstoffatom quaterniert sind.
Die Verbindungen der allgemeinen Formel (I) können gegebenenfalls durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z.B. Amino oder Alkylamino, Salze bilden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden. Salze können ebenfalls dadurch gebildet werden, dass bei geeigneten Substituenten, wie z.B. Sulfonsäuren oder Carbonsäuren, der Wasserstoff durch ein im agrochemischen Bereich geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze mit Kationen der Formel [NRR'R"R"']⁺, worin R bis R'" jeweils unabhängig einen organischen Rest, insbesondere Alkyl, Aryl, Arylalkyl oder Alkylaryl darstellen.

In der allgemeinen Formel (I) und allen übrigen Formeln in der vorliegenden Erfindung können die Reste Alkyl, Alkoxy, Haloalkyl, Haloalkoxy, Alkylamino, Alkylthio, Haloalkylthio, Alkylsulfinyl, Alkylsulfonyl, Haloalkylsulfinyl und Haloalkylsulfonyl, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen, insbesondere 1 bis 4 C-Atomen, bzw. bei ungesättigten Gruppen mit 2 bis 6 C-Atomen, insbesondere 2 bis 4 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexy und 1,4-Dimethylpentyl; Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw.

Dreifachbindung enthalten ist. Alkenyl bedeutet z.B. Vinyl, Allyl; 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Ethinyl, Propargyl, But-2-in-1-yl, But-3-in-1-yl und 1-Methyl-but-3-in-1-yl.

Halogen bedeutet Fluor, Chlor, Brom oder lod; Haloalkyl, -alkenyl und -alkinyl bedeuten durch Halogen, vorzugsweise durch Fluor, Chlor oder Brom, insbesondere durch Fluor und/oder Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Gegebenenfalls substituiertes Aryl ist vorzugsweise Phenyl, das unsubstituiert oder ein oder mehrfach, vorzugsweise bis zu dreifach durch gleiche oder verschiedene Reste ausgewählt aus der Gruppe, bestehend aus Halogen, (C₁-C₄)-Alkyl, (C₁-C₄)-Alkoxy, (C₁-C₄)- Halogenalkyl, (C₁-C₄)-Halogenalkoxy, (C₃-C₆)-Cycloalkyl, (C₁-C₆)-Alkylthio, Cyano und Nitro substituiert ist, z.B. o-, m- und p-Tolyl, Dimethylphenyle, 2-, 3- und 4-Chlorphenyl, 2-, 3- und 4-Trifluormethyl- und 2-, 3- und 4-Trichlormethylphenyl, 2,4-, 3,5-, 2,5- und 2,3-Dichlorphenyl, o-, m- und p-Methoxyphenyl substituiert ist.

Mit der Definition "mit einem oder mehreren Resten substituiert ist" sind, wenn nicht anders definiert, ein oder mehrere gleiche oder verschiedene Reste gemeint.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bei Resten mit C-Atomen sind solche mit 1 bis 6 C-Atomen, vorzugsweise 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)-Alkyl, vorzugsweise Methyl oder Ethyl, (C₁-C₄)-Haloalkyl, vorzugsweise Trifluormethyl, (C₁-C₄)-Alkoxy, vorzugsweise Methoxy oder Ethoxy, (C₁-C₄)-Haloalkoxy, Nitro und Cyano.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome (= asymmetrisch substituierte C-Atome), oder/und asymmetrische Schwefelatome in Form von Sulfoxide (n = 1), die in zwei enantiomeren Formen existieren können, oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die oben angeführten allgemeinen oder in Vorzugsbereichen angeführten Restedefinitionen gelten sowohl für die Endprodukte der Formel (I) als auch entsprechend für die jeweils zur Herstellung benötigten Ausgangs- und Zwischenprodukte. Diese Restedefinitionen können untereinander, als auch zwischen den angegebenen bevorzugten Bereichen beliebig kombiniert werden.

Die Kombinationsmöglichkeiten der verschiedenen Substituenten der allgemeinen Formel (I) sind so zu verstehen, dass die allgemeinen Grundsätze des Aufbaus chemischer Verbindungen zu beachten sind, d.h. die Formel (I) nicht Verbindungen umfasst, von denen der Fachmann weiß, dass sie chemisch nicht möglich sind.

Gegenstand der vorliegenden Erfindung sind auch Verfahren zur Herstellung der Verbindungen der allgemeinen Formel (I) und/oder deren Salze.

Die erfindungsgemäßen Verbindungen der Formel (I) können alternativ durch verschiedene Verfahren dargestellt werden.

In den nachfolgenden Verfahren werden partiell Lösemittel verwendet. In diesem Zusamenhang bezeichnen "inerte Lösemitteln" jeweils Lösemittel, die unter den jeweiligen Reaktionsbedingungen inert sind, jedoch nicht unter beliebigen Reaktionsbedingungen inert sein müssen.
a.) Zur Herstellung von optisch aktiven Sulfoxiden der Formel (III) (n = 1) bzw. Sulfonen der Formel (IV) (n = 2), worin R¹, R², R³, R⁴, R⁵ die gemäß Formel (I) zuvor angegebenen Bedeutungen haben, 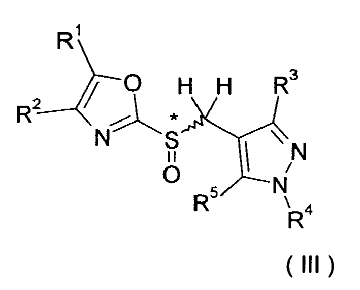 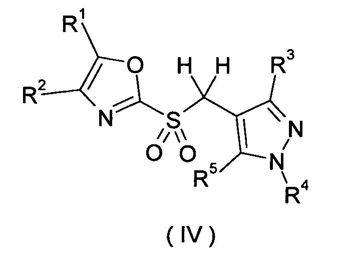 wird beispielsweise ein Thioether der allgemeinen Formel (II), 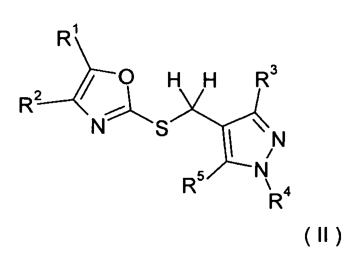 worin R¹, R², R³, R⁴, R⁵ die gemäß Formel (I) zuvor angegebenen Bedeutungen haben, mit einem Äquivalent eines Oxidationsmittels zu den optisch aktiven Sulfoxiden (III) (n = 1) oder mit zwei Äquivalenten eines Oxidationsmittels zu den Sulfonen (IV) (n = 2) oxidiert.
   Man kann die Sulfone der allgemeinen Formel (IV) auch aus den optisch aktiven Sulfoxiden der allgemeinen Formel (III) gewinnen, wobei die Sulfoxide der allgemeinen Formel (III) mit einem Äquivalent eines Oxidationsmittels zu den Sulfonen der allgemeinen Formel (IV) oxidiert werden.
   Die Oxidationsmittel, welche für diese Umsetzung verwendet werden können, unterliegen keinen besonderen Bestimmungen und es können im Allgemeinen alle Oxidationsmittel verwendet werden, welche in der Lage sind, entsprechende Schwefelverbindungen in Sulfoxidverbindungen bzw. Sulfonverbindungen zu oxidieren.
   Als Oxidationsmittel zur Herstellung der optisch aktiven Sulfoxide (n = 1) sind anorganische Peroxide wie z.B. Wasserstoffperoxid, Natriummetaperjodat, wahlweise in Gegenwart von einem Katalysator, wie z.B. Ruthenium(III) Chlorid, organische Peroxide, wie z.B. tert.-Butylhydroperoxid oder organische Persäuren, wie Peressigsäure oder bevorzugt 3-Chlor-Perbenzoesäure geeignet. Die Reaktion kann in halogenierten Kohlenwasserstoffen, z.B. Dichlormethan, 1,2-Dichlorethan, einem Alkohol, wie z.B. Methanol, oder in Dimethylformamid, Acetonitril, Wasser oder Essigsäure oder in einer Mischung der zuvor genannten Lösemittel durchgeführt werden. Die Reaktion wird in einem Temperaturbereich zwischen -80 und 120 °C, bevorzugt zwischen -20 bis 50 °C durchgeführt. Solche Verfahren sind in der Literatur bekannt und z.B. in J. Org. Chem., 58 (1993) 2791, J. Org. Chem., 68 (2003) 3849 und J. Heterocyclic Chem., 15 (1978) 1361 beschrieben.
   Als Oxidationsmittel zur Herstellung der Sulfone (n = 2) sind z.B. Wasserstoffperoxid, organische Peroxide, wie z.B. tert.-Butylhydroperoxid oder organische Persäuren, wie Peressigsäure oder bevorzugt 3-Chlor-Perbenzoesäure geeignet.
   Die enantioselektive Synthese von chiralen Sulfoxiden der Formel (III) in optisch angereicherter oder reiner Form kann, ausgehend von Thioverbindungen der Formel (II), nach Methoden erfolgen, wie sie beispielsweise in Chem. Rev., 103 (2003) 3651-3705, bzw. dort zitierter Literatur, und Adv. Synth. Catal., 347 (2005) 19-31, bzw. dort zitierter Literatur, beschrieben sind. Die absolute Konfiguration des Produkts hängt im Einzelfall von der Struktur des optisch aktiven Katalysators ab. 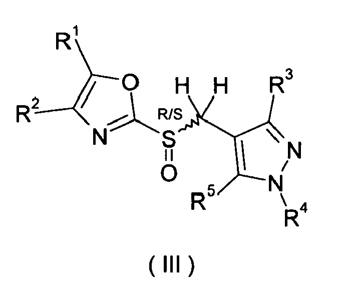 Verbindungen der allgemeinen Formel (III) bestehen aus einem Gemisch der jeweiligen, an der Sulfoxid-Funktion chiralen Enantiomere (III-*S*) und (III-*R*) 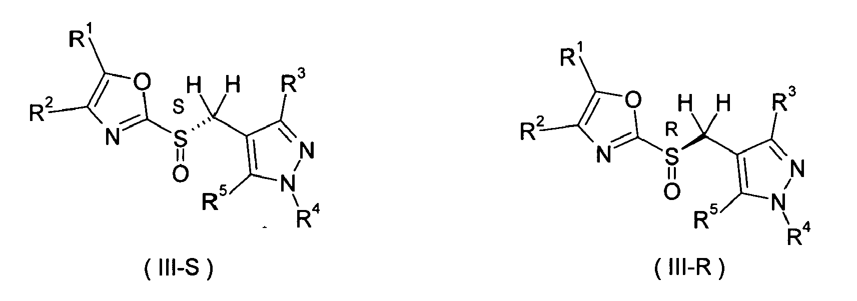 wobei die Reste R¹, R², R³, R⁴, R⁵ die gemäß Formel (I) zuvor angegebene Bedeutung haben.
   Für die Herstellung von Enantiomeren der allgemeinen Formel (III) kommen neben enantioselektiven Synthesen auch übliche Racemattrennungsmethoden in Frage (vgl. Handbücher der Stereochemie).
   Racemische Gemische, beispielsweise von optisch aktiven Sulfoxiden der allgemeinen Formel (III), lassen sich nach bekannten Verfahren trennen. Derartige Racemattrennungsmethoden sind in Handbüchern der Stereochemie beschrieben, beispielsweise in "Basic Organic Stereochemistry" (Eds.: Eliel, Ernest L.; Wilen, Samuel H.; Doyle, Michael P.; 2001; John Wiley & Sons) und "Stereochemisty of Organic Compounds (Eds.: Eliel, Ernest L.; Wilen, Samuel H.; Mander, Lewis N.; 1994; John Wiley & Sons). In Frage kommen hierbei, z.B. Adduktbildung mit einem optisch aktiven Hilfsreagens, Trennung der diastereomeren Addukte in die entsprechenden Diastereomere, z.B. durch Kristallisation, Chromatographieverfahren, vor allem Säulenchromatographie und Hochdruckflüssigchromatographie, Destillation, gegebenenfalls unter reduziertem Druck, Extraktion und andere Verfahren und anschließende Rückspaltung der Diastereomeren in die Enantiomeren. Für präparative Mengen oder im industriellen Maßstab kommen Verfahren wie die Kristallisation diastereomerer Salze, die aus den Verbindungen (III) mit optisch aktiven Säuren und gegebenenfalls bei vorhandenen sauren Gruppen mit optisch aktiven Basen erhalten werden können, in Frage.
   Zur Racemattrennung durch Kristallisation diastereomerer Salze kommen als optisch aktive Säure z.B. Camphersulfonsäure, Camphersäure, Bromcamphersulfonsäure, Chinasäure, Weinsäure, Dibenzoylweinsäure und andere analoge Säuren in Betracht; als optisch aktive Basen kommen z.B. Chinin, Cinchonin, Chinidin, Brucin, 1-Phenylethylamin und andere analoge Basen in Frage.
   Die Kristallisationen werden dann meist in wässrigen oder wässrigorganischen Lösemitteln durchgeführt, wobei das Diastereomer mit der geringeren Löslichkeit gegebenenfalls nach Animpfen zunächst ausfällt. Das eine Enantiomer der Verbindung der Formel (III) wird danach aus dem ausgefällten Salz oder das andere aus dem Kristallisat durch Ansäuern bzw. mit Base freigesetzt.
   Desweiteren können Racemate chromatographisch mit chiralen Stationärphasen getrennt werden. Derartige Enantiomerentrennungen lassen sich vom mg bis in den 100 kg Bereich mit präparativen HPLC Anlagen im Einzel- oder kontinuierlichen Betrieb durchführen.
   Die Herstellung der Thioether der allgemeinen Formel (II), welche als Edukt für die Herstellung der Sulfoxide der allgemeinen Formel (III) bzw. der Silfone der allgemeinen Formel (IV) dienen, ist unter den Verfahren b.), c.), d.), e.), f.), g.) und h.) nachfolgend beschrieben.
b.) Zur Herstellung eines Thioethers der allgemeinen Formel (II), 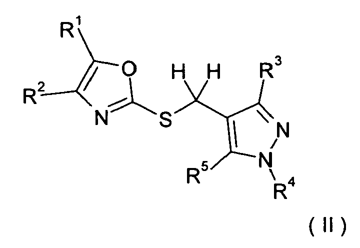 worin R¹, R², R³, R⁴, R⁵ die gemäß Formel (I) zuvor angegebenen Bedeutungen haben, wird beispielsweise 2-Mercaptooxazol oder ein Oxazol-2(3H)-thion bzw. ein Salz davon, bevorzugt ein Alkali- oder Erdalkalimetallsalz der allgemeinen Formel (V), 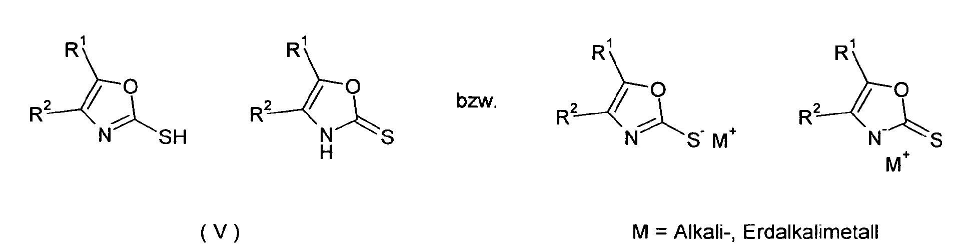 worin R¹, R² die gemäß der allgemeinen Formel (I) zuvor angegebenen Bedeutungen haben, mit einem (1 H-Pyrazol-4-ylmethyl)-Derivat der allgemeinen Formel (VI) 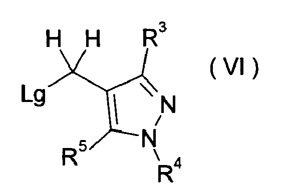 worin R³, R⁴, R⁵ die gemäß der allgemeinen Formel (I) zuvor angegebenen Bedeutungen haben und Lg eine Abgangsgruppe bedeutet, in Gegenwart einer geeigneten Alkali- oder Erdalkalimetall-Base, z.B. Kaliumcarbonat oder Natriumhydrid, oder einer organischen Base wie z.B. 1,8-Diazabicyclo(5.4.0)undec-7-en (DBU), in einem geigneten Lösemittel, z.B. Dimethylformamid, Tetrahydrofuran, Ethanol, oder bevorzugt Acetonitril, in einem Temperaturbereich zwischen 0 und 100 °C, und gegebenenfalls unter einer Inertgas-Atmosphäre, z.B. Stickstoff, umgesetzt.
   Analoge Reaktionen für die Umsetzung von 2-Mercaptooxazolen oder Oxazol-2(3H)-thione bzw. deren Salzen sind in der Literatur beschrieben, z.B.. in DE 26 25 229 A, WO 99/52874 A, WO 01/66529 A, WO 95/24403 A, Bradsher, C. K.; Jones, W. J. Jr; J. Org. Chem. 32, 2079 (1967).
   Anstelle der genannten Mercaptoverbindungen bzw. des Salzes davon können auch Mercaptanbildner, wie z.B. Isothiuroniumsalze verwendet werden.
   Als Abgangsgruppen Lg sind Chlor, Brom, lod oder Sulfonat-Gruppen, wie Methan-, Trifluormethan-, Ethan-, Benzol- oder Toluolsulfonat bevorzugt.
   Die im Verfahren b.) eingesetzten 2-Mercaptooxazol-Derivate oder Oxazol-2(3H)-thione bzw. entsprechenden Salze der 2-Mercaptooxazol-Derivate oder Oxazol-2(3H)-thione der allgemeinen Formel (V) sind dem Fachmann bekannt und teilweise kommerziell erhältlich oder nach dem Fachmann bekannten Verfahren darstellbar, beispielsweise wie beschrieben in a) Science of Synthesis, Houben-Weyl (Methods of Molecular Transformations), Category 2, Volume 11, Ed. E. Schaumann; b) Houben-Weyl (Methoden der organische Chemie), Band E8a, Hetarene III-Teil 1, Ed. E. Schaumann; c) Can. J. Chem., Vol. 50, 3082-3083 (1972); d) WO 2003/006442 A.
   Die im Verfahren b.) eingesetzten (1 H-Pyrazol-4-yl-methyl)-Derivate der allgemeinen Formel (VI) sind dem Fachmann bekannt oder kommerziell erhältlich oder nach dem Fachmann bekannten Verfahren darstellbar (siehe z.B.: a) Communications de la Faculte des Sciences de l'Universite d'Ankara, Series B: Chemistry and Chemical Engineering (1996), 41(1-2), 87-94; b) WO 2004/013106 A; c) WO 2006/024820 A).
c.) Alternativ kann die Herstellung eines Thioethers der allgemeinen Formel (II), 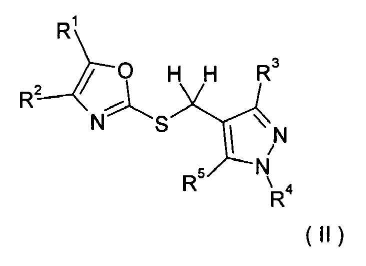 worin R¹, R², R³, R⁴, R⁵ die gemäß der allgemeinen Formel (I) zuvor angegebenen Bedeutungen haben, durch die Umsetzung eines Oxazol-Derivates der allgemeinen Formel (VII), 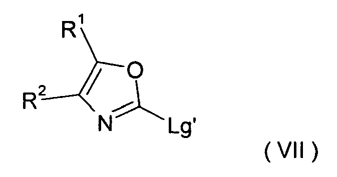 worin R¹, R² die gemäß der allgemeinen Formel (I) zuvor angegebenen Bedeutungen haben und Lg' eine Abgangsgruppe bedeutet, wobei als Abgangsgruppen u.a. Fluor, Chlor, Brom, lod, Sulfide-, Sulfoxide- oder Sulfonat-Gruppen fungieren können, mit einem [(1H-Pyrazol-4-yl-methyl)]-imidothiocarbamat-Salz der allgemeinen Formel (VIII) 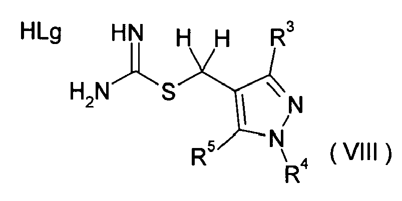 worin R³, R⁴, R⁵ die gemäß der allgemeinen Formel (I) zuvor angegebene Bedeutungen haben, Lg eine Abgangsgruppe bedeutet, in einem Eintopfverfahren in Gegenwart einer wäßrigen Alkali- oder Erdalkalimetall-Base erfolgen.
   Allgemein wird die Umsetzung durch das nachstehende Formelschema dargestellt: 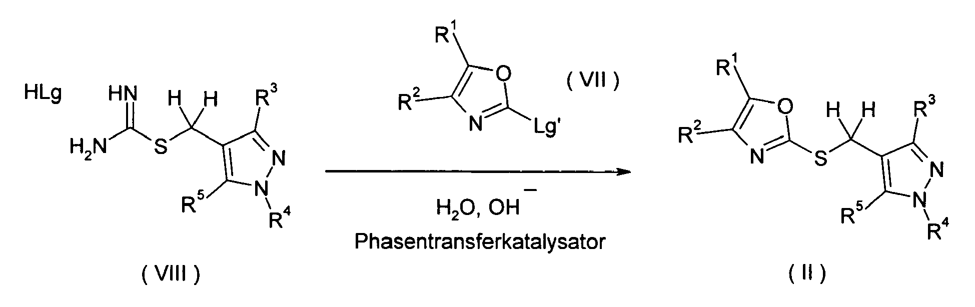 Die im Verfahren c.) eingesetzten Oxazol-Derivate der allgemeinen Formel (VII) sind dem Fachmann bekannt oder kommerziell erhältlich oder nach dem Fachmann bekannten Verfahren darstellbar (z.B. wie beschrieben in Science of Synthesis, Houben-Weyl (Methods of Molecular Transformations), Category 2, Volume 11, Ed. E. Schaumann; DE 26 25 229 A).
   Die Verwendung von Imidothiocarbamat-Salzen (Isothiuroniumsalzen) im Sinne einer Eintopfreaktion zur Verseifung des Imidothiocarbamat-Salzes (Isothiuroniumsalzes) und Umsetzung des intermediär entstandenen Mercaptans in einer Austauschreaktion ist an einem anderen Reaktionsschema z.B. in DE 39 42 946, WO 2006/024820 A und WO 2006/037945 A beschrieben und unter Verwendung von Phasen-Transfer Katalyse in WO 2007/003294 A und WO 2007/003295 A beschrieben.
   Verbindungen der allgemeinen Formel (VIII) lassen sich durch Umsetzung eines Alkylierungsmittels der allgemeinen Formel (VI), worin R³, R⁴, R⁵ die oben gemäß der allgemeinen Formel (I) angegebenen Bedeutungen haben und Lg eine Abgangsgruppe ist, mit Thioharnstoff erhalten.
   Die Herstellung der Imidothiocarbamat-Salze (VIII) durch Umsetzung eines (1 H-Pyrazol-4-yl)methylierungsmittel der allgemeinen Formel (VI) mit Thioharnstoff erfolgt nach bekannten Verfahren (wie beispielsweise dem in DD 152557 beschriebenen Verfahren), vorteilhaft durch Reaktion mit einer äquimolaren Menge an Thioharnstoff und wahlweise in Anwesenheit von Alkali-lodid, z.B. Natriumiodid oder Kaliumiodid, in einem inerten Lösemittel, wie niederen Alkoholen, wie beispielsweise Methanol, Ethanol oder Isopropanol; Kohlenwasserstoffen, wie beispielsweise Benzol oder Toluol; halogenierten Kohlenwasserstoffen, wie beispielsweise Dichlormethan oder Chloroform; oder Etherderivaten, wie beispielsweise Methyl-tert.-butylether, Tetrahydrofuran oder Dioxan bei Temperaturen zwischen 0 bis 150 °C, vorzugsweise zwischen 20 bis 100 °C.
   Die in vielen Fällen durch Kristallisation erhaltenen Verbindungen der Imidothiocarbamat-Salze der allgemeinen Formel (VIII) werden bei dem erfindungsgemäßen Verfahren im Allgemeinen ohne weitere Reinigungsschritte unter starkem Rühren mit äquimolaren Mengen der Oxazol-Derivate der allgemeinen Formel (VII) unter Phasentransferbedingungen umgesetzt.
   Vorteilhaft arbeitet man hier in einem Zweiphasensystem, wobei neben einer wäßrigen stark basischen Alkali- oder Erdalkalimetall-Hydroxid-Lösung, bevorzugt Natrium- oder Kaliumhydroxid, mit mindestens zwei Äquivalenten der Base, die organische Phase ein inertes Lösemittel wie Tetrahydrofuran, Diethylether, Acetonitril, Pentan, Hexan, Benzol, Toluol, Xylol, Chlorbenzol, Dichlormethan, Chloroform, Tetrachlorkohlenstoff, Nitrobenzol oder Mischungen dieser Lösemittel ist.
   Es ist auch möglich, das jeweils wertvollere Edukt der Formel (VIII) oder der Formel (VII) etwas im Unterschuß einzusetzen.
   Als Phasentransferkatalysatoren sind quaternäre Ammonium- oder Phosphoniumsalze sowie Kronenether, Kryptanden oder Polyethylenglykole geeignet. Beispiele solcher Katalysatoren finden sich z.B. in W. P. Weber, G. W. Gokel; Phase Transfer Catalysis in Organic Synthesis, Springer-Verlag, Berlin 1977 oder E. V. Dehmlow, S. S. Dehmlow, Phase Transfer Catalysis, Second Ed. Verlag Chemie, Weinheim 1983.
   Vorzugsweise werden die Reaktionspartner und der Katalysator bei Temperaturen von 20 bis 100 °C unter Schutzgasatmosphäre stark gerührt.
   Das unter den Reaktionsbedingungen intermediär entstehende Mercaptan der allgemeinen Formel (IX), worin R³, R⁴, R⁵ die oben gemäß der allgemeinen Formel (I) zuvor angegebene Bedeutung haben, 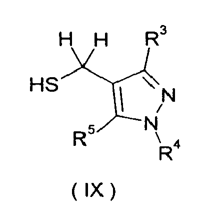 wird *in situ* sofort durch das Oxazol-Derivat der allgemeinen Formel (VII) abgefangen.
d.) Alternativ kann die Herstellung von Thioethern der Formel (II) worin R¹, R², R³, R⁴, R⁵ die gemäß Formel (I) zuvor angegebenen Bedeutungen haben, 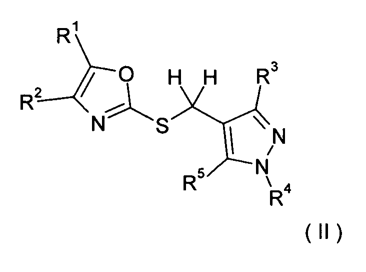 durch die Umsetzung eines Oxazol-Derivates der allgemeinen Formel (VII) 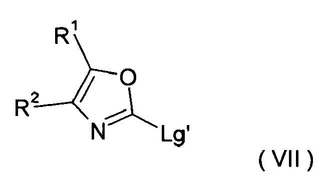 worin R¹, R² die gemäß der allgemeinen Formel (I) zuvor angegebenen Bedeutungen haben und Lg' eine Abgangsgruppe bedeutet, wobei als Abgangsgruppen u.a. Chlor, Brom oder Methylsulfonyl-Gruppen fungieren können, mit einem (1H-Pyrazol-4-ylmethyl)-Imidothiocarbamat-Salz (Isothiuroniumsalz) der allgemeinen Formel (VIII) 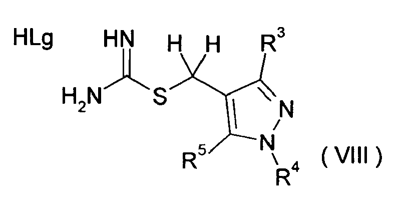 worin R³, R⁴, R⁵ die gemäß Formel (I) zuvor angegebene Bedeutungen haben, Lg eine Abgangsgruppe bedeutet, in einem Eintopfverfahren in Gegenwart einer Alkalimetall- oder Erdalkalimetall-Carbonat Base und eines Lösemittels, wie einem Alkohol, erfolgen.
   Allgemein wird die Umsetzung durch das nachstehende Formelschema dargestellt: 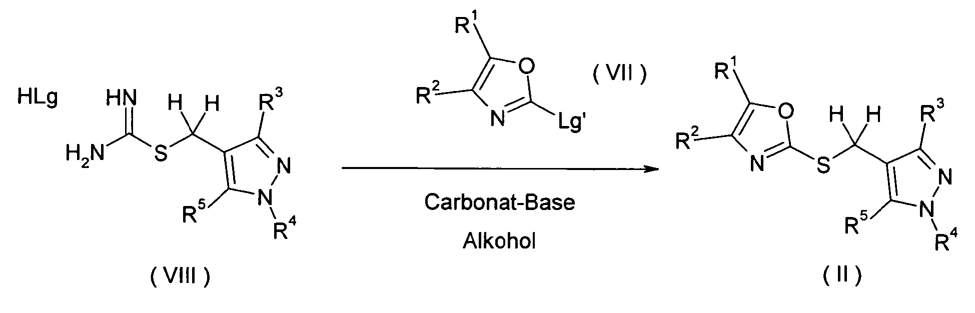 Verbindungen der allgemeinen Formel (VIII) lassen sich durch Umsetzung eines Alkylierungsmittels der allgemeinen Formel (VI), worin R³, R⁴, R⁵ die oben gemäß der allgemeinen Formel (I) angegebenen Bedeutungen haben, und Lg eine Abgangsgruppe ist, mit Thioharnstoff erhalten, wie gemäß vorstehendem Verfahren c.) beschrieben.
   Die Imidothiocarbamat-Salze (Isothiuroniumsalze) der allgemeinen Formel (VIII) werden bei dem erfindungsgemäßen Verfahren im allgemeinen ohne weitere Reinigungsschritte unter starkem Rühren mit leichtem Überschuß der Oxazol-Derivate der allgemeinen Formel (VII) und mit leichtem Überschuß einer Carbonat-Base, z.B. Kaliumcarbonat, Natriumcarbonat, oder Kaliumbicarbonat, oder eines Hydroxids, z.B. Kaliumhydroxid, oder ein Alkoxids, z.B. Natriumalkoxid, in Alkohol, z.B. Ethanol, Ether, z.B. 1,4-Dioxan, Tetrahydrofuran, einem polaren Lösemittel wie z.B. Wasser, Dimethylformamid oder einer Mischung dieser Lösemittel in einer Temperaturbereich zwischen 20 bis 200 °C, bevorzugt zwischen 50 und 150 °C, optional unter einer Inertgas-Atmosphäre, z.B. Stickstoff, oder in einer Mikrowelle-Apparatur umgesetzt.
   Die Imidothiocarbamat-Salze (Isothiuroniumsalze) der allgemeinen Formel (VIII) können auch in situ, ohne Isolierung, weiter umgesetzt werden.
   Vorteilhaft arbeitet man hier in einem Alkohol, bevorzugt Ethanol, mit mindestens 1.1 Äquivalenten der Base, bevorzugt Kaliumcarbonat (K₂CO₃).
   Solche Verfahren sind in der Literatur bekannt und sind z.B. in WO 2006/024820 A, WO 2001/012613 A und WO 2006/123088 A beschrieben.
   Die im Verfahren d.) eingesetzten Oxazol-Derivate der allgemeinen Formel (VII) sind dem Fachmann bekannt oder kommerziell erhältlich oder nach dem Fachmann bekannten Verfahren darstellbar [siehe z.B. Science of Synthesis, Houben-Weyl (Methods of Molecular Transformations), Category 2, Volume 11, Ed. E. Schaumann].
e.) Alternativ kann die Herstellung eines Thioethers der allgemeinen Formel (II), 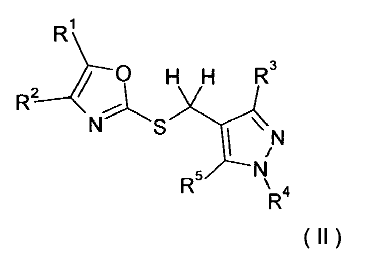 worin R¹, R², R³, R⁴, R⁵ die gemäß der allgemeinen Formel (I) zuvor angegebenen Bedeutungen haben, durch die Umsetzung eines Oxazol-Derivats der allgemeinen Formel (VII), 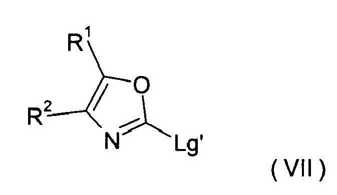 worin R¹, R² die gemäß Formel (I) zuvor angegebenen Bedeutungen haben und Lg' eine Abgangsgruppe bedeutet, wobei als Abgangsgruppen LG' u.a. Fluor, Chlor, Brom, oder Sulfonat-Gruppen fungieren können,
   mit einem (1 H-Pyrazol-4-ylmethyl)-Mercaptan der allgemeinen Formel (IX), 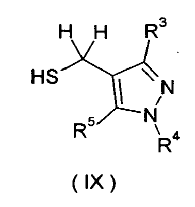 worin R³, R⁴, R⁵ die oben gemäßder allgemeinen Formel (I) zuvor angegebene Bedeutung haben, in Gegenwart einer Alkali- oder Erdalkalimetall-Base, z.B. Kaliumcarbonat oder Natriumhydrid, oder organischen Base, z.B. bevorzugt 1,8-Diazabicyclo(5.4.0)undec-7-en (DBU), optional in einem Lösemittel, z.B. Dimethylformamid, Tetrahydrofuran, Ethanol, oder bevorzugt Acetonitril, in einem Temperaturbereich zwischen 0 und 100 °C, und optional unter einer Inertgas-Atmosphäre, z.B. Stickstoff, erfolgen.
   Einige der Verfahren sind in der Literatur bekannt und sind z.B. in WO 2006/024820 A, WO 2001/012613 A und WO 2006/123088 A beschrieben.
   Nucleophile Substitutionen an Oxazol-Derivate sind in der Literatur beschrieben, z.B. in Yamanaka, H.; Ohba, S.; Sakamoto, T.; Heterocycles (1990), 31 (6), 1115-27.
   Die im Verfahren e.) eingesetzten Oxazol-Derivate der allgemeinen Formel (VII) sind dem Fachmann bekannt oder kommerziell erhältlich oder nach dem Fachmann bekannten Verfahren darstellbar [siehe z.B. Science of Synthesis, Houben-Weyl (Methods of Molecular Transformations), Category 2, Volume 11, Ed. E. Schaumann].
   Die im Verfahren e.) eingesetzten Mercaptane der allgemeinen Formel (IX) sind dem Fachmann bekannt (siehe z.B. WO 2004/013106 A) oder in Analogie zu dem Fachmann bekannten Verfahren zur Mercaptan-Herstellung darstellbar.
f.) Die Herstellung von Thioethern der Formel (II) worin R², R³, R⁴, R⁵ die gemäß Formel (I) zuvor angegebenen Bedeutungen haben, und R¹ Halogen oder Nitro bedeutet 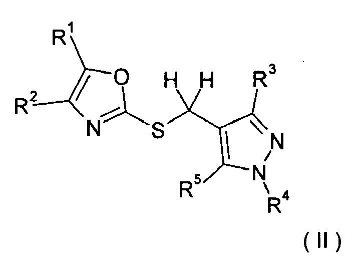 kann beispielsweise durch die Umsetzung eines Oxazol-Derivats der allgemeinen Formel (X), 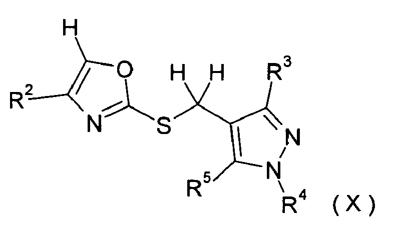 worin R², R³, R⁴, R⁵ die gemäß Formel (I) zuvor angegebene Bedeutungen haben, erfolgen. Allgemein wird die Umsetzung durch das nachstehende Formelschema dargestellt: 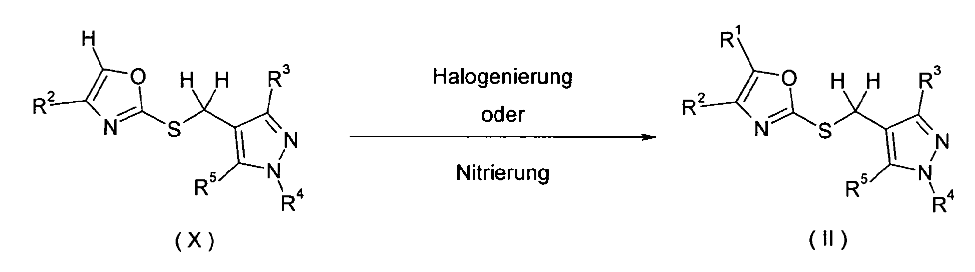 Die Verbindungen der allgemeinen Formel (X) werden mit einem halogenierenden Mittel, wie z.B. Halogen, wie Chlor, Brom, lod oder Halo-Succinimid, wie N-Chlorsuccinimid (NCS), N-Bromsuccinimid (NBS), N-lodsuccinimid (NIS) oder für Nitro mit einen Nitrierungsmittel, wie z.B. Nitriersäure, hergestellt aus Schwefelsäure und Salpetersäure, behandelt und in geeigneten Lösemittel, wie Chlorkohlenwasserstoffen, z.B. Tetrachlormethan, Dichlormethan, 1,2-Dichloroethan, oder Dimethylformamid zu Verbindungen der Formel (II) umgesetzt.
   Die im Verfahren f.) eingesetzten analogen Thioether-Derivate der allgemeinen Formel (X) sind nach dem Fachmann bekannten Verfahren (siehe z.B.: DE 26 25 229 A, WO 99/52874 A, WO 01/66529 A, WO 95/24403 A; oder nach den unter b.), c.), d.), e.) zuvor genannten Verfahren darstellbar.
g.) Die Herstellung von Thioethern der Formel (II) worin R¹, R², R³, R⁴, R⁵ die gemäß Formel (I) zuvor angegebenen Bedeutungen haben, 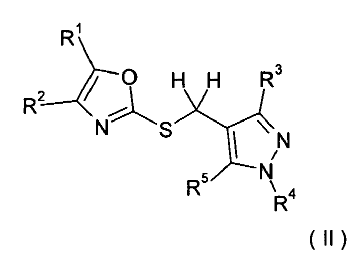 kann beispielsweise durch die Umsetzung eines Oxazol-Derivats der allgemeinen Formel (XI), 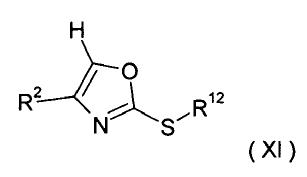 hergestellt aus einem Oxazol-Derivat der allgemeinen Formel (V), durch Umsetzung mit einem Alkylierungsmittel R¹²Lg',
   worin R² die gemäß Formel (I) zuvor angegebenen Bedeutung hat; R¹² bevorzugt (C₁-C₆)-Alkyl ist, welches unsubstituiert oder durch einen oder mehrere, gleiche oder verschiedene Reste aus der Gruppe Halogen substituiert ist, und besonders bevorzugt Methyl oder Ethyl ist, und Lg' eine Abgangsgruppe bedeutet, wobei als Abgangsgruppen u.a. Chlor, Brom oder Methylsulfonyl-Gruppen fungieren können,
   in Gegenwart einer starken Base und einem Alkylierungsmittel R¹Lg',
   worin R¹ die gemäß Formel (I) zuvor angegebenen Bedeutung hat, nach dem Schema 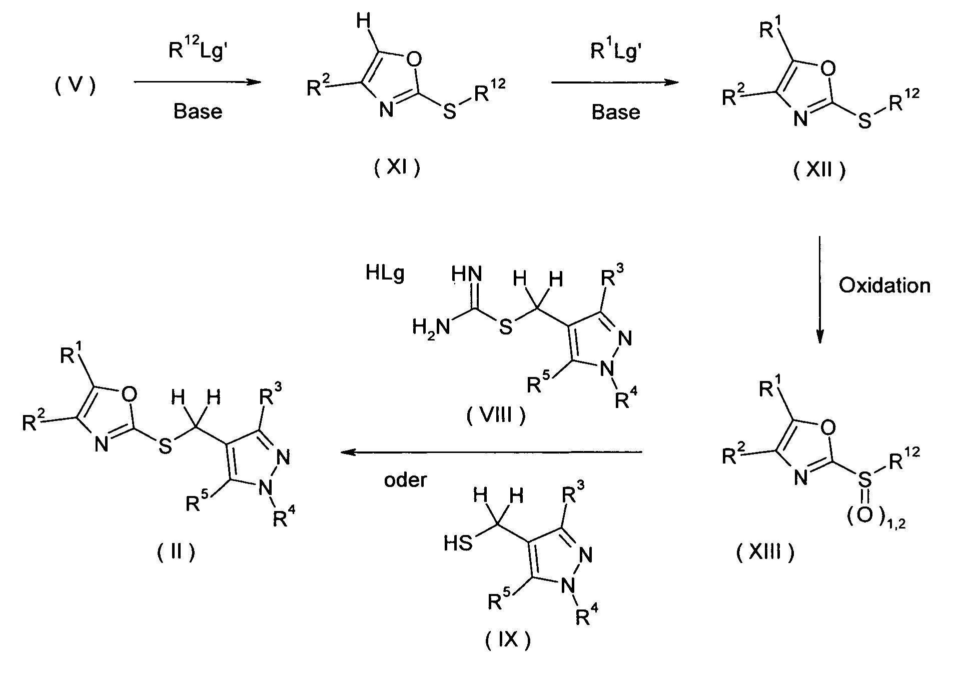 verlaufen, worin R¹, R², R³, R⁴, R⁵ die gemäß Formel (I) zuvor angegebenen Bedeutungen haben und Lg bzw. Lg' eine Abgangsgruppe bedeutet, wobei als Abgangsgruppen u.a. Fluor, Chlor, Brom, lod oder Sulfonat-Gruppen wie Methan- Trifluormethan-, Ethan-, Phenyl- oder Toluolsulfonat fungieren können, und R¹² bevorzugt die zuvor angegebene Bedeutung hat.
   Als starke Base kann Lithiumdiisopropylamid (LDA), Lithiumtetramethylpiperidin (LTMP), Lithiumhexamethyldisilazan (LHMDS), vorzugsweise LDA, verwendet werden, die nach dem Fachmann bekannten Verfahren hergestellt sein können. Hexamethylphosphoramid (HMPT) kann z.B. als Co-Lösemittel verwendet werden.
   Als Lösemittel dienen inerte Lösemittel wie Kohlenwasserstoffe, wie z.B. Hexan, Heptan, Cyclohexan, aromatische Kohlenwasserstoffe wie z.B. Benzol, Ether, wie z.B. Diethylether, Methyl-tert.-Butylether (MTBE), Tetrahydrofuran, und Dioxan, bevorzugt Tetrahydrofuran. Die zuvorgenannten Lösemittel können auch als Gemische eingesetzt werden. In dieser Reaktion werden die Verbindungen der Formel (XI) und die Base bzw. das Alkylierungsmittel R¹Lg' bevorzugt in 0.9 bis 1.5 Mol von den Letzteren pro Mol der Ersteren eingesetzt. Die Reaktion wird bevorzugt in einem Temperaturbereich zwischen -90 °C und dem Siedepunkt des Lösemittels ausgeführt. Die Reaktionszeit unterliegt keiner Begrenzung, die Reaktionen sind allgemein nach 1 bis 24 h abgeschlossen.
   Zur Herstellung der Sulfone und Sulfoxide der Verbindungen der allgemeinen Formel (11), worin R¹, R², R³, R⁴, R⁵ die gemäß der allgemeinen Formel (I) zuvor angegebenen Bedeutungen haben, kann die unter a) angegebene Methode angewendet werden.
   Besonders für den Fall dass R¹ Fluor ist, werden bevorzugt Reagenzien für die elektrophile Fluorierung eingesetzt, wie z.B. 1-Chlormethyl-4-fluor-1,4-diazabicyclo[2,2,2]oktan-bis-tetrafluorborat (F-TEDA-BF4, SelectFluor™), N-Fluorbenzolsulfonsäureimid (NFBS oder NFSi), N-Fluor-o-benzenedisulfonimid (NFOBS), 1-Fluor-4-hydroxy-1,4-diazoniabicyclo[2.2.2]-octan bis(tetrafluorborat) (NFTh, AccuFluor™) und weitere wie in "Modern Fluoroorganic Chemistry", 2004, Wiley-VCH Verlag, Ed. P. Kirsch, beschrieben.
   Die im Verfahren g.) eingesetzten 2-Mercaptooxazol-Derivate oder Oxazol-2(3H)-thione bzw. entsprechenden Salze der 2-Mercaptooxazol-Derivate oder Oxazol-2(3H)-thion-Derivate der allgemeinen Formel (V) sind dem Fachmann bekannt, oder kommerziell erhältlich oder nach dem Fachmann bekannten Verfahren darstellbar, beispielsweise wie beschrieben in Science of Synthesis, Houben-Weyl (Methods of Molecular Transformations), Category 2, Volume 11, Ed. E. Schaumann.
   Oxazol-Derivate (XI) können, in Analogie zu dem Fachmann bekannten Verfahren, in 5-Position regioselektiv deprotoniert werden. Analoge Reaktionen unter Verwendung einer Alkyl-Base wie Buthyllithium sind in der Literatur beschrieben, z.B. in Boger, D. L. et al; J. Med. Chem. (2007) 50 (33), 1058-1068 und Molinski, T. F. et al. J. Org. Chem. (1998) 63, 551-555, und mit *tert*-Butyllithium und ein Kupfer-Salz in Marino, J. P. ; Nguyen, N. Tet. Lett. (2003) 44, 7395-7398 und dort zitierte Literatur.
   Die im Verfahren g.) eingesetzten Oxazol-Derivate der allgemeinen Formel (XI) können zum Beispiel entsprechend Verfahren b.) durch Reaktion eines 2-Mercaptooxazol-Derivates oder Oxazol-2(3H)-thion-Derivates der allgemeinen Formel (V) mit einem Alkylierungsmittel R¹²Lg' oder nach dem Fachmann bekannten Verfahren [siehe z.B. Science of Synthesis, Houben-Weyl (Methods of Molecular Transformations), Category 2, Volume 11, Ed. E. Schaumann] hergestellt werden, oder sind kommerziell erhältlich.
   Die im Verfahren g.) genannten Verbindungen der Formel (XIII) können aus den Verbindungen der Formel (XII) durch Oxidation nach vorstehendem Verfahren a) oder nach dem Fachmann bekannten Verfahren hergestellt werden. Die Verbindungen der allgemeinen Formel (XIII) wiederum können nach den vorstehenden Verfahren c.) oder d.) mit (1 H-Pyrazol-4-ylmethyl)-Imidothiocarbamat-Salzen (VIII) oder mit (1 H-Pyrazol-4-ylmethyl)-Mercaptane der Formel (IX) nach vorstehenden Verfahren e.) zu Verbindungen des Typs (II) umgesetzt werden.
h.) Die Herstellung von Thioethern der Formel (II) worin R¹, R², R³, R⁴, R⁵ die gemäß Formel (I) zuvor angegebenen Bedeutungen haben, 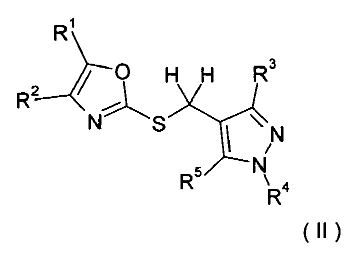 kann beispielsweise auch durch die Umsetzung eines (1H-Pyrazol-4-ylmethyl)disulfid-Derivats der allgemeinen Formel (XV) mit 2-Amino-oxazolen der Formel (XIV) und einem Diazotierungsmittel wie in folgendem Schema dargestellt, 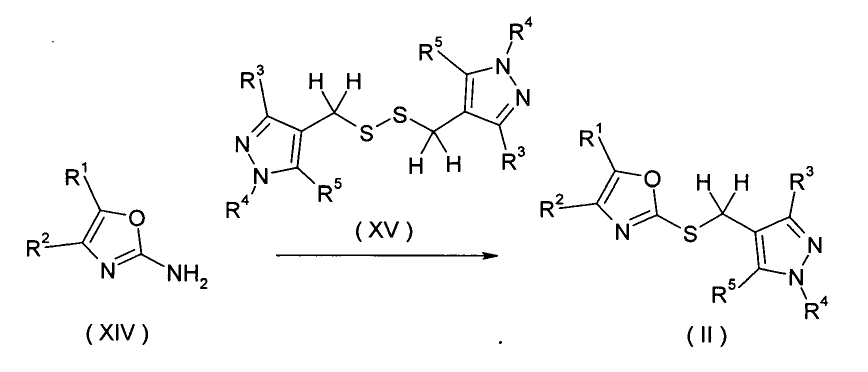 und worin R¹, R², R³, R⁴, R⁵ die gemäß Formel (I) zuvor angegebenen Bedeutungen haben, erfolgen.

Die (1 H-Pyrazol-4-ylmeth-yl)-disulfide der allgemeinen Formel (XV) werden mit einem Diazotierungsmittel und einem 2-Amino-oxazol-Derivat der allgemeinen Formel (XIV) in einem geeigneten Lösemittel zu Verbindungen der Formel (II) zur Reaktion gebracht.

Geeignete Lösemittel sind gegenüber der Reaktion inerte Lösemittel, wie z.B. Kohlenwasserstoffe, so wie Hexan, Heptan, Cyclohexan, aromatische Kohlenwasserstoffe, wie Benzol, Chlorbenzol, Toluol, Xylol, halogenierte Kohlenwasserstoffe wie z.B. Dichlormethan, Dichlorethan, Chloroform und Tetrachlorkohlenstoff, Ester wie z.B. Ethylacetat und Methylacetat, Ether wie z.B. Diethylether, Methyl-tert.-butylether, Dioxan, Nitrile so wie Acetonitril, Alkohole wie z.B. Methanol, Ethanol, Isopropylalkohol, Amide wie z.B. N,N-Dimethylformamid und Sulfoxide wie z.B. Dimethylsulfoxid.

Das Diazotierungsreagens kann z.B. ein Nitrit-Ester wie Isoamylnitrit oder ein Nitrit-Salz wie z.B. Natriumnitrit sein. Die Molverhältnisse können frei gewählt werden, bevorzugt sind equimolare Mengen der Heteroarylalkyldisulfide und der Diazotierungsmittel. Bevorzugt wird die Reaktion bei einer Temperatur zwischen -20 °C und dem Siedepunkt des gewählten Lösemittels durchgeführt und ist nach einer Zeit von 0.1 bis 40 Stunden vollständig abgeschlossen.

Die Diazotierung eines 2-Amino-oxazol-Derivat der allgemeinen Formel (XIV) ist z.B. beschrieben in Hodgetts, K. J.; Kershaw, M. T. Org. Lett. (2002), 4(17), 2905-2907.

Die im Verfahren h.) eingesetzten Oxazol-Derivate der allgemeinen Formel (XIV) sind dem Fachmann bekannt oder kommerziell erhältlich, oder nach dem Fachmann bekannten Verfahren herstellbar (siehe Science of Synthesis, Houben-Weyl (Methods of Molecular Transformations), Category 2, Volume 11, Ed. E. Schaumann).

Die (1 H-Pyrazol-4-ylmethyl)disulfide der allgemeinen Formel (XV) können nach der Fachmann bekannten Verfahren hergestellt werden, wie z.B. in Gladysz, J. A., Wong, V. K., Jick, B. S.; Tetrahedron (1979) 35, 2329.

Als Abgangsgruppen Lg sind Halogene, z.B. Chlor, Brom, Iod, oder Alkyl-oder Aryl-Sulfonyl-Gruppen, wie Methyl-, Ethyl-, Phenyl- oder Tolylsulfonyl, oder eine Haloalkylsulfonyl-Gruppe, wie Trifluormethyl-, oder Nitro, besonders aber sind Chlor und Methylsulfonyl bevorzugt.

Als Abgangsgruppen Lg' sind Halogene, z.B. Chlor, Brom, Iod, oder Alkyl-oder Arylsulfonyl-Gruppen, wie Methyl-, Ethyl-, Phenyl- oder Tolylsulfonyl, oder eine Haloalkylsulfonyl-Gruppe, wie Trifluormethyl-, oder Nitro, besonders aber sind Chlor und Methylsulfonyl bevorzugt.

Als Gruppe R¹² ist (C₁-C₆)-Alkyl bevorzugt, welche unsubstituiert oder gegebenenfalls durch einen oder mehrere, gleiche oder verschiedene Reste aus der Gruppe Halogen substituiert ist, besonders bevorzugt Methyl oder Ethyl.

Die vorliegenden Verbindungen der allgemeinen Formel (III) 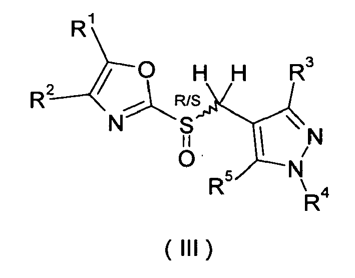 bestehen aus einem Gemisch der jeweiligen, an der Sulfoxid-Funktion chiralen Enantiomere (III-*S*) und (III-*R*):

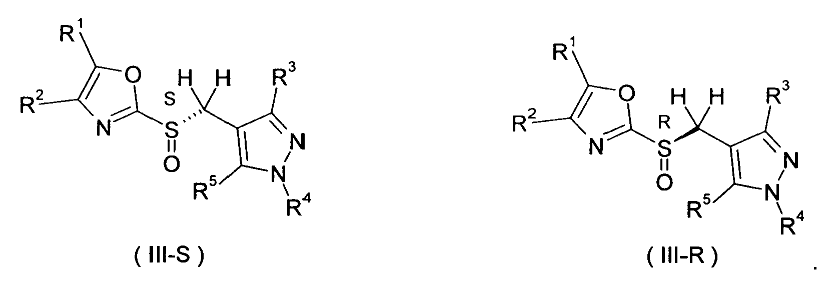

Sie weisen somit ein chirales Schwefelatom auf, welches in der oben dargestellten Struktur durch die Kennzeichneung (R/S) verdeutlicht ist. Gemäß den Regeln nach Cahn, Ingold und Prelog (CIP-Regeln) kann dieses Schwefelatom sowohl eine (R)-als auch eine (S)-Konfiguration aufweisen.

Von der vorliegenden Erfindung werden Verbindungen der allgemeinen Formel (III) sowohl mit (S)- als auch mit (R)-Konfiguration erfasst, d.h., dass die vorliegende Erfindung die Verbindungen der allgemeinen Formel (III) erfasst, in welchen das betreffende Schwefelatom
(1) eine (R)-Konfiguration; oder
(2) eine (S)-Konfiguration
aufweist.

Darüber hinaus werden im Rahmen der vorliegenden Erfindung auch
(3) beliebige Mischungen von Verbindungen der allgemeinen Formel (III), welche eine (R)-Konfiguation (Verbindungen der allgemeinen Formel (III-(R)) aufweisen, mit Verbindungen der allgemeinen Formel (III), welche eine (S)-Konfiguration (Verbindungen der allgemeinen Formel (III-S)) aufweisen,
erfasst.

Die vorliegende Erfindung umfasst Verbindungen der allgemeinen Formel (III), die racemisch sind, d.h. in welchen die Verbindungen der allgemeinen Formel (III) mit (S)-Konfiguration (Verbindungen der allgemeinen Formel (III-S)) im Vergleich zur (R)-Konfiguration (Verbindungen der allgemeinen Formel (III-R)) als 1:1 Mischung (50% stereochemischen Reinheit) vorliegen.

Allerdings sind im Rahmen der vorliegenden Erfindung auch Verbindungen der allgemeinen Formel (III) mit (S)-Konfiguration (Verbindungen der allgemeinen Formel (III-S)) im Vergleich zur (R)-Konfiguration (Verbindungen der allgemeinen Formel (III-R)) mit einer stereochemischen Reinheit von im Allgemeinen über 50 % bis 100 %, vorzugsweise 60 bis 100 %, insbesondere 80 bis 100 %, ganz besonders 90 bis 100 %, speziell 95 bis 100 %, bevorzugt, wobei die jeweilige (S)-Verbindung mit einer Enantioselektivität von jeweils mehr als 50% ee, vorzugsweise 60 bis 100% ee, insbesondere 80 bis 100% ee, ganz besonders 90 bis 100% ee, meist bevorzugt 95 bis 100% ee, bezogen auf den Gesamtgehalt an betreffender (S)-Verbindung vorliegt, bevorzugt vorliegt.

Ferner sind im Rahmen der vorliegenden Erfindung auch Verbindungen der allgemeinen Formel (III) mit (R)-Konfiguration (Verbindungen der allgemeinen Formel (III-R)) im Vergleich zur (S)-Konfiguration (Verbindungen der allgemeinen Formel (III-R)) mit einer stereochemischen Reinheit von im Allgemeinen über 50 % bis 100 %, vorzugsweise 60 bis 100 %, insbesondere 80 bis 100 %, ganz besonders 90 bis 100 %, speziell 95 bis 100 %, bevorzugt, wobei die jeweilige (R)-Verbindung mit einer Enantioselektivität von jeweils mehr als 50% ee, vorzugsweise 60 bis 100% ee, insbesondere 80 bis 100% ee, ganz besonders 90 bis 100% ee, meist bevorzugt 95 bis 100% ee, bezogen auf den Gesamtgehalt an betreffender (S)-Verbindung vorliegt, bevorzugt vorliegt.

Daher betrifft die vorliegende Erfindung auch Verbindungen der allgemeinen Formel (III), in welchen die stereochemische Konfiguration an dem mit (*) gekennzeichnet Schwefelatom (S) mit einer stereochemischen Reinheit von 60 bis 100 % (S), vorzugsweise 80 bis 100 % (S), insbesondere 90 bis 100 % (S), ganz besonders 95 bis 100 % (S), vorliegt.

Die Verbindungen der allgemeinen Formel (III) können je nach Art und Verknüpfung der Substituenten weitere Chiralitätszentren als das in Formel (III) mit einem Stern (*) markierte S-Atom enthalten und entsprechend als Stereoisomere vorliegen. Die durch ihre spezifische Raumform definierten möglichen Stereoisomeren, wie Enantiomere, Diastereomere, Z- und E-Isomere sind alle von der Formel (III) umfaßt. Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Aus-gangs-und/oder Hilfsstoffe selektiv hergestellt werden. Die Erfindung betrifft somit auch alle Stereoisomeren, die von der allgemeinen Formel (III) umfaßt, jedoch nicht mit ihrer spezifischen Stereoform angegeben sind und deren Gemische.

Sind beispielsweise eine oder mehrere Alkenylgruppen vorhanden, so können Diastereomere (Z- und E-Isomere) auftreten.

Sind beispielsweise ein oder mehrere asymmetrische Kohlenstoffatome vorhanden, so können Enantiomere und Diastereomere auftreten.
Entsprechende Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Aus-gangs-und/oder Hilfsstoffe selektiv hergestellt werden. Die Erfindung betrifft somit auch alle Stereoisomeren, die von der allgemeinen Formel (I) umfaßt werden, jedoch nicht mit ihrer spezifischen Stereoform angegeben sind und deren Gemische.

Im Rahmen der vorliegenden Erfindung können auch Säureadditionssalze der Verbindungen der allgemeinen Formel (I) verwendet werden. Zur Herstellung der Säureadditionssalze der Verbindungen der Formel (I) kommen folgende Säuren in Frauge: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphthalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösemittel wie z.B. Methanol, Aceton, Methylenchlorid oder Benzin und Hinzufügen der Säure bei Temperaturen von 0 bis 100 °C erhalten werden und in bekannter Weise, z.B. durch Abfiltrieren, isoliert und gegebenfalls durch Waschen mit einem inerten organischen Lösemittel gereinigt werden.

Die Basenadditionssalze der Verbindungen der Formel (I) werden vorzugsweise in inerten polaren Lösemitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, wie z.B. NaOH oder KOH, Alkali- und Erdalkalihydride, wie z.B. NaH, Alkali- und Erdalkalialkoholate, wie z.B. Natriummethanolat oder Kalium-tert.-butylat, Ammoniak, Ethanolamin oder quartäres Ammoniumhydroxid der Formel [NRR'R"R'"]⁺ OH⁻.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten.

Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z.B. WO 92/011376 A, WO 92/014827 A, WO 91/019806 A),
- transgene Kulturpflanzen, welche gegen bestimmte Herbizide vom Typ Glufosinate (vgl. z.B. EP 0 242 236 A, EP 0 242 246 A) oder Glyphosate (WO 92/000377 A) oder der Sulfonylharnstoffe (EP 0 257 993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum^{™} GAT^{™} (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0 142 924 A, EP 0 193 259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0 309 862 A, EP 0 464 461 A)
- gentechnisch veränderte Pflanzen mit reduzierter Photorespiration, die höhere Erträge und höhere Stresstoleranz aufweisen (EP 0 305 398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum^{™} GAT^{™} (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch Herbizide und pflanzenwachstumsregulierende Mittel, welche die erfindungsgemäßen Verbindungen enthalten.
Die Verbindungen der allgemeinen Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösemittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z. B. Insektiziden, Akariziden, Herbiziden, Fungiziden, sowie mit Safenern, Düngemitteln und/oder Wachstumsregulatoren herstellen, z. B. in Form einer Fertigformulierung oder als Tankmix.
Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z. B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-Dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösemittel z. B. Butanol, Cyclohexanon, Dimethylformamid, Xylol oder auch höhersiedenden Aromaten oder Kohlenwasserstoffen oder Mischungen der organischen Lösemittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z. B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rühren, Kolloidmühlen und/oder statischen Mischern unter Verwendung von wäßrigen organischen Lösemitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z. B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z. B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe u.a. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösemittel, Füll-, Träger- und Farbstoffe, Entschäumer, Verdunstungshemmer und den pH-Wert und die Viskosität beeinflussende Mittel.

Die Verbindungen der allgemeinen Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen.

Als Kombinationspartner für die erfindungsgemäßen Verbindungen der allgemeinen Formel (I) in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441 445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, bencarbazone, Benfluralin, Benfuresate, Bensulide, Bensulfuron-methyl, Bentazone, Benzfendizone, Benzobicyclon, Benzofenap, Benzofluor, Benzoylprop, Bifenox, Bilanafos, Bilanafosnatrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuronmethyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HOK-201, HW-02, Imazamethabenz, Imazamethabenz-methyl, Imazamox, Imazapic, Imazapyr, Imazaquin, Imazethapyr, Imazosulfuron, Inabenfide, Indanofan, Indolessigsäure (IAA), 4-Indol-3-ylbuttersäure (IBA), lodosulfuron, lodosulfuron-methyl-natrium, loxynil, Isocarbamid, Isopropalin, Isoproturon, Isouron, Isoxaben, Isoxachlortole, Isoxaflutole, Isoxapyrifop, KUH-043, KUH-071, Karbutilate, Ketospiradox, Lactofen, Lenacil, Linuron, Maleinsäurehydrazid, MCPA, MCPB, MCPB-methyl, -ethyl und - natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Methazole, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobacmethyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosatetrimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapacethyl, Tritosulfuron, Tsitodef, Uniconazole, Uniconazole-P, Vernolate, ZJ-0166, ZJ-0270, ZJ-0543, ZJ-0862 sowie die folgenden Verbindungen

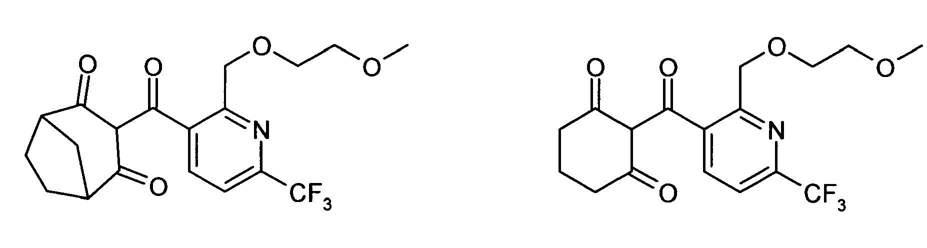

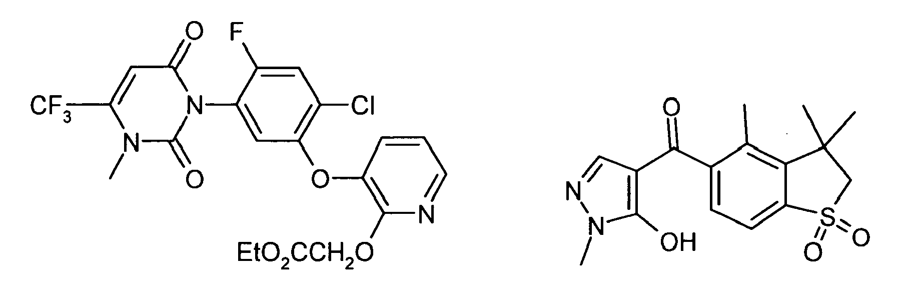

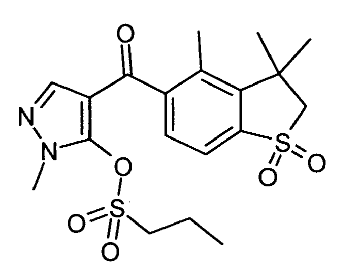

Von besonderem Interesse ist die selektive Bekämpfung von Schadpflanzen in Kulturen von Nutz- und Zierpflanzen. Obgleich die erfindungsgemäßen Verbindungen der allgemeinen Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen der allgemeinen Formel (I) von besonderem Interesse, welche die Verbindungen der allgemeinen Formel (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugswiese Getreide. Folgende Gruppen von Verbindungen kommen beispielsweise als Safener für die Verbindungen (I) alleinig oder aber in deren Kombinationen mit wieteren Pestiziden in Frage:
S1) Verbindungen der Formel (S1), 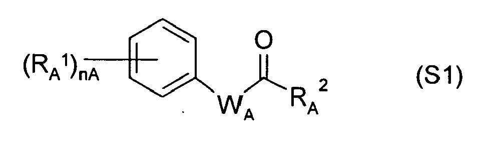 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)Alkyl, (C₁-C₄)Alkoxy, Nitro oder (C₁-C₄)Haloalkyl;
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴), 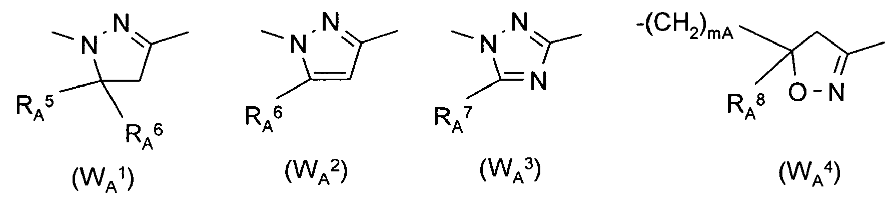
   - m_{A}: ist 0 oder 1;
   - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   - R_{A}³: ist Wasserstoff oder ein unsubstituierter oder substituierter aliphatischer Kohlenwasserstoffrest, vorzugsweise mit insgesamt 1 bis 18 C-Atomen;
   - R_{A}⁴: ist Wasserstoff, (C₁-C₆)Alkyl, (C₁-C₆)Alkoxy oder substituiertes oder unsubstituiertes Phenyl;
   - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁- C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure (S1^{a}), vorzugsweise Verbindungen wie
      1-(2,4-Dichlorphenyl)-5-(ethoxycarbonyl)-5-methyl-2-pyrazolin-3-carbonsäure,
      1-(2,4-Dichlorphenyl)-5-(ethoxycarbonyl)-5-methyl-2-pyrazolin-3-carbonsäure-ethylester (S1-1) ("Mefenpyr-diethyl"), und verwandte Verbindungen, wie sie in der WO-A-91/07874 beschrieben sind;
   b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
   c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   d) Verbindungen vom Typ der Triazolcarbonsäuren (S1^{d}), vorzugsweise Verbindungen wie Fenchlorazol(-ethylester), d.h.
      1-(2,4-Dichlorphenyl)-5-trichlormethyl-(1 H)-1,2,4-triazol-3-carbonsäureethylester (S1-7), und verwandte Verbindungen wie sie in EP-A-174 562 und EP-A-346 620 beschrieben sind;
   e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 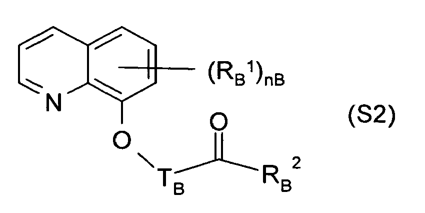 wobei die Symbole und Indizes folgende Bedeutungen haben:
   - R_{B}¹: ist Halogen, (C₁-C₄)Alkyl, (C₁-C₄)Alkoxy, Nitro oder (C₁-C₄)Haloalkyl;
   - n_{B}: ist eine natürliche Zahl von 0 bis 5, vorzugsweise 0 bis 3;
   - R_{B}²: ist OR_{B}³, SR_{B}³ oder NR_{B}³R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)Alkyl, (C₁-C₆)Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2^{a}), vorzugsweise
      (5-Chlor-8-chinolinoxy)essigsäure-(1-methylhexyl)ester ("Cloquintocet-mexyl") (S2-1),
      (5-Chlor-8-chinolinoxy)essigsäure-(1,3-dimethyl-but-1-yl)ester (S2-2),
      (5-Chlor-8-chinolinoxy)essigsäure-4-allyloxy-butylester (S2-3),
      (5-Chlor-8-chinolinoxy)essigsäure-1-allyloxy-prop-2-ylester (S2-4),
      (5-Chlor-8-chinolinoxy)essigsäureethylester (S2-5),
      (5-Chlor-8-chinolinoxy)essigsäuremethylester (S2-6),
      (5-Chlor-8-chinolinoxy)essigsäureallylester (S2-7),
      (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Verbindungen der Formel (S3) 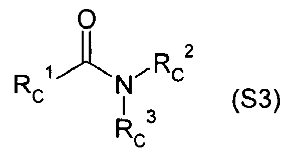 wobei die Symbole und Indizes folgende Bedeutungen haben:
   - R_{c}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   - R_{c}², R_{c}³: sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{c}² und R_{c}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
   Wirkstoffe vom Typ der Dichloracetamide, die häufig als Vorauflaufsafener (bodenwirksame Safener) angewendet werden, wie z. B. "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6),
   "AD-67" oder "MON 4660" (3-Dichloracetyl-1-oxa-3-aza-spiro[4,5]decan) der Firma Nitrokemia bzw. Monsanto (S3-7),
   "TI-35" (1-Dichloracetyl-azepan) der Firma TRI-Chemical RT (S3-8), "Diclonon" (Dicyclonon) oder "BAS145138" oder "LAB145138" (S3-9) (3-Dichloracetyl-2,5,5-trimethyl-1,3-diazabicyclo[4.3.0]nonan) der Firma BASF,
   "Furilazol" oder "MON 13900" ((RS)-3-Dichloracetyl-5-(2-furyl)-2,2-dimethyloxazolidin) (S3-10); sowie dessen (R)-Isomer (S3-11).
S4) N-Acylsulfonamide der Formel (S4) und ihre Salze, 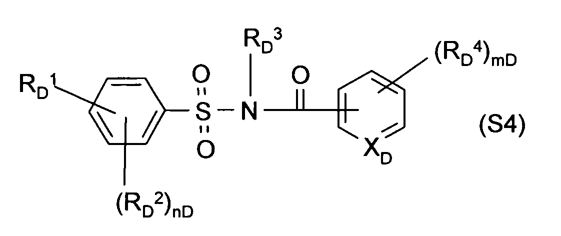 worin die Symbole und Indizes folgende Bedeutungen haben:
   - X_{D}: ist CH oder N;
   - R_{D}¹: ist CO-NR_{D}⁵R_{D}⁶ oder NHCO-R_{D}⁷;
   - R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   - R_{D}³: ist Wasserstoff, (C₁-C₄)Alkyl, (C₂-C₄)Alkenyl oder (C₂-C₄)Alkinyl;
   - R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   - R_{D}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
   - R_{D}⁶: ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
   - R_{D}⁵ und R_{D}⁶: gemeinsam mit dem dem sie tragenden Stickstoffatom einen Pyrrolidinyl- oder Piperidinyl-Rest bilden;
   - R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - n_{D}: ist 0, 1 oder 2;
   - m_{D}: ist 1 oder 2;
   - v_{D}: ist 0, 1, 2 oder 3;
   davon bevorzugt sind Verbindungen von Typ der N-Acylsulfonamide, z.B. der nachfolgenden Formel (S4^{a}), die z. B. bekannt sind aus WO-A-97/45016 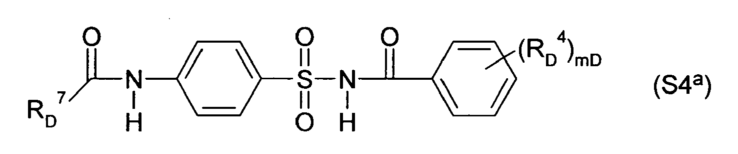 worin
   - R_{D}⁷: (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - R_{D}⁴: Halogen, (C₁-C₄)Alkyl, (C₁-C₄)Alkoxy, CF₃;
   - m_{D}: 1 oder 2;
   - v_{D}: ist 0, 1, 2 oder 3 bedeutet;
   sowie
   Acylsulfamoylbenzoesäureamide, z.B. der nachfolgenden Formel (S4^{b}), die z.B. bekannt sind aus WO-A-99/16744, 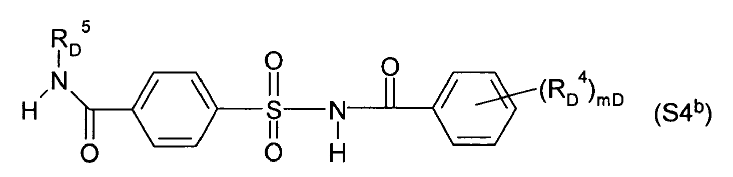 z.B. solche worin
   R_{D}⁵ = Cyclopropyl und (R_{D}⁴) = 2-OMe ist ("Cyprosulfamide", S4-1),
   R_{D}⁵ = Cyclopropyl und (R_{D}⁴) = 5-Cl-2-OMe ist (S4-2),
   R_{D}⁵ = Ethyl und (R_{D}⁴) = 2-OMe ist (S4-3),
   R_{D}⁵ = Isopropyl und (R_{D}⁴) = 5-Cl-2-OMe ist (S4-4) und
   R_{D}⁵ = Isopropyl und (R_{D}⁴) = 2-OMe ist (S4-5).
   sowie
   Verbindungen vom Typ der N-Acylsulfamoylphenylharnstoffe der Formel (S4^{c}), die z.B. bekannt sind aus der EP-A-365484, 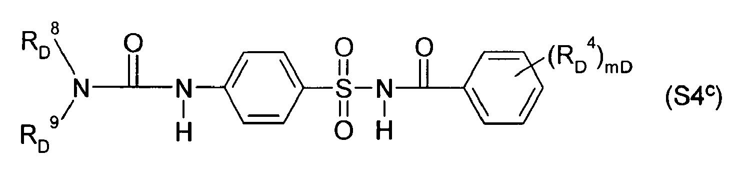 worin
   - R_{D}⁸ und R_{D}⁹: unabhängig voneinander Wasserstoff, (C₁-C₈)Alkyl, (C₃-C₈)Cycloalkyl, (C₃-C₆)Alkenyl, (C₃-C₆)Alkinyl,
   - R_{D}⁴: Halogen, (C₁-C₄)Alkyl, (C₁-C₄)Alkoxy, CF₃
   - m_{D}: 1 oder 2 bedeutet;
   beispielsweise
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff.
S5) Wirkstoffe aus der Klasse der Hydroxyaromaten und der aromatischaliphatischen Carbonsäurederivate (S5), z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydrochinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7),wie sie in der WO-A-1998/38856 beschrieben sind 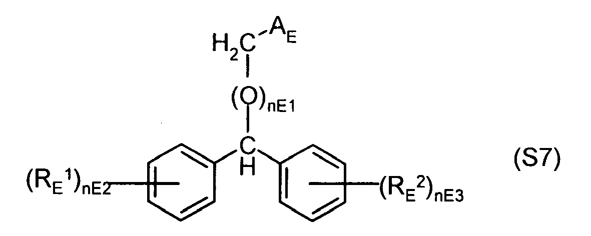 worin die Symbole und Indizes folgende Bedeutungen haben:
   - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - A_{E}: ist COOR_{E}³ oder COSR_{E}⁴
   - R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - n_{E}¹: ist 0 oder 1
   - n_{E}², n_{E}³: sind unabhängig voneinander 0, 1 oder 2,
   vorzugsweise:
   Diphenylmethoxyessigsäure,
   Diphenylmethoxyessigsäureethylester,
   Diphenylmethoxyessigsäuremethylester (CAS-Reg.Nr. 41858-19-9) (S7-1). S8) Verbindungen der Formel (S8),wie sie in der WO-A-98/27049 beschrieben
   sind 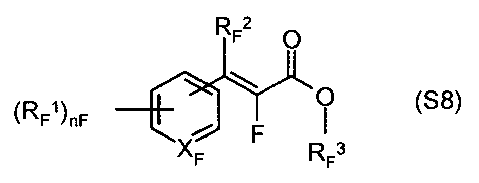 worin
   - X_{F}: CH oder N,
   - n_{F}: für den Fall, dass X_{F}=N ist, eine ganze Zahl von 0 bis 4 und
   für den Fall, dass X_{F}=CH ist, eine ganze Zahl von 0 bis 5 ,
   - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   - R_{F}²: Wasserstoff oder (C₁-C₄)Alkyl
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze,
   vorzugsweise Verbindungen worin
   - X_{F}: CH,
   - n_{F}: eine ganze Zahl von 0 bis 2 ,
   - R_{F}¹: Halogen, (C₁-C₄)Alkyl, (C₁-C₄)Haloalkyl, (C₁-C₄)Alkoxy, (C₁-C₄)Haloalkoxy,
   - R_{F}²: Wasserstoff oder (C₁-C₄)Alkyl,
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolylcarbonyl)-2-chinolon (CAS-Reg.Nr. 95855-00-8), wie sie in der WO-A-1999/000020 beschrieben sind.
S10) Verbindungen der Formeln (S10^{a}) oder (S10^{b}), wie sie in der WO-A-2007/023719 und WO-A-2007/023764 beschrieben sind 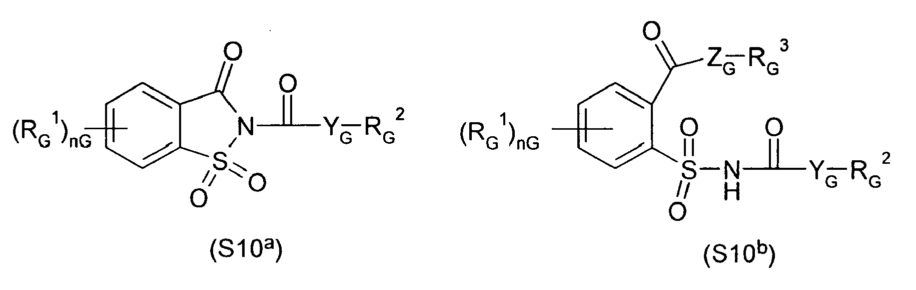 worin
   - R_{G}¹: Halogen, (C₁-C₄)Alkyl, Methoxy, Nitro, Cyano, CF₃, OCF₃
   - Y_{G}, Z_{G}: unabhängig voneinander O oder S,
   - n_{G}: eine ganze Zahl von 0 bis 4,
   - R_{G}²: (C₁-C₁₆)Alkyl, (C₂-C₆)Alkenyl, (C₃-C₆)Cycloalkyl, Aryl; Benzyl, Halogenbenzyl,
   - R_{G}³: Wasserstoff oder (C₁-C₆)Alkyl bedeutet.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8)
      (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG-838" (CAS-Reg.Nr. 133993-74-5)
      (2-propenyl 1-oxa-4-azaspiro[4.5]decane-4-carbodithioate) (S13-6) der Firma Nitrokemia,
   "Disulfoton" (O,O-Diethyl S-2-ethylthioethyl phosphordithioat) (S13-7),
   "Dietholate" (O,O-Diethyl-O-phenylphosphorotioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (S-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist, "Cumyluron" = "JC-940" (3-(2-Chlorphenylmethyl)-1-(1-methyl-1-phenylethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Einige der Safener sind bereits als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der Formel (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Formulierungen gegebenenfalls in üblicher Weise verdünnt z. B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der allgemeinen Formel (I). Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

Die vorliegende Erfindung wird ahand der nachfolgenden Beispiele näher erläutert, welche die Erfindung jedoch keinesfalls beschränken.

### Synthesebeispiele

Nachfolgend sind einige Synthesebeispiele von Verbindungen der allgemeinen Formel (I) oder deren Salze beispielhaft beschrieben.

### 2-({[5-(Difluormethoxy)-1-methyl-3-(trifluormethyl)-1H-pyrazol-4-yl]methyl}sulfanyl)-1,3-oxazol (Bsp. 49)

1,3-Oxazol-2(3H)-thion (2.960 g, 29 mmol; hergestellt wie beschrieben in WO 2003/006442 A) wird in 50 ml Acetonitril vorgelegt. Unter Eisbadkühlung wird 1,8-Diazabicyclo(5.4.0)undec-7-en (DBU, 4.81 ml, 32 mmol) zugetropft. Es wird 30 Minuten bei 25 °C nachgerührt. Eine Lösung von 4-(Brommethyl)-5-(difluormethoxy)-1-methyl-3-(trifluormethyl)-1 H-pyrazol (9.045 g, 29 mmol) gelöst in Acetonitril wird zugetropft. Man rührt weitere 4 Stunden bei 25 °C und lässt über Nacht stehen. Zur Aufarbeitung wird das Reaktionsgemisch auf Wasser gegeben und zweimal mit Dichlormethan extrahiert, danach mit Wasser und schließlich mit gesättigter NaCl-Lösung gewaschen. Die vereinigten organischen Phasen werden über Magnesiumsulfat getrocknet, abfiltriert und eingeengt. Das Rohprodukt wird chromatographisch gereinigt (Heptan : Ethylacetat, Gradient 10:0 bis 7:3). Man erhält 7.6 g Produkt (74.9 % d. Th.).
NMR (CDCl₃, 400 MHz): 3.82 (s, 3H, CH₃); 4.32 (s, 2H, SCH₂); 6.75 (t, 1H, OCHF₂); 7.11 (br s, 1H); 7.68 (br s, 1H).

### 2-({[5-(Difluormethoxy)-1-methyl-3-(trifluormethyl)-1H-pyrazol-4-yl]methyl}sulfinyl)-1,3-oxazol (Bsp. 50)

2-({[5-(Difluormethoxy)-1-methyl-3-(trifluormethyl)-1H-pyrazol-4-yl]methyl}sulfanyl)-1,3-oxazol (2.00 g, 6 mmol) wird in 333 ml Dichlormethan unter einer Argonatmosphäre vorgelegt. Dann wird unter Rühren und Eiskühlung portionsweise 3-Chlor-perbenzoesäure (1.362 g, 6 mmol, 77 %ig) zugegeben und weitere 6 Stunden bei 0 °C gerührt. Zur Aufarbeitung wird das Reaktionsgemisch zweimal mit 2 molarer Natriumhydroxid-Lösung, danach mit Wasser und schließlich mit gesättigter NaCl-Lösung gewaschen. Die vereinigten organischen Phasen werden über Magnesiumsulfat getrocknet, abfiltriert und eingeengt. Das Rohprodukt wird chromatographisch gereinigt (Heptan : Ethylacetat, Gradient 10:0 bis 6:4). Man erhält 1.98 g Produkt (89.7 % d. Th.).
NMR (CDCl₃, 400 MHz): 3.86 (s, 3H, CH₃); 4.36 (d, 1H, S(O)CH₂); 4.49 (d, 1H, S(O)CH₂); 6.97 (dd, 1H, OCHF₂); 7.39 (br s, 1H); 7.93 (br s, 1H).

### 2-({[5-(Difluormethoxy)-1-methyl-3-(trifluormethyl)-1H-pyrazol-4-yl]methyl}sulfonyl)-1,3-oxazol (Bsp. 51)

2-({[5-(Difluormethoxy)-1-methyl-3-(trifluormethyl)-1H-pyrazol-4-yl]methyl}sulfanyl)-1,3-oxazol (0.30 g, 1 mmol) wird in 50 ml Dichlormethan unter einer Argonatmosphäre vorgelegt. Dann wird unter Rühren und Eiskühlung portionsweise 3-Chlor-perbenzoesäure (0.449 g, 2 mmol, 77 %ig) zugegeben und weitere 3 Stunden bei 25 °C gerührt und über Nacht stehengelassen. Es werden weitere 100 mg 3-Chlor-perbenzoesäure zugegeben und weitere 4 Stunden bei 25 °C gerührt. Zur Aufarbeitung wird das Reaktionsgemisch zweimal mit 2 molarer Natriumhydroxid-Lösung, danach mit Wasser und schließlich mit gesättigter NaCl-Lösung gewaschen. Die vereinigten organischen Phasen werden über Magnesiumsulfat getrocknet, abfiltriert und eingeengt. Das Rohprodukt wird mittels präparativer HPLC (Reverse Phase) gereinigt. Man erhält 0.097 g Produkt (28 % d. Th.).
NMR (CDCl₃, 400 MHz): 3.87 (s, 3H, CH₃); 4.59 (s, 2H, S(O)₂CH₂); 6.84 (t, 1H, OCHF₂); 7.4 (br s, 1H); 7.89 (br s, 1H).

### 5-Brom-2-({[5-(difluormethoxy)-1-methyl-3-(trifluormethyl)-1H-pyrazol-4-yl]methyl}sulfanyl)-1,3-oxazol (Bsp. 733)

### a) Herstellung von 2-({[5-(Difluormethoxy)-1-methyl-3-(trifluormethyl)-1H-pyrazol-4-yl]methyl}sulfanyl)-1,3-oxazol [nach Can. J. Chem., Vol. 50, 3082-3083 (1972) ]

Dihydroxyfumarsäure (5.00 g, 34 mmol) wird portionsweise unter einer Argonatmosphäre zu 60 °C heißem Wasser (20 ml) gegeben (Gasentwicklung). Es wird so lange bei 60 °C gerührt bis keine Gasentwicklung mehr stattfindet (ca. 1h; Lösung 1). Kaliumrhodanid (3.282 g, 34 mmol) wird in Ethanol vorgelegt (25 ml). Bei 0 °C wird konzentrierte Salzsäure (4 ml, 4.76 g, 48 mmol) zugetropft und eine Stunde bei 25 °C gerührt. Das entstandene ausgefallene Kaliumchlorid wird abgesaugt und das Filtrat wird bei Raumtemperatur langsam zu Lösung 1 zugetropft und anschließend 12 Stunden bei Rückfluss weiter gerührt. Die Reaktionslösung wird eingeengt, und der wäßrige Rückstand wird mit Acetontril (50 ml) versetzt. Man gibt erst Kaliumcarbonat (7.00 g, 51 mmol) und dann 4-(Brommethyl)-5-(difluormethoxy)-1-methyl-3-(trifluormethyl)-1H-pyrazol (5.218 g, 17 mmol) zu und rührt weitere 7 Stunden bei 60 °C. Zur Aufarbeitung wird das Reaktionsgemisch auf Wasser gegeben und zweimal mit Ethylacetat extrahiert, danach mit Wasser und schließlich mit gesättigter NaCl-Lösung gewaschen. Die vereinigten organischen Phasen werden über Magnesiumsulfat getrocknet, abfiltriert und eingeengt. Das Rohprodukt wird chromatographisch gereinigt (Heptan : Ethylacetat, Gradient 10:0 bis 7:3). Man erhält 3.09 g Produkt (26.4 % d. Th.).

### b) Herstellung von 5-Brom-2-({[5-(difluormethoxy)-1-methyl-3-(trifluormethyl)-1H-pyrazol-4-yl]methyl}sulfanyl)-1,3-oxazol

2-({[5-(Difluormethoxy)-1-methyl-3-(trifluormethyl)-1H-pyrazol-4-yl]methyl}sulfanyl)-1,3-oxazol (1.24 g, 4 mmol) wird in 20 ml Dimethylformamid vorgelegt. Dann wird unter Rühren N-Bromsuccinimid (0.892 g, 5 mmol) portionsweise zugegeben. Man rührt weitere 6 Stunden bei 40 °C. Zur Aufarbeitung wird das Reaktionsgemisch auf Wasser gegeben, zweimal mit Dichlormethan extrahiert, danach mit Wasser und schließlich mit gesättigter NaCl-Lösung gewaschen. Die vereinigten organischen Phasen werden über Magnesiumsulfat getrocknet, abfiltriert und eingeengt. Das Rohprodukt wird chromatographisch gereinigt (Heptan : Ethylacetat, Gradient 10:0 bis 7:3). Man erhält 0.70 g Produkt (43.2 % d. Th.).
NMR (CDCl₃, 400 MHz): 3.81 (s, 3H, CH₃); 4.28 (s, 2H, SCH₂); 6.71 (t, 1H, OCHF₂); 6.98 (s, 1H).

### 5-Chlor-2-({[5-(difluormethoxy)-1-methyl-3-(trifluormethyl)-1H-pyrazol-4-yl]methyl}sulfanyl)-1,3-oxazol (Bsp. 505)

2-({[5-(Difluormethoxy)-1-methyl-3-(trifluormethyl)-1H-pyrazol-4-yl]methyl}sulfanyl)-1,3-oxazol (1.02 g, 3 mmol) wird in 15 ml Dimethylformamid vorgelegt. Dann wird unter Rühren N-Chlorsuccinimid (0.550 g, 4 mmol) portionsweise zugegeben. Man rührt weitere 4 Stunden bei 40 °C. Zur Aufarbeitung wird das Reaktionsgemisch auf Wasser gegeben, zweimal mit Dichlormethan extrahiert, danach mit Wasser und schließlich mit gesättigter NaCl-Lösung gewaschen. Die vereinigten organischen Phasen werden über Magnesiumsulfat getrocknet, abfiltriert und eingeengt. Das Rohprodukt wird chromatographisch gereinigt (Heptan : Ethylacetat, Gradient 10:0 bis 7:3). Man erhält 0.45 g Produkt (35.9 % d. Th.).
NMR (CDCl₃, 400 MHz): 3.80 (s, 3H, CH₃); 4.28 (s, 2H, SCH₂); 6.69 (t, 1H, OCHF₂); 6.88 (s, 1H).

### 5-lod-2-({[5-(difluormethoxy)-1-methyl-3-(trifluormethyl)-1H-pyrazol-4-yl]methyl}sulfanyl)-1,3-oxazol (Bsp. 961)

2-({[5-(Difluormethoxy)-1-methyl-3-(trifluormethyl)-1H-pyrazol-4-yl]methyl}sulfanyl)-1,3-oxazol (0.700 g, 2 mmol) wird in 10 ml Dimethylformamid vorgelegt. Dann wird unter Rühren N-lodsuccinimid (0.750 g, 3.3 mmol) portionsweise zugegeben. Man rührt weiter 16 Stunden bei 40 °C. Zur Aufarbeitung wird das Reaktionsgemisch auf Wasser gegeben, und zweimal mit Dichlormethan extrahiert, danach mit Wasser und schließlich mit gesättigter NaCl-Lösung gewaschen. Die vereinigten organischen Phasen werden über Magnesiumsulfat getrocknet, abfiltriert und eingeengt. Das Rohprodukt wird chromatographisch gereinigt (Heptan : Ethylacetat, Gradient 10:0 bis 8:2). Man erhält 0.25 g Produkt (24.5 % d. Th.).
NMR (CDCl₃, 400 MHz): 3.80 (s, 3H, CH₃); 4.29 (s, 2H, SCH₂); 6.70 (t, 1H, OCHF₂); 7.11 (s, 1H).

### 2-({[5-(Chlor)-1-methyl-3-(trifluormethyl)-1H-pyrazol-4-yl]methyl}sulfanyl)-1,3-oxazol (Bsp. 34)

### a) Herstellung von 2-(Methylsulfonyl)-1,3-oxazol

1,3-Oxazole-2(3H)-thione (1.00 g, 10 mmol; hergestellt wie beschrieben in WO 2003/006442 A) wird unter Schutzgasatmosphäre in 20 ml Acetonitril vorgelegt. lodmethan (1.544 g, 0.677 ml, 11 mmol) wird zugetropft, dann wird Kaliumcarbonat (1.503 ml, 11 mmol) addiert. Es wird 6 Stunden bei 25 °C nachgerührt. Zur Aufarbeitung wird das Reaktionsgemisch auf Wasser gegeben und zweimal mit Dichlormethan extrahiert (100 ml), danach mit Wasser und schließlich mit gesättigter NaCl-Lösung gewaschen. Die vereinigten organischen Phasen werden über Magnesiumsulfat getrocknet, abfiltriert und direkt weiter umgesetzt. Zu dieser Lösung in Dichlormethan wird dann unter Rühren und Eiskühlung portionsweise 3-Chlor-perbenzoesäure (5.100 g, 23 mmol, 77 %ig) zugegeben, weitere 6 Stunden bei 25 °C nachgerührt und über Nacht stehengelassen. Zur Aufarbeitung wird das Reaktionsgemisch zweimal mit 2 molarer Natriumhydroxid-Lösung, danach mit Wasser und schließlich mit gesättigter NaCl-Lösung gewaschen. Die vereinigten organischen Phasen werden über Magnesiumsulfat getrocknet, abfiltriert und eingeengt. Man erhält 0.820 g Produkt (50.7 % d. Th.).
NMR (CDCl₃, 400 MHz): 3.35 (s, 3H, CH₃); 7.38 (br s, 1 H); 7.88 (br s, 1H).

### b) Herstellung von 2-({[5-(Chlor)-1-methyl-3-(trifluormethyl)-1H-pyrazol-4-yl]methyl}sulfanyl)-1,3-oxazol

Zu einem heftig gerührten Gemisch bestehend aus 50 ml Toluol und 50 %iger wäßriger Natronlauge (21 g) wird [5-Chloro-1-methyl-3-(trifluoromethyl)-1 H-pyrazol-4-yl]methyl imidothiocarbamathydrobromid (1.934 g, 5 mmol) gegeben und weitere 1,5 Stunden heftig gerührt. Danach werden Tetra-n-butylammoniumbromid (0.494 g, 2 mmol) und 2-(Methylsulfonyl)-1,3-oxazol (0.805 g, 5 mmol) zugegeben und weitere 6 Stunden bei 25 °C stark gerührt. Zur Aufarbeitung wird die Reaktionslösung auf Wasser gegeben und mit Toluol extrahiert. Die vereinigten organischen Phasen werden getrocknet und eingeengt. Man erhält 1.290 g Produkt (75.2 % d. Th.).
NMR (CDCl₃, 400 MHz): 3.89 (s, 3H, CH₃); 4.33 (s, 2H, SCH₂); 7.12 (br s, 1H); 7.68 (br s, 1H).

Die in den nachfolgenden Tabellen 1 - 3 beschriebenen Verbindungen erhält man gemäß oder analog zu den oben beschriebenen Synthesebeispielen.

In den Tabellen bedeuten:
- Me: = Methyl
- Et: = Ethyl
- Ph: = Phenyl
- Pr: = n-Propyl
- cPr: = Cyclopropyl
- iPr: = Isopropyl
- tBu: = tert.-Butyl
- cPen: = Cyclopentyl

**Tabelle 1: Verbindungen der Formel (I)**

| | | | | | | |
|---|---|---|---|---|---|---|
| 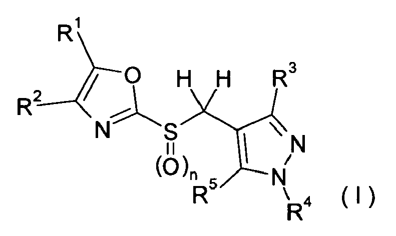 | | | | | | |

| Bsp. Nr. | R¹ | R² | R³ | R⁴ | R⁵ | n |
|---|---|---|---|---|---|---|
| 1. | H | H | CF₃ | Ph | Cl | 0 |
| 2. | H | H | CF₃ | Ph | Cl | 1 |
| 3. | H | H | CF₃ | Ph | Cl | 2 |
| 4. | H | H | CF₃ | tBu | Cl | 0 |
| 5. | H | H | CF₃ | tBu | Cl | 1 |
| 6. | H | H | CF₃ | tBu | Cl | 2 |
| 7. | H | H | CF₃ | CHF₂ | Cl | 0 |
| 8. | H | H | CF₃ | CHF₂ | Cl | 1 |
| 9. | H | H | CF₃ | CHF₂ | Cl | 2 |
| 10. | H | H | Cl | CHF₂ | CF₃ | 0 |
| 11. | H | H | Cl | CHF₂ | CF₃ | 1 |
| 12. | H | H | Cl | CHF₂ | CF₃ | 2 |
| 13. | H | H | CF₃ | Me | OMe | 0 |
| 14. | H | H | CF₃ | Me | OMe | 1 |
| 15. | H | H | CF₃ | Me | OMe | 2 |
| 16. | H | H | CF₃ | Me | CN | 0 |
| 17. | H | H | CF₃ | Me | CN | 1 |
| 18. | H | H | CF₃ | Me | CN | 2 |
| 19. | H | H | Cl | Et | Cl | 0 |
| 20. | H | H | Cl | Et | Cl | 1 |
| 21. | H | H | Cl | Et | Cl | 2 |
| 22. | H | H | CHF₂ | Me | Cl | 0 |
| 23. | H | H | CHF₂ | Me | Cl | 1 |
| 24. | H | H | CHF₂ | Me | Cl | 2 |
| 25. | H | H | Me | Me | Me | 0 |
| 26. | H | H | Me | Me | Me | 1 |
| 27. | H | H | Me | Me | Me | 2 |
| 28. | H | H | Me | Me | Cl | 0 |
| 29. | H | H | Me | Me | Cl | 1 |
| 30. | H | H | Me | Me | Cl | 2 |
| 31. | H | H | Cl | Me | Cl | 0 |
| 32. | H | H | Cl | Me | Cl | 1 |
| 33. | H | H | Cl | Me | Cl | 2 |
| 34. | H | H | CF₃ | Me | Cl | 0 |
| 35. | H | H | CF₃ | Me | Cl | 1 |
| 36. | H | H | CF₃ | Me | Cl | 2 |
| 37. | H | H | Cl | Me | CF₃ | 0 |
| 38. | H | H | Cl | Me | CF₃ | 1 |
| 39. | H | H | Cl | Me | CF₃ | 2 |
| 40. | H | H | CF₃ | Me | F | 0 |
| 41. | H | H | CF₃ | Me | F | 1 |
| 42. | H | H | CF₃ | Me | F | 2 |
| 43. | H | H | OMe | Me | CF₃ | 0 |
| 44. | H | H | OMe | Me | CF₃ | 1 |
| 45. | H | H | OMe | Me | CF₃ | 2 |
| 46. | H | H | CF₃ | Me | OEt | 0 |
| 47. | H | H | CF₃ | Me | OEt | 1 |
| 48. | H | H | CF₃ | Me | OEt | 2 |
| 49. | H | H | CF₃ | Me | OCHF₂ | 0 |
| 50. | H | H | CF₃ | Me | OCHF₂ | 1 |
| 51. | H | H | CF₃ | Me | OCHF₂ | 2 |
| 52. | H | H | OCHF₂ | Me | CF₃ | 0 |
| 53. | H | H | OCHF₂ | Me | CF₃ | 1 |
| 54. | H | H | OCHF₂ | Me | CF₃ | 2 |
| 55. | H | H | CF₃ | Me | OCH₂CHF₂ | 0 |
| 56. | H | H | CF₃ | Me | OCH₂CHF₂ | 1 |
| 57. | H | H | CF₃ | Me | OCH₂CHF2 | 2 |
| 58. | H | H | CF₃ | Me | OCH₂CF₃ | 0 |
| 59. | H | H | CF₃ | Me | OCH₂CF₃ | 1 |
| 60. | H | H | CF₃ | Me | OCH₂CF₃ | 2 |
| 61. | H | H | CF₃ | Me | OCH₂CN | 0 |
| 62. | H | H | CF₃ | Me | OCH₂CN | 1 |
| 63. | H | H | CF₃ | Me | OCH₂CN | 2 |
| 64. | H | H | CF₃ | Me | SO₂Me | 0 |
| 65. | H | H | CF₃ | Me | SO₂Me | 1 |
| 66. | H | H | CF₃ | Me | SO₂Me | 2 |
| 67. | H | H | CF₃ | Me | SEt | 0 |
| 68. | H | H | CF₃ | Me | SEt | 1 |
| 69. | H | H | CF₃ | Me | SEt | 2 |
| 70. | H | H | CF₃ | Me | Me | 0 |
| 71. | H | H | CF₃ | Me | Me | 1 |
| 72. | H | H | CF₃ | Me | Me | 2 |
| 73. | H | H | CF₃ | Me | Et | 0 |
| 74. | H | H | CF₃ | Me | Et | 1 |
| 75. | H | H | CF₃ | Me | Et | 2 |
| 76. | H | H | CF₃ | Et | Cl | 0 |
| 77. | H | H | CF₃ | Et | Cl | 1 |
| 78. | H | H | CF₃ | Et | Cl | 2 |
| 79. | H | H | Cl | Et | CF₃ | 0 |
| 80. | H | H | Cl | Et | CF₃ | 1 |
| 81. | H | H | Cl | Et | CF₃ | 2 |
| 82. | H | H | CF₃ | iPr | Cl | 0 |
| 83. | H | H | CF₃ | iPr | Cl | 1 |
| 84. | H | H | CF₃ | iPr | Cl | 2 |
| 85. | H | H | Cl | iPr | CF₃ | 0 |
| 86. | H | H | Cl | iPr | CF₃ | 1 |
| 87. | H | H | Cl | iPr | CF₃ | 2 |
| 88. | H | H | CF₃ | tBu | Cl | 0 |
| 89. | H | H | CF₃ | tBu | Cl | 1 |
| 90. | H | H | CF₃ | tBu | Cl | 2 |
| 91. | H | H | Cl | tBu | CF₃ | 0 |
| 92. | H | H | Cl | tBu | CF₃ | 1 |
| 93. | H | H | Cl | tBu | CF₃ | 2 |
| 94. | H | H | CF₃ | cPen | Cl | 0 |
| 95. | H | H | CF₃ | cPen | Cl | 1 |
| 96. | H | H | CF₃ | cPen | Cl | 2 |
| 97. | H | H | Cl | cPen | CF₃ | 0 |
| 98. | H | H | Cl | cPen | CF₃ | 1 |
| 99. | H | H | Cl | cPen | CF₃ | 2 |
| 100. | H | H | CF₃ | CH₂cPr | Cl | 0 |
| 101. | H | H | CF₃ | CH₂cPr | Cl | 1 |
| 102. | H | H | CF₃ | CH₂cPr | Cl | 2 |
| 103. | H | H | Cl | CH₂cPr | CF₃ | 0 |
| 104. | H | H | Cl | CH₂cPr | CF₃ | 1 |
| 105. | H | H | Cl | CH₂cPr | CF₃ | 2 |
| 106. | H | H | CF₃ | CH₂CH=CH₂ | Cl | 0 |
| 107. | H | H | CF₃ | CH₂CH=CH₂ | Cl | 1 |
| 108. | H | H | CF₃ | CH₂CH=CH₂ | Cl | 2 |
| 109. | H | H | Cl | CH₂CH=CH₂ | CF₃ | 0 |
| 110. | H | H | Cl | CH₂CH=CH₂ | CF₃ | 1 |
| 111. | H | H | Cl | CH₂CH=CH₂ | CF₃ | 2 |
| 112. | H | H | CF₃ | CHF₂ | OMe | 0 |
| 113. | H | H | CF₃ | CHF₂ | OMe | 1 |
| 114. | H | H | CF₃ | CHF₂ | OMe | 2 |
| 115. | H | H | OMe | CHF₂ | CF₃ | 0 |
| 116. | H | H | OMe | CHF₂ | CF₃ | 1 |
| 117. | H | H | OMe | CHF₂ | CF₃ | 2 |
| 118. | H | H | CF₃ | CH₂CF₃ | Cl | 0 |
| 119. | H | H | CF₃ | CH₂CF₃ | Cl | 1 |
| 120. | H | H | CF₃ | CH₂CF₃ | Cl | 2 |
| 121. | H | H | Cl | CH₂CF₃ | CF₃ | 0 |
| 122. | H | H | Cl | CH₂CF₃ | CF₃ | 1 |
| 123. | H | H | Cl | CH₂CF₃ | CF₃ | 2 |
| 124. | H | H | CF₃ | CH₂OMe | Cl | 0 |
| 125. | H | H | CF₃ | CH₂OMe | Cl | 1 |
| 126. | H | H | CF₃ | CH₂OMe | Cl | 2 |
| 127. | H | H | Cl | CH₂OMe | CF₃ | 0 |
| 128. | H | H | Cl | CH₂OMe | CF₃ | 1 |
| 129. | H | H | Cl | CH₂OMe | CF₃ | 2 |
| 130. | H | H | CF₃ | CH₂CN | Cl | 0 |
| 131. | H | H | CF₃ | CH₂CN | Cl | 1 |
| 132. | H | H | CF₃ | CH₂CN | Cl | 2 |
| 133. | H | H | Me | Ph | Me | 0 |
| 134. | H | H | Me | Ph | Me | 1 |
| 135. | H | H | Me | Ph | Me | 2 |
| 136. | H | H | Me | Ph | Cl | 0 |
| 137. | H | H | Me | Ph | Cl | 1 |
| 138. | H | H | Me | Ph | Cl | 2 |
| 139. | H | H | Et | Ph | Cl | 0 |
| 140. | H | H | Et | Ph | Cl | 1 |
| 141. | H | H | Et | Ph | Cl | 2 |
| 142. | H | H | Pr | Ph | Cl | 0 |
| 143. | H | H | Pr | Ph | Cl | 1 |
| 144. | H | H | Pr | Ph | Cl | 2 |
| 145. | H | H | iPr | Ph | Cl | 0 |
| 146. | H | H | iPr | Ph | Cl | 1 |
| 147. | H | H | iPr | Ph | Cl | 2 |
| 148. | H | H | CF₃ | Ph | Cl | 0 |
| 149. | H | H | CF₃ | Ph | Cl | 1 |
| 150. | H | H | CF₃ | Ph | Cl | 2 |
| 151. | H | H | CF₃ | Ph | Me | 0 |
| 152. | H | H | CF₃ | Ph | Me | 1 |
| 153. | H | H | CF₃ | Ph | Me | 2 |
| 154. | H | H | CF₃ | Ph | CF₃ | 0 |
| 155. | H | H | CF₃ | Ph | CF₃ | 1 |
| 156. | H | H | CF₃ | Ph | CF₃ | 2 |
| 157. | H | H | CF₃ | Ph | F | 0 |
| 158. | H | H | CF₃ | Ph | F | 1 |
| 159. | H | H | CF₃ | Ph | F | 2 |
| 160. | H | H | CF₃ | Ph | OMe | 0 |
| 161. | H | H | CF₃ | Ph | OMe | 1 |
| 162. | H | H | CF₃ | Ph | OMe | 2 |
| 163. | H | H | CF₃ | Ph | OEt | 0 |
| 164. | H | H | CF₃ | Ph | OEt | 1 |
| 165. | H | H | CF₃ | Ph | OEt | 2 |
| 166. | H | H | CF₃ | Ph | OCHF₂ | 0 |
| 167. | H | H | CF₃ | Ph | OCHF₂ | 1 |
| 168. | H | H | CF₃ | Ph | OCHF₂ | 2 |
| 169. | H | H | CF₃ | Ph | CN | 0 |
| 170. | H | H | CF₃ | Ph | CN | 1 |
| 171. | H | H | CF₃ | Ph | CN | 2 |
| 172. | H | H | CF₃ | Ph(4-Cl) | Cl | 0 |
| 173. | H | H | CF₃ | Ph(4-Cl) | Cl | 1 |
| 174. | H | H | CF₃ | Ph(4-Cl) | Cl | 2 |
| 175. | H | H | Me | Me | OCH₂CF₃ | 0 |
| 176. | H | H | Me | Me | OCH₂CF₃ | 1 |
| 177. | H | H | Me | Me | OCH₂CF₃ | 2 |
| 178. | H | H | CF₃ | Me | 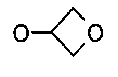 | 0 |
| 179. | H | H | CF₃ | Me | 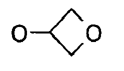 | 1 |
| 180. | H | H | CF₃ | Me | 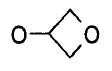 | 2 |
| 181. | H | H | CF₃ | Me | H | 0 |
| 182. | H | H | CF₃ | Me | H | 1 |
| 183. | H | H | CF₃ | Me | H | 2 |
| 184. | H | H | CF3 | Me | OCH₂CH₂OMe | 0 |
| 185. | H | H | CF3 | Me | OCH₂CH₂OMe | 1 |
| 186. | H | H | CF₃ | Me | OCH₂CH₂OMe | 2 |
| 187. | H | H | CF₃ | Me | SMe | 0 |
| 188. | H | H | CF₃ | Me | SMe | 1 |
| 189. | H | H | CF₃ | Me | SMe | 2 |
| 190. | H | H | CF₃ | Me | OCH₂CH₂CH₂F | 0 |
| 191. | H | H | CF₃ | Me | OCH₂CH₂CH₂F | 1 |
| 192. | H | H | CF₃ | Me | OCH₂CH₂CH₂F | 2 |
| 193. | H | H | CF₃ | Me | OCH(CH₂F)₂ | 0 |
| 194. | H | H | CF₃ | Me | OCH(CH₂F)₂ | 1 |
| 195. | H | H | CF₃ | Me | OCH(CH₂F)₂ | 2 |
| 196. | H | H | CF₃ | Me | OCH₂CF₂CHF₂ | 0 |
| 197. | H | H | CF₃ | Me | OCH₂CF₂CHF₂ | 1 |
| 198. | H | H | CF₃ | Me | OCH₂CF₂CHF₂ | 2 |
| 199. | H | H | CF₃ | Me | OCH₂CF=CH₂ | 0 |
| 200. | H | H | CF₃ | Me | OCH₂CF=CH₂ | 1 |
| 201. | H | H | CF₃ | Me | OCH₂CF=CH₂ | 2 |
| 202. | H | H | CF₃ | Me | OCH(Me)CF₃ | 0 |
| 203. | H | H | CF₃ | Me | OCH(Me)CF₃ | 1 |
| 204. | H | H | CF₃ | Me | OCH(Me)CF₃ | 2 |
| 205. | H | H | CF₃ | Me | OCH(Me)CH₂F | 0 |
| 206. | H | H | CF₃ | Me | OCH(Me)CH₂F | 1 |
| 207. | H | H | CF₃ | Me | OCH(Me)CH₂F | 2 |
| 208. | H | H | OCH₂CF₃ | Me | CF₃ | 0 |
| 209. | H | H | OCH₂CF₃ | Me | CF₃ | 1 |
| 210. | H | H | OCH₂CF₃ | Me | CF₃ | 2 |
| 211. | H | H | OCH₂CF₃ | Me | CHF₂ | 0 |
| 212. | H | H | OCH₂CF₃ | Me | CHF₂ | 1 |
| 213. | H | H | OCH₂CF₃ | Me | CHF₂ | 2 |
| 214. | H | H | CHF₂ | Me | CHF₂ | 0 |
| 215. | H | H | CHF₂ | Me | CHF₂ | 1 |
| 216. | H | H | CHF₂ | Me | CHF₂ | 2 |
| 217. | H | H | CF₃ | Me | CHF₂ | 0 |
| 218. | H | H | CF₃ | Me | CHF₂ | 1 |
| 219. | H | H | CF₃ | Me | CHF₂ | 2 |
| 220. | H | H | Cl | Me | OCHF₂ | 0 |
| 221. | H | H | Cl | Me | OCHF₂ | 1 |
| 222. | H | H | Cl | Me | OCHF₂ | 2 |
| 223. | H | H | Br | Me | OCHF₂ | 0 |
| 224. | H | H | Br | Me | OCHF₂ | 1 |
| 225. | H | H | Br | Me | OCHF₂ | 2 |
| 226. | H | H | Br | Me | CF₃ | 0 |
| 227. | H | H | Br | Me | CF₃ | 1 |
| 228. | H | H | Br | Me | CF₃ | 2 |
| 229. | F | H | CF₃ | Ph | Cl | 0 |
| 230. | F | H | CF₃ | Ph | Cl | 1 |
| 231. | F | H | CF₃ | Ph | Cl | 2 |
| 232. | F | H | CF₃ | tBu | Cl | 0 |
| 233. | F | H | CF₃ | tBu | Cl | 1 |
| 234. | F | H | CF₃ | tBu | Cl | 2 |
| 235. | F | H | CF₃ | CHF₂ | Cl | 0 |
| 236. | F | H | CF₃ | CHF₂ | Cl | 1 |
| 237. | F | H | CF₃ | CHF₂ | Cl | 2 |
| 238. | F | H | Cl | CHF₂ | CF₃ | 0 |
| 239. | F | H | Cl | CHF₂ | CF₃ | 1 |
| 240. | F | H | Cl | CHF₂ | CF₃ | 2 |
| 241. | F | H | CF₃ | Me | OMe | 0 |
| 242. | F | H | CF₃ | Me | OMe | 1 |
| 243. | F | H | CF₃ | Me | OMe | 2 |
| 244. | F | H | CF₃ | Me | CN | 0 |
| 245. | F | H | CF₃ | Me | CN | 1 |
| 246. | F | H | CF₃ | Me | CN | 2 |
| 247. | F | H | Cl | Et | Cl | 0 |
| 248. | F | H | Cl | Et | Cl | 1 |
| 249. | F | H | Cl | Et | Cl | 2 |
| 250. | F | H | CHF₂ | Me | Cl | 0 |
| 251. | F | H | CHF₂ | Me | Cl | 1 |
| 252. | F | H | CHF₂ | Me | Cl | 2 |
| 253. | F | H | Me | Me | Me | 0 |
| 254. | F | H | Me | Me | Me | 1 |
| 255. | F | H | Me | Me | Me | 2 |
| 256. | F | H | Me | Me | Cl | 0 |
| 257. | F | H | Me | Me | Cl | 1 |
| 258. | F | H | Me | Me | Cl | 2 |
| 259. | F | H | Cl | Me | Cl | 0 |
| 260. | F | H | Cl | Me | Cl | 1 |
| 261. | F | H | Cl | Me | Cl | 2 |
| 262. | F | H | CF₃ | Me | Cl | 0 |
| 263. | F | H | CF₃ | Me | Cl | 1 |
| 264. | F | H | CF₃ | Me | Cl | 2 |
| 265. | F | H | Cl | Me | CF₃ | 0 |
| 266. | F | H | Cl | Me | CF₃ | 1 |
| 267. | F | H | Cl | Me | CF₃ | 2 |
| 268. | F | H | CF₃ | Me | F | 0 |
| 269. | F | H | CF₃ | Me | F | 1 |
| 270. | F | H | CF₃ | Me | F | 2 |
| 271. | F | H | OMe | Me | CF₃ | 0 |
| 272. | F | H | OMe | Me | CF₃ | 1 |
| 273. | F | H | OMe | Me | CF₃ | 2 |
| 274. | F | H | CF₃ | Me | OEt | 0 |
| 275. | F | H | CF₃ | Me | OEt | 1 |
| 276. | F | H | CF₃ | Me | OEt | 2 |
| 277. | F | H | CF₃ | Me | OCHF₂ | 0 |
| 278. | F | H | CF₃ | Me | OCHF₂ | 1 |
| 279. | F | H | CF₃ | Me | OCHF₂ | 2 |
| 280. | F | H | OCHF₂ | Me | CF₃ | 0 |
| 281. | F | H | OCHF₂ | Me | CF₃ | 1 |
| 282. | F | H | OCHF₂ | Me | CF₃ | 2 |
| 283. | F | H | CF₃ | Me | OCH₂CHF₂ | 0 |
| 284. | F | H | CF₃ | Me | OCH₂CHF₂ | 1 |
| 285. | F | H | CF₃ | Me | OCH₂CHF₂ | 2 |
| 286. | F | H | CF₃ | Me | OCH₂CF₃ | 0 |
| 287. | F | H | CF₃ | Me | OCH₂CF₃ | 1 |
| 288. | F | H | CF₃ | Me | OCH₂CF₃ | 2 |
| 289. | F | H | CF₃ | Me | OCH₂CN | 0 |
| 290. | F | H | CF₃ | Me | OCH₂CN | 1 |
| 291. | F | H | CF₃ | Me | OCH₂CN | 2 |
| 292. | F | H | CF₃ | Me | SO₂Me | 0 |
| 293. | F | H | CF₃ | Me | SO₂Me | 1 |
| 294. | F | H | CF₃ | Me | SO₂Me | 2 |
| 295. | F | H | CF₃ | Me | SEt | 0 |
| 296. | F | H | CF₃ | Me | SEt | 1 |
| 297. | F | H | CF₃ | Me | SEt | 2 |
| 298. | F | H | CF₃ | Me | Me | 0 |
| 299. | F | H | CF₃ | Me | Me | 1 |
| 300. | F | H | CF₃ | Me | Me | 2 |
| 301. | F | H | CF₃ | Me | Et | 0 |
| 302. | F | H | CF₃ | Me | Et | 1 |
| 303. | F | H | CF₃ | Me | Et | 2 |
| 304. | F | H | CF₃ | Et | Cl | 0 |
| 305. | F | H | CF₃ | Et | Cl | 1 |
| 306. | F | H | CF₃ | Et | Cl | 2 |
| 307. | F | H | Cl | Et | CF₃ | 0 |
| 308. | F | H | Cl | Et | CF₃ | 1 |
| 309. | F | H | Cl | Et | CF₃ | 2 |
| 310. | F | H | CF₃ | iPr | Cl | 0 |
| 311. | F | H | CF₃ | iPr | Cl | 1 |
| 312. | F | H | CF₃ | iPr | Cl | 2 |
| 313. | F | H | Cl | iPr | CF₃ | 0 |
| 314. | F | H | Cl | iPr | CF₃ | 1 |
| 315. | F | H | Cl | iPr | CF₃ | 2 |
| 316. | F | H | CF₃ | tBu | Cl | 0 |
| 317. | F | H | CF₃ | tBu | Cl | 1 |
| 318. | F | H | CF₃ | tBu | Cl | 2 |
| 319. | F | H | Cl | tBu | CF₃ | 0 |
| 320. | F | H | Cl | tBu | CF₃ | 1 |
| 321. | F | H | Cl | tBu | CF₃ | 2 |
| 322. | F | H | CF₃ | cPen | Cl | 0 |
| 323. | F | H | CF₃ | cPen | Cl | 1 |
| 324. | F | H | CF₃ | cPen | Cl | 2 |
| 325. | F | H | Cl | cPen | CF₃ | 0 |
| 326. | F | H | Cl | cPen | CF₃ | 1 |
| 327. | F | H | Cl | cPen | CF₃ | 2 |
| 328. | F | H | CF₃ | CH₂cPr | Cl | 0 |
| 329. | F | H | CF₃ | CH₂cPr | Cl | 1 |
| 330. | F | H | CF₃ | CH₂cPr | Cl | 2 |
| 331. | F | H | Cl | CH₂cPr | CF₃ | 0 |
| 332. | F | H | Cl | CH₂cPr | CF₃ | 1 |
| 333. | F | H | Cl | CH₂cPr | CF₃ | 2 |
| 334. | F | H | CF₃ | CH₂CH=CH₂ | Cl | 0 |
| 335. | F | H | CF₃ | CH₂CH=CH₂ | Cl | 1 |
| 336. | F | H | CF₃ | CH₂CH=CH₂ | Cl | 2 |
| 337. | F | H | Cl | CH₂CH=CH₂ | CF₃ | 0 |
| 338. | F | H | Cl | CH₂CH=CH₂ | CF₃ | 1 |
| 339. | F | H | Cl | CH₂CH=CH₂ | CF₃ | 2 |
| 340. | F | H | CF₃ | CHF₂ | OMe | 0 |
| 341. | F | H | CF₃ | CHF₂ | OMe | 1 |
| 342. | F | H | CF₃ | CHF₂ | OMe | 2 |
| 343. | F | H | OMe | CHF₂ | CF₃ | 0 |
| 344. | F | H | OMe | CHF₂ | CF₃ | 1 |
| 345. | F | H | OMe | CHF₂ | CF₃ | 2 |
| 346. | F | H | CF₃ | CH₂CF₃ | Cl | 0 |
| 347. | F | H | CF₃ | CH₂CF₃ | Cl | 1 |
| 348. | F | H | CF₃ | CH₂CF₃ | Cl | 2 |
| 349. | F | H | Cl | CH₂CF₃ | CF₃ | 0 |
| 350. | F | H | Cl | CH₂CF₃ | CF₃ | 1 |
| 351. | F | H | Cl | CH₂CF₃ | CF₃ | 2 |
| 352. | F | H | CF₃ | CH₂OMe | Cl | 0 |
| 353. | F | H | CF₃ | CH₂OMe | Cl | 1 |
| 354. | F | H | CF₃ | CH₂OMe | Cl | 2 |
| 355. | F | H | Cl | CH₂OMe | CF₃ | 0 |
| 356. | F | H | Cl | CH₂OMe | CF₃ | 1 |
| 357. | F | H | Cl | CH₂OMe | CF₃ | 2 |
| 358. | F | H | CF₃ | CH₂CN | Cl | 0 |
| 359. | F | H | CF₃ | CH₂CN | Cl | 1 |
| 360. | F | H | CF₃ | CH₂CN | Cl | 2 |
| 361. | F | H | Me | Ph | Me | 0 |
| 362. | F | H | Me | Ph | Me | 1 |
| 363. | F | H | Me | Ph | Me | 2 |
| 364. | F | H | Me | Ph | Cl | 0 |
| 365. | F | H | Me | Ph | Cl | 1 |
| 366. | F | H | Me | Ph | Cl | 2 |
| 367. | F | H | Et | Ph | Cl | 0 |
| 368. | F | H | Et | Ph | Cl | 1 |
| 369. | F | H | Et | Ph | Cl | 2 |
| 370. | F | H | Pr | Ph | Cl | 0 |
| 371. | F | H | Pr | Ph | Cl | 1 |
| 372. | F | H | Pr | Ph | Cl | 2 |
| 373. | F | H | iPr | Ph | Cl | 0 |
| 374. | F | H | iPr | Ph | Cl | 1 |
| 375. | F | H | iPr | Ph | Cl | 2 |
| 376. | F | H | CF₃ | Ph | Cl | 0 |
| 377. | F | H | CF₃ | Ph | Cl | 1 |
| 378. | F | H | CF₃ | Ph | Cl | 2 |
| 379. | F | H | CF₃ | Ph | Me | 0 |
| 380. | F | H | CF₃ | Ph | Me | 1 |
| 381. | F | H | CF₃ | Ph | Me | 2 |
| 382. | F | H | CF₃ | Ph | CF₃ | 0 |
| 383. | F | H | CF₃ | Ph | CF₃ | 1 |
| 384. | F | H | CF₃ | Ph | CF₃ | 2 |
| 385. | F | H | CF₃ | Ph | F | 0 |
| 386. | F | H | CF₃ | Ph | F | 1 |
| 387. | F | H | CF₃ | Ph | F | 2 |
| 388. | F | H | CF₃ | Ph | OMe | 0 |
| 389. | F | H | CF₃ | Ph | OMe | 1 |
| 390. | F | H | CF₃ | Ph | OMe | 2 |
| 391. | F | H | CF₃ | Ph | OEt | 0 |
| 392. | F | H | CF₃ | Ph | OEt | 1 |
| 393. | F | H | CF₃ | Ph | OEt | 2 |
| 394. | F | H | CF₃ | Ph | OCHF₂ | 0 |
| 395. | F | H | CF₃ | Ph | OCHF₂ | 1 |
| 396. | F | H | CF₃ | Ph | OCHF₂ | 2 |
| 397. | F | H | CF₃ | Ph | CN | 0 |
| 398. | F | H | CF₃ | Ph | CN | 1 |
| 399. | F | H | CF₃ | Ph | CN | 2 |
| 400. | F | H | CF₃ | Ph(4-Cl) | Cl | 0 |
| 401. | F | H | CF₃ | Ph(4-Cl) | Cl | 1 |
| 402. | F | H | CF₃ | Ph(4-Cl) | Cl | 2 |
| 403. | F | H | Me | Me | OCH₂CF₃ | 0 |
| 404. | F | H | Me | Me | OCH₂CF₃ | 1 |
| 405. | F | H | Me | Me | OCH₂CF₃ | 2 |
| 406. | F | H | CF₃ | Me | 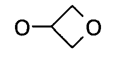 | 0 |
| 407. | F | H | CF₃ | Me | 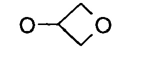 | 1 |
| 408. | F | H | CF₃ | Me | 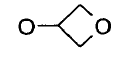 | 2 |
| 409. | F | H | CF₃ | Me | H | 0 |
| 410. | F | H | CF₃ | Me | H | 1 |
| 411. | F | H | CF₃ | Me | H | 2 |
| 412. | F | H | CF₃ | Me | OCH₂CH₂OMe | 0 |
| 413. | F | H | CF₃ | Me | OCH₂CH₂OMe | 1 |
| 414. | F | H | CF₃ | Me | OCH₂CH₂OMe | 2 |
| 415. | F | H | CF₃ | Me | SMe | 0 |
| 416. | F | H | CF₃ | Me | SMe | 1 |
| 417. | F | H | CF₃ | Me | SMe | 2 |
| 418. | F | H | CF₃ | Me | OCH₂CH₂CH₂F | 0 |
| 419. | F | H | CF₃ | Me | OCH₂CH₂CH₂F | 1 |
| 420. | F | H | CF₃ | Me | OCH₂CH₂CH₂F | 2 |
| 421. | F | H | CF₃ | Me | OCH(CH₂F)₂ | 0 |
| 422. | F | H | CF₃ | Me | OCH(CH₂F)₂ | 1 |
| 423. | F | H | CF₃ | Me | OCH(CH₂F)₂ | 2 |
| 424. | F | H | CF₃ | Me | OCH₂CF₂CHF₂ | 0 |
| 425. | F | H | CF₃ | Me | OCH₂CF₂CHF₂ | 1 |
| 426. | F | H | CF₃ | Me | OCH₂CF₂CHF₂ | 2 |
| 427. | F | H | CF₃ | Me | OCH₂CF=CH₂ | 0 |
| 428. | F | H | CF₃ | Me | OCH₂CF=CH₂ | 1 |
| 429. | F | H | CF₃ | Me | OCH₂CF=CH₂ | 2 |
| 430. | F | H | CF₃ | Me | OCH(Me)CF₃ | 0 |
| 431. | F | H | CF₃ | Me | OCH(Me)CF₃ | 1 |
| 432. | F | H | CF₃ | Me | OCH(Me)CF₃ | 2 |
| 433. | F | H | CF₃ | Me | OCH(Me)CH₂F | 0 |
| 434. | F | H | CF₃ | Me | OCH(Me)CH₂F | 1 |
| 435. | F | H | CF₃ | Me | OCH(Me)CH₂F | 2 |
| 436. | F | H | OCH₂CF₃ | Me | CF₃ | 0 |
| 437. | F | H | OCH₂CF₃ | Me | CF₃ | 1 |
| 438. | F | H | OCH₂CF₃ | Me | CF₃ | 2 |
| 439. | F | H | OCH₂CF₃ | Me | CHF₂ | 0 |
| 440. | F | H | OCH₂CF₃ | Me | CHF₂ | 1 |
| 441. | F | H | OCH₂CF₃ | Me | CHF₂ | 2 |
| 442. | F | H | CHF₂ | Me | CHF₂ | 0 |
| 443. | F | H | CHF₂ | Me | CHF₂ | 1 |
| 444. | F | H | CHF₂ | Me | CHF₂ | 2 |
| 445. | F | H | CF₃ | Me | CHF₂ | 0 |
| 446. | F | H | CF₃ | Me | CHF₂ | 1 |
| 447. | F | H | CF₃ | Me | CHF₂ | 2 |
| 448. | F | H | Cl | Me | OCHF₂ | 0 |
| 449. | F | H | Cl | Me | OCHF₂ | 1 |
| 450. | F | H | Cl | Me | OCHF₂ | 2 |
| 451. | F | H | Br | Me | OCHF₂ | 0 |
| 452. | F | H | Br | Me | OCHF₂ | 1 |
| 453. | F | H | Br | Me | OCHF₂ | 2 |
| 454. | F | H | Br | Me | CF₃ | 0 |
| 455. | F | H | Br | Me | CF₃ | 1 |
| 456. | F | H | Br | Me | CF₃ | 2 |
| 457. | Cl | H | CF₃ | Ph | Cl | 0 |
| 458. | Cl | H | CF₃ | Ph | Cl | 1 |
| 459. | Cl | H | CF₃ | Ph | Cl | 2 |
| 460. | Cl | H | CF₃ | tBu | Cl | 0 |
| 461. | Cl | H | CF₃ | tBu | Cl | 1 |
| 462. | Cl | H | CF₃ | tBu | Cl | 2 |
| 463. | Cl | H | CF₃ | CHF₂ | Cl | 0 |
| 464. | Cl | H | CF₃ | CHF₂ | Cl | 1 |
| 465. | Cl | H | CF₃ | CHF₂ | Cl | 2 |
| 466. | Cl | H | Cl | CHF₂ | CF₃ | 0 |
| 467. | Cl | H | Cl | CHF₂ | CF₃ | 1 |
| 468. | Cl | H | Cl | CHF₂ | CF₃ | 2 |
| 469. | Cl | H | CF₃ | Me | OMe | 0 |
| 470. | Cl | H | CF₃ | Me | OMe | 1 |
| 471. | Cl | H | CF₃ | Me | OMe | 2 |
| 472. | Cl | H | CF₃ | Me | CN | 0 |
| 473. | Cl | H | CF₃ | Me | CN | 1 |
| 474. | Cl | H | CF₃ | Me | CN | 2 |
| 475. | Cl | H | Cl | Et | Cl | 0 |
| 476. | Cl | H | Cl | Et | Cl | 1 |
| 477. | Cl | H | Cl | Et | Cl | 2 |
| 478. | Cl | H | CHF₂ | Me | Cl | 0 |
| 479. | Cl | H | CHF₂ | Me | Cl | 1 |
| 480. | Cl | H | CHF₂ | Me | Cl | 2 |
| 481. | Cl | H | Me | Me | Me | 0 |
| 482. | Cl | H | Me | Me | Me | 1 |
| 483. | Cl | H | Me | Me | Me | 2 |
| 484. | Cl | H | Me | Me | Cl | 0 |
| 485. | Cl | H | Me | Me | Cl | 1 |
| 486. | Cl | H | Me | Me | Cl | 2 |
| 487. | Cl | H | Cl | Me | Cl | 0 |
| 488. | Cl | H | Cl | Me | Cl | 1 |
| 489. | Cl | H | Cl | Me | Cl | 2 |
| 490. | Cl | H | CF₃ | Me | Cl | 0 |
| 491. | Cl | H | CF₃ | Me | Cl | 1 |
| 492. | Cl | H | CF₃ | Me | Cl | 2 |
| 493. | Cl | H | Cl | Me | CF₃ | 0 |
| 494. | Cl | H | Cl | Me | CF₃ | 1 |
| 495. | Cl | H | Cl | Me | CF₃ | 2 |
| 496. | Cl | H | CF₃ | Me | F | 0 |
| 497. | Cl | H | CF₃ | Me | F | 1 |
| 498. | Cl | H | CF₃ | Me | F | 2 |
| 499. | Cl | H | OMe | Me | CF₃ | 0 |
| 500. | Cl | H | OMe | Me | CF₃ | 1 |
| 501. | Cl | H | OMe | Me | CF₃ | 2 |
| 502. | Cl | H | CF₃ | Me | OEt | 0 |
| 503. | Cl | H | CF₃ | Me | OEt | 1 |
| 504. | Cl | H | CF₃ | Me | OEt | 2 |
| 505. | Cl | H | CF₃ | Me | OCHF₂ | 0 |
| 506. | Cl | H | CF₃ | Me | OCHF₂ | 1 |
| 507. | Cl | H | CF₃ | Me | OCHF₂ | 2 |
| 508. | Cl | H | OCHF₂ | Me | CF₃ | 0 |
| 509. | Cl | H | OCHF₂ | Me | CF₃ | 1 |
| 510. | Cl | H | OCHF₂ | Me | CF₃ | 2 |
| 511. | Cl | H | CF₃ | Me | OCH₂CHF₂ | 0 |
| 512. | Cl | H | CF₃ | Me | OCH₂CHF₂ | 1 |
| 513. | Cl | H | CF₃ | Me | OCH₂CHF₂ | 2 |
| 514. | Cl | H | CF₃ | Me | OCH₂CF₃ | 0 |
| 515. | Cl | H | CF₃ | Me | OCH₂CF₃ | 1 |
| 516. | Cl | H | CF₃ | Me | OCH₂CF₃ | 2 |
| 517. | Cl | H | CF₃ | Me | OCH₂CN | 0 |
| 518. | Cl | H | CF₃ | Me | OCH₂CN | 1 |
| 519. | Cl | H | CF₃ | Me | OCH₂CN | 2 |
| 520. | Cl | H | CF₃ | Me | SO₂Me | 0 |
| 521. | Cl | H | CF₃ | Me | SO₂Me | 1 |
| 522. | Cl | H | CF₃ | Me | SO₂Me | 2 |
| 523. | Cl | H | CF₃ | Me | SEt | 0 |
| 524. | Cl | H | CF₃ | Me | SEt | 1 |
| 525. | Cl | H | CF₃ | Me | SEt | 2 |
| 526. | Cl | H | CF₃ | Me | Me | 0 |
| 527. | Cl | H | CF₃ | Me | Me | 1 |
| 528. | Cl | H | CF₃ | Me | Me | 2 |
| 529. | Cl | H | CF₃ | Me | Et | 0 |
| 530. | Cl | H | CF₃ | Me | Et | 1 |
| 531. | Cl | H | CF₃ | Me | Et | 2 |
| 532. | Cl | H | CF₃ | Et | Cl | 0 |
| 533. | Cl | H | CF₃ | Et | Cl | 1 |
| 534. | Cl | H | CF₃ | Et | Cl | 2 |
| 535. | Cl | H | Cl | Et | CF₃ | 0 |
| 536. | Cl | H | Cl | Et | CF₃ | 1 |
| 537. | Cl | H | Cl | Et | CF₃ | 2 |
| 538. | Cl | H | CF₃ | iPr | Cl | 0 |
| 539. | Cl | H | CF₃ | iPr | Cl | 1 |
| 540. | Cl | H | CF₃ | iPr | Cl | 2 |
| 541. | Cl | H | Cl | iPr | CF₃ | 0 |
| 542. | Cl | H | Cl | iPr | CF₃ | 1 |
| 543. | Cl | H | Cl | iPr | CF₃ | 2 |
| 544. | Cl | H | CF₃ | tBu | Cl | 0 |
| 545. | Cl | H | CF₃ | tBu | Cl | 1 |
| 546. | Cl | H | CF₃ | tBu | Cl | 2 |
| 547. | Cl | H | Cl | tBu | CF₃ | 0 |
| 548. | Cl | H | Cl | tBu | CF₃ | 1 |
| 549. | Cl | H | Cl | tBu | CF₃ | 2 |
| 550. | Cl | H | CF₃ | cPen | Cl | 0 |
| 551. | Cl | H | CF₃ | cPen | Cl | 1 |
| 552. | Cl | H | CF₃ | cPen | Cl | 2 |
| 553. | Cl | H | Cl | cPen | CF₃ | 0 |
| 554. | Cl | H | Cl | cPen | CF₃ | 1 |
| 555. | Cl | H | Cl | cPen | CF₃ | 2 |
| 556. | Cl | H | CF₃ | CH₂cPr | Cl | 0 |
| 557. | Cl | H | CF₃ | CH₂cPr | Cl | 1 |
| 558. | Cl | H | CF₃ | CH₂cPr | Cl | 2 |
| 559. | Cl | H | Cl | CH₂cPr | CF₃ | 0 |
| 560. | Cl | H | Cl | CH₂cPr | CF₃ | 1 |
| 561. | Cl | H | Cl | CH₂cPr | CF₃ | 2 |
| 562. | Cl | H | CF₃ | CH₂CH=CH₂ | Cl | 0 |
| 563. | Cl | H | CF₃ | CH₂CH=CH₂ | Cl | 1 |
| 564. | Cl | H | CF₃ | CH₂CH=CH₂ | Cl | 2 |
| 565. | Cl | H | Cl | CH₂CH=CH₂ | CF₃ | 0 |
| 566. | Cl | H | Cl | CH₂CH=CH₂ | CF₃ | 1 |
| 567. | Cl | H | Cl | CH₂CH=CH₂ | CF₃ | 2 |
| 568. | Cl | H | CF₃ | CHF₂ | OMe | 0 |
| 569. | Cl | H | CF₃ | CHF₂ | OMe | 1 |
| 570. | Cl | H | CF₃ | CHF₂ | OMe | 2 |
| 571. | Cl | H | OMe | CHF₂ | CF₃ | 0 |
| 572. | Cl | H | OMe | CHF₂ | CF₃ | 1 |
| 573. | Cl | H | OMe | CHF₂ | CF₃ | 2 |
| 574. | Cl | H | CF₃ | CH₂CF₃ | Cl | 0 |
| 575. | Cl | H | CF₃ | CH₂CF₃ | Cl | 1 |
| 576. | Cl | H | CF₃ | CH₂CF₃ | Cl | 2 |
| 577. | Cl | H | Cl | CH₂CF₃ | CF₃ | 0 |
| 578. | Cl | H | Cl | CH₂CF₃ | CF₃ | 1 |
| 579. | Cl | H | Cl | CH₂CF₃ | CF₃ | 2 |
| 580. | Cl | H | CF₃ | CH₂OMe | Cl | 0 |
| 581. | Cl | H | CF₃ | CH₂OMe | Cl | 1 |
| 582. | Cl | H | CF₃ | CH₂OMe | Cl | 2 |
| 583. | Cl | H | Cl | CH₂OMe | CF₃ | 0 |
| 584. | Cl | H | Cl | CH₂OMe | CF₃ | 1 |
| 585. | Cl | H | Cl | CH₂OMe | CF₃ | 2 |
| 586. | Cl | H | CF₃ | CH₂CN | Cl | 0 |
| 587. | Cl | H | CF₃ | CH₂CN | Cl | 1 |
| 588. | Cl | H | CF₃ | CH₂CN | Cl | 2 |
| 589. | Cl | H | Me | Ph | Me | 0 |
| 590. | Cl | H | Me | Ph | Me | 1 |
| 591. | Cl | H | Me | Ph | Me | 2 |
| 592. | Cl | H | Me | Ph | Cl | 0 |
| 593. | Cl | H | Me | Ph | Cl | 1 |
| 594. | Cl | H | Me | Ph | Cl | 2 |
| 595. | Cl | H | Et | Ph | Cl | 0 |
| 596. | Cl | H | Et | Ph | Cl | 1 |
| 597. | Cl | H | Et | Ph | Cl | 2 |
| 598. | Cl | H | Pr | Ph | Cl | 0 |
| 599. | Cl | H | Pr | Ph | Cl | 1 |
| 600. | Cl | H | Pr | Ph | Cl | 2 |
| 601. | Cl | H | iPr | Ph | Cl | 0 |
| 602. | Cl | H | iPr | Ph | Cl | 1 |
| 603. | Cl | H | iPr | Ph | Cl | 2 |
| 604. | Cl | H | CF₃ | Ph | Cl | 0 |
| 605. | Cl | H | CF₃ | Ph | Cl | 1 |
| 606. | Cl | H | CF₃ | Ph | Cl | 2 |
| 607. | Cl | H | CF₃ | Ph | Me | 0 |
| 608. | Cl | H | CF₃ | Ph | Me | 1 |
| 609. | Cl | H | CF₃ | Ph | Me | 2 |
| 610. | Cl | H | CF₃ | Ph | CF₃ | 0 |
| 611. | Cl | H | CF₃ | Ph | CF₃ | 1 |
| 612. | Cl | H | CF₃ | Ph | CF₃ | 2 |
| 613. | Cl | H | CF₃ | Ph | F | 0 |
| 614. | Cl | H | CF₃ | Ph | F | 1 |
| 615. | Cl | H | CF₃ | Ph | F | 2 |
| 616. | Cl | H | CF₃ | Ph | OMe | 0 |
| 617. | Cl | H | CF₃ | Ph | OMe | 1 |
| 618. | Cl | H | CF₃ | Ph | OMe | 2 |
| 619. | Cl | H | CF₃ | Ph | OEt | 0 |
| 620. | Cl | H | CF₃ | Ph | OEt | 1 |
| 621. | Cl | H | CF₃ | Ph | OEt | 2 |
| 622. | Cl | H | CF₃ | Ph | OCHF₂ | 0 |
| 623. | Cl | H | CF₃ | Ph | OCHF₂ | 1 |
| 624. | Cl | H | CF₃ | Ph | OCHF₂ | 2 |
| 625. | Cl | H | CF₃ | Ph | CN | 0 |
| 626. | Cl | H | CF₃ | Ph | CN | 1 |
| 627. | Cl | H | CF₃ | Ph | CN | 2 |
| 628. | Cl | H | CF₃ | Ph(4-Cl) | Cl | 0 |
| 629. | Cl | H | CF₃ | Ph(4-Cl) | Cl | 1 |
| 630. | Cl | H | CF₃ | Ph(4-Cl) | Cl | 2 |
| 631. | Cl | H | Me | Me | OCH₂CF₃ | 0 |
| 632. | Cl | H | Me | Me | OCH₂CF₃ | 1 |
| 633. | Cl | H | Me | Me | OCH₂CF₃ | 2 |
| 634. | Cl | H | CF₃ | Me | 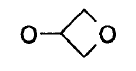 | 0 |
| 635. | Cl | H | CF₃ | Me | 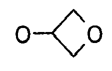 | 1 |
| 636. | Cl | H | CF₃ | Me | 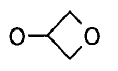 | 2 |
| 637. | Cl | H | CF₃ | Me | H | 0 |
| 638. | Cl | H | CF₃ | Me | H | 1 |
| 639. | Cl | H | CF₃ | Me | H | 2 |
| 640. | Cl | H | CF₃ | Me | OCH₂CH₂OMe | 0 |
| 641. | Cl | H | CF₃ | Me | OCH₂CH₂OMe | 1 |
| 642. | Cl | H | CF₃ | Me | OCH₂CH₂OMe | 2 |
| 643. | Cl | H | CF₃ | Me | SMe | 0 |
| 644. | Cl | H | CF₃ | Me | SMe | 1 |
| 645. | Cl | H | CF₃ | Me | SMe | 2 |
| 646. | Cl | H | CF₃ | Me | OCH₂CH₂CH₂F | 0 |
| 647. | Cl | H | CF₃ | Me | OCH₂CH₂CH₂F | 1 |
| 648. | Cl | H | CF₃ | Me | OCH₂CH₂CH₂F | 2 |
| 649. | Cl | H | CF₃ | Me | OCH(CH₂F)₂ | 0 |
| 650. | Cl | H | CF₃ | Me | OCH(CH₂F)₂ | 1 |
| 651. | Cl | H | CF₃ | Me | OCH(CH₂F)₂ | 2 |
| 652. | Cl | H | CF₃ | Me | OCH₂CF₂CHF₂ | 0 |
| 653. | Cl | H | CF₃ | Me | OCH₂CF₂CHF₂ | 1 |
| 654. | Cl | H | CF₃ | Me | OCH₂CF₂CHF₂ | 2 |
| 655. | Cl | H | CF₃ | Me | OCH₂CF=CH₂ | 0 |
| 656. | Cl | H | CF₃ | Me | OCH₂CF=CH₂ | 1 |
| 657. | Cl | H | CF₃ | Me | OCH₂CF=CH₂ | 2 |
| 658. | Cl | H | CF₃ | Me | OCH(Me)CF₃ | 0 |
| 659. | Cl | H | CF₃ | Me | OCH(Me)CF₃ | 1 |
| 660. | Cl | H | CF₃ | Me | OCH(Me)CF₃ | 2 |
| 661. | Cl | H | CF₃ | Me | OCH(Me)CH₂F | 0 |
| 662. | Cl | H | CF₃ | Me | OCH(Me)CH₂F | 1 |
| 663. | Cl | H | CF₃ | Me | OCH(Me)CH₂F | 2 |
| 664. | Cl | H | OCH₂CF₃ | Me | CF₃ | 0 |
| 665. | Cl | H | OCH₂CF₃ | Me | CF₃ | 1 |
| 666. | Cl | H | OCH₂CF₃ | Me | CF₃ | 2 |
| 667. | Cl | H | OCH₂CF₃ | Me | CHF₂ | 0 |
| 668. | Cl | H | OCH₂CF₃ | Me | CHF₂ | 1 |
| 669. | Cl | H | OCH₂CF₃ | Me | CHF₂ | 2 |
| 670. | Cl | H | CHF₂ | Me | CHF₂ | 0 |
| 671. | Cl | H | CHF₂ | Me | CHF₂ | 1 |
| 672. | Cl | H | CHF₂ | Me | CHF₂ | 2 |
| 673. | Cl | H | CF₃ | Me | CHF₂ | 0 |
| 674. | Cl | H | CF₃ | Me | CHF₂ | 1 |
| 675. | Cl | H | CF₃ | Me | CHF₂ | 2 |
| 676. | Cl | H | Cl | Me | OCHF₂ | 0 |
| 677. | Cl | H | Cl | Me | OCHF₂ | 1 |
| 678. | Cl | H | Cl | Me | OCHF₂ | 2 |
| 679. | Cl | H | Br | Me | OCHF₂ | 0 |
| 680. | Cl | H | Br | Me | OCHF₂ | 1 |
| 681. | Cl | H | Br | Me | OCHF₂ | 2 |
| 682. | Cl | H | Br | Me | CF₃ | 0 |
| 683. | Cl | H | Br | Me | CF₃ | 1 |
| 684. | Cl | H | Br | Me | CF₃ | 2 |
| 685. | Br | H | CF₃ | Ph | Cl | 0 |
| 686. | Br | H | CF₃ | Ph | Cl | 1 |
| 687. | Br | H | CF₃ | Ph | Cl | 2 |
| 688. | Br | H | CF₃ | tBu | Cl | 0 |
| 689. | Br | H | CF₃ | tBu | Cl | 1 |
| 690. | Br | H | CF₃ | tBu | Cl | 2 |
| 691. | Br | H | CF₃ | CHF₂ | Cl | 0 |
| 692. | Br | H | CF₃ | CHF₂ | Cl | 1 |
| 693. | Br | H | CF₃ | CHF₂ | Cl | 2 |
| 694. | Br | H | Cl | CHF₂ | CF₃ | 0 |
| 695. | Br | H | Cl | CHF₂ | CF₃ | 1 |
| 696. | Br | H | Cl | CHF₂ | CF₃ | 2 |
| 697. | Br | H | CF₃ | Me | OMe | 0 |
| 698. | Br | H | CF₃ | Me | OMe | 1 |
| 699. | Br | H | CF₃ | Me | OMe | 2 |
| 700. | Br | H | CF₃ | Me | CN | 0 |
| 701. | Br | H | CF₃ | Me | CN | 1 |
| 702. | Br | H | CF₃ | Me | CN | 2 |
| 703. | Br | H | Cl | Et | Cl | 0 |
| 704. | Br | H | Cl | Et | Cl | 1 |
| 705. | Br | H | Cl | Et | Cl | 2 |
| 706. | Br | H | CHF₂ | Me | Cl | 0 |
| 707. | Br | H | CHF₂ | Me | Cl | 1 |
| 708. | Br | H | CHF₂ | Me | Cl | 2 |
| 709. | Br | H | Me | Me | Me | 0 |
| 710. | Br | H | Me | Me | Me | 1 |
| 711. | Br | H | Me | Me | Me | 2 |
| 712. | Br | H | Me | Me | Cl | 0 |
| 713. | Br | H | Me | Me | Cl | 1 |
| 714. | Br | H | Me | Me | Cl | 2 |
| 715. | Br | H | Cl | Me | Cl | 0 |
| 716. | Br | H | Cl | Me | Cl | 1 |
| 717. | Br | H | Cl | Me | Cl | 2 |
| 718. | Br | H | CF₃ | Me | Cl | 0 |
| 719. | Br | H | CF₃ | Me | Cl | 1 |
| 720. | Br | H | CF₃ | Me | Cl | 2 |
| 721. | Br | H | Cl | Me | CF₃ | 0 |
| 722. | Br | H | Cl | Me | CF₃ | 1 |
| 723. | Br | H | Cl | Me | CF₃ | 2 |
| 724. | Br | H | CF₃ | Me | F | 0 |
| 725. | Br | H | CF₃ | Me | F | 1 |
| 726. | Br | H | CF₃ | Me | F | 2 |
| 727. | Br | H | OMe | Me | CF₃ | 0 |
| 728. | Br | H | OMe | Me | CF₃ | 1 |
| 729. | Br | H | OMe | Me | CF₃ | 2 |
| 730. | Br | H | CF₃ | Me | OEt | 0 |
| 731. | Br | H | CF₃ | Me | OEt | 1 |
| 732. | Br | H | CF₃ | Me | OEt | 2 |
| 733. | Br | H | CF₃ | Me | OCHF₂ | 0 |
| 734. | Br | H | CF₃ | Me | OCHF₂ | 1 |
| 735. | Br | H | CF₃ | Me | OCHF₂ | 2 |
| 736. | Br | H | OCHF₂ | Me | CF₃ | 0 |
| 737. | Br | H | OCHF₂ | Me | CF₃ | 1 |
| 738. | Br | H | OCHF₂ | Me | CF₃ | 2 |
| 739. | Br | H | CF₃ | Me | OCH₂CHF₂ | 0 |
| 740. | Br | H | CF₃ | Me | OCH₂CHF₂ | 1 |
| 741. | Br | H | CF₃ | Me | OCH₂CHF₂ | 2 |
| 742. | Br | H | CF₃ | Me | OCH₂CF₃ | 0 |
| 743. | Br | H | CF₃ | Me | OCH₂CF₃ | 1 |
| 744. | Br | H | CF₃ | Me | OCH₂CF₃ | 2 |
| 745. | Br | H | CF₃ | Me | OCH₂CN | 0 |
| 746. | Br | H | CF₃ | Me | OCH₂CN | 1 |
| 747. | Br | H | CF₃ | Me | OCH₂CN | 2 |
| 748. | Br | H | CF₃ | Me | SO₂Me | 0 |
| 749. | Br | H | CF₃ | Me | SO₂Me | 1 |
| 750. | Br | H | CF₃ | Me | SO₂Me | 2 |
| 751. | Br | H | CF₃ | Me | SEt | 0 |
| 752. | Br | H | CF₃ | Me | SEt | 1 |
| 753. | Br | H | CF₃ | Me | SEt | 2 |
| 754. | Br | H | CF₃ | Me | Me | 0 |
| 755. | Br | H | CF₃ | Me | Me | 1 |
| 756. | Br | H | CF₃ | Me | Me | 2 |
| 757. | Br | H | CF₃ | Me | Et | 0 |
| 758. | Br | H | CF₃ | Me | Et | 1 |
| 759. | Br | H | CF₃ | Me | Et | 2 |
| 760. | Br | H | CF₃ | Et | Cl | 0 |
| 761. | Br | H | CF₃ | Et | Cl | 1 |
| 762. | Br | H | CF₃ | Et | Cl | 2 |
| 763. | Br | H | Cl | Et | CF₃ | 0 |
| 764. | Br | H | Cl | Et | CF₃ | 1 |
| 765. | Br | H | Cl | Et | CF₃ | 2 |
| 766. | Br | H | CF₃ | iPr | Cl | 0 |
| 767. | Br | H | CF₃ | iPr | Cl | 1 |
| 768. | Br | H | CF₃ | iPr | Cl | 2 |
| 769. | Br | H | Cl | iPr | CF₃ | 0 |
| 770. | Br | H | Cl | iPr | CF₃ | 1 |
| 771. | Br | H | Cl | iPr | CF₃ | 2 |
| 772. | Br | H | CF₃ | tBu | Cl | 0 |
| 773. | Br | H | CF₃ | tBu | Cl | 1 |
| 774. | Br | H | CF₃ | tBu | Cl | 2 |
| 775. | Br | H | Cl | tBu | CF₃ | 0 |
| 776. | Br | H | Cl | tBu | CF₃ | 1 |
| 777. | Br | H | Cl | tBu | CF₃ | 2 |
| 778. | Br | H | CF₃ | cPen | Cl | 0 |
| 779. | Br | H | CF₃ | cPen | Cl | 1 |
| 780. | Br | H | CF₃ | cPen | Cl | 2 |
| 781. | Br | H | Cl | cPen | CF₃ | 0 |
| 782. | Br | H | Cl | cPen | CF₃ | 1 |
| 783. | Br | H | Cl | cPen | CF₃ | 2 |
| 784. | Br | H | CF₃ | CH₂cPr | Cl | 0 |
| 785. | Br | H | CF₃ | CH₂cPr | Cl | 1 |
| 786. | Br | H | CF₃ | CH₂cPr | Cl | 2 |
| 787. | Br | H | Cl | CH₂cPr | CF₃ | 0 |
| 788. | Br | H | Cl | CH₂cPr | CF₃ | 1 |
| 789. | Br | H | Cl | CH₂cPr | CF₃ | 2 |
| 790. | Br | H | CF₃ | CH₂CH=CH₂ | Cl | 0 |
| 791. | Br | H | CF₃ | CH₂CH=CH₂ | Cl | 1 |
| 792. | Br | H | CF₃ | CH₂CH=CH₂ | Cl | 2 |
| 793. | Br | H | Cl | CH₂CH=CH₂ | CF₃ | 0 |
| 794. | Br | H | Cl | CH₂CH=CH₂ | CF₃ | 1 |
| 795. | Br | H | Cl | CH₂CH=CH₂ | CF₃ | 2 |
| 796. | Br | H | CF₃ | CHF₂ | OMe | 0 |
| 797. | Br | H | CF₃ | CHF₂ | OMe | 1 |
| 798. | Br | H | CF₃ | CHF₂ | OMe | 2 |
| 799. | Br | H | OMe | CHF₂ | CF₃ | 0 |
| 800. | Br | H | OMe | CHF₂ | CF₃ | 1 |
| 801. | Br | H | OMe | CHF₂ | CF₃ | 2 |
| 802. | Br | H | CF₃ | CH₂CF₃ | Cl | 0 |
| 803. | Br | H | CF₃ | CH₂CF₃ | Cl | 1 |
| 804. | Br | H | CF₃ | CH₂CF₃ | Cl | 2 |
| 805. | Br | H | Cl | CH₂CF₃ | CF₃ | 0 |
| 806. | Br | H | Cl | CH₂CF₃ | CF₃ | 1 |
| 807. | Br | H | Cl | CH₂CF₃ | CF₃ | 2 |
| 808. | Br | H | CF₃ | CH₂OMe | Cl | 0 |
| 809. | Br | H | CF₃ | CH₂OMe | Cl | 1 |
| 810. | Br | H | CF₃ | CH₂OMe | Cl | 2 |
| 811. | Br | H | Cl | CH₂OMe | CF₃ | 0 |
| 812. | Br | H | Cl | CH₂OMe | CF₃ | 1 |
| 813. | Br | H | Cl | CH₂OMe | CF₃ | 2 |
| 814. | Br | H | CF₃ | CH₂CN | Cl | 0 |
| 815. | Br | H | CF₃ | CH₂CN | Cl | 1 |
| 816. | Br | H | CF₃ | CH₂CN | Cl | 2 |
| 817. | Br | H | Me | Ph | Me | 0 |
| 818. | Br | H | Me | Ph | Me | 1 |
| 819. | Br | H | Me | Ph | Me | 2 |
| 820. | Br | H | Me | Ph | Cl | 0 |
| 821. | Br | H | Me | Ph | Cl | 1 |
| 822. | Br | H | Me | Ph | Cl | 2 |
| 823. | Br | H | Et | Ph | Cl | 0 |
| 824. | Br | H | Et | Ph | Cl | 1 |
| 825. | Br | H | Et | Ph | Cl | 2 |
| 826. | Br | H | Pr | Ph | Cl | 0 |
| 827. | Br | H | Pr | Ph | Cl | 1 |
| 828. | Br | H | Pr | Ph | Cl | 2 |
| 829. | Br | H | iPr | Ph | Cl | 0 |
| 830. | Br | H | iPr | Ph | Cl | 1 |
| 831. | Br | H | iPr | Ph | Cl | 2 |
| 832. | Br | H | CF₃ | Ph | Cl | 0 |
| 833. | Br | H | CF₃ | Ph | Cl | 1 |
| 834. | Br | H | CF₃ | Ph | Cl | 2 |
| 835. | Br | H | CF₃ | Ph | Me | 0 |
| 836. | Br | H | CF₃ | Ph | Me | 1 |
| 837. | Br | H | CF₃ | Ph | Me | 2 |
| 838. | Br | H | CF₃ | Ph | CF₃ | 0 |
| 839. | Br | H | CF₃ | Ph | CF₃ | 1 |
| 840. | Br | H | CF₃ | Ph | CF₃ | 2 |
| 841. | Br | H | CF₃ | Ph | F | 0 |
| 842. | Br | H | CF₃ | Ph | F | 1 |
| 843. | Br | H | CF₃ | Ph | F | 2 |
| 844. | Br | H | CF₃ | Ph | OMe | 0 |
| 845. | Br | H | CF₃ | Ph | OMe | 1 |
| 846. | Br | H | CF₃ | Ph | OMe | 2 |
| 847. | Br | H | CF₃ | Ph | OEt | 0 |
| 848. | Br | H | CF₃ | Ph | OEt | 1 |
| 849. | Br | H | CF₃ | Ph | OEt | 2 |
| 850. | Br | H | CF₃ | Ph | OCHF₂ | 0 |
| 851. | Br | H | CF₃ | Ph | OCHF₂ | 1 |
| 852. | Br | H | CF₃ | Ph | OCHF₂ | 2 |
| 853. | Br | H | CF₃ | Ph | CN | 0 |
| 854. | Br | H | CF₃ | Ph | CN | 1 |
| 855. | Br | H | CF₃ | Ph | CN | 2 |
| 856. | Br | H | CF₃ | Ph(4-Cl) | Cl | 0 |
| 857. | Br | H | CF₃ | Ph(4-Cl) | Cl | 1 |
| 858. | Br | H | CF₃ | Ph(4-Cl) | Cl | 2 |
| 859. | Br | H | Me | Me | OCH₂CF₃ | 0 |
| 860. | Br | H | Me | Me | OCH₂CF₃ | 1 |
| 861. | Br | H | Me | Me | OCH₂CF₃ | 2 |
| 862. | Br | H | CF₃ | Me | 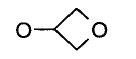 | 0 |
| 863. | Br | H | CF₃ | Me | 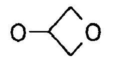 | 1 |
| 864. | Br | H | CF₃ | Me | 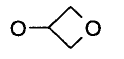 | 2 |
| 865. | Br | H | CF₃ | Me | H | 0 |
| 866. | Br | H | CF₃ | Me | H | 1 |
| 867. | Br | H | CF₃ | Me | H | 2 |
| 868. | Br | H | CF₃ | Me | OCH₂CH₂OMe | 0 |
| 869. | Br | H | CF₃ | Me | OCH₂CH₂OMe | 1 |
| 870. | Br | H | CF₃ | Me | OCH₂CH₂OMe | 2 |
| 871. | Br | H | CF₃ | Me | SMe | 0 |
| 872. | Br | H | CF₃ | Me | SMe | 1 |
| 873. | Br | H | CF₃ | Me | SMe | 2 |
| 874. | Br | H | CF₃ | Me | OCH₂CH₂CH₂F | 0 |
| 875. | Br | H | CF₃ | Me | OCH₂CH₂CH₂F | 1 |
| 876. | Br | H | CF₃ | Me | OCH₂CH₂CH₂F | 2 |
| 877. | Br | H | CF₃ | Me | OCH(CH₂F)₂ | 0 |
| 878. | Br | H | CF₃ | Me | OCH(CH₂F)₂ | 1 |
| 879. | Br | H | CF₃ | Me | OCH(CH₂F)₂ | 2 |
| 880. | Br | H | CF₃ | Me | OCH₂CF₂CHF₂ | 0 |
| 881. | Br | H | CF₃ | Me | OCH₂CF₂CHF₂ | 1 |
| 882. | Br | H | CF₃ | Me | OCH₂CF₂CHF₂ | 2 |
| 883. | Br | H | CF₃ | Me | OCH₂CF=CH₂ | 0 |
| 884. | Br | H | CF₃ | Me | OCH₂CF=CH₂ | 1 |
| 885. | Br | H | CF₃ | Me | OCH₂CF=CH₂ | 2 |
| 886. | Br | H | CF₃ | Me | OCH(Me)CF₃ | 0 |
| 887. | Br | H | CF₃ | Me | OCH(Me)CF₃ | 1 |
| 888. | Br | H | CF₃ | Me | OCH(Me)CF₃ | 2 |
| 889. | Br | H | CF₃ | Me | OCH(Me)CH₂F | 0 |
| 890. | Br | H | CF₃ | Me | OCH(Me)CH₂F | 1 |
| 891. | Br | H | CF₃ | Me | OCH(Me)CH₂F | 2 |
| 892. | Br | H | OCH₂CF₃ | Me | CF₃ | 0 |
| 893. | Br | H | OCH₂CF₃ | Me | CF₃ | 1 |
| 894. | Br | H | OCH₂CF₃ | Me | CF₃ | 2 |
| 895. | Br | H | OCH₂CF₃ | Me | CHF₂ | 0 |
| 896. | Br | H | OCH₂CF₃ | Me | CHF₂ | 1 |
| 897. | Br | H | OCH₂CF₃ | Me | CHF₂ | 2 |
| 898. | Br | H | CHF₂ | Me | CHF₂ | 0 |
| 899. | Br | H | CHF₂ | Me | CHF₂ | 1 |
| 900. | Br | H | CHF₂ | Me | CHF₂ | 2 |
| 901. | Br | H | CF₃ | Me | CHF₂ | 0 |
| 902. | Br | H | CF₃ | Me | CHF₂ | 1 |
| 903. | Br | H | CF₃ | Me | CHF₂ | 2 |
| 904. | Br | H | Cl | Me | OCHF₂ | 0 |
| 905. | Br | H | Cl | Me | OCHF₂ | 1 |
| 906. | Br | H | Cl | Me | OCHF₂ | 2 |
| 907. | Br | H | Br | Me | OCHF₂ | 0 |
| 908. | Br | H | Br | Me | OCHF₂ | 1 |
| 909. | Br | H | Br | Me | OCHF₂ | 2 |
| 910. | Br | H | Br | Me | CF₃ | 0 |
| 911. | Br | H | Br | Me | CF₃ | 1 |
| 912. | Br | H | Br | Me | CF₃ | 2 |
| 913. | I | H | CF₃ | Ph | Cl | 0 |
| 914. | I | H | CF₃ | Ph | Cl | 1 |
| 915. | I | H | CF₃ | Ph | Cl | 2 |
| 916. | I | H | CF₃ | tBu | Cl | 0 |
| 917. | I | H | CF₃ | tBu | Cl | 1 |
| 918. | I | H | CF₃ | tBu | Cl | 2 |
| 919. | I | H | CF₃ | CHF₂ | Cl | 0 |
| 920. | I | H | CF₃ | CHF₂ | Cl | 1 |
| 921. | I | H | CF₃ | CHF₂ | Cl | 2 |
| 922. | I | H | Cl | CHF₂ | CF₃ | 0 |
| 923. | I | H | Cl | CHF₂ | CF₃ | 1 |
| 924. | I | H | Cl | CHF₂ | CF₃ | 2 |
| 925. | I | H | CF₃ | Me | OMe | 0 |
| 926. | I | H | CF₃ | Me | OMe | 1 |
| 927. | I | H | CF₃ | Me | OMe | 2 |
| 928. | I | H | CF₃ | Me | CN | 0 |
| 929. | I | H | CF₃ | Me | CN | 1 |
| 930. | I | H | CF₃ | Me | CN | 2 |
| 931. | I | H | Cl | Et | Cl | 0 |
| 932. | I | H | Cl | Et | Cl | 1 |
| 933. | I | H | Cl | Et | Cl | 2 |
| 934. | I | H | CHF₂ | Me | Cl | 0 |
| 935. | I | H | CHF₂ | Me | Cl | 1 |
| 936. | I | H | CHF₂ | Me | Cl | 2 |
| 937. | I | H | Me | Me | Me | 0 |
| 938. | I | H | Me | Me | Me | 1 |
| 939. | I | H | Me | Me | Me | 2 |
| 940. | I | H | Me | Me | Cl | 0 |
| 941. | I | H | Me | Me | Cl | 1 |
| 942. | I | H | Me | Me | Cl | 2 |
| 943. | I | H | Cl | Me | Cl | 0 |
| 944. | I | H | Cl | Me | Cl | 1 |
| 945. | I | H | Cl | Me | Cl | 2 |
| 946. | I | H | CF₃ | Me | Cl | 0 |
| 947. | I | H | CF₃ | Me | Cl | 1 |
| 948. | I | H | CF₃ | Me | Cl | 2 |
| 949. | I | H | Cl | Me | CF₃ | 0 |
| 950. | I | H | Cl | Me | CF₃ | 1 |
| 951. | I | H | Cl | Me | CF₃ | 2 |
| 952. | I | H | CF₃ | Me | F | 0 |
| 953. | I | H | CF₃ | Me | F | 1 |
| 954. | I | H | CF₃ | Me | F | 2 |
| 955. | I | H | OMe | Me | CF₃ | 0 |
| 956. | I | H | OMe | Me | CF₃ | 1 |
| 957. | I | H | OMe | Me | CF₃ | 2 |
| 958. | I | H | CF₃ | Me | OEt | 0 |
| 959. | I | H | CF₃ | Me | OEt | 1 |
| 960. | I | H | CF₃ | Me | OEt | 2 |
| 961. | I | H | CF₃ | Me | OCHF₂ | 0 |
| 962. | I | H | CF₃ | Me | OCHF₂ | 1 |
| 963. | I | H | CF₃ | Me | OCHF₂ | 2 |
| 964. | I | H | OCHF₂ | Me | CF₃ | 0 |
| 965. | I | H | OCHF₂ | Me | CF₃ | 1 |
| 966. | I | H | OCHF₂ | Me | CF₃ | 2 |
| 967. | I | H | CF₃ | Me | OCH₂CHF₂ | 0 |
| 968. | I | H | CF₃ | Me | OCH₂CHF₂ | 1 |
| 969. | I | H | CF₃ | Me | OCH₂CHF₂ | 2 |
| 970. | I | H | CF₃ | Me | OCH₂CF₃ | 0 |
| 971. | I | H | CF₃ | Me | OCH₂CF₃ | 1 |
| 972. | I | H | CF₃ | Me | OCH₂CF₃ | 2 |
| 973. | I | H | CF₃ | Me | OCH₂CN | 0 |
| 974. | I | H | CF₃ | Me | OCH₂CN | 1 |
| 975. | I | H | CF₃ | Me | OCH₂CN | 2 |
| 976. | I | H | CF₃ | Me | SO₂Me | 0 |
| 977. | I | H | CF₃ | Me | SO₂Me | 1 |
| 978. | I | H | CF₃ | Me | SO₂Me | 2 |
| 979. | I | H | CF₃ | Me | SEt | 0 |
| 980. | I | H | CF₃ | Me | SEt | 1 |
| 981. | I | H | CF₃ | Me | SEt | 2 |
| 982. | I | H | CF₃ | Me | Me | 0 |
| 983. | I | H | CF₃ | Me | Me | 1 |
| 984. | I | H | CF₃ | Me | Me | 2 |
| 985. | I | H | CF₃ | Me | Et | 0 |
| 986. | I | H | CF₃ | Me | Et | 1 |
| 987. | I | H | CF₃ | Me | Et | 2 |
| 988. | I | H | CF₃ | Et | Cl | 0 |
| 989. | I | H | CF₃ | Et | Cl | 1 |
| 990. | I | H | CF₃ | Et | Cl | 2 |
| 991. | I | H | Cl | Et | CF₃ | 0 |
| 992. | I | H | Cl | Et | CF₃ | 1 |
| 993. | I | H | Cl | Et | CF₃ | 2 |
| 994. | I | H | CF₃ | iPr | Cl | 0 |
| 995. | I | H | CF₃ | iPr | Cl | 1 |
| 996. | I | H | CF₃ | iPr | Cl | 2 |
| 997. | I | H | Cl | iPr | CF₃ | 0 |
| 998. | I | H | Cl | iPr | CF₃ | 1 |
| 999. | I | H | Cl | iPr | CF₃ | 2 |
| 1000. | I | H | CF₃ | tBu | Cl | 0 |
| 1001. | I | H | CF₃ | tBu | Cl | 1 |
| 1002. | I | H | CF₃ | tBu | Cl | 2 |
| 1003. | I | H | Cl | tBu | CF₃ | 0 |
| 1004. | I | H | Cl | tBu | CF₃ | 1 |
| 1005. | I | H | Cl | tBu | CF₃ | 2 |
| 1006. | I | H | CF₃ | cPen | Cl | 0 |
| 1007. | I | H | CF₃ | cPen | Cl | 1 |
| 1008. | I | H | CF₃ | cPen | Cl | 2 |
| 1009. | I | H | Cl | cPen | CF₃ | 0 |
| 1010. | I | H | Cl | cPen | CF₃ | 1 |
| 1011. | I | H | Cl | cPen | CF₃ | 2 |
| 1012. | I | H | CF₃ | CH₂cPr | Cl | 0 |
| 1013. | I | H | CF₃ | CH₂cPr | Cl | 1 |
| 1014. | I | H | CF₃ | CH₂cPr | Cl | 2 |
| 1015. | I | H | Cl | CH₂cPr | CF₃ | 0 |
| 1016. | I | H | Cl | CH₂cPr | CF₃ | 1 |
| 1017. | I | H | Cl | CH₂cPr | CF₃ | 2 |
| 1018. | I | H | CF₃ | CH₂CH=CH₂ | Cl | 0 |
| 1019. | I | H | CF₃ | CH₂CH=CH₂ | Cl | 1 |
| 1020. | I | H | CF₃ | CH₂CH=CH₂ | Cl | 2 |
| 1021. | I | H | Cl | CH₂CH=CH₂ | CF₃ | 0 |
| 1022. | I | H | Cl | CH₂CH=CH₂ | CF₃ | 1 |
| 1023. | I | H | Cl | CH₂CH=CH₂ | CF₃ | 2 |
| 1024. | I | H | CF₃ | CHF₂ | OMe | 0 |
| 1025. | I | H | CF₃ | CHF₂ | OMe | 1 |
| 1026. | I | H | CF₃ | CHF₂ | OMe | 2 |
| 1027. | I | H | OMe | CHF₂ | CF₃ | 0 |
| 1028. | I | H | OMe | CHF₂ | CF₃ | 1 |
| 1029. | I | H | OMe | CHF₂ | CF₃ | 2 |
| 1030. | I | H | CF₃ | CH₂CF₃ | Cl | 0 |
| 1031. | I | H | CF₃ | CH₂CF₃ | Cl | 1 |
| 1032. | I | H | CF₃ | CH₂CF₃ | Cl | 2 |
| 1033. | I | H | Cl | CH₂CF₃ | CF₃ | 0 |
| 1034. | I | H | Cl | CH₂CF₃ | CF₃ | 1 |
| 1035. | I | H | Cl | CH₂CF₃ | CF₃ | 2 |
| 1036. | I | H | CF₃ | CH₂OMe | Cl | 0 |
| 1037. | I | H | CF₃ | CH₂OMe | Cl | 1 |
| 1038. | I | H | CF₃ | CH₂OMe | Cl | 2 |
| 1039. | I | H | Cl | CH₂OMe | CF₃ | 0 |
| 1040. | I | H | Cl | CH₂OMe | CF₃ | 1 |
| 1041. | I | H | Cl | CH₂OMe | CF₃ | 2 |
| 1042. | I | H | CF₃ | CH₂CN | Cl | 0 |
| 1043. | I | H | CF₃ | CH₂CN | Cl | 1 |
| 1044. | I | H | CF₃ | CH₂CN | Cl | 2 |
| 1045. | I | H | Me | Ph | Me | 0 |
| 1046. | I | H | Me | Ph | Me | 1 |
| 1047. | I | H | Me | Ph | Me | 2 |
| 1048. | I | H | Me | Ph | Cl | 0 |
| 1049. | I | H | Me | Ph | Cl | 1 |
| 1050. | I | H | Me | Ph | Cl | 2 |
| 1051. | I | H | Et | Ph | Cl | 0 |
| 1052. | I | H | Et | Ph | Cl | 1 |
| 1053. | I | H | Et | Ph | Cl | 2 |
| 1054. | I | H | Pr | Ph | Cl | 0 |
| 1055. | I | H | Pr | Ph | Cl | 1 |
| 1056. | I | H | Pr | Ph | Cl | 2 |
| 1057. | I | H | iPr | Ph | Cl | 0 |
| 1058. | I | H | iPr | Ph | Cl | 1 |
| 1059. | I | H | iPr | Ph | Cl | 2 |
| 1060. | I | H | CF₃ | Ph | Cl | 0 |
| 1061. | I | H | CF₃ | Ph | Cl | 1 |
| 1062. | I | H | CF₃ | Ph | Cl | 2 |
| 1063. | I | H | CF₃ | Ph | Me | 0 |
| 1064. | I | H | CF₃ | Ph | Me | 1 |
| 1065. | I | H | CF₃ | Ph | Me | 2 |
| 1066. | I | H | CF₃ | Ph | CF₃ | 0 |
| 1067. | I | H | CF₃ | Ph | CF₃ | 1 |
| 1068. | I | H | CF₃ | Ph | CF₃ | 2 |
| 1069. | I | H | CF₃ | Ph | F | 0 |
| 1070. | I | H | CF₃ | Ph | F | 1 |
| 1071. | I | H | CF₃ | Ph | F | 2 |
| 1072. | I | H | CF₃ | Ph | OMe | 0 |
| 1073. | I | H | CF₃ | Ph | OMe | 1 |
| 1074. | I | H | CF₃ | Ph | OMe | 2 |
| 1075. | I | H | CF₃ | Ph | OEt | 0 |
| 1076. | I | H | CF₃ | Ph | OEt | 1 |
| 1077. | I | H | CF₃ | Ph | OEt | 2 |
| 1078. | I | H | CF₃ | Ph | OCHF₂ | 0 |
| 1079. | I | H | CF₃ | Ph | OCHF₂ | 1 |
| 1080. | I | H | CF₃ | Ph | OCHF₂ | 2 |
| 1081. | I | H | CF₃ | Ph | CN | 0 |
| 1082. | I | H | CF₃ | Ph | CN | 1 |
| 1083. | I | H | CF₃ | Ph | CN | 2 |
| 1084. | I | H | CF₃ | Ph(4-Cl) | Cl | 0 |
| 1085. | I | H | CF₃ | Ph(4-Cl) | Cl | 1 |
| 1086. | I | H | CF₃ | Ph(4-Cl) | Cl | 2 |
| 1087. | I | H | Me | Me | OCH₂CF₃ | 0 |
| 1088. | I | H | Me | Me | OCH₂CF₃ | 1 |
| 1089. | I | H | Me | Me | OCH₂CF₃ | 2 |
| 1090. | I | H | CF₃ | Me | 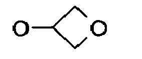 | 0 |
| 1091. | I | H | CF₃ | Me | 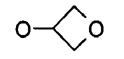 | 1 |
| 1092. | I | H | CF₃ | Me | 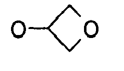 | 2 |
| 1093. | I | H | CF₃ | Me | H | 0 |
| 1094. | I | H | CF₃ | Me | H | 1 |
| 1095. | I | H | CF₃ | Me | H | 2 |
| 1096. | I | H | CF₃ | Me | OCH₂CH₂OMe | 0 |
| 1097. | I | H | CF₃ | Me | OCH₂CH₂OMe | 1 |
| 1098. | I | H | CF₃ | Me | OCH₂CH₂OMe | 2 |
| 1099. | I | H | CF₃ | Me | SMe | 0 |
| 1100. | I | H | CF₃ | Me | SMe | 1 |
| 1101. | I | H | CF₃ | Me | SMe | 2 |
| 1102. | I | H | CF₃ | Me | OCH₂CH₂CH₂F | 0 |
| 1103. | I | H | CF₃ | Me | OCH₂CH₂CH₂F | 1 |
| 1104. | I | H | CF₃ | Me | OCH₂CH₂CH₂F | 2 |
| 1105. | I | H | CF₃ | Me | OCH(CH₂F)₂ | 0 |
| 1106. | I | H | CF₃ | Me | OCH(CH₂F)₂ | 1 |
| 1107. | I | H | CF₃ | Me | OCH(CH₂F)₂ | 2 |
| 1108. | I | H | CF₃ | Me | OCH₂CF₂CHF₂ | 0 |
| 1109. | I | H | CF₃ | Me | OCH₂CF₂CHF₂ | 1 |
| 1110. | I | H | CF₃ | Me | OCH₂CF₂CHF₂ | 2 |
| 1111. | I | H | CF₃ | Me | OCH₂CF=CH₂ | 0 |
| 1112. | I | H | CF₃ | Me | OCH₂CF=CH₂ | 1 |
| 1113. | I | H | CF₃ | Me | OCH₂CF=CH₂ | 2 |
| 1114. | I | H | CF₃ | Me | OCH(Me)CF₃ | 0 |
| 1115. | I | H | CF₃ | Me | OCH(Me)CF₃ | 1 |
| 1116. | I | H | CF₃ | Me | OCH(Me)CF₃ | 2 |
| 1117. | I | H | CF₃ | Me | OCH(Me)CH₂F | 0 |
| 1118. | I | H | CF₃ | Me | OCH(Me)CH₂F | 1 |
| 1119. | I | H | CF₃ | Me | OCH(Me)CH₂F | 2 |
| 1120. | I | H | OCH₂CF₃ | Me | CF₃ | 0 |
| 1121. | I | H | OCH₂CF₃ | Me | CF₃ | 1 |
| 1122. | I | H | OCH₂CF₃ | Me | CF₃ | 2 |
| 1123. | I | H | OCH₂CF₃ | Me | CHF₂ | 0 |
| 1124. | I | H | OCH₂CF₃ | Me | CHF₂ | 1 |
| 1125. | I | H | OCH₂CF₃ | Me | CHF₂ | 2 |
| 1126. | I | H | CHF₂ | Me | CHF₂ | 0 |
| 1127. | I | H | CHF₂ | Me | CHF₂ | 1 |
| 1128. | I | H | CHF₂ | Me | CHF₂ | 2 |
| 1129. | I | H | CF₃ | Me | CHF₂ | 0 |
| 1130. | I | H | CF₃ | Me | CHF₂ | 1 |
| 1131. | I | H | CF₃ | Me | CHF₂ | 2 |
| 1132. | I | H | Cl | Me | OCHF₂ | 0 |
| 1133. | I | H | Cl | Me | OCHF₂ | 1 |
| 1134. | I | H | Cl | Me | OCHF₂ | 2 |
| 1135. | I | H | Br | Me | OCHF₂ | 0 |
| 1136. | I | H | Br | Me | OCHF₂ | 1 |
| 1137. | I | H | Br | Me | OCHF₂ | 2 |
| 1138. | I | H | Br | Me | CF₃ | 0 |
| 1139. | I | H | Br | Me | CF₃ | 1 |
| 1140. | I | H | Br | Me | CF₃ | 2 |
| 1141. | H | F | CF₃ | Ph | Cl | 0 |
| 1142. | H | F | CF₃ | Ph | Cl | 1 |
| 1143. | H | F | CF₃ | Ph | Cl | 2 |
| 1144. | H | F | CF₃ | tBu | Cl | 0 |
| 1145. | H | F | CF₃ | tBu | Cl | 1 |
| 1146. | H | F | CF₃ | tBu | Cl | 2 |
| 1147. | H | F | CF₃ | CHF₂ | Cl | 0 |
| 1148. | H | F | CF₃ | CHF₂ | Cl | 1 |
| 1149. | H | F | CF₃ | CHF₂ | Cl | 2 |
| 1150. | H | F | Cl | CHF₂ | CF₃ | 0 |
| 1151. | H | F | Cl | CHF₂ | CF₃ | 1 |
| 1152. | H | F | Cl | CHF₂ | CF₃ | 2 |
| 1153. | H | F | CF₃ | Me | OMe | 0 |
| 1154. | H | F | CF₃ | Me | OMe | 1 |
| 1155. | H | F | CF₃ | Me | OMe | 2 |
| 1156. | H | F | CF₃ | Me | CN | 0 |
| 1157. | H | F | CF₃ | Me | CN | 1 |
| 1158. | H | F | CF₃ | Me | CN | 2 |
| 1159. | H | F | Cl | Et | Cl | 0 |
| 1160. | H | F | Cl | Et | Cl | 1 |
| 1161. | H | F | Cl | Et | Cl | 2 |
| 1162. | H | F | CHF₂ | Me | Cl | 0 |
| 1163. | H | F | CHF₂ | Me | Cl | 1 |
| 1164. | H | F | CHF₂ | Me | Cl | 2 |
| 1165. | H | F | Me | Me | Me | 0 |
| 1166. | H | F | Me | Me | Me | 1 |
| 1167. | H | F | Me | Me | Me | 2 |
| 1168. | H | F | Me | Me | Cl | 0 |
| 1169. | H | F | Me | Me | Cl | 1 |
| 1170. | H | F | Me | Me | Cl | 2 |
| 1171. | H | F | Cl | Me | Cl | 0 |
| 1172. | H | F | Cl | Me | Cl | 1 |
| 1173. | H | F | Cl | Me | Cl | 2 |
| 1174. | H | F | CF₃ | Me | Cl | 0 |
| 1175. | H | F | CF₃ | Me | Cl | 1 |
| 1176. | H | F | CF₃ | Me | Cl | 2 |
| 1177. | H | F | Cl | Me | CF₃ | 0 |
| 1178. | H | F | Cl | Me | CF₃ | 1 |
| 1179. | H | F | Cl | Me | CF₃ | 2 |
| 1180. | H | F | CF₃ | Me | F | 0 |
| 1181. | H | F | CF₃ | Me | F | 1 |
| 1182. | H | F | CF₃ | Me | F | 2 |
| 1183. | H | F | OMe | Me | CF₃ | 0 |
| 1184. | H | F | OMe | Me | CF₃ | 1 |
| 1185. | H | F | OMe | Me | CF₃ | 2 |
| 1186. | H | F | CF₃ | Me | OEt | 0 |
| 1187. | H | F | CF₃ | Me | OEt | 1 |
| 1188. | H | F | CF₃ | Me | OEt | 2 |
| 1189. | H | F | CF₃ | Me | OCHF₂ | 0 |
| 1190. | H | F | CF₃ | Me | OCHF₂ | 1 |
| 1191. | H | F | CF₃ | Me | OCHF₂ | 2 |
| 1192. | H | F | OCHF₂ | Me | CF₃ | 0 |
| 1193. | H | F | OCHF₂ | Me | CF₃ | 1 |
| 1194. | H | F | OCHF₂ | Me | CF₃ | 2 |
| 1195. | H | F | CF₃ | Me | OCH₂CHF₂ | 0 |
| 1196. | H | F | CF₃ | Me | OCH₂CHF₂ | 1 |
| 1197. | H | F | CF₃ | Me | OCH₂CHF₂ | 2 |
| 1198. | H | F | CF₃ | Me | OCH₂CF₃ | 0 |
| 1199. | H | F | CF₃ | Me | OCH₂CF₃ | 1 |
| 1200. | H | F | CF₃ | Me | OCH₂CF₃ | 2 |
| 1201. | H | F | CF₃ | Me | OCH₂CN | 0 |
| 1202. | H | F | CF₃ | Me | OCH₂CN | 1 |
| 1203. | H | F | CF₃ | Me | OCH₂CN | 2 |
| 1204. | H | F | CF₃ | Me | SO₂Me | 0 |
| 1205. | H | F | CF₃ | Me | SO₂Me | 1 |
| 1206. | H | F | CF₃ | Me | SO₂Me | 2 |
| 1207. | H | F | CF₃ | Me | SEt | 0 |
| 1208. | H | F | CF₃ | Me | SEt | 1 |
| 1209. | H | F | CF₃ | Me | SEt | 2 |
| 1210. | H | F | CF₃ | Me | Me | 0 |
| 1211. | H | F | CF₃ | Me | Me | 1 |
| 1212. | H | F | CF₃ | Me | Me | 2 |
| 1213. | H | F | CF₃ | Me | Et | 0 |
| 1214. | H | F | CF₃ | Me | Et | 1 |
| 1215. | H | F | CF₃ | Me | Et | 2 |
| 1216. | H | F | CF₃ | Et | Cl | 0 |
| 1217. | H | F | CF₃ | Et | Cl | 1 |
| 1218. | H | F | CF₃ | Et | Cl | 2 |
| 1219. | H | F | Cl | Et | CF₃ | 0 |
| 1220. | H | F | Cl | Et | CF₃ | 1 |
| 1221. | H | F | Cl | Et | CF₃ | 2 |
| 1222. | H | F | CF₃ | iPr | Cl | 0 |
| 1223. | H | F | CF₃ | iPr | Cl | 1 |
| 1224. | H | F | CF₃ | iPr | Cl | 2 |
| 1225. | H | F | Cl | iPr | CF₃ | 0 |
| 1226. | H | F | Cl | iPr | CF₃ | 1 |
| 1227. | H | F | Cl | iPr | CF₃ | 2 |
| 1228. | H | F | CF₃ | tBu | Cl | 0 |
| 1229. | H | F | CF₃ | tBu | Cl | 1 |
| 1230. | H | F | CF₃ | tBu | Cl | 2 |
| 1231. | H | F | Cl | tBu | CF₃ | 0 |
| 1232. | H | F | Cl | tBu | CF₃ | 1 |
| 1233. | H | F | Cl | tBu | CF₃ | 2 |
| 1234. | H | F | CF₃ | cPen | Cl | 0 |
| 1235. | H | F | CF₃ | cPen | Cl | 1 |
| 1236. | H | F | CF₃ | cPen | Cl | 2 |
| 1237. | H | F | Cl | cPen | CF₃ | 0 |
| 1238. | H | F | Cl | cPen | CF₃ | 1 |
| 1239. | H | F | Cl | cPen | CF₃ | 2 |
| 1240. | H | F | CF₃ | CH₂cPr | Cl | 0 |
| 1241. | H | F | CF₃ | CH₂cPr | Cl | 1 |
| 1242. | H | F | CF₃ | CH₂cPr | Cl | 2 |
| 1243. | H | F | Cl | CH₂cPr | CF₃ | 0 |
| 1244. | H | F | Cl | CH₂cPr | CF₃ | 1 |
| 1245. | H | F | Cl | CH₂cPr | CF₃ | 2 |
| 1246. | H | F | CF₃ | CH₂CH=CH₂ | Cl | 0 |
| 1247. | H | F | CF₃ | CH₂CH=CH₂ | Cl | 1 |
| 1248. | H | F | CF₃ | CH₂CH=CH₂ | Cl | 2 |
| 1249. | H | F | Cl | CH₂CH=CH₂ | CF₃ | 0 |
| 1250. | H | F | Cl | CH₂CH=CH₂ | CF₃ | 1 |
| 1251. | H | F | Cl | CH₂CH=CH₂ | CF₃ | 2 |
| 1252. | H | F | CF₃ | CHF₂ | OMe | 0 |
| 1253. | H | F | CF₃ | CHF₂ | OMe | 1 |
| 1254. | H | F | CF₃ | CHF₂ | OMe | 2 |
| 1255. | H | F | OMe | CHF₂ | CF₃ | 0 |
| 1256. | H | F | OMe | CHF₂ | CF₃ | 1 |
| 1257. | H | F | OMe | CHF₂ | CF₃ | 2 |
| 1258. | H | F | CF₃ | CH₂CF₃ | Cl | 0 |
| 1259. | H | F | CF₃ | CH₂CF₃ | Cl | 1 |
| 1260. | H | F | CF₃ | CH₂CF₃ | Cl | 2 |
| 1261. | H | F | Cl | CH₂CF₃ | CF₃ | 0 |
| 1262. | H | F | Cl | CH₂CF₃ | CF₃ | 1 |
| 1263. | H | F | Cl | CH₂CF₃ | CF₃ | 2 |
| 1264. | H | F | CF₃ | CH₂OMe | Cl | 0 |
| 1265. | H | F | CF₃ | CH₂OMe | Cl | 1 |
| 1266. | H | F | CF₃ | CH₂OMe | Cl | 2 |
| 1267. | H | F | Cl | CH₂OMe | CF₃ | 0 |
| 1268. | H | F | Cl | CH₂OMe | CF₃ | 1 |
| 1269. | H | F | Cl | CH₂OMe | CF₃ | 2 |
| 1270. | H | F | CF₃ | CH₂CN | Cl | 0 |
| 1271. | H | F | CF₃ | CH₂CN | Cl | 1 |
| 1272. | H | F | CF₃ | CH₂CN | Cl | 2 |
| 1273. | H | F | Me | Ph | Me | 0 |
| 1274. | H | F | Me | Ph | Me | 1 |
| 1275. | H | F | Me | Ph | Me | 2 |
| 1276. | H | F | Me | Ph | Cl | 0 |
| 1277. | H | F | Me | Ph | Cl | 1 |
| 1278. | H | F | Me | Ph | Cl | 2 |
| 1279. | H | F | Et | Ph | Cl | 0 |
| 1280. | H | F | Et | Ph | Cl | 1 |
| 1281. | H | F | Et | Ph | Cl | 2 |
| 1282. | H | F | Pr | Ph | Cl | 0 |
| 1283. | H | F | Pr | Ph | Cl | 1 |
| 1284. | H | F | Pr | Ph | Cl | 2 |
| 1285. | H | F | iPr | Ph | Cl | 0 |
| 1286. | H | F | iPr | Ph | Cl | 1 |
| 1287. | H | F | iPr | Ph | Cl | 2 |
| 1288. | H | F | CF₃ | Ph | Cl | 0 |
| 1289. | H | F | CF₃ | Ph | Cl | 1 |
| 1290. | H | F | CF₃ | Ph | Cl | 2 |
| 1291. | H | F | CF₃ | Ph | Me | 0 |
| 1292. | H | F | CF₃ | Ph | Me | 1 |
| 1293. | H | F | CF₃ | Ph | Me | 2 |
| 1294. | H | F | CF₃ | Ph | CF₃ | 0 |
| 1295. | H | F | CF₃ | Ph | CF₃ | 1 |
| 1296. | H | F | CF₃ | Ph | CF₃ | 2 |
| 1297. | H | F | CF₃ | Ph | F | 0 |
| 1298. | H | F | CF₃ | Ph | F | 1 |
| 1299. | H | F | CF₃ | Ph | F | 2 |
| 1300. | H | F | CF₃ | Ph | OMe | 0 |
| 1301. | H | F | CF₃ | Ph | OMe | 1 |
| 1302. | H | F | CF₃ | Ph | OMe | 2 |
| 1303. | H | F | CF₃ | Ph | OEt | 0 |
| 1304. | H | F | CF₃ | Ph | OEt | 1 |
| 1305. | H | F | CF₃ | Ph | OEt | 2 |
| 1306. | H | F | CF₃ | Ph | OCHF₂ | 0 |
| 1307. | H | F | CF₃ | Ph | OCHF₂ | 1 |
| 1308. | H | F | CF₃ | Ph | OCHF₂ | 2 |
| 1309. | H | F | CF₃ | Ph | CN | 0 |
| 1310. | H | F | CF₃ | Ph | CN | 1 |
| 1311. | H | F | CF₃ | Ph | CN | 2 |
| 1312. | H | F | CF₃ | Ph(4-Cl) | Cl | 0 |
| 1313. | H | F | CF₃ | Ph(4-Cl) | Cl | 1 |
| 1314. | H | F | CF₃ | Ph(4-Cl) | Cl | 2 |
| 1315. | H | F | Me | Me | OCH₂CF₃ | 0 |
| 1316. | H | F | Me | Me | OCH₂CF₃ | 1 |
| 1317. | H | F | Me | Me | OCH₂CF₃ | 2 |
| 1318. | H | F | CF₃ | Me | 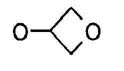 | 0 |
| 1319. | H | F | CF₃ | Me | 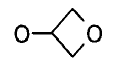 | 1 |
| 1320. | H | F | CF₃ | Me | 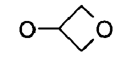 | 2 |
| 1321. | H | F | CF₃ | Me | H | 0 |
| 1322. | H | F | CF₃ | Me | H | 1 |
| 1323. | H | F | CF₃ | Me | H | 2 |
| 1324. | H | F | CF₃ | Me | OCH₂CH₂OMe | 0 |
| 1325. | H | F | CF₃ | Me | OCH₂CH₂OMe | 1 |
| 1326. | H | F | CF₃ | Me | OCH₂CH₂OMe | 2 |
| 1327. | H | F | CF₃ | Me | SMe | 0 |
| 1328. | H | F | CF₃ | Me | SMe | 1 |
| 1329. | H | F | CF₃ | Me | SMe | 2 |
| 1330. | H | F | CF₃ | Me | OCH₂CH₂CH₂F | 0 |
| 1331. | H | F | CF₃ | Me | OCH₂CH₂CH₂F | 1 |
| 1332. | H | F | CF₃ | Me | OCH₂CH₂CH₂F | 2 |
| 1333. | H | F | CF₃ | Me | OCH(CH₂F)₂ | 0 |
| 1334. | H | F | CF₃ | Me | OCH(CH₂F)₂ | 1 |
| 1335. | H | F | CF₃ | Me | OCH(CH₂F)₂ | 2 |
| 1336. | H | F | CF₃ | Me | OCH₂CF₂CHF₂ | 0 |
| 1337. | H | F | CF₃ | Me | OCH₂CF₂CHF₂ | 1 |
| 1338. | H | F | CF₃ | Me | OCH₂CF₂CHF₂ | 2 |
| 1339. | H | F | CF₃ | Me | OCH₂CF=CH₂ | 0 |
| 1340. | H | F | CF₃ | Me | OCH₂CF=CH₂ | 1 |
| 1341. | H | F | CF₃ | Me | OCH₂CF=CH₂ | 2 |
| 1342. | H | F | CF₃ | Me | OCH(Me)CF₃ | 0 |
| 1343. | H | F | CF₃ | Me | OCH(Me)CF₃ | 1 |
| 1344. | H | F | CF₃ | Me | OCH(Me)CF₃ | 2 |
| 1345. | H | F | CF₃ | Me | OCH(Me)CH₂F | 0 |
| 1346. | H | F | CF₃ | Me | OCH(Me)CH₂F | 1 |
| 1347. | H | F | CF₃ | Me | OCH(Me)CH₂F | 2 |
| 1348. | H | F | OCH₂CF₃ | Me | CF₃ | 0 |
| 1349. | H | F | OCH₂CF₃ | Me | CF₃ | 1 |
| 1350. | H | F | OCH₂CF₃ | Me | CF₃ | 2 |
| 1351. | H | F | OCH₂CF₃ | Me | CHF₂ | 0 |
| 1352. | H | F | OCH₂CF₃ | Me | CHF₂ | 1 |
| 1353. | H | F | OCH₂CF₃ | Me | CHF₂ | 2 |
| 1354. | H | F | CHF₂ | Me | CHF₂ | 0 |
| 1355. | H | F | CHF₂ | Me | CHF₂ | 1 |
| 1356. | H | F | CHF₂ | Me | CHF₂ | 2 |
| 1357. | H | F | CF₃ | Me | CHF₂ | 0 |
| 1358. | H | F | CF₃ | Me | CHF₂ | 1 |
| 1359. | H | F | CF₃ | Me | CHF₂ | 2 |
| 1360. | H | F | Cl | Me | OCHF₂ | 0 |
| 1361. | H | F | Cl | Me | OCHF₂ | 1 |
| 1362. | H | F | Cl | Me | OCHF₂ | 2 |
| 1363. | H | F | Br | Me | OCHF₂ | 0 |
| 1364. | H | F | Br | Me | OCHF₂ | 1 |
| 1365. | H | F | Br | Me | OCHF₂ | 2 |
| 1366. | H | F | Br | Me | CF₃ | 0 |
| 1367. | H | F | Br | Me | CF₃ | 1 |
| 1368. | H | F | Br | Me | CF₃ | 2 |
| 1369. | H | Cl | CF₃ | tBu | Cl | 0 |
| 1370. | H | Cl | CF₃ | tBu | Cl | 1 |
| 1371. | H | Cl | CF₃ | tBu | Cl | 2 |
| 1372. | H | Cl | CF₃ | CHF₂ | Cl | 0 |
| 1373. | H | Cl | CF₃ | CHF₂ | Cl | 1 |
| 1374. | H | Cl | CF₃ | CHF₂ | Cl | 2 |
| 1375. | H | Cl | Cl | CHF₂ | CF₃ | 0 |
| 1376. | H | Cl | Cl | CHF₂ | CF₃ | 1 |
| 1377. | H | Cl | Cl | CHF₂ | CF₃ | 2 |
| 1378. | H | Cl | CF₃ | Me | OMe | 0 |
| 1379. | H | Cl | CF₃ | Me | OMe | 1 |
| 1380. | H | Cl | CF₃ | Me | OMe | 2 |
| 1381. | H | Cl | CF₃ | Me | CN | 0 |
| 1382. | H | Cl | CF₃ | Me | CN | 1 |
| 1383. | H | Cl | CF₃ | Me | CN | 2 |
| 1384. | H | Cl | CHF₂ | Me | Cl | 0 |
| 1385. | H | Cl | CHF₂ | Me | Cl | 1 |
| 1386. | H | Cl | CHF₂ | Me | Cl | 2 |
| 1387. | H | Cl | Me | Me | Me | 0 |
| 1388. | H | Cl | Me | Me | Me | 1 |
| 1389. | H | Cl | Me | Me | Me | 2 |
| 1390. | H | Cl | Me | Me | Cl | 0 |
| 1391. | H | Cl | Me | Me | Cl | 1 |
| 1392. | H | Cl | Me | Me | Cl | 2 |
| 1393. | H | Cl | CF₃ | Me | Cl | 0 |
| 1394. | H | Cl | CF₃ | Me | Cl | 1 |
| 1395. | H | Cl | CF₃ | Me | Cl | 2 |
| 1396. | H | Cl | Cl | Me | CF₃ | 0 |
| 1397. | H | Cl | Cl | Me | CF₃ | 1 |
| 1398. | H | Cl | Cl | Me | CF₃ | 2 |
| 1399. | H | Cl | CF₃ | Me | F | 0 |
| 1400. | H | Cl | CF₃ | Me | F | 1 |
| 1401. | H | Cl | CF₃ | Me | F | 2 |
| 1402. | H | Cl | OMe | Me | CF₃ | 0 |
| 1403. | H | Cl | OMe | Me | CF₃ | 1 |
| 1404. | H | Cl | OMe | Me | CF₃ | 2 |
| 1405. | H | Cl | CF₃ | Me | OCHF₂ | 0 |
| 1406. | H | Cl | CF₃ | Me | OCHF₂ | 1 |
| 1407. | H | Cl | CF₃ | Me | OCHF₂ | 2 |
| 1408. | H | Cl | OCHF₂ | Me | CF₃ | 0 |
| 1409. | H | Cl | OCHF₂ | Me | CF₃ | 1 |
| 1410. | H | Cl | OCHF₂ | Me | CF₃ | 2 |
| 1411. | H | Cl | CF₃ | Me | OCH₂CHF₂ | 0 |
| 1412. | H | Cl | CF₃ | Me | OCH₂CHF₂ | 1 |
| 1413. | H | Cl | CF₃ | Me | OCH₂CHF₂ | 2 |
| 1414. | H | Cl | CF₃ | Me | OCH₂CF₃ | 0 |
| 1415. | H | Cl | CF₃ | Me | OCH₂CF₃ | 1 |
| 1416. | H | Cl | CF₃ | Me | OCH₂CF₃ | 2 |
| 1417. | H | Cl | CF₃ | Me | SO₂Me | 0 |
| 1418. | H | Cl | CF₃ | Me | SO₂Me | 1 |
| 1419. | H | Cl | CF₃ | Me | SO₂Me | 2 |
| 1420. | H | Cl | CF₃ | Me | SEt | 0 |
| 1421. | H | Cl | CF₃ | Me | SEt | 1 |
| 1422. | H | Cl | CF₃ | Me | SEt | 2 |
| 1423. | H | Cl | CF₃ | Et | Cl | 0 |
| 1424. | H | Cl | CF₃ | Et | Cl | 1 |
| 1425. | H | Cl | CF₃ | Et | Cl | 2 |
| 1426. | H | Cl | CF₃ | iPr | Cl | 0 |
| 1427. | H | Cl | CF₃ | iPr | Cl | 1 |
| 1428. | H | Cl | CF₃ | iPr | Cl | 2 |
| 1429. | H | Cl | CF₃ | tBu | Cl | 0 |
| 1430. | H | Cl | CF₃ | tBu | Cl | 1 |
| 1431. | H | Cl | CF₃ | tBu | Cl | 2 |
| 1432. | H | Cl | Cl | tBu | CF₃ | 0 |
| 1433. | H | Cl | Cl | tBu | CF₃ | 1 |
| 1434. | H | Cl | Cl | tBu | CF₃ | 2 |
| 1435. | H | Cl | CF₃ | cPen | Cl | 0 |
| 1436. | H | Cl | CF₃ | cPen | Cl | 1 |
| 1437. | H | Cl | CF₃ | cPen | Cl | 2 |
| 1438. | H | Cl | CF₃ | CHF₂ | OMe | 0 |
| 1439. | H | Cl | CF₃ | CHF₂ | OMe | 1 |
| 1440. | H | Cl | CF₃ | CHF₂ | OMe | 2 |
| 1441. | H | Cl | CF₃ | CH₂CF₃ | Cl | 0 |
| 1442. | H | Cl | CF₃ | CH₂CF₃ | Cl | 1 |
| 1443. | H | Cl | CF₃ | CH₂CF₃ | Cl | 2 |
| 1444. | H | Cl | CF₃ | Ph | OCHF₂ | 0 |
| 1445. | H | Cl | CF₃ | Ph | OCHF₂ | 1 |
| 1446. | H | Cl | CF₃ | Ph | OCHF₂ | 2 |
| 1447. | H | Cl | CF₃ | Ph | Cl | 0 |
| 1448. | H | Cl | CF₃ | Ph | Cl | 1 |
| 1449. | H | Cl | CF₃ | Ph | Cl | 2 |
| 1450. | H | Cl | Me | Me | OCH₂CF₃ | 0 |
| 1451. | H | Cl | Me | Me | OCH₂CF₃ | 1 |
| 1452. | H | Cl | Me | Me | OCH₂CF₃ | 2 |
| 1453. | H | Cl | CF₃ | Me | 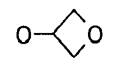 | 0 |
| 1454. | H | Cl | CF₃ | Me | 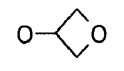 | 1 |
| 1455. | H | Cl | CF₃ | Me | 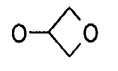 | 2 |
| 1456. | H | Cl | CF₃ | Me | H | 0 |
| 1457. | H | Cl | CF₃ | Me | H | 1 |
| 1458. | H | Cl | CF₃ | Me | H | 2 |
| 1459. | H | Cl | CF₃ | Me | OCH₂CH₂OMe | 0 |
| 1460. | H | Cl | CF₃ | Me | OCH₂CH₂OMe | 1 |
| 1461. | H | Cl | CF₃ | Me | OCH₂CH₂OMe | 2 |
| 1462. | H | Cl | CF₃ | Me | SMe | 0 |
| 1463. | H | Cl | CF₃ | Me | SMe | 1 |
| 1464. | H | Cl | CF₃ | Me | SMe | 2 |
| 1465. | H | Cl | CF₃ | Me | OCH₂CH₂CH₂F | 0 |
| 1466. | H | Cl | CF₃ | Me | OCH₂CH₂CH₂F | 1 |
| 1467. | H | Cl | CF₃ | Me | OCH₂CH₂CH₂F | 2 |
| 1468. | H | Cl | CF₃ | Me | OCH(CH₂F)₂ | 0 |
| 1469. | H | Cl | CF₃ | Me | OCH(CH₂F)₂ | 1 |
| 1470. | H | Cl | CF₃ | Me | OCH(CH₂F)₂ | 2 |
| 1471. | H | Cl | CF₃ | Me | OCH₂CF₂CHF₂ | 0 |
| 1472. | H | Cl | CF₃ | Me | OCH₂CF₂CHF₂ | 1 |
| 1473. | H | Cl | CF₃ | Me | OCH₂CF₂CHF₂ | 2 |
| 1474. | H | Cl | CF₃ | Me | OCH₂CF=CH₂ | 0 |
| 1475. | H | Cl | CF₃ | Me | OCH₂CF=CH₂ | 1 |
| 1476. | H | Cl | CF₃ | Me | OCH₂CF=CH₂ | 2 |
| 1477. | H | Cl | CF₃ | Me | OCH(Me)CF₃ | 0 |
| 1478. | H | Cl | CF₃ | Me | OCH(Me)CF₃ | 1 |
| 1479. | H | Cl | CF₃ | Me | OCH(Me)CF₃ | 2 |
| 1480. | H | Cl | CF₃ | Me | OCH(Me)CH₂F | 0 |
| 1481. | H | Cl | CF₃ | Me | OCH(Me)CH₂F | 1 |
| 1482. | H | Cl | CF₃ | Me | OCH(Me)CH₂F | 2 |
| 1483. | H | Cl | OCH₂CF₃ | Me | CF₃ | 0 |
| 1484. | H | Cl | OCH₂CF₃ | Me | CF₃ | 1 |
| 1485. | H | Cl | OCH₂CF₃ | Me | CF₃ | 2 |
| 1486. | H | Cl | OCH₂CF₃ | Me | CHF₂ | 0 |
| 1487. | H | Cl | OCH₂CF₃ | Me | CHF₂ | 1 |
| 1488. | H | Cl | OCH₂CF₃ | Me | CHF₂ | 2 |
| 1489. | H | Cl | CHF₂ | Me | CHF₂ | 0 |
| 1490. | H | Cl | CHF₂ | Me | CHF₂ | 1 |
| 1491. | H | Cl | CHF₂ | Me | CHF₂ | 2 |
| 1492. | H | Cl | CF₃ | Me | CHF₂ | 0 |
| 1493. | H | Cl | CF₃ | Me | CHF₂ | 1 |
| 1494. | H | Cl | CF₃ | Me | CHF₂ | 2 |
| 1495. | H | Cl | Cl | Me | OCHF₂ | 0 |
| 1496. | H | Cl | Cl | Me | OCHF₂ | 1 |
| 1497. | H | Cl | Cl | Me | OCHF₂ | 2 |
| 1498. | H | Cl | Br | Me | OCHF₂ | 0 |
| 1499. | H | Cl | Br | Me | OCHF₂ | 1 |
| 1500. | H | Cl | Br | Me | OCHF₂ | 2 |
| 1501. | H | Cl | Br | Me | CF₃ | 0 |
| 1502. | H | Cl | Br | Me | CF₃ | 1 |
| 1503. | H | Cl | Br | Me | CF₃ | 2 |
| 1504. | H | Br | CF₃ | tBu | Cl | 0 |
| 1505. | H | Br | CF₃ | tBu | Cl | 1 |
| 1506. | H | Br | CF₃ | tBu | Cl | 2 |
| 1507. | H | Br | CF₃ | CHF₂ | Cl | 0 |
| 1508. | H | Br | CF₃ | CHF₂ | Cl | 1 |
| 1509. | H | Br | CF₃ | CHF₂ | Cl | 2 |
| 1510. | H | Br | Cl | CHF₂ | CF₃ | 0 |
| 1511. | H | Br | Cl | CHF₂ | CF₃ | 1 |
| 1512. | H | Br | Cl | CHF₂ | CF₃ | 2 |
| 1513. | H | Br | CF₃ | Me | OMe | 0 |
| 1514. | H | Br | CF₃ | Me | OMe | 1 |
| 1515. | H | Br | CF₃ | Me | OMe | 2 |
| 1516. | H | Br | CF₃ | Me | CN | 0 |
| 1517. | H | Br | CF₃ | Me | CN | 1 |
| 1518. | H | Br | CF₃ | Me | CN | 2 |
| 1519. | H | Br | CHF₂ | Me | Cl | 0 |
| 1520. | H | Br | CHF₂ | Me | Cl | 1 |
| 1521. | H | Br | CHF₂ | Me | Cl | 2 |
| 1522. | H | Br | Me | Me | Me | 0 |
| 1523. | H | Br | Me | Me | Me | 1 |
| 1524. | H | Br | Me | Me | Me | 2 |
| 1525. | H | Br | Me | Me | Cl | 0 |
| 1526. | H | Br | Me | Me | Cl | 1 |
| 1527. | H | Br | Me | Me | Cl | 2 |
| 1528. | H | Br | CF₃ | Me | Cl | 0 |
| 1529. | H | Br | CF₃ | Me | Cl | 1 |
| 1530. | H | Br | CF₃ | Me | Cl | 2 |
| 1531. | H | Br | Cl | Me | CF₃ | 0 |
| 1532. | H | Br | Cl | Me | CF₃ | 1 |
| 1533. | H | Br | Cl | Me | CF₃ | 2 |
| 1534. | H | Br | CF₃ | Me | F | 0 |
| 1535. | H | Br | CF₃ | Me | F | 1 |
| 1536. | H | Br | CF₃ | Me | F | 2 |
| 1537. | H | Br | OMe | Me | CF₃ | 0 |
| 1538. | H | Br | OMe | Me | CF₃ | 1 |
| 1539. | H | Br | OMe | Me | CF₃ | 2 |
| 1540. | H | Br | CF₃ | Me | OCHF₂ | 0 |
| 1541. | H | Br | CF₃ | Me | OCHF₂ | 1 |
| 1542. | H | Br | CF₃ | Me | OCHF₂ | 2 |
| 1543. | H | Br | OCHF₂ | Me | CF₃ | 0 |
| 1544. | H | Br | OCHF₂ | Me | CF₃ | 1 |
| 1545. | H | Br | OCHF₂ | Me | CF₃ | 2 |
| 1546. | H | Br | CF₃ | Me | OCH₂CHF₂ | 0 |
| 1547. | H | Br | CF₃ | Me | OCH₂CHF₂ | 1 |
| 1548. | H | Br | CF₃ | Me | OCH₂CHF₂ | 2 |
| 1549. | H | Br | CF₃ | Me | OCH₂CF₃ | 0 |
| 1550. | H | Br | CF₃ | Me | OCH₂CF₃ | 1 |
| 1551. | H | Br | CF₃ | Me | OCH₂CF₃ | 2 |
| 1552. | H | Br | CF₃ | Me | SO₂Me | 0 |
| 1553. | H | Br | CF₃ | Me | SO₂Me | 1 |
| 1554. | H | Br | CF₃ | Me | SO₂Me | 2 |
| 1555. | H | Br | CF₃ | Me | SEt | 0 |
| 1556. | H | Br | CF₃ | Me | SEt | 1 |
| 1557. | H | Br | CF₃ | Me | SEt | 2 |
| 1558. | H | Br | CF₃ | Et | Cl | 0 |
| 1559. | H | Br | CF₃ | Et | Cl | 1 |
| 1560. | H | Br | CF₃ | Et | Cl | 2 |
| 1561. | H | Br | CF₃ | iPr | Cl | 0 |
| 1562. | H | Br | CF₃ | iPr | Cl | 1 |
| 1563. | H | Br | CF₃ | iPr | Cl | 2 |
| 1564. | H | Br | CF₃ | tBu | Cl | 0 |
| 1565. | H | Br | CF₃ | tBu | Cl | 1 |
| 1566. | H | Br | CF₃ | tBu | Cl | 2 |
| 1567. | H | Br | Cl | tBu | CF₃ | 0 |
| 1568. | H | Br | Cl | tBu | CF₃ | 1 |
| 1569. | H | Br | Cl | tBu | CF₃ | 2 |
| 1570. | H | Br | CF₃ | cPen | Cl | 0 |
| 1571. | H | Br | CF₃ | cPen | Cl | 1 |
| 1572. | H | Br | CF₃ | cPen | Cl | 2 |
| 1573. | H | Br | CF₃ | CHF₂ | OMe | 0 |
| 1574. | H | Br | CF₃ | CHF₂ | OMe | 1 |
| 1575. | H | Br | CF₃ | CHF₂ | OMe | 2 |
| 1576. | H | Br | CF₃ | CH₂CF₃ | Cl | 0 |
| 1577. | H | Br | CF₃ | CH₂CF₃ | Cl | 1 |
| 1578. | H | Br | CF₃ | CH₂CF₃ | Cl | 2 |
| 1579. | H | Br | CF₃ | Ph | OCHF₂ | 0 |
| 1580. | H | Br | CF₃ | Ph | OCHF₂ | 1 |
| 1581. | H | Br | CF₃ | Ph | OCHF₂ | 2 |
| 1582. | H | Br | CF₃ | Ph | Cl | 0 |
| 1583. | H | Br | CF₃ | Ph | Cl | 1 |
| 1584. | H | Br | CF₃ | Ph | Cl | 2 |
| 1585. | H | Br | Me | Me | OCH₂CF₃ | 0 |
| 1586. | H | Br | Me | Me | OCH₂CF₃ | 1 |
| 1587. | H | Br | Me | Me | OCH₂CF₃ | 2 |
| 1588. | H | Br | CF₃ | Me | 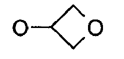 | 0 |
| 1589. | H | Br | CF₃ | Me | 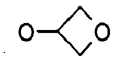 | 1 |
| 1590. | H | Br | CF₃ | Me | 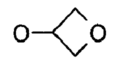 | 2 |
| 1591. | H | Br | CF₃ | Me | H | 0 |
| 1592. | H | Br | CF₃ | Me | H | 1 |
| 1593. | H | Br | CF₃ | Me | H | 2 |
| 1594. | H | Br | CF₃ | Me | OCH₂CH₂OMe | 0 |
| 1595. | H | Br | CF₃ | Me | OCH₂CH₂OMe | 1 |
| 1596. | H | Br | CF₃ | Me | OCH₂CH₂OMe | 2 |
| 1597. | H | Br | CF₃ | Me | SMe | 0 |
| 1598. | H | Br | CF₃ | Me | SMe | 1 |
| 1599. | H | Br | CF₃ | Me | SMe | 2 |
| 1600. | H | Br | CF₃ | Me | OCH₂CH₂CH₂F | 0 |
| 1601. | H | Br | CF₃ | Me | OCH₂CH₂CH₂F | 1 |
| 1602. | H | Br | CF₃ | Me | OCH₂CH₂CH₂F | 2 |
| 1603. | H | Br | CF₃ | Me | OCH(CH₂F)₂ | 0 |
| 1604. | H | Br | CF₃ | Me | OCH(CH₂F)₂ | 1 |
| 1605. | H | Br | CF₃ | Me | OCH(CH₂F)₂ | 2 |
| 1606. | H | Br | CF₃ | Me | OCH₂CF₂CHF₂ | 0 |
| 1607. | H | Br | CF₃ | Me | OCH₂CF₂CHF₂ | 1 |
| 1608. | H | Br | CF₃ | Me | OCH₂CF₂CHF₂ | 2 |
| 1609. | H | Br | CF₃ | Me | OCH₂CF=CH₂ | 0 |
| 1610. | H | Br | CF₃ | Me | OCH₂CF=CH₂ | 1 |
| 1611. | H | Br | CF₃ | Me | OCH₂CF=CH₂ | 2 |
| 1612. | H | Br | CF₃ | Me | OCH(Me)CF₃ | 0 |
| 1613. | H | Br | CF₃ | Me | OCH(Me)CF₃ | 1 |
| 1614. | H | Br | CF₃ | Me | OCH(Me)CF₃ | 2 |
| 1615. | H | Br | CF₃ | Me | OCH(Me)CH₂F | 0 |
| 1616. | H | Br | CF₃ | Me | OCH(Me)CH₂F | 1 |
| 1617. | H | Br | CF₃ | Me | OCH(Me)CH₂F | 2 |
| 1618. | H | Br | OCH₂CF₃ | Me | CF₃ | 0 |
| 1619. | H | Br | OCH₂CF₃ | Me | CF₃ | 1 |
| 1620. | H | Br | OCH₂CF₃ | Me | CF₃ | 2 |
| 1621. | H | Br | OCH₂CF₃ | Me | CHF₂ | 0 |
| 1622. | H | Br | OCH₂CF₃ | Me | CHF₂ | 1 |
| 1623. | H | Br | OCH₂CF₃ | Me | CHF₂ | 2 |
| 1624. | H | Br | CHF₂ | Me | CHF₂ | 0 |
| 1625. | H | Br | CHF₂ | Me | CHF₂ | 1 |
| 1626. | H | Br | CHF₂ | Me | CHF₂ | 2 |
| 1627. | H | Br | CF₃ | Me | CHF₂ | 0 |
| 1628. | H | Br | CF₃ | Me | CHF₂ | 1 |
| 1629. | H | Br | CF₃ | Me | CHF₂ | 2 |
| 1630. | H | Br | Cl | Me | OCHF₂ | 0 |
| 1631. | H | Br | Cl | Me | OCHF₂ | 1 |
| 1632. | H | Br | Cl | Me | OCHF₂ | 2 |
| 1633. | H | Br | Br | Me | OCHF₂ | 0 |
| 1634. | H | Br | Br | Me | OCHF₂ | 1 |
| 1635. | H | Br | Br | Me | OCHF₂ | 2 |
| 1636. | H | Br | Br | Me | CF₃ | 0 |
| 1637. | H | Br | Br | Me | CF₃ | 1 |
| 1638. | H | Br | Br | Me | CF₃ | 2 |
| 1639. | Me | H | CF₃ | tBu | Cl | 0 |
| 1640. | Me | H | CF₃ | tBu | Cl | 1 |
| 1641. | Me | H | CF₃ | tBu | Cl | 2 |
| 1642. | Me | H | CF₃ | CHF₂ | Cl | 0 |
| 1643. | Me | H | CF₃ | CHF₂ | Cl | 1 |
| 1644. | Me | H | CF₃ | CHF₂ | Cl | 2 |
| 1645. | Me | H | Cl | CHF₂ | CF₃ | 0 |
| 1646. | Me | H | Cl | CHF₂ | CF₃ | 1 |
| 1647. | Me | H | Cl | CHF₂ | CF₃ | 2 |
| 1648. | Me | H | CF₃ | Me | OMe | 0 |
| 1649. | Me | H | CF₃ | Me | OMe | 1 |
| 1650. | Me | H | CF₃ | Me | OMe | 2 |
| 1651. | Me | H | CF₃ | Me | CN | 0 |
| 1652. | Me | H | CF₃ | Me | CN | 1 |
| 1653. | Me | H | CF₃ | Me | CN | 2 |
| 1654. | Me | H | CHF₂ | Me | Cl | 0 |
| 1655. | Me | H | CHF₂ | Me | Cl | 1 |
| 1656. | Me | H | CHF₂ | Me | Cl | 2 |
| 1657. | Me | H | Me | Me | Me | 0 |
| 1658. | Me | H | Me | Me | Me | 1 |
| 1659. | Me | H | Me | Me | Me | 2 |
| 1660. | Me | H | Me | Me | Cl | 0 |
| 1661. | Me | H | Me | Me | Cl | 1 |
| 1662. | Me | H | Me | Me | Cl | 2 |
| 1663. | Me | H | CF₃ | Me | Cl | 0 |
| 1664. | Me | H | CF₃ | Me | Cl | 1 |
| 1665. | Me | H | CF₃ | Me | Cl | 2 |
| 1666. | Me | H | Cl | Me | CF₃ | 0 |
| 1667. | Me | H | Cl | Me | CF₃ | 1 |
| 1668. | Me | H | Cl | Me | CF₃ | 2 |
| 1669. | Me | H | CF₃ | Me | F | 0 |
| 1670. | Me | H | CF₃ | Me | F | 1 |
| 1671. | Me | H | CF₃ | Me | F | 2 |
| 1672. | Me | H | OMe | Me | CF₃ | 0 |
| 1673. | Me | H | OMe | Me | CF₃ | 1 |
| 1674. | Me | H | OMe | Me | CF₃ | 2 |
| 1675. | Me | H | CF₃ | Me | OCHF₂ | 0 |
| 1676. | Me | H | CF₃ | Me | OCHF₂ | 1 |
| 1677. | Me | H | CF₃ | Me | OCHF₂ | 2 |
| 1678. | Me | H | OCHF₂ | Me | CF₃ | 0 |
| 1679. | Me | H | OCHF₂ | Me | CF₃ | 1 |
| 1680. | Me | H | OCHF₂ | Me | CF₃ | 2 |
| 1681. | Me | H | CF₃ | Me | OCH₂CHF₂ | 0 |
| 1682. | Me | H | CF₃ | Me | OCH₂CHF₂ | 1 |
| 1683. | Me | H | CF₃ | Me | OCH₂CHF₂ | 2 |
| 1684. | Me | H | CF₃ | Me | OCH₂CF₃ | 0 |
| 1685. | Me | H | CF₃ | Me | OCH₂CF₃ | 1 |
| 1686. | Me | H | CF₃ | Me | OCH₂CF₃ | 2 |
| 1687. | Me | H | CF₃ | Me | SO₂Me | 0 |
| 1688. | Me | H | CF₃ | Me | SO₂Me | 1 |
| 1689. | Me | H | CF₃ | Me | SO₂Me | 2 |
| 1690. | Me | H | CF₃ | Me | SEt | 0 |
| 1691. | Me | H | CF₃ | Me | SEt | 1 |
| 1692. | Me | H | CF₃ | Me | SEt | 2 |
| 1693. | Me | H | CF₃ | Et | Cl | 0 |
| 1694. | Me | H | CF₃ | Et | Cl | 1 |
| 1695. | Me | H | CF₃ | Et | Cl | 2 |
| 1696. | Me | H | CF₃ | iPr | Cl | 0 |
| 1697. | Me | H | CF₃ | iPr | Cl | 1 |
| 1698. | Me | H | CF₃ | iPr | Cl | 2 |
| 1699. | Me | H | CF₃ | tBu | Cl | 0 |
| 1700. | Me | H | CF₃ | tBu | Cl | 1 |
| 1701. | Me | H | CF₃ | tBu | Cl | 2 |
| 1702. | Me | H | Cl | tBu | CF₃ | 0 |
| 1703. | Me | H | Cl | tBu | CF₃ | 1 |
| 1704. | Me | H | Cl | tBu | CF₃ | 2 |
| 1705. | Me | H | CF₃ | cPen | Cl | 0 |
| 1706. | Me | H | CF₃ | cPen | Cl | 1 |
| 1707. | Me | H | CF₃ | cPen | Cl | 2 |
| 1708. | Me | H | CF₃ | CHF₂ | OMe | 0 |
| 1709. | Me | H | CF₃ | CHF₂ | OMe | 1 |
| 1710. | Me | H | CF₃ | CHF₂ | OMe | 2 |
| 1711. | Me | H | CF₃ | CH₂CF₃ | Cl | 0 |
| 1712. | Me | H | CF₃ | CH₂CF₃ | Cl | 1 |
| 1713. | Me | H | CF₃ | CH₂CF₃ | Cl | 2 |
| 1714. | Me | H | CF₃ | Ph | OCHF₂ | 0 |
| 1715. | Me | H | CF₃ | Ph | OCHF₂ | 1 |
| 1716. | Me | H | CF₃ | Ph | OCHF₂ | 2 |
| 1717. | Me | H | CF₃ | Ph | Cl | 0 |
| 1718. | Me | H | CF₃ | Ph | Cl | 1 |
| 1719. | Me | H | CF₃ | Ph | Cl | 2 |
| 1720. | Me | H | Me | Me | OCH₂CF₃ | 0 |
| 1721. | Me | H | Me | Me | OCH₂CF₃ | 1 |
| 1722. | Me | H | Me | Me | OCH₂CF₃ | 2 |
| 1723. | | Me H | CF₃ | Me | 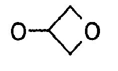 | 0 |
| 1724. | | Me H | CF₃ | Me | 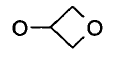 | 1 |
| 1725. | | Me H | CF₃ | Me | 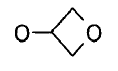 | 2 |
| 1726. | Me | H | CF₃ | Me | H | 0 |
| 1727. | Me | H | CF₃ | Me | H | 1 |
| 1728. | Me | H | CF₃ | Me | H | 2 |
| 1729. | Me | H | CF₃ | Me | OCH₂CH₂OMe | 0 |
| 1730. | Me | H | CF₃ | Me | OCH₂CH₂OMe | 1 |
| 1731. | Me | H | CF₃ | Me | OCH₂CH₂OMe | 2 |
| 1732. | Me | H | CF₃ | Me | SMe | 0 |
| 1733. | Me | H | CF₃ | Me | SMe | 1 |
| 1734. | Me | H | CF₃ | Me | SMe | 2 |
| 1735. | Me | H | CF₃ | Me | OCH₂CH₂CH₂F | 0 |
| 1736. | Me | H | CF₃ | Me | OCH₂CH₂CH₂F | 1 |
| 1737. | Me | H | CF₃ | Me | OCH₂CH₂CH₂F | 2 |
| 1738. | Me | H | CF₃ | Me | OCH(CH₂F)₂ | 0 |
| 1739. | Me | H | CF₃ | Me | OCH(CH₂F)₂ | 1 |
| 1740. | Me | H | CF₃ | Me | OCH(CH₂F)₂ | 2 |
| 1741. | Me | H | CF₃ | Me | OCH₂CF₂CHF₂ | 0 |
| 1742. | Me | H | CF₃ | Me | OCH₂CF₂CHF₂ | 1 |
| 1743. | Me | H | CF₃ | Me | OCH₂CF₂CHF₂ | 2 |
| 1744. | Me | H | CF₃ | Me | OCH₂CF=CH₂ | 0 |
| 1745. | Me | H | CF₃ | Me | OCH₂CF=CH₂ | 1 |
| 1746. | Me | H | CF₃ | Me | OCH₂CF=CH₂ | 2 |
| 1747. | Me | H | CF₃ | Me | OCH(Me)CF₃ | 0 |
| 1748. | Me | H | CF₃ | Me | OCH(Me)CF₃ | 1 |
| 1749. | Me | H | CF₃ | Me | OCH(Me)CF₃ | 2 |
| 1750. | Me | H | CF₃ | Me | OCH(Me)CH₂F | 0 |
| 1751. | Me | H | CF₃ | Me | OCH(Me)CH₂F | 1 |
| 1752. | Me | H | CF₃ | Me | OCH(Me)CH₂F | 2 |
| 1753. | Me | H | OCH₂CF₃ | Me | CF₃ | 0 |
| 1754. | Me | H | OCH₂CF₃ | Me | CF₃ | 1 |
| 1755. | Me | H | OCH₂CF₃ | Me | CF₃ | 2 |
| 1756. | Me | H | OCH₂CF₃ | Me | CHF₂ | 0 |
| 1757. | Me | H | OCH₂CF₃ | Me | CHF₂ | 1 |
| 1758. | Me | H | OCH₂CF₃ | Me | CHF₂ | 2 |
| 1759. | Me | H | CHF₂ | Me | CHF₂ | 0 |
| 1760. | Me | H | CHF₂ | Me | CHF₂ | 1 |
| 1761. | Me | H | CHF₂ | Me | CHF₂ | 2 |
| 1762. | Me | H | CF₃ | Me | CHF₂ | 0 |
| 1763. | Me | H | CF₃ | Me | CHF₂ | 1 |
| 1764. | Me | H | CF₃ | Me | CHF₂ | 2 |
| 1765. | Me | H | Cl | Me | OCHF₂ | 0 |
| 1766. | Me | H | Cl | Me | OCHF₂ | 1 |
| 1767. | Me | H | Cl | Me | OCHF₂ | 2 |
| 1768. | Me | H | Br | Me | OCHF₂ | 0 |
| 1769. | Me | H | Br | Me | OCHF₂ | 1 |
| 1770. | Me | H | Br | Me | OCHF₂ | 2 |
| 1771. | Me | H | Br | Me | CF₃ | 0 |
| 1772. | Me | H | Br | Me | CF₃ | 1 |
| 1773. | Me | H | Br | Me | CF₃ | 2 |
| 1774. | NO₂ | H | CF₃ | tBu | Cl | 0 |
| 1775. | NO₂ | H | CF₃ | tBu | Cl | 1 |
| 1776. | NO₂ | H | CF₃ | tBu | Cl | 2 |
| 1777. | NO₂ | H | CF₃ | CHF₂ | Cl | 0 |
| 1778. | NO₂ | H | CF₃ | CHF₂ | Cl | 1 |
| 1779. | NO₂ | H | CF₃ | CHF₂ | Cl | 2 |
| 1780. | NO₂ | H | Cl | CHF₂ | CF₃ | 0 |
| 1781. | NO₂ | H | Cl | CHF₂ | CF₃ | 1 |
| 1782. | NO₂ | H | Cl | CHF₂ | CF₃ | 2 |
| 1783. | NO₂ | H | CF₃ | Me | OMe | 0 |
| 1784. | NO₂ | H | CF₃ | Me | OMe | 1 |
| 1785. | NO₂ | H | CF₃ | Me | OMe | 2 |
| 1786. | NO₂ | H | CF₃ | Me | CN | 0 |
| 1787. | NO₂ | H | CF₃ | Me | CN | 1 |
| 1788. | NO₂ | H | CF₃ | Me | CN | 2 |
| 1789. | NO₂ | H | CHF₂ | Me | Cl | 0 |
| 1790. | NO₂ | H | CHF₂ | Me | Cl | 1 |
| 1791. | NO₂ | H | CHF₂ | Me | Cl | 2 |
| 1792. | NO₂ | H | Me | Me | Me | 0 |
| 1793. | NO₂ | H | Me | Me | Me | 1 |
| 1794. | NO₂ | H | Me | Me | Me | 2 |
| 1795. | NO₂ | H | Me | Me | Cl | 0 |
| 1796. | NO₂ | H | Me | Me | Cl | 1 |
| 1797. | NO₂ | H | Me | Me | Cl | 2 |
| 1798. | NO₂ | H | CF₃ | Me | Cl | 0 |
| 1799. | NO₂ | H | CF₃ | Me | Cl | 1 |
| 1800. | NO₂ | H | CF₃ | Me | Cl | 2 |
| 1801. | NO₂ | H | Cl | Me | CF₃ | 0 |
| 1802. | NO₂ | H | Cl | Me | CF₃ | 1 |
| 1803. | NO₂ | H | Cl | Me | CF₃ | 2 |
| 1804. | NO₂ | H | CF₃ | Me | F | 0 |
| 1805. | NO₂ | H | CF₃ | Me | F | 1 |
| 1806. | NO₂ | H | CF₃ | Me | F | 2 |
| 1807. | NO₂ | H | OMe | Me | CF₃ | 0 |
| 1808. | NO₂ | H | OMe | Me | CF₃ | 1 |
| 1809. | NO₂ | H | OMe | Me | CF₃ | 2 |
| 1810. | NO₂ | H | CF₃ | Me | OCHF₂ | 0 |
| 1811. | NO₂ | H | CF₃ | Me | OCHF₂ | 1 |
| 1812. | NO₂ | H | CF₃ | Me | OCHF₂ | 2 |
| 1813. | NO₂ | H | OCHF₂ | Me | CF₃ | 0 |
| 1814. | NO₂ | H | OCHF₂ | Me | CF₃ | 1 |
| 1815. | NO₂ | H | OCHF₂ | Me | CF₃ | 2 |
| 1816. | NO₂ | H | CF₃ | Me | OCH₂CHF₂ | 0 |
| 1817. | NO₂ | H | CF₃ | Me | OCH₂CHF₂ | 1 |
| 1818. | NO₂ | H | CF₃ | Me | OCH₂CHF₂ | 2 |
| 1819. | NO₂ | H | CF₃ | Me | OCH₂CF₃ | 0 |
| 1820. | NO₂ | H | CF₃ | Me | OCH₂CF₃ | 1 |
| 1821. | NO₂ | H | CF₃ | Me | OCH₂CF₃ | 2 |
| 1822. | NO₂ | H | CF₃ | Me | SO₂Me | 0 |
| 1823. | NO₂ | H | CF₃ | Me | SO₂Me | 1 |
| 1824. | NO₂ | | H CF₃ | Me | SO₂Me | 2 |
| 1825. | NO₂ | H | CF₃ | Me | SEt | 0 |
| 1826. | NO₂ | H | CF₃ | Me | SEt | 1 |
| 1827. | NO₂ | H | CF₃ | Me | SEt | 2 |
| 1828. | NO₂ | H | CF₃ | Et | Cl | 0 |
| 1829. | NO₂ | H | CF₃ | Et | Cl | 1 |
| 1830. | NO₂ | H | CF₃ | Et | Cl | 2 |
| 1831 | NO₂ | H | CF₃ | iPr | Cl | 0 |
| 1832. | NO₂ | H | CF₃ | iPr | Cl | 1 |
| 1833. | NO₂ | H | CF₃ | iPr | Cl | 2 |
| 1834. | NO₂ | H | CF₃ | tBu | Cl | 0 |
| 1835. | NO₂ | H | CF₃ | tBu | Cl | 1 |
| 1836. | NO₂ | H | CF₃ | tBu | Cl | 2 |
| 1837. | NO₂ | H | Cl | tBu | CF₃ | 0 |
| 1838. | NO₂ | H | Cl | tBu | CF₃ | 1 |
| 1839. | NO₂ | H | Cl | tBu | CF₃ | 2 |
| 1840. | NO₂ | H | CF₃ | cPen | Cl | 0 |
| 1841. | NO₂ | H | CF₃ | cPen | Cl | 1 |
| 1842. | NO₂ | H | CF₃ | cPen | Cl | 2 |
| 1843. | NO₂ | H | CF₃ | CHF₂ | OMe | 0 |
| 1844. | NO₂ | H | CF₃ | CHF₂ | OMe | 1 |
| 1845. | NO₂ | H | CF₃ | CHF₂ | OMe | 2 |
| 1846. | NO₂ | H | CF₃ | CH₂CF₃ | Cl | 0 |
| 1847. | NO₂ | H | CF₃ | CH₂CF₃ | Cl | 1 |
| 1848. | NO₂ | H | CF₃ | CH₂CF₃ | Cl | 2 |
| 1849. | NO₂ | H | CF₃ | Ph | OCHF₂ | 0 |
| 1850. | NO₂ | H | CF₃ | Ph | OCHF₂ | 1 |
| 1851. | NO₂ | H | CF₃ | Ph | OCHF₂ | 2 |
| 1852. | NO₂ | H | CF₃ | Ph | Cl | 0 |
| 1853. | NO₂ | H | CF₃ | Ph | Cl | 1 |
| 1854. | NO₂ | H | CF₃ | Ph | Cl | 2 |
| 1855. | NO₂ | H | Me | Me | OCH₂CF₃ | 0 |
| 1856. | NO₂ | H | Me | Me | OCH₂CF₃ | 1 |
| 1857. | NO₂ | H | Me | Me | OCH₂CF₃ | 2 |
| 1858. | NO₂ | H | CF₃ | Me | 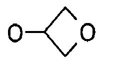 | 0 |
| 1859. | NO₂ | H | CF₃ | Me | 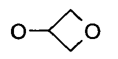 | 1 |
| 1860. | NO₂ | H | CF₃ | Me | 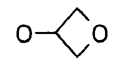 | 2 |
| 1861. | NO₂ | H | CF₃ | Me | H | 0 |
| 1862. | NO₂ | H | CF₃ | Me | H | 1 |
| 1863. | NO₂ | H | CF₃ | Me | H | 2 |
| 1864. | NO₂ | H | CF₃ | Me | OCH₂CH₂OMe | 0 |
| 1865. | NO₂ | H | CF₃ | Me | OCH₂CH₂OMe | 1 |
| 1866. | NO₂ | H | CF₃ | Me | OCH₂CH₂OMe | 2 |
| 1867. | NO₂ | H | CF₃ | Me | SMe | 0 |
| 1868. | NO₂ | H | CF₃ | Me | SMe | 1 |
| 1869. | NO₂ | H | CF₃ | Me | SMe | 2 |
| 1870. | NO₂ | H | CF₃ | Me | OCH₂CH₂CH₂F | 0 |
| 1871. | NO₂ | H | CF₃ | Me | OCH₂CH₂CH₂F | 1 |
| 1872. | NO₂ | H | CF₃ | Me | OCH₂CH₂CH₂F | 2 |
| 1873. | NO₂ | H | CF₃ | Me | OCH(CH₂F)₂ | 0 |
| 1874. | NO₂ | H | CF₃ | Me | OCH(CH₂F)₂ | 1 |
| 1875. | NO₂ | H | CF₃ | Me | OCH(CH₂F)₂ | 2 |
| 1876. | NO₂ | H | CF₃ | Me | OCH₂CF₂CHF₂ | 0 |
| 1877. | NO₂ | H | CF₃ | Me | OCH₂CF₂CHF₂ | 1 |
| 1878. | NO₂ | H | CF₃ | Me | OCH₂CF₂CHF₂ | 2 |
| 1879. | NO₂ | H | CF₃ | Me | OCH₂CF=CH₂ | 0 |
| 1880. | NO₂ | H | CF₃ | Me | OCH₂CF=CH₂ | 1 |
| 1881. | NO₂ | H | CF₃ | Me | OCH₂CF=CH₂ | 2 |
| 1882. | NO₂ | H | CF₃ | Me | OCH(Me)CF₃ | 0 |
| 1883. | NO₂ | H | CF₃ | Me | OCH(Me)CF₃ | 1 |
| 1884. | NO₂ | H | CF₃ | Me | OCH(Me)CF₃ | 2 |
| 1885. | NO₂ | H | CF₃ | Me | OCH(Me)CH₂F | 0 |
| 1886. | NO₂ | H | CF₃ | Me | OCH(Me)CH₂F | 1 |
| 1887. | NO₂ | H | CF₃ | Me | OCH(Me)CH₂F | 2 |
| 1888. | NO₂ | H | OCH₂CF₃ | Me | CF₃ | 0 |
| 1889. | NO₂ | H | OCH₂CF₃ | Me | CF₃ | 1 |
| 1890. | NO₂ | H | OCH₂CF₃ | Me | CF₃ | 2 |
| 1891. | NO₂ | H | OCH₂CF₃ | Me | CHF₂ | 0 |
| 1892. | NO₂ | H | OCH₂CF₃ | Me | CHF₂ | 1 |
| 1893. | NO₂ | H | OCH₂CF₃ | Me | CHF₂ | 2 |
| 1894. | NO₂ | H | CHF₂ | Me | CHF₂ | 0 |
| 1895. | NO₂ | H | CHF₂ | Me | CHF₂ | 1 |
| 1896. | NO₂ | H | CHF₂ | Me | CHF₂ | 2 |
| 1897. | NO₂ | H | CF₃ | Me | CHF₂ | 0 |
| 1898. | NO₂ | H | CF₃ | Me | CHF₂ | 1 |
| 1899. | NO₂ | H | CF₃ | Me | CHF₂ | 2 |
| 1900. | NO₂ | H | Cl | Me | OCHF₂ | 0 |
| 1901. | NO₂ | H | Cl | Me | OCHF₂ | 1 |
| 1902. | NO₂ | H | Cl | Me | OCHF₂ | 2 |
| 1903. | NO₂ | H | Br | Me | OCHF₂ | 0 |
| 1904. | NO₂ | H | Br | Me | OCHF₂ | 1 |
| 1905. | NO₂ | H | Br | Me | OCHF₂ | 2 |
| 1906. | NO₂ | H | Br | Me | CF₃ | 0 |
| 1907. | NO₂ | H | Br | Me | CF₃ | 1 |
| 1908. | NO₂ | H | Br | Me | CF₃ | 2 |
| 1909. | Cl | Cl | CF₃ | tBu | Cl | 0 |
| 1910. | Cl | Cl | CF₃ | tBu | Cl | 1 |
| 1911. | Cl | Cl | CF₃ | tBu | Cl | 2 |
| 1912. | CI | Cl | CF₃ | CHF₂ | Cl | 0 |
| 1913. | Cl | Cl | CF₃ | CHF₂ | Cl | 1 |
| 1914. | Cl | Cl | CF₃ | CHF₂ | Cl | 2 |
| 1915. | Cl | Cl | Cl | CHF₂ | CF₃ | 0 |
| 1916. | Cl | Cl | Cl | CHF₂ | CF₃ | 1 |
| 1917. | Cl | Cl | Cl | CHF₂ | CF₃ | 2 |
| 1918. | Cl | Cl | CF₃ | Me | OMe | 0 |
| 1919. | Cl | Cl | CF₃ | Me | OMe | 1 |
| 1920. | Cl | Cl | CF₃ | Me | OMe | 2 |
| 1921. | Cl | Cl | CF₃ | Me | CN | 0 |
| 1922. | Cl | Cl | CF₃ | Me | CN | 1 |
| 1923. | Cl | Cl | CF₃ | Me | CN | 2 |
| 1924. | Cl | Cl | CHF₂ | Me | Cl | 0 |
| 1925. | Cl | Cl | CHF₂ | Me | Cl | 1 |
| 1926. | Cl | Cl | CHF₂ | Me | Cl | 2 |
| 1927. | Cl | Cl | Me | Me | Me | 0 |
| 1928. | Cl | Cl | Me | Me | Me | 1 |
| 1929. | Cl | Cl | Me | Me | Me | 2 |
| 1930. | Cl | Cl | Me | Me | Cl | 0 |
| 1931. | Cl | Cl | Me | Me | Cl | 1 |
| 1932. | Cl | Cl | Me | Me | Cl | 2 |
| 1933. | Cl | Cl | CF₃ | Me | Cl | 0 |
| 1934. | Cl | Cl | CF₃ | Me | Cl | 1 |
| 1935. | Cl | Cl | CF₃ | Me | Cl | 2 |
| 1936. | Cl | Cl | Cl | Me | CF₃ | 0 |
| 1937. | Cl | Cl | Cl | Me | CF₃ | 1 |
| 1938. | Cl | Cl | Cl | Me | CF₃ | 2 |
| 1939. | Cl | Cl | CF₃ | Me | F | 0 |
| 1940. | Cl | Cl | CF₃ | Me | F | 1 |
| 1941. | Cl | Cl | CF₃ | Me | F | 2 |
| 1942. | Cl | Cl | OMe | Me | CF₃ | 0 |
| 1943. | Cl | Cl | OMe | Me | CF₃ | 1 |
| 1944. | Cl | Cl | OMe | Me | CF₃ | 2 |
| 1945. | Cl | Cl | CF₃ | Me | OCHF₂ | 0 |
| 1946. | Cl | Cl | CF₃ | Me | OCHF₂ | 1 |
| 1947. | Cl | Cl | CF₃ | Me | OCHF₂ | 2 |
| 1948. | Cl | Cl | OCHF₂ | Me | CF₃ | 0 |
| 1949. | Cl | Cl | OCHF₂ | Me | CF₃ | 1 |
| 1950. | Cl | Cl | OCHF₂ | Me | CF₃ | 2 |
| 1951. | Cl | Cl | CF₃ | Me | OCH₂CHF₂ | 0 |
| 1952. | Cl | Cl | CF₃ | Me | OCH₂CHF₂ | 1 |
| 1953. | Cl | Cl | CF₃ | Me | OCH₂CHF₂ | 2 |
| 1954. | Cl | Cl | CF₃ | Me | OCH₂CF₃ | 0 |
| 1955. | Cl | Cl | CF₃ | Me | OCH₂CF₃ | 1 |
| 1956. | Cl | Cl | CF₃ | Me | OCH₂CF₃ | 2 |
| 1957. | Cl | Cl | CF₃ | Me | SO₂Me | 0 |
| 1958. | Cl | Cl | CF₃ | Me | SO₂Me | 1 |
| 1959. | Cl | Cl | CF₃ | Me | SO₂Me | 2 |
| 1960. | Cl | Cl | CF₃ | Me | SEt | 0 |
| 1961. | Cl | Cl | CF₃ | Me | SEt | 1 |
| 1962. | Cl | Cl | CF₃ | Me | SEt | 2 |
| 1963. | Cl | Cl | CF₃ | Et | Cl | 0 |
| 1964. | Cl | Cl | CF₃ | Et | Cl | 1 |
| 1965. | Cl | Cl | CF₃ | Et | Cl | 2 |
| 1966. | Cl | Cl | CF₃ | iPr | Cl | 0 |
| 1967. | Cl | Cl | CF₃ | iPr | Cl | 1 |
| 1968. | Cl | Cl | CF₃ | iPr | Cl | 2 |
| 1969. | Cl | Cl | CF₃ | tBu | Cl | 0 |
| 1970. | Cl | Cl | CF₃ | tBu | Cl | 1 |
| 1971. | Cl | Cl | CF₃ | tBu | Cl | 2 |
| 1972. | Cl | Cl | Cl | tBu | CF₃ | 0 |
| 1973. | Cl | Cl | Cl | tBu | CF₃ | 1 |
| 1974. | Cl | Cl | Cl | tBu | CF₃ | 2 |
| 1975. | Cl | Cl | CF₃ | cPen | Cl | 0 |
| 1976. | Cl | Cl | CF₃ | cPen | Cl | 1 |
| 1977. | Cl | Cl | CF₃ | cPen | Cl | 2 |
| 1978. | Cl | Cl | CF₃ | CHF₂ | OMe | 0 |
| 1979. | Cl | Cl | CF₃ | CHF₂ | OMe | 1 |
| 1980. | Cl | Cl | CF₃ | CHF₂ | OMe | 2 |
| 1981. | Cl | Cl | CF₃ | CH₂CF₃ | Cl | 0 |
| 1982. | Cl | Cl | CF₃ | CH₂CF₃ | Cl | 1 |
| 1983. | Cl | Cl | CF₃ | CH₂CF₃ | Cl | 2 |
| 1984. | Cl | Cl | CF₃ | Ph | OCHF₂ | 0 |
| 1985. | Cl | Cl | CF₃ | Ph | OCHF₂ | 1 |
| 1986. | Cl | Cl | CF₃ | Ph | OCHF₂ | 2 |
| 1987. | Cl | Cl | CF₃ | Ph | Cl | 0 |
| 1988. | Cl | Cl | CF₃ | Ph | Cl | 1 |
| 1989. | Cl | Cl | CF₃ | Ph | Cl | 2 |
| 1990. | Cl | Cl | Me | Me | OCH₂CF₃ | 0 |
| 1991. | Cl | Cl | Me | Me | OCH₂CF₃ | 1 |
| 1992. | Cl | Cl | Me | Me | OCH₂CF₃ | 2 |
| 1993. | Cl | Cl | CF₃ | Me | 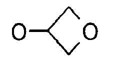 | 0 |
| 1994. | Cl | Cl | CF₃ | Me | 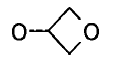 | 1 |
| 1995. | Cl | Cl | CF₃ | Me | 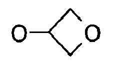 | 2 |
| 1996. | Cl | Cl | CF₃ | Me | H | 0 |
| 1997. | Cl | Cl | CF₃ | Me | H | 1 |
| 1998. | Cl | Cl | CF₃ | Me | H | 2 |
| 1999. | Cl | Cl | CF₃ | Me | OCH₂CH₂OMe | 0 |
| 2000. | Cl | Cl | CF₃ | Me | OCH₂CH₂OMe | 1 |
| 2001. | Cl | Cl | CF₃ | Me | OCH₂CH₂OMe | 2 |
| 2002. | Cl | Cl | CF₃ | Me | SMe | 0 |
| 2003. | Cl | Cl | CF₃ | Me | SMe | 1 |
| 2004. | Cl | Cl | CF₃ | Me | SMe | 2 |
| 2005. | Cl | Cl | CF₃ | Me | OCH₂CH₂CH₂F | 0 |
| 2006. | Cl | Cl | CF₃ | Me | OCH₂CH₂CH₂F | 1 |
| 2007. | Cl | Cl | CF₃ | Me | OCH₂CH₂CH₂F | 2 |
| 2008. | Cl | Cl | CF₃ | Me | OCH(CH₂F)₂ | 0 |
| 2009. | Cl | Cl | CF₃ | Me | OCH(CH₂F)₂ | 1 |
| 2010. | Cl | Cl | CF₃ | Me | OCH(CH₂F)₂ | 2 |
| 2011. | Cl | Cl | CF₃ | Me | OCH₂CF₂CHF₂ | 0 |
| 2012. | Cl | Cl | CF₃ | Me | OCH₂CF₂CHF₂ | 1 |
| 2013. | Cl | Cl | CF₃ | Me | OCH₂CF₂CHF₂ | 2 |
| 2014. | Cl | Cl | CF₃ | Me | OCH₂CF=CH₂ | 0 |
| 2015. | Cl | Cl | CF₃ | Me | OCH₂CF=CH₂ | 1 |
| 2016. | Cl | Cl | CF₃ | Me | OCH₂CF=CH₂ | 2 |
| 2017. | Cl | Cl | CF₃ | Me | OCH(Me)CF₃ | 0 |
| 2018. | Cl | Cl | CF₃ | Me | OCH(Me)CF₃ | 1 |
| 2019. | Cl | Cl | CF₃ | Me | OCH(Me)CF₃ | 2 |
| 2020. | Cl | Cl | CF₃ | Me | OCH(Me)CH₂F | 0 |
| 2021. | Cl | Cl | CF₃ | Me | OCH(Me)CH₂F | 1 |
| 2022. | Cl | Cl | CF₃ | Me | OCH(Me)CH₂F | 2 |
| 2023. | Cl | Cl | OCH₂CF₃ | Me | CF₃ | 0 |
| 2024. | Cl | Cl | OCH₂CF₃ | Me | CF₃ | 1 |
| 2025. | Cl | Cl | OCH₂CF₃ | Me | CF₃ | 2 |
| 2026. | Cl | Cl | OCH₂CF₃ | Me | CHF₂ | 0 |
| 2027. | Cl | Cl | OCH₂CF₃ | Me | CHF₂ | 1 |
| 2028. | Cl | Cl | OCH₂CF₃ | Me | CHF₂ | 2 |
| 2029. | Cl | Cl | CHF₂ | Me | CHF₂ | 0 |
| 2030. | Cl | Cl | CHF₂ | Me | CHF₂ | 1 |
| 2031. | Cl | Cl | CHF₂ | Me | CHF₂ | 2 |
| 2032. | Cl | Cl | CF₃ | Me | CHF₂ | 0 |
| 2033. | Cl | Cl | CF₃ | Me | CHF₂ | 1 |
| 2034. | Cl | Cl | CF₃ | Me | CHF₂ | 2 |
| 2035. | Cl | Cl | Cl | Me | OCHF₂ | 0 |
| 2036. | Cl | Cl | Cl | Me | OCHF₂ | 1 |
| 2037. | Cl | Cl | Cl | Me | OCHF₂ | 2 |
| 2038. | Cl | Cl | Br | Me | OCHF₂ | 0 |
| 2039. | Cl | Cl | Br | Me | OCHF₂ | 1 |
| 2040. | Cl | Cl | Br | Me | OCHF₂ | 2 |
| 2041. | Cl | Cl | Br | Me | CF₃ | 0 |
| 2042. | Cl | Cl | Br | Me | CF₃ | 1 |
| 2043. | Cl | Cl | Br | Me | CF₃ | 2 |
| 2044. | Cl | Me | CF₃ | tBu | Cl | 0 |
| 2045. | Cl | Me | CF₃ | tBu | Cl | 1 |
| 2046. | Cl | Me | CF₃ | tBu | Cl | 2 |
| 2047. | Cl | Me | CF₃ | CHF₂ | Cl | 0 |
| 2048. | Cl | Me | CF₃ | CHF₂ | Cl | 1 |
| 2049. | Cl | Me | CF₃ | CHF₂ | Cl | 2 |
| 2050. | Cl | Me | Cl | CHF₂ | CF₃ | 0 |
| 2051. | Cl | Me | Cl | CHF₂ | CF₃ | 1 |
| 2052. | Cl | Me | Cl | CHF₂ | CF₃ | 2 |
| 2053. | Cl | Me | CF₃ | Me | OMe | 0 |
| 2054. | Cl | Me | CF₃ | Me | OMe | 1 |
| 2055. | Cl | Me | CF₃ | Me | OMe | 2 |
| 2056. | Cl | Me | CF₃ | Me | CN | 0 |
| 2057. | Cl | Me | CF₃ | Me | CN | 1 |
| 2058. | Cl | Me | CF₃ | Me | CN | 2 |
| 2059. | Cl | Me | CHF₂ | Me | Cl | 0 |
| 2060. | Cl | Me | CHF₂ | Me | Cl | 1 |
| 2061. | Cl | Me | CHF₂ | Me | Cl | 2 |
| 2062. | Cl | Me | Me | Me | Me | 0 |
| 2063. | Cl | Me | Me | Me | Me | 1 |
| 2064. | Cl | Me | Me | Me | Me | 2 |
| 2065. | Cl | Me | Me | Me | Cl | 0 |
| 2066. | Cl | Me | Me | Me | Cl | 1 |
| 2067. | Cl | Me | Me | Me | Cl | 2 |
| 2068. | Cl | Me | CF₃ | Me | Cl | 0 |
| 2069. | Cl | Me | CF₃ | Me | Cl | 1 |
| 2070. | Cl | Me | CF₃ | Me | Cl | 2 |
| 2071. | Cl | Me | Cl | Me | CF₃ | 0 |
| 2072. | Cl | Me | Cl | Me | CF₃ | 1 |
| 2073. | Cl | Me | Cl | Me | CF₃ | 2 |
| 2074. | Cl | Me | CF₃ | Me | F | 0 |
| 2075. | Cl | Me | CF₃ | Me | F | 1 |
| 2076. | Cl | Me | CF₃ | Me | F | 2 |
| 2077. | Cl | Me | OMe | Me | CF₃ | 0 |
| 2078. | Cl | Me | OMe | Me | CF₃ | 1 |
| 2079. | Cl | Me | OMe | Me | CF₃ | 2 |
| 2080. | Cl | Me | CF₃ | Me | OCHF₂ | 0 |
| 2081. | Cl | Me | CF₃ | Me | OCHF₂ | 1 |
| 2082. | Cl | Me | CF₃ | Me | OCHF₂ | 2 |
| 2083. | Cl | Me | OCHF₂ | Me | CF₃ | 0 |
| 2084. | Cl | Me | OCHF₂ | Me | CF₃ | 1 |
| 2085. | Cl | Me | OCHF₂ | Me | CF₃ | 2 |
| 2086. | Cl | Me | CF₃ | Me | OCH₂CHF₂ | 0 |
| 2087. | Cl | Me | CF₃ | Me | OCH₂CHF₂ | 1 |
| 2088. | Cl | Me | CF₃ | Me | OCH₂CHF₂ | 2 |
| 2089. | Cl | Me | CF₃ | Me | OCH₂CF₃ | 0 |
| 2090. | Cl | Me | CF₃ | Me | OCH₂CF₃ | 1 |
| 2091. | Cl | Me | CF₃ | Me | OCH₂CF₃ | 2 |
| 2092. | Cl | Me | CF₃ | Me | SO₂Me | 0 |
| 2093. | Cl | Me | CF₃ | Me | SO₂Me | 1 |
| 2094. | Cl | Me | CF₃ | Me | SO₂Me | 2 |
| 2095. | Cl | Me | CF₃ | Me | SEt | 0 |
| 2096. | Cl | Me | CF₃ | Me | SEt | 1 |
| 2097. | Cl | Me | CF₃ | Me | SEt | 2 |
| 2098. | Cl | Me | CF₃ | Et | Cl | 0 |
| 2099. | Cl | Me | CF₃ | Et | Cl | 1 |
| 2100. | Cl | Me | CF₃ | Et | Cl | 2 |
| 2101. | Cl | Me | CF₃ | iPr | Cl | 0 |
| 2102. | Cl | Me | CF₃ | iPr | Cl | 1 |
| 2103. | Cl | Me | CF₃ | iPr | Cl | 2 |
| 2104. | Cl | Me | CF₃ | tBu | Cl | 0 |
| 2105. | Cl | Me | CF₃ | tBu | Cl | 1 |
| 2106. | Cl | Me | CF₃ | tBu | Cl | 2 |
| 2107. | Cl | Me | Cl | tBu | CF₃ | 0 |
| 2108. | Cl | Me | Cl | tBu | CF₃ | 1 |
| 2109. | Cl | Me | Cl | tBu | CF₃ | 2 |
| 2110. | Cl | Me | CF₃ | cPen | Cl | 0 |
| 2111. | Cl | Me | CF₃ | cPen | Cl | 1 |
| 2112. | Cl | Me | CF₃ | cPen | Cl | 2 |
| 2113. | Cl | Me | CF₃ | CHF₂ | OMe | 0 |
| 2114. | Cl | Me | CF₃ | CHF₂ | OMe | 1 |
| 2115. | Cl | Me | CF₃ | CHF₂ | OMe | 2 |
| 2116. | Cl | Me | CF₃ | CH₂CF₃ | Cl | 0 |
| 2117. | Cl | Me | CF₃ | CH₂CF₃ | Cl | 1 |
| 2118. | Cl | Me | CF₃ | CH₂CF₃ | Cl | 2 |
| 2119. | Cl | Me | CF₃ | Ph | OCHF₂ | 0 |
| 2120. | Cl | Me | CF₃ | Ph | OCHF₂ | 1 |
| 2121. | Cl | Me | CF₃ | Ph | OCHF₂ | 2 |
| 2122. | Cl | Me | CF₃ | Ph | Cl | 0 |
| 2123. | Cl | Me | CF₃ | Ph | Cl | 1 |
| 2124. | Cl | Me | CF₃ | Ph | Cl | 2 |
| 2125. | Cl | Me | Me | Me | OCH₂CF₃ | 0 |
| 2126. | Cl | Me | Me | Me | OCH₂CF₃ | 1 |
| 2127. | Cl | Me | Me | Me | OCH₂CF₃ | 2 |
| 2128. | Cl | Me | CF₃ | Me | 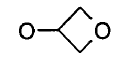 | 0 |
| 2129. | Cl | Me | CF₃ | Me | 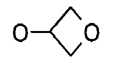 | 1 |
| 2130. | Cl | Me | CF₃ | Me | 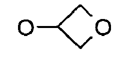 | 2 |
| 2131. | Cl | Me | CF₃ | Me | H | 0 |
| 2132. | Cl | Me | CF₃ | Me | H | 1 |
| 2133. | Cl | Me | CF₃ | Me | H | 2 |
| 2134. | Cl | Me | CF₃ | Me | OCH₂CH₂OMe | 0 |
| 2135. | Cl | Me | CF₃ | Me | OCH₂CH₂OMe | 1 |
| 2136. | Cl | Me | CF₃ | Me | OCH₂CH₂OMe | 2 |
| 2137. | Cl | Me | CF₃ | Me | SMe | 0 |
| 2138. | Cl | Me | CF₃ | Me | SMe | 1 |
| 2139. | Cl | Me | CF₃ | Me | SMe | 2 |
| 2140. | Cl | Me | CF₃ | Me | OCH₂CH₂CH₂F | 0 |
| 2141. | Cl | Me | CF₃ | Me | OCH₂CH₂CH₂F | 1 |
| 2142. | Cl | Me | CF₃ | Me | OCH₂CH₂CH₂F | 2 |
| 2143. | Cl | Me | CF₃ | Me | OCH(CH₂F)₂ | 0 |
| 2144. | Cl | Me | CF₃ | Me | OCH(CH₂F)₂ | 1 |
| 2145. | Cl | Me | CF₃ | Me | OCH(CH₂F)₂ | 2 |
| 2146. | Cl | Me | CF₃ | Me | OCH₂CF₂CHF₂ | 0 |
| 2147. | Cl | Me | CF₃ | Me | OCH₂CF₂CHF₂ | 1 |
| 2148. | Cl | Me | CF₃ | Me | OCH₂CF₂CHF₂ | 2 |
| 2149. | Cl | Me | CF₃ | Me | OCH₂CF=CH₂ | 0 |
| 2150. | Cl | Me | CF₃ | Me | OCH₂CF=CH₂ | 1 |
| 2151. | Cl | Me | CF₃ | Me | OCH₂CF=CH₂ | 2 |
| 2152. | Cl | Me | CF₃ | Me | OCH(Me)CF₃ | 0 |
| 2153. | Cl | Me | CF₃ | Me | OCH(Me)CF₃ | 1 |
| 2154. | Cl | Me | CF₃ | Me | OCH(Me)CF₃ | 2 |
| 2155. | Cl | Me | CF₃ | Me | OCH(Me)CH₂F | 0 |
| 2156. | Cl | Me | CF₃ | Me | OCH(Me)CH₂F | 1 |
| 2157. | Cl | Me | CF₃ | Me | OCH(Me)CH₂F | 2 |
| 2158. | Cl | Me | OCH₂CF₃ | Me | CF₃ | 0 |
| 2159. | Cl | Me | OCH₂CF₃ | Me | CF₃ | 1 |
| 2160. | Cl | Me | OCH₂CF₃ | Me | CF₃ | 2 |
| 2161. | Cl | Me | OCH₂CF₃ | Me | CHF₂ | 0 |
| 2162. | Cl | Me | OCH₂CF₃ | Me | CHF₂ | 1 |
| 2163. | Cl | Me | OCH₂CF₃ | Me | CHF₂ | 2 |
| 2164. | Cl | Me | CHF₂ | Me | CHF₂ | 0 |
| 2165. | Cl | Me | CHF₂ | Me | CHF₂ | 1 |
| 2166. | Cl | Me | CHF₂ | Me | CHF₂ | 2 |
| 2167. | Cl | Me | CF₃ | Me | CHF₂ | 0 |
| 2168. | Cl | Me | CF₃ | Me | CHF₂ | 1 |
| 2169. | Cl | Me | CF₃ | Me | CHF₂ | 2 |
| 2170. | Cl | Me | Cl | Me | OCHF₂ | 0 |
| 2171. | Cl | Me | Cl | Me | OCHF₂ | 1 |
| 2172. | Cl | Me | Cl | Me | OCHF₂ | 2 |
| 2173. | Cl | Me | Br | Me | OCHF₂ | 0 |
| 2174. | Cl | Me | Br | Me | OCHF₂ | 1 |
| 2175. | Cl | Me | Br | Me | OCHF₂ | 2 |
| 2176. | Cl | Me | Br | Me | CF₃ | 0 |
| 2177. | Cl | Me | Br | Me | CF₃ | 1 |
| 2178. | Cl | Me | Br | Me | CF₃ | 2 |

NMR Daten augewählten Verbindungen der Tabelle 1:
5-Chlor-2-({[5-(difluormethoxy)-1-methyl-3-(trifluormethyl)-1H-pyrazol-4-yl]methyl}sulfinyl)-1,3-oxazol
   Verbindung Nr. 506: NMR (CDCl₃, 400 MHz): 3.87 (s, 3H, CH₃); 4.38 (d, 1H, S(O)CH₂); 4.50 (d, 1H, S(O)CH₂); 6.92 (dd, 1H, OCHF₂); 7.16 (s, 1H).
5-Chlor-2-({[5-(difluormethoxy)-1-methyl-3-(trifluormethyl)-1H-pyrazol-4-yl]methyl}sulfonyl)-1,3-oxazol
   Verbindung Nr. 507: NMR (CDCl₃, 400 MHz): 3.88 (s, 3H, CH₃); 4.59 (s, 2H, S(O)₂CH₂); 6.81 (t, 1H, OCHF₂); 7.19 (s, 1H).
5-Brom-2-({[5-(difluormethoxy)-1-methyl-3-(trifluormethyl)-1H-pyrazol-4-yl]methyl}sulfinyl)-1,3-oxazol
   Verbindung Nr. 734: NMR (CDCl₃, 400 MHz): 3.85 (s, 3H, CH₃); 4.38 (d, 1H, S(O)CH₂); 4.49 (d, 1H, S(O)CH₂); 6.91 (dd, 1H, OCHF₂); 7.24 (s, 1H).
5-Brom-2-({[5-(difluormethoxy)-1-methyl-3-(trifluormethyl)-1H-pyrazol-4-yl]methyl}sulfonyl)-1,3-oxazol
   Verbindung Nr. 735: NMR (CDCl₃, 400 MHz): 3.88 (s, 3H, CH₃); 4.59 (s, 2H, S(O)₂CH₂); 6.81 (t, 1H, OCHF₂); 7.28 (s, 1H).
5-lod-2-({[5-(difluormethoxy)-1-methyl-3-(trifluormethyl)-1H-pyrazol-4-yl]methyl}sulfinyl)-1,3-oxazol
   Verbindung Nr. 962: NMR (CDCl₃, 400 MHz): 3.85 (s, 3H, CH₃); 4.37 (d, 1H, S(O)CH₂); 4.48 (d, 1H, S(O)CH₂); 6.92 (dd, 1H, OCHF₂); 7.38 (s, 1H).
5-lod-2-({[5-(difluormethoxy)-1-methyl-3-(trifluormethyl)-1H-pyrazol-4-yl]methyl}sulfonyl)-1,3-oxazol
   Verbindung Nr. 963: NMR (CDCl₃, 400 MHz): 3.88 (s, 3H, CH₃); 4.59 (s, 2H, S(O)₂CH₂); 6.81 (t, 1H, OCHF₂); 7.40 (s, 1H).
2-({[5-(Chlor)-1-methyl-3-(trifluormethyl)-1H-pyrazol-4-yl]methyl}sulfinyl)-1,3-oxazol Verbindung Nr. 35: NMR (CDCl₃, 400 MHz): 4.00 (s, 3H, CH₃); 4.50 (br s, 2H, S(O)CH₂); 7.36 (br d, 1H); 7.91 (br d, 1H).
2-({[5-(Chlor)-1-methyl-3-(trifluormethyl)-1H-pyrazol-4-yl]methyl}sulfonyl)-1,3-oxazol Verbindung Nr. 36: NMR (CDCl₃, 400 MHz): 3.20 (s, 3H, CH₃); 4.61 (s, 2H, S(O)₂CH₂); 7.40 (br d, 1H); 7.89 (br d, 1H).

**Tabelle 2: Verbindungen der Formel (III-S)**

| | | | | | |
|---|---|---|---|---|---|
| 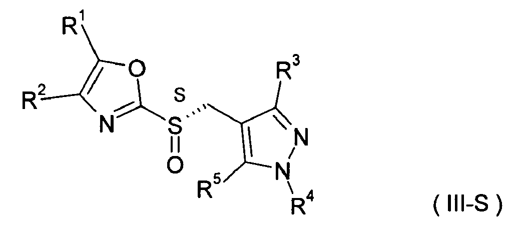 | | | | | |

| Bsp. Nr. | R¹ | R² | R³ | R⁴ | R⁵ |
|---|---|---|---|---|---|
| 2179. | H | H | CF₃ | Ph | Cl |
| 2180. | H | H | CF₃ | tBu | Cl |
| 2181. | H | H | CF₃ | CHF₂ | Cl |
| 2182. | H | H | Cl | CHF₂ | CF₃ |
| 2183. | H | H | CF₃ | Me | OMe |
| 2184. | H | H | CF₃ | Me | CN |
| 2185. | H | H | Cl | Et | Cl |
| 2186. | H | H | CHF₂ | Me | Cl |
| 2187. | H | H | Me | Me | Me |
| 2188. | H | H | Me | Me | Cl |
| 2189. | H | H | Cl | Me | Cl |
| 2190. | H | H | CF₃ | Me | Cl |
| 2191. | H | H | Cl | Me | CF₃ |
| 2192. | H | H | CF₃ | Me | F |
| 2193. | H | H | OMe | Me | CF₃ |
| 2194. | H | H | CF₃ | Me | OEt |
| 2195. | H | H | CF₃ | Me | OCHF₂ |
| 2196. | H | H | OCHF₂ | Me | CF₃ |
| 2197. | H | H | CF₃ | Me | OCH₂CHF₂ |
| 2198. | H | H | CF₃ | Me | OCH₂CF₃ |
| 2199. | H | H | CF₃ | Me | OCH₂CN |
| 2200. | H | H | CF₃ | Me | SO₂Me |
| 2201. | H | H | CF₃ | Me | SEt |
| 2202. | H | H | CF₃ | Me | Me |
| 2203. | H | H | CF₃ | Me | Et |
| 2204. | H | H | CF₃ | Et | Cl |
| 2205. | H | H | Cl | Et | CF₃ |
| 2206. | H | H | CF₃ | iPr | Cl |
| 2207. | H | H | Cl | iPr | CF₃ |
| 2208. | H | H | CF₃ | tBu | Cl |
| 2209. | H | H | Cl | tBu | CF₃ |
| 2210. | H | H | CF₃ | cPen | Cl |
| 2211. | H | H | Cl | cPen | CF₃ |
| 2212. | H | H | CF₃ | CH₂cPr | Cl |
| 2213. | H | H | Cl | CH₂cPr | CF₃ |
| 2214. | H | H | CF₃ | CH₂CH=CH₂ | Cl |
| 2215. | H | H | Cl | CH₂CH=CH₂ | CF₃ |
| 2216. | H | H | CF₃ | CHF₂ | OMe |
| 2217. | H | H | OMe | CHF₂ | CF₃ |
| 2218. | H | H | CF₃ | CH₂CF₃ | Cl |
| 2219. | H | H | Cl | CH₂CF₃ | CF₃ |
| 2220. | H | H | CF₃ | CH₂OMe | Cl |
| 2221. | H | H | Cl | CH₂OMe | CF₃ |
| 2222. | H | H | CF₃ | CH₂CN | Cl |
| 2223. | H | H | Me | Ph | Me |
| 2224. | H | H | Me | Ph | Cl |
| 2225. | H | H | Et | Ph | Cl |
| 2226. | H | H | Pr | Ph | Cl |
| 2227. | H | H | iPr | Ph | Cl |
| 2228. | H | H | CF₃ | Ph | Cl |
| 2229. | H | H | CF₃ | Ph | Me |
| 2230. | H | H | CF₃ | Ph | CF₃ |
| 2231. | H | H | CF₃ | Ph | F |
| 2232. | H | H | CF₃ | Ph | OMe |
| 2233. | H | H | CF₃ | Ph | OEt |
| 2234. | H | H | CF₃ | Ph | OCHF₂ |
| 2235. | H | H | CF₃ | Ph | CN |
| 2236. | H | H | CF₃ | Ph(4-Cl) | Cl |
| 2237. | H | H | Me | Me | OCH₂CF₃ |
| 2238. | H | H | CF₃ | Me | 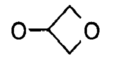 |
| 2239. | H | H | CF₃ | Me | H |
| 2240. | H | H | CF₃ | Me | OCH₂CH₂OMe |
| 2241. | H | H | CF₃ | Me | SMe |
| 2242. | H | H | CF₃ | Me | OCH₂CH₂CH₂F |
| 2243. | H | H | CF₃ | Me | OCH(CH₂F)₂ |
| 2244. | H | H | CF₃ | Me | OCH₂CF₂CHF₂ |
| 2245. | H | H | CF₃ | Me | OCH₂CF=CH₂ |
| 2246. | H | H | CF₃ | Me | OCH(Me)CF₃ |
| 2247. | H | H | CF₃ | Me | OCH(Me)CH₂F |
| 2248. | H | H | OCH₂CF₃ | Me | CF₃ |
| 2249. | H | H | OCH₂CF₃ | Me | CHF₂ |
| 2250. | H | H | CHF₂ | Me | CHF₂ |
| 2251. | H | H | CF₃ | Me | CHF₂ |
| 2252. | H | H | Cl | Me | OCHF₂ |
| 2253. | H | H | Br | Me | OCHF₂ |
| 2254. | H | H | Br | Me | CF₃ |
| 2255. | F | H | CF₃ | Ph | Cl |
| 2256. | F | H | CF₃ | tBu | Cl |
| 2257. | F | H | CF₃ | CHF₂ | Cl |
| 2258. | F | H | Cl | CHF₂ | CF₃ |
| 2259. | F | H | CF₃ | Me | OMe |
| 2260. | F | H | CF₃ | Me | CN |
| 2261. | F | H | Cl | Et | Cl |
| 2262. | F | H | CHF₂ | Me | Cl |
| 2263. | F | H | Me | Me | Me |
| 2264. | F | H | Me | Me | Cl |
| 2265. | F | H | Cl | Me | Cl |
| 2266. | F | H | CF₃ | Me | Cl |
| 2267. | F | H | Cl | Me | CF₃ |
| 2268. | F | H | CF₃ | Me | F |
| 2269. | F | H | OMe | Me | CF₃ |
| 2270. | F | H | CF₃ | Me | OEt |
| 2271. | F | H | CF₃ | Me | OCHF₂ |
| 2272. | F | H | OCHF₂ | Me | CF₃ |
| 2273. | F | H | CF₃ | Me | OCH₂CHF₂ |
| 2274. | F | H | CF₃ | Me | OCH₂CF₃ |
| 2275. | F | H | CF₃ | Me | OCH₂CN |
| 2276. | F | H | CF₃ | Me | SO₂Me |
| 2277. | F | H | CF₃ | Me | SEt |
| 2278. | F | H | CF₃ | Me | Me |
| 2279. | F | H | CF₃ | Me | Et |
| 2280. | F | H | CF₃ | Et | Cl |
| 2281. | F | H | Cl | Et | CF₃ |
| 2282. | F | H | CF₃ | iPr | Cl |
| 2283. | F | H | Cl | iPr | CF₃ |
| 2284. | F | H | CF₃ | tBu | Cl |
| 2285. | F | H | Cl | tBu | CF₃ |
| 2286. | F | H | CF₃ | cPen | Cl |
| 2287. | F | H | Cl | cPen | CF₃ |
| 2288. | F | H | CF₃ | CH₂cPr | Cl |
| 2289. | F | H | Cl | CH₂cPr | CF₃ |
| 2290. | F | H | CF₃ | CH₂CH=CH₂ | Cl |
| 2291. | F | H | Cl | CH₂CH=CH₂ | CF₃ |
| 2292. | F | H | CF₃ | CHF₂ | OMe |
| 2293. | F | H | OMe | CHF₂ | CF₃ |
| 2294. | F | H | CF₃ | CH₂CF₃ | Cl |
| 2295. | F | H | Cl | CH₂CF₃ | CF₃ |
| 2296. | F | H | CF₃ | CH₂OMe | Cl |
| 2297. | F | H | Cl | CH₂OMe | CF₃ |
| 2298. | F | H | CF₃ | CH₂CN | Cl |
| 2299. | F | H | Me | Ph | Me |
| 2300. | F | H | Me | Ph | Cl |
| 2301. | F | H | Et | Ph | Cl |
| 2302. | F | H | Pr | Ph | Cl |
| 2303. | F | H | iPr | Ph | Cl |
| 2304. | F | H | CF₃ | Ph | Cl |
| 2305. | F | H | CF₃ | Ph | Me |
| 2306. | F | H | CF₃ | Ph | CF₃ |
| 2307. | F | H | CF₃ | Ph | F |
| 2308. | F | H | CF₃ | Ph | OMe |
| 2309. | F | H | CF₃ | Ph | OEt |
| 2310. | F | H | CF₃ | Ph | OCHF₂ |
| 2311. | F | H | CF₃ | Ph | CN |
| 2312. | F | H | CF₃ | Ph(4-Cl) | Cl |
| 2313. | F | H | Me | Me | OCH₂CF₃ |
| 2314. | F | H | CF₃ | Me | 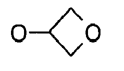 |
| 2315. | F | H | CF₃ | Me | H |
| 2316. | F | H | CF₃ | Me | OCH₂CH₂OMe |
| 2317. | F | H | CF₃ | Me | SMe |
| 2318. | F | H | CF₃ | Me | OCH₂CH₂CH₂F |
| 2319. | F | H | CF₃ | Me | OCH(CH₂F)₂ |
| 2320. | F | H | CF₃ | Me | OCH₂CF₂CHF₂ |
| 2321. | F | H | CF₃ | Me | OCH₂CF=CH₂ |
| 2322. | F | H | CF₃ | Me | OCH(Me)CF₃ |
| 2323. | F | H | CF₃ | Me | OCH(Me)CH₂F |
| 2324. | F | H | OCH₂CF₃ | Me | CF₃ |
| 2325. | F | H | OCH₂CF₃ | Me | CHF₂ |
| 2326. | F | H | CHF₂ | Me | CHF₂ |
| 2327. | F | H | CF₃ | Me | CHF₂ |
| 2328. | F | H | Cl | Me | OCHF₂ |
| 2329. | F | H | Br | Me | OCHF₂ |
| 2330. | F | H | Br | Me | CF₃ |
| 2331. | Cl | H | CF₃ | Ph | Cl |
| 2332. | Cl | H | CF₃ | tBu | Cl |
| 2333. | Cl | H | CF₃ | CHF₂ | Cl |
| 2334. | Cl | H | Cl | CHF₂ | CF₃ |
| 2335. | Cl | H | CF₃ | Me | OMe |
| 2336. | Cl | H | CF₃ | Me | CN |
| 2337. | Cl | H | Cl | Et | Cl |
| 2338. | Cl | H | CHF₂ | Me | Cl |
| 2339. | Cl | H | Me | Me | Me |
| 2340. | Cl | H | Me | Me | Cl |
| 2341. | Cl | H | Cl | Me | Cl |
| 2342. | Cl | H | CF₃ | Me | Cl |
| 2343. | Cl | H | Cl | Me | CF₃ |
| 2344. | Cl | H | CF₃ | Me | F |
| 2345. | Cl | H | OMe | Me | CF₃ |
| 2346. | Cl | H | CF₃ | Me | OEt |
| 2347. | Cl | H | CF₃ | Me | OCHF₂ |
| 2348. | Cl | H | OCHF₂ | Me | CF₃ |
| 2349. | Cl | H | CF₃ | Me | OCH₂CHF₂ |
| 2350. | Cl | H | CF₃ | Me | OCH₂CF₃ |
| 2351. | Cl | H | CF₃ | Me | OCH₂CN |
| 2352. | Cl | H | CF₃ | Me | SO₂Me |
| 2353. | Cl | H | CF₃ | Me | SEt |
| 2354. | Cl | H | CF₃ | Me | Me |
| 2355. | Cl | H | CF₃ | Me | Et |
| 2356. | Cl | H | CF₃ | Et | Cl |
| 2357. | Cl | H | Cl | Et | CF₃ |
| 2358. | Cl | H | CF₃ | iPr | Cl |
| 2359. | Cl | H | Cl | iPr | CF₃ |
| 2360. | Cl | H | CF₃ | tBu | Cl |
| 2361. | Cl | H | Cl | tBu | CF₃ |
| 2362. | Cl | H | CF₃ | cPen | Cl |
| 2363. | Cl | H | Cl | cPen | CF₃ |
| 2364. | Cl | H | CF₃ | CH₂cPr | Cl |
| 2365. | Cl | H | Cl | CH₂cPr | CF₃ |
| 2366. | Cl | H | CF₃ | CH₂CH=CH₂ | Cl |
| 2367. | Cl | H | Cl | CH₂CH=CH₂ | CF₃ |
| 2368. | Cl | H | CF₃ | CHF₂ | OMe |
| 2369. | Cl | H | OMe | CHF₂ | CF₃ |
| 2370. | Cl | H | CF₃ | CH₂CF₃ | Cl |
| 2371. | Cl | H | Cl | CH₂CF₃ | CF₃ |
| 2372. | Cl | H | CF₃ | CH₂OMe | Cl |
| 2373. | Cl | H | Cl | CH₂OMe | CF₃ |
| 2374. | Cl | H | CF₃ | CH₂CN | Cl |
| 2375. | Cl | H | Me | Ph | Me |
| 2376. | Cl | H | Me | Ph | Cl |
| 2377. | Cl | H | Et | Ph | Cl |
| 2378. | Cl | H | Pr | Ph | Cl |
| 2379. | Cl | H | iPr | Ph | Cl |
| 2380. | Cl | H | CF₃ | Ph | Cl |
| 2381. | Cl | H | CF₃ | Ph | Me |
| 2382. | Cl | H | CF₃ | Ph | CF₃ |
| 2383. | Cl | H | CF₃ | Ph | F |
| 2384. | Cl | H | CF₃ | Ph | OMe |
| 2385. | Cl | H | CF₃ | Ph | OEt |
| 2386. | Cl | H | CF₃ | Ph | OCHF₂ |
| 2387. | Cl | H | CF₃ | Ph | CN |
| 2388. | Cl | H | CF₃ | Ph(4-Cl) | Cl |
| 2389. | Cl | H | Me | Me | OCH₂CF₃ |
| 2390. | Cl | H | CF₃ | Me | 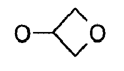 |
| 2391. | Cl | H | CF₃ | Me | H |
| 2392. | Cl | H | CF₃ | Me | OCH₂CH₂OMe |
| 2393. | Cl | H | CF₃ | Me | SMe |
| 2394. | Cl | H | CF₃ | Me | OCH₂CH₂CH₂F |
| 2395. | Cl | H | CF₃ | Me | OCH(CH₂F)₂ |
| 2396. | Cl | H | CF₃ | Me | OCH₂CF₂CHF₂ |
| 2397. | Cl | H | CF₃ | Me | OCH₂CF=CH₂ |
| 2398. | Cl | H | CF₃ | Me | OCH(Me)CF₃ |
| 2399. | Cl | H | CF₃ | Me | OCH(Me)CH₂F |
| 2400. | Cl | H | OCH₂CF₃ | Me | CF₃ |
| 2401. | Cl | H | OCH₂CF₃ | Me | CHF₂ |
| 2402. | Cl | H | CHF₂ | Me | CHF₂ |
| 2403. | Cl | H | CF₃ | Me | CHF₂ |
| 2404. | Cl | H | Cl | Me | OCHF₂ |
| 2405. | Cl | H | Br | Me | OCHF₂ |
| 2406. | Cl | H | Br | Me | CF₃ |
| 2407. | Br | H | CF₃ | Ph | Cl |
| 2408. | Br | H | CF₃ | tBu | Cl |
| 2409. | Br | H | CF₃ | CHF₂ | Cl |
| 2410. | Br | H | Cl | CHF₂ | CF₃ |
| 2411. | Br | H | CF₃ | Me | OMe |
| 2412. | Br | H | CF₃ | Me | CN |
| 2413. | Br | H | Cl | Et | Cl |
| 2414. | Br | H | CHF₂ | Me | Cl |
| 2415. | Br | H | Me | Me | Me |
| 2416. | Br | H | Me | Me | Cl |
| 2417. | Br | H | Cl | Me | Cl |
| 2418. | Br | H | CF₃ | Me | Cl |
| 2419. | Br | H | Cl | Me | CF₃ |
| 2420. | Br | H | CF₃ | Me | F |
| 2421. | Br | H | OMe | Me | CF₃ |
| 2422. | Br | H | CF₃ | Me | OEt |
| 2423. | Br | H | CF₃ | Me | OCHF₂ |
| 2424. | Br | H | OCHF₂ | Me | CF₃ |
| 2425. | Br | H | CF₃ | Me | OCH₂CHF₂ |
| 2426. | Br | H | CF₃ | Me | OCH₂CF₃ |
| 2427. | Br | H | CF₃ | Me | OCH₂CN |
| 2428. | Br | H | CF₃ | Me | SO₂Me |
| 2429. | Br | H | CF₃ | Me | SEt |
| 2430. | Br | H | CF₃ | Me | Me |
| 2431. | Br | H | CF₃ | Me | Et |
| 2432. | Br | H | CF₃ | Et | Cl |
| 2433. | Br | H | Cl | Et | CF₃ |
| 2434. | Br | H | CF₃ | iPr | Cl |
| 2435. | Br | H | Cl | iPr | CF₃ |
| 2436. | Br | H | CF₃ | tBu | Cl |
| 2437. | Br | H | Cl | tBu | CF₃ |
| 2438. | Br | H | CF₃ | cPen | Cl |
| 2439. | Br | H | Cl | cPen | CF₃ |
| 2440. | Br | H | CF₃ | CH₂cPr | Cl |
| 2441. | Br | H | Cl | CH₂cPr | CF₃ |
| 2442. | Br | H | CF₃ | CH₂CH=CH₂ | Cl |
| 2443. | Br | H | Cl | CH₂CH=CH₂ | CF₃ |
| 2444. | Br | H | CF₃ | CHF₂ | OMe |
| 2445. | Br | H | OMe | CHF₂ | CF₃ |
| 2446. | Br | H | CF₃ | CH₂CF₃ | Cl |
| 2447. | Br | H | Cl | CH₂CF₃ | CF₃ |
| 2448. | Br | H | CF₃ | CH₂OMe | Cl |
| 2449. | Br | H | Cl | CH₂OMe | CF3 |
| 2450. | Br | H | CF₃ | CH₂CN | Cl |
| 2451. | Br | H | Me | Ph | Me |
| 2452. | Br | H | Me | Ph | Cl |
| 2453. | Br | H | Et | Ph | Cl |
| 2454. | Br | H | Pr | Ph | Cl |
| 2455. | Br | H | iPr | Ph | Cl |
| 2456. | Br | H | CF₃ | Ph | Cl |
| 2457. | Br | H | CF₃ | Ph | Me |
| 2458. | Br | H | CF₃ | Ph | CF₃ |
| 2459. | Br | H | CF₃ | Ph | F |
| 2460. | Br | H | CF₃ | Ph | OMe |
| 2461. | Br | H | CF₃ | Ph | OEt |
| 2462. | Br | H | CF₃ | Ph | OCHF₂ |
| 2463. | Br | H | CF₃ | Ph | CN |
| 2464. | Br | H | CF₃ | Ph(4-Cl) | Cl |
| 2465. | Br | H | Me | Me | OCH₂CF₃ |
| 2466. | Br | H | CF₃ | Me | 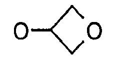 |
| 2467. | Br | H | CF₃ | Me | H |
| 2468. | Br | H | CF₃ | Me | OCH₂CH₂OMe |
| 2469. | Br | H | CF₃ | Me | SMe |
| 2470. | Br | H | CF₃ | Me | OCH₂CH₂CH₂F |
| 2471. | Br | H | CF₃ | Me | OCH(CH₂F)₂ |
| 2472. | Br | H | CF₃ | Me | OCH₂CF₂CHF₂ |
| 2473. | Br | H | CF₃ | Me | OCH₂CF=CH₂ |
| 2474. | Br | H | CF₃ | Me | OCH(Me)CF₃ |
| 2475. | Br | H | CF₃ | Me | OCH(Me)CH₂F |
| 2476. | Br | H | OCH₂CF₃ | Me | CF₃ |
| 2477. | Br | H | OCH₂CF₃ | Me | CHF₂ |
| 2478. | Br | H | CHF₂ | Me | CHF₂ |
| 2479. | Br | H | CF₃ | Me | CHF₂ |
| 2480. | Br | H | Cl | Me | OCHF₂ |
| 2481. | Br | H | Br | Me | OCHF₂ |
| 2482. | Br | H | Br | Me | CF₃ |
| 2483. | I | H | CF₃ | Ph | Cl |
| 2484. | I | H | CF₃ | tBu | Cl |
| 2485. | I | H | CF₃ | CHF₂ | Cl |
| 2486. | I | H | Cl | CHF₂ | CF₃ |
| 2487. | I | H | CF₃ | Me | OMe |
| 2488. | I | H | CF₃ | Me | CN |
| 2489. | I | H | Cl | Et | Cl |
| 2490. | I | H | CHF₂ | Me | Cl |
| 2491. | I | H | Me | Me | Me |
| 2492. | I | H | Me | Me | Cl |
| 2493. | I | H | Cl | Me | Cl |
| 2494. | I | H | CF₃ | Me | Cl |
| 2495. | I | H | Cl | Me | CF₃ |
| 2496. | I | H | CF₃ | Me | F |
| 2497. | I | H | OMe | Me | CF₃ |
| 2498. | I | H | CF₃ | Me | OEt |
| 2499. | I | H | CF₃ | Me | OCHF₂ |
| 2500. | I | H | OCHF₂ | Me | CF₃ |
| 2501. | I | H | CF₃ | Me | OCH₂CHF₂ |
| 2502. | I | H | CF₃ | Me | OCH₂CF₃ |
| 2503. | I | H | CF₃ | Me | OCH₂CN |
| 2504. | I | H | CF₃ | Me | SO₂Me |
| 2505. | I | H | CF₃ | Me | SEt |
| 2506. | I | H | CF₃ | Me | Me |
| 2507. | I | H | CF₃ | Me | Et |
| 2508. | I | H | CF₃ | Et | Cl |
| 2509. | I | H | Cl | Et | CF₃ |
| 2510. | I | H | CF₃ | iPr | Cl |
| 2511. | I | H | Cl | iPr | CF₃ |
| 2512. | I | H | CF₃ | tBu | Cl |
| 2513. | I | H | Cl | tBu | CF₃ |
| 2514. | I | H | CF₃ | cPen | Cl |
| 2515. | I | H | Cl | cPen | CF₃ |
| 2516. | I | H | CF₃ | CH₂cPr | Cl |
| 2517. | I | H | Cl | CH₂cPr | CF₃ |
| 2518. | I | H | CF₃ | CH₂CH=CH₂ | Cl |
| 2519. | I | H | Cl | CH₂CH=CH₂ | CF₃ |
| 2520. | I | H | CF₃ | CHF₂ | OMe |
| 2521. | I | H | OMe | CHF₂ | CF₃ |
| 2522. | I | H | CF₃ | CH₂CF₃ | Cl |
| 2523. | I | H | Cl | CH₂CF₃ | CF₃ |
| 2524. | I | H | CF₃ | CH₂OMe | Cl |
| 2525. | I | H | Cl | CH₂OMe | CF₃ |
| 2526. | I | H | CF₃ | CH₂CN | Cl |
| 2527. | I | H | Me | Ph | Me |
| 2528. | I | H | Me | Ph | Cl |
| 2529. | I | H | Et | Ph | Cl |
| 2530. | I | H | Pr | Ph | Cl |
| 2531. | I | H | iPr | Ph | Cl |
| 2532. | I | H | CF₃ | Ph | Cl |
| 2533. | I | H | CF₃ | Ph | Me |
| 2534. | I | H | CF₃ | Ph | CF₃ |
| 2535. | I | H | CF₃ | Ph | F |
| 2536. | I | H | CF₃ | Ph | OMe |
| 2537. | I | H | CF₃ | Ph | OEt |
| 2538. | I | H | CF₃ | Ph | OCHF₂ |
| 2539. | I | H | CF₃ | Ph | CN |
| 2540. | I | H | CF₃ | Ph(4-Cl) | Cl |
| 2541. | I | H | Me | Me | OCH₂CF₃ |
| 2542. | I | H | CF₃ | Me | 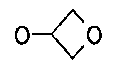 |
| 2543. | I | H | CF₃ | Me | H |
| 2544. | I | H | CF₃ | Me | OCH₂CH₂OMe |
| 2545. | I | H | CF₃ | Me | SMe |
| 2546. | I | H | CF₃ | Me | OCH₂CH₂CH₂F |
| 2547. | I | H | CF₃ | Me | OCH(CH₂F)₂ |
| 2548. | I | H | CF₃ | Me | OCH₂CF₂CHF₂ |
| 2549. | I | H | CF₃ | Me | OCH₂CF=CH₂ |
| 2550. | I | H | CF₃ | Me | OCH(Me)CF₃ |
| 2551. | I | H | CF₃ | Me | OCH(Me)CH₂F |
| 2552. | I | H | OCH₂CF₃ | Me | CF₃ |
| 2553. | I | H | OCH₂CF₃ | Me | CHF₂ |
| 2554. | I | H | CHF₂ | Me | CHF₂ |
| 2555. | I | H | CF₃ | Me | CHF₂ |
| 2556. | I | H | Cl | Me | OCHF₂ |
| 2557. | I | H | Br | Me | OCHF₂ |
| 2558. | I | H | Br | Me | CF₃ |
| 2559. | H | F | CF₃ | Ph | Cl |
| 2560. | H | F | CF₃ | tBu | Cl |
| 2561. | H | F | CF₃ | CHF₂ | Cl |
| 2562. | H | F | Cl | CHF₂ | CF₃ |
| 2563. | H | F | CF₃ | Me | OMe |
| 2564. | H | F | CF₃ | Me | CN |
| 2565. | H | F | Cl | Et | Cl |
| 2566. | H | F | CHF₂ | Me | Cl |
| 2567. | H | F | Me | Me | Me |
| 2568. | H | F | Me | Me | Cl |
| 2569. | H | F | Cl | Me | Cl |
| 2570. | H | F | CF₃ | Me | Cl |
| 2571. | H | F | Cl | Me | CF₃ |
| 2572. | H | F | CF₃ | Me | F |
| 2573. | H | F | OMe | Me | CF₃ |
| 2574. | H | F | CF₃ | Me | OEt |
| 2575. | H | F | CF₃ | Me | OCHF₂ |
| 2576. | H | F | OCHF₂ | Me | CF₃ |
| 2577. | H | F | CF₃ | Me | OCH₂CHF₂ |
| 2578. | H | F | CF₃ | Me | OCH₂CF₃ |
| 2579. | H | F | CF₃ | Me | OCH₂CN |
| 2580. | H | F | CF₃ | Me | SO₂Me |
| 2581. | H | F | CF₃ | Me | SEt |
| 2582. | H | F | CF₃ | Me | Me |
| 2583. | H | F | CF₃ | Me | Et |
| 2584. | H | F | CF₃ | Et | Cl |
| 2585. | H | F | Cl | Et | CF₃ |
| 2586. | H | F | CF₃ | iPr | Cl |
| 2587. | H | F | Cl | iPr | CF₃ |
| 2588. | H | F | CF₃ | tBu | Cl |
| 2589. | H | F | Cl | tBu | CF₃ |
| 2590. | H | F | CF₃ | cPen | Cl |
| 2591. | H | F | Cl | cPen | CF₃ |
| 2592. | H | F | CF₃ | CH₂cPr | Cl |
| 2593. | H | F | Cl | CH₂cPr | CF₃ |
| 2594. | H | F | CF₃ | CH₂CH=CH₂ | Cl |
| 2595. | H | F | Cl | CH₂CH=CH₂ | CF₃ |
| 2596. | H | F | CF₃ | CHF₂ | OMe |
| 2597. | H | F | OMe | CHF₂ | CF₃ |
| 2598. | H | F | CF₃ | CH₂CF₃ | Cl |
| 2599. | H | F | Cl | CH₂CF₃ | CF₃ |
| 2600. | H | F | CF₃ | CH₂OMe | Cl |
| 2601. | H | F | Cl | CH₂OMe | CF₃ |
| 2602. | H | F | CF₃ | CH₂CN | Cl |
| 2603. | H | F | Me | Ph | Me |
| 2604. | H | F | Me | Ph | Cl |
| 2605. | H | F | Et | Ph | Cl |
| 2606. | H | F | Pr | Ph | Cl |
| 2607. | H | F | iPr | Ph | Cl |
| 2608. | H | F | CF₃ | Ph | Cl |
| 2609. | H | F | CF₃ | Ph | Me |
| 2610. | H | F | CF₃ | Ph | CF₃ |
| 2611. | H | F | CF₃ | Ph | F |
| 2612. | H | F | CF₃ | Ph | OMe |
| 2613. | H | F | CF₃ | Ph | OEt |
| 2614. | H | F | CF₃ | Ph | OCHF₂ |
| 2615. | H | F | CF₃ | Ph | CN |
| 2616. | H | F | CF₃ | Ph(4-Cl) | Cl |
| 2617. | H | F | Me | Me | OCH₂CF₃ |
| 2618. | H | F | CF₃ | Me | 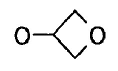 |
| 2619. | H | F | CF₃ | Me | H |
| 2620. | H | F | CF₃ | Me | OCH₂CH₂OMe |
| 2621. | H | F | CF₃ | Me | SMe |
| 2622. | H | F | CF₃ | Me | OCH₂CH₂CH₂F |
| 2623. | H | F | CF₃ | Me | OCH(CH₂F)₂ |
| 2624. | H | F | CF₃ | Me | OCH₂CF₂CHF2 |
| 2625. | H | F | CF₃ | Me | OCH₂CF=CH₂ |
| 2626. | H | F | CF₃ | Me | OCH(Me)CF₃ |
| 2627. | H | F | CF₃ | Me | OCH(Me)CH₂F |
| 2628. | H | F | OCH₂CF₃ | Me | CF₃ |
| 2629. | H | F | OCH₂CF₃ | Me | CHF₂ |
| 2630. | H | F | CHF₂ | Me | CHF₂ |
| 2631. | H | F | CF₃ | Me | CHF₂ |
| 2632. | H | F | Cl | Me | OCHF₂ |
| 2633. | H | F | Br | Me | OCHF₂ |
| 2634. | H | F | Br | Me | CF₃ |
| 2635. | H | Cl | CF₃ | tBu | Cl |
| 2636. | H | Cl | CF₃ | CHF₂ | Cl |
| 2637. | H | Cl | Cl | CHF₂ | CF₃ |
| 2638. | H | Cl | CF₃ | Me | OMe |
| 2639. | H | Cl | CF₃ | Me | CN |
| 2640. | H | Cl | CHF₂ | Me | Cl |
| 2641. | H | Cl | Me | Me | Me |
| 2642. | H | Cl | Me | Me | Cl |
| 2643. | H | Cl | CF₃ | Me | Cl |
| 2644. | H | Cl | Cl | Me | CF₃ |
| 2645. | H | Cl | CF₃ | Me | F |
| 2646. | H | Cl | OMe | Me | CF₃ |
| 2647. | H | Cl | CF₃ | Me | OCHF₂ |
| 2648. | H | Cl | OCHF₂ | Me | CF₃ |
| 2649. | H | Cl | CF₃ | Me | OCH₂CHF₂ |
| 2650. | H | Cl | CF₃ | Me | OCH₂CF₃ |
| 2651. | H | Cl | CF₃ | Me | SO₂Me |
| 2652. | H | Cl | CF₃ | Me | SEt |
| 2653. | H | Cl | CF₃ | Et | Cl |
| 2654. | H | Cl | CF₃ | iPr | Cl |
| 2655. | H | Cl | CF₃ | tBu | Cl |
| 2656. | H | Cl | Cl | tBu | CF₃ |
| 2657. | H | Cl | CF₃ | cPen | Cl |
| 2658. | H | Cl | CF₃ | CHF₂ | OMe |
| 2659. | H | Cl | CF₃ | CH₂CF₃ | Cl |
| 2660. | H | Cl | CF₃ | Ph | OCHF₂ |
| 2661. | H | Cl | CF₃ | Ph | Cl |
| 2662. | H | Cl | Me | Me | OCH₂CF₃ |
| 2663. | H | Cl | CF₃ | Me | 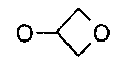 |
| 2664. | H | Cl | CF₃ | Me | H |
| 2665. | H | Cl | CF₃ | Me | OCH₂CH₂OMe |
| 2666. | H | Cl | CF₃ | Me | SMe |
| 2667. | H | Cl | CF₃ | Me | OCH₂CH₂CH₂F |
| 2668. | H | Cl | CF₃ | Me | OCH(CH₂F)₂ |
| 2669. | H | Cl | CF₃ | Me | OCH₂CF₂CHF₂ |
| 2670. | H | Cl | CF₃ | Me | OCH₂CF=CH₂ |
| 2671. | H | Cl | CF₃ | Me | OCH(Me)CF₃ |
| 2672. | H | Cl | CF₃ | Me | OCH(Me)CH₂F |
| 2673. | H | Cl | OCH₂CF₃ | Me | CF₃ |
| 2674. | H | Cl | OCH₂CF₃ | Me | CHF₂ |
| 2675. | H | Cl | CHF₂ | Me | CHF₂ |
| 2676. | H | Cl | CF₃ | Me | CHF₂ |
| 2677. | H | Cl | Cl | Me | OCHF₂ |
| 2678. | H | Cl | Br | Me | OCHF₂ |
| 2679. | H | Cl | Br | Me | CF₃ |
| 2680. | H | Br | CF₃ | tBu | Cl |
| 2681. | H | Br | CF₃ | CHF₂ | Cl |
| 2682. | H | Br | Cl | CHF₂ | CF₃ |
| 2683. | H | Br | CF₃ | Me | OMe |
| 2684. | H | Br | CF₃ | Me | CN |
| 2685. | H | Br | CHF₂ | Me | Cl |
| 2686. | H | Br | Me | Me | Me |
| 2687. | H | Br | Me | Me | Cl |
| 2688. | H | Br | CF₃ | Me | Cl |
| 2689. | H | Br | Cl | Me | CF₃ |
| 2690. | H | Br | CF₃ | Me | F |
| 2691. | H | Br | OMe | Me | CF₃ |
| 2692. | H | Br | CF₃ | Me | OCHF₂ |
| 2693. | H | Br | OCHF₂ | Me | CF₃ |
| 2694. | H | Br | CF₃ | Me | OCH₂CHF₂ |
| 2695. | H | Br | CF₃ | Me | OCH₂CF₃ |
| 2696. | H | Br | CF₃ | Me | SO₂Me |
| 2697. | H | Br | CF₃ | Me | SEt |
| 2698. | H | Br | CF₃ | Et | Cl |
| 2699. | H | Br | CF₃ | iPr | Cl |
| 2700. | H | Br | CF₃ | tBu | Cl |
| 2701. | H | Br | Cl | tBu | CF₃ |
| 2702. | H | Br | CF₃ | cPen | Cl |
| 2703: | H | Br | CF₃ | CHF₂ | OMe |
| 2704. | H | Br | CF₃ | CH₂CF₃ | Cl |
| 2705. | H | Br | CF₃ | Ph | OCHF₂ |
| 2706. | H | Br | CF₃ | Ph | Cl |
| 2707. | H | Br | Me | Me | OCH₂CF₃ |
| 2708. | H | Br | CF₃ | Me | 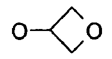 |
| 2709. | H | Br | CF₃ | Me | H |
| 2710. | H | Br | CF₃ | Me | OCH₂CH₂OMe |
| 2711. | H | Br | CF₃ | Me | SMe |
| 2712. | H | Br | CF₃ | Me | OCH₂CH₂CH₂F |
| 2713. | H | Br | CF₃ | Me | OCH(CH₂F)₂ |
| 2714. | H | Br | CF₃ | Me | OCH₂CF₂CHF₂ |
| 2715. | H | Br | CF₃ | Me | OCH₂CF=CH₂ |
| 2716. | H | Br | CF₃ | Me | OCH(Me)CF₃ |
| 2717. | H | Br | CF₃ | Me | OCH(Me)CH₂F |
| 2718. | H | Br | OCH₂CF₃ | Me | CF₃ |
| 2719. | H | Br | OCH₂CF₃ | Me | CH₂ |
| 2720. | H | Br | CHF₂ | Me | CHF₂ |
| 2721. | H | Br | CF₃ | Me | CHF₂ |
| 2722. | H | Br | Cl | Me | OCHF₂ |
| 2723. | H | Br | Br | Me | OCHF₂ |
| 2724. | H | Br | Br | Me | CF₃ |
| 2725. | Me | H | CF₃ | tBu | Cl |
| 2726. | Me | H | CF₃ | CHF₂ | Cl |
| 2727. | Me | H | Cl | CHF₂ | CF₃ |
| 2728. | Me | H | CF₃ | Me | OMe |
| 2729. | Me | H | CF₃ | Me | CN |
| 2730. | Me | H | CHF₂ | Me | Cl |
| 2731. | Me | H | Me | Me | Me |
| 2732. | Me | H | Me | Me | Cl |
| 2733. | Me | H | CF₃ | Me | Cl |
| 2734. | Me | H | Cl | Me | CF₃ |
| 2735. | Me | H | CF₃ | Me | F |
| 2736. | Me | H | OMe | Me | CF₃ |
| 2737. | Me | H | CF₃ | Me | OCHF₂ |
| 2738. | Me | H | OCHF₂ | Me | CF₃ |
| 2739. | Me | H | CF₃ | Me | OCH₂CHF₂ |
| 2740. | Me | H | CF₃ | Me | OCH₂CF₃ |
| 2741. | Me | H | CF₃ | Me | SO₂Me |
| 2742. | Me | H | CF₃ | Me | SEt |
| 2743. | Me | H | CF₃ | Et | Cl |
| 2744. | Me | H | CF₃ | iPr | Cl |
| 2745. | Me | H | CF₃ | tBu | Cl |
| 2746. | Me | H | Cl | tBu | CF₃ |
| 2747. | Me | H | CF₃ | cPen | Cl |
| 2748. | Me | H | CF₃ | CHF₂ | OMe |
| 2749. | Me | H | CF₃ | CH₂CF₃ | Cl |
| 2750. | Me | H | CF₃ | Ph | OCHF₂ |
| 2751. | Me | H | CF₃ | Ph | Cl |
| 2752. | Me | H | Me | Me | OCH₂CF₃ |
| 2753. | Me H | | CF₃ | Me | 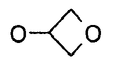 |
| 2754. | Me | H | CF₃ | Me | H |
| 2755. | Me | H | CF₃ | Me | OCH₂CH₂OMe |
| 2756. | Me | H | CF₃ | Me | SMe |
| 2757. | Me | H | CF₃ | Me | OCH₂CH₂CH₂F |
| 2758. | Me | H | CF₃ | Me | OCH(CH₂F)₂ |
| 2759. | Me | H | CF₃ | Me | OCH₂CF₂CHF₂ |
| 2760. | Me | H | CF₃ | Me | OCH₂CF=CH₂ |
| 2761. | Me | H | CF₃ | Me | OCH(Me)CF₃ |
| 2762. | Me | H | CF₃ | Me | OCH(Me)CH₂F |
| 2763. | Me | H | OCH₂CF₃ | Me | CF₃ |
| 2764. | Me | H | OCH₂CF₃ | Me | CHF₂ |
| 2765. | Me | H | CHF₂ | Me | CHF₂ |
| 2766. | Me | H | CF₃ | Me | CHF₂ |
| 2767. | Me | H | Cl | Me | OCHF₂ |
| 2768. | Me | H | Br | Me | OCHF₂ |
| 2769. | Me | H | Br | Me | CF₃ |
| 2770. | NO₂ | H | CF₃ | tBu | Cl |
| 2771. | NO₂ | H | CF₃ | CHF₂ | Cl |
| 2772. | NO₂ | H | Cl | CHF₂ | CF₃ |
| 2773. | NO₂ | H | CF₃ | Me | OMe |
| 2774. | NO₂ | H | CF₃ | Me | CN |
| 2775. | NO₂ | H | CHF₂ | Me | Cl |
| 2776. | NO₂ | H | Me | Me | Me |
| 2777. | NO₂ | H | Me | Me | Cl |
| 2778. | NO₂ | H | CF₃ | Me | Cl |
| 2779. | NO₂ | H | Cl | Me | CF₃ |
| 2780. | NO₂ | H | CF₃ | Me | F |
| 2781. | NO₂ | H | OMe | Me | CF₃ |
| 2782. | NO₂ | H | CF₃ | Me | OCHF₂ |
| 2783. | NO₂ | H | OCHF₂ | Me | CF₃ |
| 2784. | NO₂ | H | CF₃ | Me | OCH₂CHF₂ |
| 2785. | NO₂ | H | CF₃ | Me | OCH₂CF₃ |
| 2786. | NO₂ | H | CF₃ | Me | SO₂Me |
| 2787. | NO₂ | H | CF₃ | Me | SEt |
| 2788. | NO₂ | H | CF₃ | Et | Cl |
| 2789. | NO₂ | H | CF₃ | iPr | Cl |
| 2790. | NO₂ | H | CF₃ | tBu | Cl |
| 2791. | NO₂ | H | Cl | tBu | CF₃ |
| 2792. | NO₂ | H | CF₃ | cPen | Cl |
| 2793. | NO₂ | H | CF₃ | CHF₂ | OMe |
| 2794. | NO₂ | H | CF₃ | CH₂CF₃ | Cl |
| 2795. | NO₂ | H | CF₃ | Ph | OCHF₂ |
| 2796. | NO₂ | H | CF₃ | Ph | Cl |
| 2797. | NO₂ | H | Me | Me | OCH₂CF₃ |
| 2798. | NO₂ | H | CF₃ | Me | 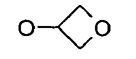 |
| 2799. | NO₂ | H | CF₃ | Me | H |
| 2800. | NO₂ | H | CF₃ | Me | OCH₂CH₂OMe |
| 2801. | NO₂ | H | CF₃ | Me | SMe |
| 2802. | NO₂ | H | CF₃ | Me | OCH₂CH₂CH₂F |
| 2803. | NO₂ | H | CF₃ | Me | OCH(CH₂F)₂ |
| 2804. | NO₂ | H | CF₃ | Me | OCH₂CF₂CHF₂ |
| 2805. | NO₂ | H | CF₃ | Me | OCH₂CF=CH₂ |
| 2806. | NO₂ | H | CF₃ | Me | OCH(Me)CF₃ |
| 2807. | NO₂ | H | CF₃ | Me | OCH(Me)CH₂F |
| 2808. | NO₂ | H | OCH₂CF₃ | Me | CF₃ |
| 2809. | NO₂ | H | OCH₂CF₃ | Me | CHF₂ |
| 2810. | NO₂ | H | CHF₂ | Me | CHF₂ |
| 2811. | NO₂ | H | CF₃ | Me | CHF₂ |
| 2812. | NO₂ | H | Cl | Me | OCHF₂ |
| 2813. | NO₂ | H | Br | Me | OCHF₂ |
| 2814. | NO₂ | H | Br | Me | CF₃ |
| 2815. | Cl | Cl | CF₃ | tBu | Cl |
| 2816. | Cl | Cl | CF₃ | CHF₂ | Cl |
| 2817. | Cl | Cl | Cl | CHF₂ | CF₃ |
| 2818. | Cl | Cl | CF₃ | Me | OMe |
| 2819. | Cl | Cl | CF₃ | Me | CN |
| 2820. | Cl | Cl | CHF₂ | Me | Cl |
| 2821. | Cl | Cl | Me | Me | Me |
| 2822. | Cl | Cl | Me | Me | Cl |
| 2823. | Cl | Cl | CF₃ | Me | Cl |
| 2824. | Cl | Cl | Cl | Me | CF₃ |
| 2825. | Cl | Cl | CF₃ | Me | F |
| 2826. | Cl | Cl | OMe | Me | CF₃ |
| 2827. | Cl | Cl | CF₃ | Me | OCHF₂ |
| 2828. | Cl | Cl | OCHF₂ | Me | CF₃ |
| 2829. | Cl | Cl | CF₃ | Me | OCH₂CHF₂ |
| 2830. | Cl | Cl | CF₃ | Me | OCH₂CF₃ |
| 2831. | Cl | Cl | CF₃ | Me | SO₂Me |
| 2832. | Cl | Cl | CF₃ | Me | SEt |
| 2833. | Cl | Cl | CF₃ | Et | Cl |
| 2834. | Cl | Cl | CF₃ | iPr | Cl |
| 2835. | Cl | Cl | CF₃ | tBu | Cl |
| 2836. | Cl | Cl | Cl | tBu | CF₃ |
| 2837. | Cl | Cl | CF₃ | cPen | Cl |
| 2838. | Cl | Cl | CF₃ | CHF₂ | OMe |
| 2839. | Cl | Cl | CF₃ | CH₂CF₃ | Cl |
| 2840. | Cl | Cl | CF₃ | Ph | OCHF₂ |
| 2841. | Cl | Cl | CF₃ | Ph | Cl |
| 2842. | Cl | Cl | Me | Me | OCH₂CF₃ |
| 2843. | Cl | Cl | CF₃ | Me | 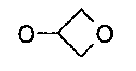 |
| 2844. | Cl | Cl | CF₃ | Me | H |
| 2845. | Cl | Cl | CF₃ | Me | OCH₂CH₂OMe |
| 2846. | Cl | Cl | CF₃ | Me | SMe |
| 2847. | Cl | Cl | CF₃ | Me | OCH₂CH₂CH₂F |
| 2848. | Cl | Cl | CF₃ | Me | OCH(CH₂F)₂ |
| 2849. | Cl | Cl | CF₃ | Me | OCH₂CF₂CHF₂ |
| 2850. | Cl | Cl | CF₃ | Me | OCH₂CF=CH₂ |
| 2851. | Cl | Cl | CF₃ | Me | OCH(Me)CF₃ |
| 2852. | Cl | Cl | CF₃ | Me | OCH(Me)CH₂F |
| 2853. | Cl | Cl | OCH₂CF₃ | Me | CF₃ |
| 2854. | Cl | Cl | OCH₂CF₃ | Me | CHF₂ |
| 2855. | Cl | Cl | CHF₂ | Me | CHF₂ |
| 2856. | Cl | Cl | CF₃ | Me | CHF₂ |
| 2857. | Cl | Cl | Cl | Me | OCHF₂ |
| 2858. | Cl | Cl | Br | Me | OCHF₂ |
| 2859. | Cl | Cl | Br | Me | CF₃ |
| 2860. | Cl | Me | CF₃ | tBu | Cl |
| 2861. | Cl | Me | CF₃ | CHF₂ | Cl |
| 2862. | Cl | Me | Cl | CHF₂ | CF₃ |
| 2863. | Cl | Me | CF₃ | Me | OMe |
| 2864. | Cl | Me | CF₃ | Me | CN |
| 2865. | Cl | Me | CHF₂ | Me | Cl |
| 2866. | Cl | Me | Me | Me | Me |
| 2867. | Cl | Me | Me | Me | Cl |
| 2868. | Cl | Me | CF₃ | Me | Cl |
| 2869. | Cl | Me | Cl | Me | CF₃ |
| 2870. | Cl | Me | CF₃ | Me | F |
| 2871. | Cl | Me | OMe | Me | CF₃ |
| 2872. | Cl | Me | CF₃ | Me | OCHF₂ |
| 2873. | Cl | Me | OCHF₂ | Me | CF₃ |
| 2874. | Cl | Me | CF₃ | Me | OCH₂CHF₂ |
| 2875. | Cl | Me | CF₃ | Me | OCH₂CF₃ |
| 2876. | Cl | Me | CF₃ | Me | SO₂Me |
| 2877. | Cl | Me | CF₃ | Me | SEt |
| 2878. | Cl | Me | CF₃ | Et | Cl |
| 2879. | Cl | Me | CF₃ | iPr | Cl |
| 2880. | Cl | Me | CF₃ | tBu | Cl |
| 2881. | Cl | Me | Cl | tBu | CF₃ |
| 2882. | Cl | Me | CF₃ | cPen | Cl |
| 2883. | Cl | Me | CF₃ | CHF₂ | OMe |
| 2884. | Cl | Me | CF₃ | CH₂CF₃ | Cl |
| 2885. | Cl | Me | CF₃ | Ph | OCHF₂ |
| 2886. | Cl | Me | CF₃ | Ph | Cl |
| 2887. | Cl | Me | Me | Me | OCH₂CF₃ |
| 2888. | Cl | Me | CF₃ | Me | 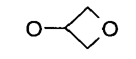 |
| 2889. | Cl | Me | CF₃ | Me | H |
| 2890. | Cl | Me | CF₃ | Me | OCH₂CH₂OMe |
| 2891. | Cl | Me | CF₃ | Me | SMe |
| 2892. | Cl | Me | CF₃ | Me | OCH₂CH₂CH₂F |
| 2893. | Cl | Me | CF₃ | Me | OCH(CH₂F)₂ |
| 2894. | Cl | Me | CF₃ | Me | OCH₂CF₂CHF₂ |
| 2895. | Cl | Me | CF₃ | Me | OCH₂CF=CH₂ |
| 2896. | Cl | Me | CF₃ | Me | OCH(Me)CF₃ |
| 2897. | Cl | Me | CF₃ | Me | OCH(Me)CH₂F |
| 2898. | Cl | Me | OCH₂CF₃ | Me | CF₃ |
| 2899. | Cl | Me | OCH₂CF₃ | Me | CHF₂ |
| 2900. | Cl | Me | CHF₂ | Me | CHF₂ |
| 2901. | Cl | Me | CF₃ | Me | CHF₂ |
| 2902. | Cl | Me | Cl | Me | OCHF₂ |
| 2903. | Cl | Me | Br | Me | OCHF₂ |
| 2904. | Cl | Me | Br | Me | CF₃ |

**Tabelle 3: Verbindungen der Formel (III-R)**

| | | | | | |
|---|---|---|---|---|---|
| 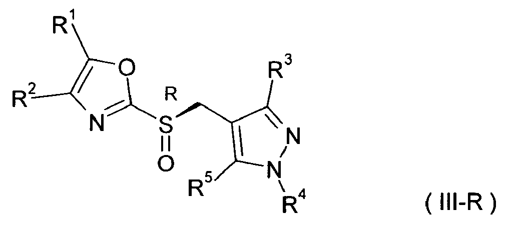 | | | | | |

| Bsp. Nr. | R¹ | R² | R³ | R⁴ | R⁵ |
|---|---|---|---|---|---|
| 2905. | H | H | CF₃ | Ph | Cl |
| 2906. | H | H | CF₃ | tBu | Cl |
| 2907. | H | H | CF₃ | CHF₂ | Cl |
| 2908. | H | H | Cl | CHF₂ | CF₃ |
| 2909. | H | H | CF₃ | Me | OMe |
| 2910. | H | H | CF₃ | Me | CN |
| 2911. | H | H | Cl | Et | Cl |
| 2912. | H | H | CHF₂ | Me | Cl |
| 2913. | H | H | Me | Me | Me |
| 2914. | H | H | Me | Me | Cl |
| 2915. | H | H | Cl | Me | Cl |
| 2916. | H | H | CF₃ | Me | Cl |
| 2917. | H | H | Cl | Me | CF₃ |
| 2918. | H | H | CF₃ | Me | F |
| 2919. | H | H | OMe | Me | CF₃ |
| 2920. | H | H | CF₃ | Me | OEt |
| 2921. | H | H | CF₃ | Me | OCHF₂ |
| 2922. | H | H | OCHF₂ | Me | CF₃ |
| 2923. | H | H | CF₃ | Me | OCH₂CHF₂ |
| 2924. | H | H | CF₃ | Me | OCH₂CF₃ |
| 2925. | H | H | CF₃ | Me | OCH₂CN |
| 2926. | H | H | CF₃ | Me | SO₂Me |
| 2927. | H | H | CF₃ | Me | SEt |
| 2928. | H | H | CF₃ | Me | Me |
| 2929. | H | H | CF₃ | Me | Et |
| 2930. | H | H | CF₃ | Et | Cl |
| 2931. | H | H | Cl | Et | CF₃ |
| 2932. | H | H | CF₃ | iPr | Cl |
| 2933. | H | H | Cl | iPr | CF₃ |
| 2934. | H | H | CF₃ | tBu | Cl |
| 2935. | H | H | Cl | tBu | CF₃ |
| 2936. | H | H | CF₃ | cPen | Cl |
| 2937. | H | H | Cl | cPen | CF₃ |
| 2938. | H | H | CF₃ | CH₂cPr | Cl |
| 2939. | H | H | Cl | CH₂cPr | CF₃ |
| 2940. | H | H | CF₃ | CH₂CH=CH₂ | Cl |
| 2941. | H | H | Cl | CH₂CH=CH₂ | CF₃ |
| 2942. | H | H | CF₃ | CHF₂ | OMe |
| 2943. | H | H | OMe | CHF₂ | CF₃ |
| 2944. | H | H | CF₃ | CH₂CF₃ | Cl |
| 2945. | H | H | Cl | CH₂CF₃ | CF₃ |
| 2946. | H | H | CF₃ | CH₂OMe | Cl |
| 2947. | H | H | Cl | CH₂OMe | CF₃ |
| 2948. | H | H | CF₃ | CH₂CN | Cl |
| 2949. | H | H | Me | Ph | Me |
| 2950. | H | H | Me | Ph | Cl |
| 2951. | H | H | Et | Ph | Cl |
| 2952. | H | H | Pr | Ph | Cl |
| 2953. | H | H | iPr | Ph | Cl |
| 2954. | H | H | CF₃ | Ph | Cl |
| 2955. | H | H | CF₃ | Ph | Me |
| 2956. | H | H | CF₃ | Ph | CF₃ |
| 2957. | H | H | CF₃ | Ph | F |
| 2958. | H | H | CF₃ | Ph | OMe |
| 2959. | H | H | CF₃ | Ph | OEt |
| 2960. | H | H | CF₃ | Ph | OCHF₂ |
| 2961. | H | H | CF₃ | Ph | CN |
| 2962. | H | H | CF₃ | Ph(4-Cl) | Cl |
| 2963. | H | H | Me | Me | OCH₂CF₃ |
| 2964. | H | H | CF₃ | Me | 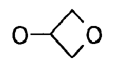 |
| 2965. | H | H | CF₃ | Me | H |
| 2966. | H | H | CF₃ | Me | OCH₂CH₂OMe |
| 2967. | H | H | CF₃ | Me | SMe |
| 2968. | H | H | CF₃ | Me | OCH₂CH₂CH₂F |
| 2969. | H | H | CF₃ | Me | OCH(CH₂F)₂ |
| 2970. | H | H | CF₃ | Me | OCH₂CF₂CHF₂ |
| 2971. | H | H | CF₃ | Me | OCH₂CF=CH₂ |
| 2972. | H | H | CF₃ | Me | OCH(Me)CF₃ |
| 2973. | H | H | CF₃ | Me | OCH(Me)CH₂F |
| 2974. | H | H | OCH₂CF₃ | Me | CF₃ |
| 2975. | H | H | OCH₂CF₃ | Me | CHF₂ |
| 2976. | H | H | CHF₂ | Me | CHF₂ |
| 2977. | H | H | CF₃ | Me | CHF₂ |
| 2978. | H | H | Cl | Me | OCHF₂ |
| 2979. | H | H | Br | Me | OCHF₂ |
| 2980. | H | H | Br | Me | CF₃ |
| 2981. | F | H | CF₃ | Ph | Cl |
| 2982. | F | H | CF₃ | tBu | Cl |
| 2983. | F | H | CF₃ | CHF₂ | Cl |
| 2984. | F | H | Cl | CHF₂ | CF₃ |
| 2985. | F | H | CF₃ | Me | OMe |
| 2986. | F | H | CF₃ | Me | CN |
| 2987. | F | H | Cl | Et | Cl |
| 2988. | F | H | CHF₂ | Me | Cl |
| 2989. | F | H | Me | Me | Me |
| 2990. | F | H | Me | Me | Cl |
| 2991. | F | H | Cl | Me | Cl |
| 2992. | F | H | CF₃ | Me | Cl |
| 2993. | F | H | Cl | Me | CF₃ |
| 2994. | F | H | CF₃ | Me | F |
| 2995. | F | H | OMe | Me | CF₃ |
| 2996. | F | H | CF₃ | Me | OEt |
| 2997. | F | H | CF₃ | Me | OCHF₂ |
| 2998. | F | H | OCHF₂ | Me | CF₃ |
| 2999. | F | H | CF₃ | Me | OCH₂CHF₂ |
| 3000. | F | H | CF₃ | Me | OCH₂CF₃ |
| 3001. | F | H | CF₃ | Me | OCH₂CN |
| 3002. | F | H | CF₃ | Me | SO₂Me |
| 3003. | F | H | CF₃ | Me | SEt |
| 3004. | F | H | CF₃ | Me | Me |
| 3005. | F | H | CF₃ | Me | Et |
| 3006. | F | H | CF₃ | Et | Cl |
| 3007. | F | H | Cl | Et | CF₃ |
| 3008. | F | H | CF₃ | iPr | Cl |
| 3009. | F | H | Cl | iPr | CF₃ |
| 3010. | F | H | CF₃ | tBu | Cl |
| 3011. | F | H | Cl | tBu | CF₃ |
| 3012. | F | H | CF₃ | cPen | Cl |
| 3013. | F | H | Cl | cPen | CF₃ |
| 3014. | F | H | CF₃ | CH₂cPr | Cl |
| 3015. | F | H | Cl | CH₂cPr | CF₃ |
| 3016. | F | H | CF₃ | CH₂CH=CH₂ | Cl |
| 3017. | F | H | Cl | CH₂CH=CH₂ | CF₃ |
| 3018. | F | H | CF₃ | CHF₂ | OMe |
| 3019. | F | H | OMe | CHF₂ | CF₃ |
| 3020. | F | H | CF₃ | CH₂CF₃ | Cl |
| 3021. | F | H | Cl | CH₂CF₃ | CF₃ |
| 3022. | F | H | CF₃ | CH₂OMe | Cl |
| 3023. | F | H | Cl | CH₂OMe | CF₃ |
| 3024. | F | H | CF₃ | CH₂CN | Cl |
| 3025. | F | H | Me | Ph | Me |
| 3026. | F | H | Me | Ph | Cl |
| 3027. | F | H | Et | Ph | Cl |
| 3028. | F | H | Pr | Ph | Cl |
| 3029. | F | H | iPr | Ph | Cl |
| 3030. | F | H | CF₃ | Ph | Cl |
| 3031. | F | H | CF₃ | Ph | Me |
| 3032. | F | H | CF₃ | Ph | CF₃ |
| 3033. | F | H | CF₃ | Ph | F |
| 3034. | F | H | CF₃ | Ph | OMe |
| 3035. | F | H | CF₃ | Ph | OEt |
| 3036. | F | H | CF₃ | Ph | OCHF₂ |
| 3037. | F | H | CF₃ | Ph | CN |
| 3038. | F | H | CF₃ | Ph(4-Cl) | Cl |
| 3039. | F | H | Me | Me | OCH₂CF₃ |
| 3040. | F | H | CF₃ | Me | 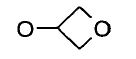 |
| 3041. | F | H | CF₃ | Me | H |
| 3042. | F | H | CF₃ | Me | OCH₂CH₂OMe |
| 3043. | F | H | CF₃ | Me | SMe |
| 3044. | F | H | CF₃ | Me | OCH₂CH₂CH₂F |
| 3045. | F | H | CF₃ | Me | OCH(CH₂F)₂ |
| 3046. | F | H | CF₃ | Me | OCH₂CF₂CHF₂ |
| 3047. | F | H | CF₃ | Me | OCH₂CF=CH₂ |
| 3048. | F | H | CF₃ | Me | OCH(Me)CF₃ |
| 3049. | F | H | CF₃ | Me | OCH(Me)CH₂F |
| 3050. | F | H | OCH₂CF₃ | Me | CF₃ |
| 3051. | F | H | OCH₂CF₃ | Me | CHF₂ |
| 3052. | F | H | CHF₂ | Me | CHF₂ |
| 3053. | F | H | CF₃ | Me | CHF₂ |
| 3054. | F | H | Cl | Me | OCHF₂ |
| 3055. | F | H | Br | Me | OCHF₂ |
| 3056. | F | H | Br | Me | CF₃ |
| 3057. | Cl | H | CF₃ | Ph | Cl |
| 3058. | Cl | H | CF₃ | tBu | Cl |
| 3059. | Cl | H | CF₃ | CHF₂ | Cl |
| 3060. | Cl | H | Cl | CHF₂ | CF₃ |
| 3061. | Cl | H | CF₃ | Me | OMe |
| 3062. | Cl | H | CF₃ | Me | CN |
| 3063. | Cl | H | Cl | Et | Cl |
| 3064. | Cl | H | CHF₂ | Me | Cl |
| 3065. | Cl | H | Me | Me | Me |
| 3066. | Cl | H | Me | Me | Cl |
| 3067. | Cl | H | Cl | Me | Cl |
| 3068. | Cl | H | CF₃ | Me | Cl |
| 3069. | Cl | H | Cl | Me | CF₃ |
| 3070. | Cl | H | CF₃ | Me | F |
| 3071. | Cl | H | OMe | Me | CF₃ |
| 3072. | Cl | H | CF₃ | Me | OEt |
| 3073. | Cl | H | CF₃ | Me | OCHF₂ |
| 3074. | Cl | H | OCHF₂ | Me | CF₃ |
| 3075. | Cl | H | CF₃ | Me | OCH₂CHF₂ |
| 3076. | Cl | H | CF₃ | Me | OCH₂CF₃ |
| 3077. | Cl | H | CF₃ | Me | OCH₂CN |
| 3078. | Cl | H | CF₃ | Me | SO₂Me |
| 3079. | Cl | H | CF₃ | Me | SEt |
| 3080. | Cl | H | CF₃ | Me | Me |
| 3081. | Cl | H | CF₃ | Me | Et |
| 3082. | Cl | H | CF₃ | Et | Cl |
| 3083. | Cl | H | Cl | Et | CF₃ |
| 3084. | Cl | H | CF₃ | iPr | Cl |
| 3085. | Cl | H | Cl | iPr | CF₃ |
| 3086. | Cl | H | CF₃ | tBu | Cl |
| 3087. | Cl | H | Cl | tBu | CF₃ |
| 3088. | Cl | H | CF₃ | cPen | Cl |
| 3089. | Cl | H | Cl | cPen | CF₃ |
| 3090. | Cl | H | CF₃ | CH₂cPr | Cl |
| 3091. | Cl | H | Cl | CH₂cPr | CF₃ |
| 3092. | Cl | H | CF₃ | CH₂CH=CH₂ | Cl |
| 3093. | Cl | H | Cl | CH₂CH=CH₂ | CF₃ |
| 3094. | Cl | H | CF₃ | CHF₂ | OMe |
| 3095. | Cl | H | OMe | CHF₂ | CF₃ |
| 3096. | Cl | H | CF₃ | CH₂CF₃ | Cl |
| 3097. | Cl | H | Cl | CH₂CF₃ | CF₃ |
| 3098. | Cl | H | CF₃ | CH₂OMe | Cl |
| 3099. | Cl | H | Cl | CH₂OMe | CF₃ |
| 3100. | Cl | H | CF₃ | CH₂CN | Cl |
| 3101. | Cl | H | Me | Ph | Me |
| 3102. | Cl | H | Me | Ph | Cl |
| 3103. | Cl | H | Et | Ph | Cl |
| 3104. | Cl | H | Pr | Ph | Cl |
| 3105. | Cl | H | iPr | Ph | Cl |
| 3106. | Cl | H | CF₃ | Ph | Cl |
| 3107. | Cl | H | CF₃ | Ph | Me |
| 3108. | Cl | H | CF₃ | Ph | CF₃ |
| 3109. | Cl | H | CF₃ | Ph | F |
| 3110. | Cl | H | CF₃ | Ph | OMe |
| 3111. | Cl | H | CF₃ | Ph | OEt |
| 3112. | Cl | H | CF₃ | Ph | OCHF₂ |
| 3113. | Cl | H | CF₃ | Ph | CN |
| 3114. | Cl | H | CF₃ | Ph(4-Cl) | Cl |
| 3115. | Cl | H | Me | Me | OCH₂CF₃ |
| 3116. | Cl | H | CF₃ | Me | 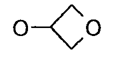 |
| 3117. | Cl | H | CF₃ | Me | H |
| 3118. | Cl | H | CF₃ | Me | OCH₂CH₂OMe |
| 3119. | Cl | H | CF₃ | Me | SMe |
| 3120. | Cl | H | CF₃ | Me | OCH₂CH₂CH₂F |
| 3121. | Cl | H | CF₃ | Me | OCH(CH₂F)₂ |
| 3122. | Cl | H | CF₃ | Me | OCH₂CF₂CHF₂ |
| 3123. | Cl | H | CF₃ | Me | OCH₂CF=CH₂ |
| 3124. | Cl | H | CF₃ | Me | OCH(Me)CF₃ |
| 3125. | Cl | H | CF₃ | Me | OCH(Me)CH₂F |
| 3126. | Cl | H | OCH₂CF₃ | Me | CF₃ |
| 3127. | Cl | H | OCH₂CF₃ | Me | CHF₂ |
| 3128. | Cl | H | CHF₂ | Me | CHF₂ |
| 3129. | Cl | H | CF₃ | Me | CHF₂ |
| 3130. | Cl | H | Cl | Me | OCHF₂ |
| 3131. | Cl | H | Br | Me | OCHF₂ |
| 3132. | Cl | H | Br | Me | CF₃ |
| 3133. | Br | H | CF₃ | Ph | Cl |
| 3134. | Br | H | CF₃ | tBu | Cl |
| 3135. | Br | H | CF₃ | CHF₂ | Cl |
| 3136. | Br | H | Cl | CHF₂ | CF₃ |
| 3137. | Br | H | CF₃ | Me | OMe |
| 3138. | Br | H | CF₃ | Me | CN |
| 3139. | Br | H | Cl | Et | Cl |
| 3140. | Br | H | CHF₂ | Me | Cl |
| 3141. | Br | H | Me | Me | Me |
| 3142. | Br | H | Me | Me | Cl |
| 3143. | Br | H | Cl | Me | Cl |
| 3144. | Br | H | CF₃ | Me | Cl |
| 3145. | Br | H | Cl | Me | CF₃ |
| 3146. | Br | H | CF₃ | Me | F |
| 3147. | Br | H | OMe | Me | CF₃ |
| 3148. | Br | H | CF₃ | Me | OEt |
| 3149. | Br | H | CF₃ | Me | OCHF₂ |
| 3150. | Br | H | OCHF₂ | Me | CF₃ |
| 3151. | Br | H | CF₃ | Me | OCH₂CHF₂ |
| 3152. | Br | H | CF₃ | Me | OCH₂CF₃ |
| 3153. | Br | H | CF₃ | Me | OCH₂CN |
| 3154. | Br | H | CF₃ | Me | SO₂Me |
| 3155. | Br | H | CF₃ | Me | SEt |
| 3156. | Br | H | CF₃ | Me | Me |
| 3157. | Br | H | CF₃ | Me | Et |
| 3158. | Br | H | CF₃ | Et | Cl |
| 3159. | Br | H | Cl | Et | CF₃ |
| 3160. | Br | H | CF₃ | iPr | Cl |
| 3161. | Br | H | Cl | iPr | CF₃ |
| 3162. | Br | H | CF₃ | tBu | Cl |
| 3163. | Br | H | Cl | tBu | CF₃ |
| 3164. | Br | H | CF₃ | cPen | Cl |
| 3165. | Br | H | Cl | cPen | CF₃ |
| 3166. | Br | H | CF₃ | CH₂cPr | Cl |
| 3167. | Br | H | Cl | CH₂cPr | CF₃ |
| 3168. | Br | H | CF₃ | CH₂CH=CH₂ | Cl |
| 3169. | Br | H | Cl | CH₂CH=CH₂ | CF₃ |
| 3170. | Br | H | CF₃ | CHF₂ | OMe |
| 3171. | Br | H | OMe | CHF₂ | CF₃ |
| 3172. | Br | H | CF₃ | CH₂CF₃ | Cl |
| 3173. | Br | H | Cl | CH₂CF₃ | CF₃ |
| 3174. | Br | H | CF₃ | CH₂OMe | Cl |
| 3175. | Br | H | Cl | CH₂OMe | CF₃ |
| 3176. | Br | H | CF₃ | CH₂CN | Cl |
| 3177. | Br | H | Me | Ph | Me |
| 3178. | Br | H | Me | Ph | Cl |
| 3179. | Br | H | Et | Ph | Cl |
| 3180. | Br | H | Pr | Ph | Cl |
| 3181. | Br | H | iPr | Ph | Cl |
| 3182. | Br | H | CF₃ | Ph | Cl |
| 3183. | Br | H | CF₃ | Ph | Me |
| 3184. | Br | H | CF₃ | Ph | CF₃ |
| 3185. | Br | H | CF₃ | Ph | F |
| 3186. | Br | H | CF₃ | Ph | OMe |
| 3187. | Br | H | CF₃ | Ph | OEt |
| 3188. | Br | H | CF₃ | Ph | OCHF₂ |
| 3189. | Br | H | CF₃ | Ph | CN |
| 3190. | Br | H | CF₃ | Ph(4-Cl) | Cl |
| 3191. | Br | H | Me | Me | OCH₂CF₃ |
| 3192. | Br | H | CF₃ | Me | 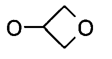 |
| 3193. | Br | H | CF₃ | Me | H |
| 3194. | Br | H | CF₃ | Me | OCH₂CH₂OMe |
| 3195. | Br | H | CF₃ | Me | SMe |
| 3196. | Br | H | CF₃ | Me | OCH₂CH₂CH₂F |
| 3197. | Br | H | CF₃ | Me | OCH(CH₂F)₂ |
| 3198. | Br | H | CF₃ | Me | OCH₂CF₂CHF₂ |
| 3199. | Br | H | CF₃ | Me | OCH₂CF=CH₂ |
| 3200. | Br | H | CF₃ | Me | OCH(Me)CF₃ |
| 3201. | Br | H | CF₃ | Me | OCH(Me)CH₂F |
| 3202. | Br | H | OCH₂CF₃ | Me | CF₃ |
| 3203. | Br | H | OCH₂CF₃ | Me | CHF₂ |
| 3204. | Br | H | CHF₂ | Me | CHF₂ |
| 3205. | Br | H | CF₃ | Me | CHF₂ |
| 3206. | Br | H | Cl | Me | OCHF₂ |
| 3207. | Br | H | Br | Me | OCHF₂ |
| 3208. | Br | H | Br | Me | CF₃ |
| 3209. | I | H | CF₃ | Ph | Cl |
| 3210. | I | H | CF₃ | tBu | Cl |
| 3211. | I | H | CF₃ | CHF₂ | Cl |
| 3212. | I | H | Cl | CHF₂ | CF₃ |
| 3213. | I | H | CF₃ | Me | OMe |
| 3214. | I | H | CF₃ | Me | CN |
| 3215. | I | H | Cl | Et | Cl |
| 3216. | I | H | CHF₂ | Me | Cl |
| 3217. | I | H | Me | Me | Me |
| 3218. | I | H | Me | Me | Cl |
| 3219. | I | H | Cl | Me | Cl |
| 3220. | I | H | CF₃ | Me | Cl |
| 3221. | I | H | Cl | Me | CF₃ |
| 3222. | I | H | CF₃ | Me | F |
| 3223. | I | H | OMe | Me | CF₃ |
| 3224. | I | H | CF₃ | Me | OEt |
| 3225. | I | H | CF₃ | Me | OCHF₂ |
| 3226. | I | H | OCHF₂ | Me | CF₃ |
| 3227. | I | H | CF₃ | Me | OCH₂CHF₂ |
| 3228. | I | H | CF₃ | Me | OCH₂CF₃ |
| 3229. | I | H | CF₃ | Me | OCH₂CN |
| 3230. | I | H | CF₃ | Me | SO₂Me |
| 3231. | I | H | CF₃ | Me | SEt |
| 3232. | I | H | CF₃ | Me | Me |
| 3233. | I | H | CF₃ | Me | Et |
| 3234. | I | H | CF₃ | Et | Cl |
| 3235. | I | H | Cl | Et | CF₃ |
| 3236. | I | H | CF₃ | iPr | Cl |
| 3237. | I | H | Cl | iPr | CF₃ |
| 3238. | I | H | CF₃ | tBu | Cl |
| 3239. | I | H | Cl | tBu | CF₃ |
| 3240. | I | H | CF₃ | cPen | Cl |
| 3241. | I | H | Cl | cPen | CF₃ |
| 3242. | I | H | CF₃ | CH₂cPr | Cl |
| 3243. | I | H | Cl | CH₂cPr | CF₃ |
| 3244. | I | H | CF₃ | CH₂CH=CH₂ | Cl |
| 3245. | I | H | Cl | CH₂CH=CH₂ | CF₃ |
| 3246. | I | H | CF₃ | CHF₂ | OMe |
| 3247. | I | H | OMe | CHF₂ | CF₃ |
| 3248. | I | H | CF₃ | CH₂CF₃ | Cl |
| 3249. | I | H | Cl | CH₂CF₃ | CF₃ |
| 3250. | I | H | CF₃ | CH₂OMe | Cl |
| 3251. | I | H | Cl | CH₂OMe | CF₃ |
| 3252. | I | H | CF₃ | CH₂CN | Cl |
| 3253. | I | H | Me | Ph | Me |
| 3254. | I | H | Me | Ph | Cl |
| 3255. | I | H | Et | Ph | Cl |
| 3256. | I | H | Pr | Ph | Cl |
| 3257. | I | H | iPr | Ph | Cl |
| 3258. | I | H | CF₃ | Ph | Cl |
| 3259. | I | H | CF₃ | Ph | Me |
| 3260. | I | H | CF₃ | Ph | CF₃ |
| 3261. | I | H | CF₃ | Ph | F |
| 3262. | I | H | CF₃ | Ph | OMe |
| 3263. | I | H | CF₃ | Ph | OEt |
| 3264. | I | H | CF₃ | Ph | OCHF₂ |
| 3265. | I | H | CF₃ | Ph | CN |
| 3266. | I | H | CF₃ | Ph(4-Cl) | Cl |
| 3267. | I | H | Me | Me | OCH₂CF₃ |
| 3268. | I | H | CF₃ | Me | 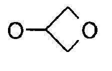 |
| 3269. | I | H | CF₃ | Me | H |
| 3270. | I | H | CF₃ | Me | OCH₂CH₂OMe |
| 3271. | I | H | CF₃ | Me | SMe |
| 3272. | I | H | CF₃ | Me | OCH₂CH₂CH₂F |
| 3273. | I | H | CF₃ | Me | OCH(CH₂F)₂ |
| 3274. | I | H | CF₃ | Me | OCH₂CF₂CHF₂ |
| 3275. | I | H | CF₃ | Me | OCH₂CF=CH₂ |
| 3276. | I | H | CF₃ | Me | OCH(Me)CF₃ |
| 3277. | I | H | CF₃ | Me | OCH(Me)CH₂F |
| 3278. | I | H | OCH₂CF₃ | Me | CF₃ |
| 3279. | I | H | OCH₂CF₃ | Me | CHF₂ |
| 3280. | I | H | CHF₂ | Me | CHF₂ |
| 3281. | I | H | CF₃ | Me | CHF₂ |
| 3282. | I | H | Cl | Me | OCHF₂ |
| 3283. | I | H | Br | Me | OCHF₂ |
| 3284. | I | H | Br | Me | CF₃ |
| 3285. | H | F | CF₃ | Ph | Cl |
| 3286. | H | F | CF₃ | tBu | Cl |
| 3287. | H | F | CF₃ | CHF₂ | Cl |
| 3288. | H | F | Cl | CHF₂ | CF₃ |
| 3289. | H | F | CF₃ | Me | OMe |
| 3290. | H | F | CF₃ | Me | CN |
| 3291. | H | F | Cl | Et | Cl |
| 3292. | H | F | CHF₂ | Me | Cl |
| 3293. | H | F | Me | Me | Me |
| 3294. | H | F | Me | Me | Cl |
| 3295. | H | F | Cl | Me | Cl |
| 3296. | H | F | CF₃ | Me | Cl |
| 3297. | H | F | Cl | Me | CF₃ |
| 3298. | H | F | CF₃ | Me | F |
| 3299. | H | F | OMe | Me | CF₃ |
| 3300. | H | F | CF₃ | Me | OEt |
| 3301. | H | F | CF₃ | Me | OCHF₂ |
| 3302. | H | F | OCHF₂ | Me | CF₃ |
| 3303. | H | F | CF₃ | Me | OCH₂CHF₂ |
| 3304. | H | F | CF₃ | Me | OCH₂CF₃ |
| 3305. | H | F | CF₃ | Me | OCH₂CN |
| 3306. | H | F | CF₃ | Me | SO₂Me |
| 3307. | H | F | CF₃ | Me | SEt |
| 3308. | H | F | CF₃ | Me | Me |
| 3309. | H | F | CF₃ | Me | Et |
| 3310. | H | F | CF₃ | Et | Cl |
| 3311. | H | F | Cl | Et | CF₃ |
| 3312. | H | F | CF₃ | iPr | Cl |
| 3313. | H | F | Cl | iPr | CF₃ |
| 3314. | H | F | CF₃ | tBu | Cl |
| 3315. | H | F | Cl | tBu | CF₃ |
| 3316. | H | F | CF₃ | cPen | Cl |
| 3317. | H | F | Cl | cPen | CF₃ |
| 3318. | H | F | CF₃ | CH₂cPr | Cl |
| 3319. | H | F | Cl | CH₂cPr | CF₃ |
| 3320. | H | F | CF₃ | CH₂CH=CH₂ | Cl |
| 3321. | H | F | Cl | CH₂CH=CH₂ | CF₃ |
| 3322. | H | F | CF₃ | CHF₂ | OMe |
| 3323. | H | F | OMe | CHF₂ | CF₃ |
| 3324. | H | F | CF₃ | CH₂CF₃ | Cl |
| 3325. | H | F | Cl | CH₂CF₃ | CF₃ |
| 3326. | H | F | CF₃ | CH₂OMe | Cl |
| 3327. | H | F | Cl | CH₂OMe | CF₃ |
| 3328. | H | F | CF₃ | CH₂CN | Cl |
| 3329. | H | F | Me | Ph | Me |
| 3330. | H | F | Me | Ph | Cl |
| 3331. | H | F | Et | Ph | Cl |
| 3332. | H | F | Pr | Ph | Cl |
| 3333. | H | F | iPr | Ph | Cl |
| 3334. | H | F | CF₃ | Ph | Cl |
| 3335. | H | F | CF₃ | Ph | Me |
| 3336. | H | F | CF₃ | Ph | CF₃ |
| 3337. | H | F | CF₃ | Ph | F |
| 3338. | H | F | CF₃ | Ph | OMe |
| 3339. | H | F | CF₃ | Ph | OEt |
| 3340. | H | F | CF₃ | Ph | OCHF₂ |
| 3341. | H | F | CF₃ | Ph | CN |
| 3342. | H | F | CF₃ | Ph(4-Cl) | Cl |
| 3343. | H | F | Me | Me | OCH₂CF₃ |
| 3344. | H | F | CF₃ | Me | 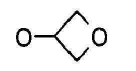 |
| 3345. | H | F | CF₃ | Me | H |
| 3346. | H | F | CF₃ | Me | OCH₂CH₂OMe |
| 3347. | H | F | CF₃ | Me | SMe |
| 3348. | H | F | CF₃ | Me | OCH₂CH₂CH₂F |
| 3349. | H | F | CF₃ | Me | OCH(CH₂F)₂ |
| 3350. | H | F | CF₃ | Me | OCH₂CF₂CHF₂ |
| 3351. | H | F | CF₃ | Me | OCH₂CF=CH₂ |
| 3352. | H | F | CF₃ | Me | OCH(Me)CF₃ |
| 3353. | H | F | CF₃ | Me | OCH(Me)CH₂F |
| 3354. | H | F | OCH₂CF₃ | Me | CF₃ |
| 3355. | H | F | OCH₂CF₃ | Me | CHF₂ |
| 3356. | H | F | CHF₂ | Me | CHF₂ |
| 3357. | H | F | CF₃ | Me | CHF₂ |
| 3358. | H | F | Cl | Me | OCHF₂ |
| 3359. | H | F | Br | Me | OCHF₂ |
| 3360. | H | F | Br | Me | CF₃ |
| 3361. | H | Cl | CF₃ | tBu | Cl |
| 3362. | H | Cl | CF₃ | CHF₂ | Cl |
| 3363. | H | Cl | Cl | CHF₂ | CF₃ |
| 3364. | H | Cl | CF₃ | Me | OMe |
| 3365. | H | Cl | CF₃ | Me | CN |
| 3366. | H | Cl | CHF₂ | Me | Cl |
| 3367. | H | Cl | Me | Me | Me |
| 3368. | H | Cl | Me | Me | Cl |
| 3369. | H | Cl | CF₃ | Me | Cl |
| 3370. | H | Cl | Cl | Me | CF₃ |
| 3371. | H | Cl | CF₃ | Me | F |
| 3372. | H | Cl | OMe | Me | CF₃ |
| 3373. | H | Cl | CF₃ | Me | OCHF₂ |
| 3374. | H | Cl | OCHF₂ | Me | CF₃ |
| 3375. | H | Cl | CF₃ | Me | OCH₂CHF₂ |
| 3376. | H | Cl | CF₃ | Me | OCH₂CF₃ |
| 3377. | H | Cl | CF₃ | Me | SO₂Me |
| 3378. | H | Cl | CF₃ | Me | SEt |
| 3379. | H | Cl | CF₃ | Et | Cl |
| 3380. | H | Cl | CF₃ | iPr | Cl |
| 3381. | H | Cl | CF₃ | tBu | Cl |
| 3382. | H | Cl | Cl | tBu | CF₃ |
| 3383. | H | Cl | CF₃ | cPen | Cl |
| 3384. | H | Cl | CF₃ | CHF₂ | OMe |
| 3385. | H | Cl | CF₃ | CH₂CF₃ | Cl |
| 3386. | H | Cl | CF₃ | Ph | OCHF₂ |
| 3387. | H | Cl | CF₃ | Ph | Cl |
| 3388. | H | Cl | Me | Me | OCH₂CF₃ |
| 3389. | H | Cl | CF₃ | Me | 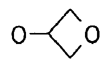 |
| 3390. | H | Cl | CF₃ | Me | H |
| 3391. | H | Cl | CF₃ | Me | OCH₂CH₂OMe |
| 3392. | H | Cl | CF₃ | Me | SMe |
| 3393. | H | Cl | CF₃ | Me | OCH₂CH₂CH₂F |
| 3394. | H | Cl | CF₃ | Me | OCH(CH₂F)₂ |
| 3395. | H | Cl | CF₃ | Me | OCH₂CF₂CHF₂ |
| 3396. | H | Cl | CF₃ | Me | OCH₂CF=CH₂ |
| 3397. | H | Cl | CF₃ | Me | OCH(Me)CF₃ |
| 3398. | H | Cl | CF₃ | Me | OCH(Me)CH₂F |
| 3399. | H | Cl | OCH₂CF₃ | Me | CF₃ |
| 3400. | H | Cl | OCH₂CF₃ | Me | CHF₂ |
| 3401. | H | Cl | CHF₂ | Me | CHF₂ |
| 3402. | H | Cl | CF₃ | Me | CHF₂ |
| 3403. | H | Cl | Cl | Me | OCHF₂ |
| 3404. | H | Cl | Br | Me | OCHF₂ |
| 3405. | H | Cl | Br | Me | CF₃ |
| 3406. | H | Br | CF₃ | tBu | Cl |
| 3407. | H | Br | CF₃ | CHF₂ | Cl |
| 3408. | H | Br | Cl | CHF₂ | CF₃ |
| 3409. | H | Br | CF₃ | Me | OMe |
| 3410. | H | Br | CF₃ | Me | CN |
| 3411. | H | Br | CHF₂ | Me | Cl |
| 3412. | H | Br | Me | Me | Me |
| 3413. | H | Br | Me | Me | Cl |
| 3414. | H | Br | CF₃ | Me | Cl |
| 3415. | H | Br | Cl | Me | CF₃ |
| 3416. | H | Br | CF₃ | Me | F |
| 3417. | H | Br | OMe | Me | CF₃ |
| 3418. | H | Br | CF₃ | Me | OCHF₂ |
| 3419. | H | Br | OCHF₂ | Me | CF₃ |
| 3420. | H | Br | CF₃ | Me | OCH₂CHF₂ |
| 3421. | H | Br | CF₃ | Me | OCH₂CF₃ |
| 3422. | H | Br | CF₃ | Me | SO₂Me |
| 3423. | H | Br | CF₃ | Me | SEt |
| 3424. | H | Br | CF₃ | Et | Cl |
| 3425. | H | Br | CF₃ | iPr | Cl |
| 3426. | H | Br | CF₃ | tBu | Cl |
| 3427. | H | Br | Cl | tBu | CF₃ |
| 3428. | H | Br | CF₃ | cPen | Cl |
| 3429. | H | Br | CF₃ | CHF₂ | OMe |
| 3430. | H | Br | CF₃ | CH₂CF₃ | Cl |
| 3431. | H | Br | CF₃ | Ph | OCHF₂ |
| 3432. | H | Br | CF₃ | Ph | Cl |
| 3433. | H | Br | Me | Me | OCH₂CF₃ |
| 3434. | H | Br | CF₃ | Me | 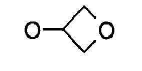 |
| 3435. | H | Br | CF₃ | Me | H |
| 3436. | H | Br | CF₃ | Me | OCH₂CH₂OMe |
| 3437. | H | Br | CF₃ | Me | SMe |
| 3438. | H | Br | CF₃ | Me | OCH₂CH₂CH₂F |
| 3439. | H | Br | CF₃ | Me | OCH(CH₂F)₂ |
| 3440. | H | Br | CF₃ | Me | OCH₂CF₂CHF₂ |
| 3441. | H | Br | CF₃ | Me | OCH₂CF=CH₂ |
| 3442. | H | Br | CF₃ | Me | OCH(Me)CF₃ |
| 3443. | H | Br | CF₃ | Me | OCH(Me)CH₂F |
| 3444. | H | Br | OCH₂CF₃ | Me | CF₃ |
| 3445. | H | Br | OCH₂CF₃ | Me | CHF₂ |
| 3446. | H | Br | CHF₂ | Me | CHF₂ |
| 3447. | H | Br | CF₃ | Me | CHF₂ |
| 3448. | H | Br | Cl | Me | OCHF₂ |
| 3449. | H | Br | Br | Me | OCHF₂ |
| 3450. | H | Br | Br | Me | CF₃ |
| 3451. | Me | H | CF₃ | tBu | Cl |
| 3452. | Me | H | CF₃ | CHF₂ | Cl |
| 3453. | Me | H | Cl | CHF₂ | CF₃ |
| 3454. | Me | H | CF₃ | Me | OMe |
| 3455. | Me | H | CF₃ | Me | CN |
| 3456. | Me | H | CHF₂ | Me | Cl |
| 3457. | Me | H | Me | Me | Me |
| 3458. | Me | H | Me | Me | Cl |
| 3459. | Me | H | CF₃ | Me | Cl |
| 3460. | Me | H | Cl | Me | CF₃ |
| 3461. | Me | H | CF₃ | Me | F |
| 3462. | Me | H | OMe | Me | CF₃ |
| 3463. | Me | H | CF₃ | Me | OCHF₂ |
| 3464. | Me | H | OCHF₂ | Me | CF₃ |
| 3465. | Me | H | CF₃ | Me | OCH₂CHF₂ |
| 3466. | Me | H | CF₃ | Me | OCH₂CF₃ |
| 3467. | Me | H | CF₃ | Me | SO₂Me |
| 3468. | Me | H | CF₃ | Me | SEt |
| 3469. | Me | H | CF₃ | Et | Cl |
| 3470. | Me | H | CF₃ | iPr | Cl |
| 3471. | Me | H | CF₃ | tBu | Cl |
| 3472. | Me | H | Cl | tBu | CF₃ |
| 3473. | Me | H | CF₃ | cPen | Cl |
| 3474. | Me | H | CF₃ | CHF₂ | OMe |
| 3475. | Me | H | CF₃ | CH₂CF₃ | Cl |
| 3476. | Me | H | CF₃ | Ph | OCHF₂ |
| 3477. | Me | H | CF₃ | Ph | Cl |
| 3478. | Me | H | Me | Me | OCH₂CF₃ |
| 3479. | Me | H | CF₃ | Me | 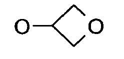 |
| 3480. | Me | H | CF₃ | Me | H |
| 3481. | Me | H | CF₃ | Me | OCH₂CH₂OMe |
| 3482. | Me | H | CF₃ | Me | SMe |
| 3483. | Me | H | CF₃ | Me | OCH₂CH₂CH₂F |
| 3484. | Me | H | CF₃ | Me | OCH(CH₂F)₂ |
| 3485. | Me | H | CF₃ | Me | OCH₂CF₂CHF₂ |
| 3486. | Me | H | CF₃ | Me | OCH₂CF=CH₂ |
| 3487. | Me | H | CF₃ | Me | OCH(Me)CF₃ |
| 3488. | Me | H | CF₃ | Me | OCH(Me)CH₂F |
| 3489. | Me | H | OCH₂CF₃ | Me | CF₃ |
| 3490. | Me | H | OCH₂CF₃ | Me | CHF₂ |
| 3491. | Me | H | CHF₂ | Me | CHF₂ |
| 3492. | Me | H | CF₃ | Me | CHF₂ |
| 3493. | Me | H | Cl | Me | OCHF₂ |
| 3494. | Me | H | Br | Me | OCHF₂ |
| 3495. | Me | H | Br | Me | CF₃ |
| 3496. | NO₂ | H | CF₃ | tBu | Cl |
| 3497. | NO₂ | H | CF₃ | CHF₂ | Cl |
| 3498. | NO₂ | H | Cl | CHF₂ | CF₃ |
| 3499. | NO₂ | H | CF₃ | Me | OMe |
| 3500. | NO₂ | H | CF₃ | Me | CN |
| 3501. | NO₂ | H | CHF₂ | Me | Cl |
| 3502. | NO₂ | H | Me | Me | Me |
| 3503. | NO₂ | H | Me | Me | Cl |
| 3504. | NO₂ | H | CF₃ | Me | Cl |
| 3505. | NO₂ | H | Cl | Me | CF₃ |
| 3506. | NO₂ | H | CF₃ | Me | F |
| 3507. | NO₂ | H | OMe | Me | CF₃ |
| 3508. | NO₂ | H | CF₃ | Me | OCHF₂ |
| 3509. | NO₂ | H | OCHF₂ | Me | CF₃ |
| 3510. | NO₂ | H | CF₃ | Me | OCH₂CHF₂ |
| 3511. | NO₂ | H | CF₃ | Me | OCH₂CF₃ |
| 3512. | NO₂ | H | CF₃ | Me | SO₂Me |
| 3513. | NO₂ | H | CF₃ | Me | SEt |
| 3514. | NO₂ | H | CF₃ | Et | Cl |
| 3515. | NO₂ | H | CF₃ | iPr | Cl |
| 3516. | NO₂ | H | CF₃ | tBu | Cl |
| 3517. | NO₂ | H | Cl | tBu | CF₃ |
| 3518. | NO₂ | H | CF₃ | cPen | Cl |
| 3519. | NO₂ | H | CF₃ | CHF₂ | OMe |
| 3520. | NO₂ | H | CF₃ | CH₂CF₃ | Cl |
| 3521. | NO₂ | H | CF₃ | Ph | OCHF₂ |
| 3522. | NO₂ | H | CF₃ | Ph | Cl |
| 3523. | NO₂ | H | Me | Me | OCH₂CF₃ |
| 3524. | NO₂ H | | CF₃ | Me | 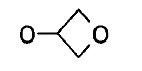 |
| 3525. | NO₂ | H | CF₃ | Me | H |
| 3526. | NO₂ | H | CF₃ | Me | OCH₂CH₂OMe |
| 3527. | NO₂ | H | CF₃ | Me | SMe |
| 3528. | NO₂ | H | CF₃ | Me | OCH₂CH₂CH₂F |
| 3529. | NO₂ | H | CF₃ | Me | OCH(CH₂F)₂ |
| 3530. | NO₂ | H | CF₃ | Me | OCH₂CF₂CHF₂ |
| 3531. | NO₂ | H | CF₃ | Me | OCH₂CF=CH₂ |
| 3532. | NO₂ | H | CF₃ | Me | OCH(Me)CF₃ |
| 3533. | NO₂ | H | CF₃ | Me | OCH(Me)CH₂F |
| 3534. | NO₂ | H | OCH₂CF₃ | Me | CF₃ |
| 3535. | NO₂ | H | OCH₂CF₃ | Me | CHF₂ |
| 3536. | NO₂ | H | CHF₂ | Me | CHF₂ |
| 3537. | NO₂ | H | CF₃ | Me | CHF₂ |
| 3538. | NO₂ | H | Cl | Me | OCHF₂ |
| 3539. | NO₂ | H | Br | Me | OCHF₂ |
| 3540. | NO₂ | H | Br | Me | CF₃ |
| 3541. | Cl | Cl | CF₃ | tBu | Cl |
| 3542. | Cl | Cl | CF₃ | CHF₂ | Cl |
| 3543. | Cl | Cl | Cl | CHF₂ | CF₃ |
| 3544. | Cl | Cl | CF₃ | Me | OMe |
| 3545. | Cl | Cl | CF₃ | Me | CN |
| 3546. | Cl | Cl | CHF₂ | Me | Cl |
| 3547. | Cl | Cl | Me | Me | Me |
| 3548. | Cl | Cl | Me | Me | Cl |
| 3549. | Cl | Cl | CF₃ | Me | Cl |
| 3550. | Cl | Cl | Cl | Me | CF₃ |
| 3551. | Cl | Cl | CF₃ | Me | F |
| 3552. | Cl | Cl | OMe | Me | CF₃ |
| 3553. | Cl | Cl | CF₃ | Me | OCHF₂ |
| 3554. | Cl | Cl | OCHF₂ | Me | CF₃ |
| 3555. | Cl | Cl | CF₃ | Me | OCH₂CHF₂ |
| 3556. | Cl | Cl | CF₃ | Me | OCH₂CF₃ |
| 3557. | Cl | Cl | CF₃ | Me | SO₂Me |
| 3558. | Cl | Cl | CF₃ | Me | SEt |
| 3559. | Cl | Cl | CF₃ | Et | Cl |
| 3560. | Cl | Cl | CF₃ | iPr | Cl |
| 3561. | Cl | Cl | CF₃ | tBu | Cl |
| 3562. | Cl | Cl | Cl | tBu | CF₃ |
| 3563. | Cl | Cl | CF₃ | cPen | Cl |
| 3564. | Cl | Cl | CF₃ | CHF₂ | OMe |
| 3565. | Cl | Cl | CF₃ | CH₂CF₃ | Cl |
| 3566. | Cl | Cl | CF₃ | Ph | OCHF₂ |
| 3567. | Cl | Cl | CF₃ | Ph | Cl |
| 3568. | Cl | Cl | Me | Me | OCH₂CF₃ |
| 3569. | Cl | Cl | CF₃ | Me | 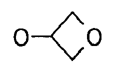 |
| 3570. | Cl | Cl | CF₃ | Me | H |
| 3571. | Cl | Cl | CF₃ | Me | OCH₂CH₂OMe |
| 3572. | Cl | Cl | CF₃ | Me | SMe |
| 3573. | Cl | Cl | CF₃ | Me | OCH₂CH₂CH₂F |
| 3574. | Cl | Cl | CF₃ | Me | OCH(CH₂F)₂ |
| 3575. | Cl | Cl | CF₃ | Me | OCH₂CF₂CHF₂ |
| 3576. | Cl | Cl | CF₃ | Me | OCH₂CF=CH₂ |
| 3577. | Cl | Cl | CF₃ | Me | OCH(Me)CF₃ |
| 3578. | Cl | Cl | CF₃ | Me | OCH(Me)CH₂F |
| 3579. | Cl | Cl | OCH₂CF₃ | Me | CF₃ |
| 3580. | Cl | Cl | OCH₂CF₃ | Me | CHF₂ |
| 3581. | Cl | Cl | CHF₂ | Me | CHF₂ |
| 3582. | Cl | Cl | CF₃ | Me | CHF₂ |
| 3583. | Cl | Cl | Cl | Me | OCHF₂ |
| 3584. | Cl | Cl | Br | Me | OCHF₂ |
| 3585. | Cl | Cl | Br | Me | CF₃ |
| 3586. | Cl | Me | CF₃ | tBu | Cl |
| 3587. | Cl | Me | CF₃ | CHF₂ | Cl |
| 3588. | Cl | Me | Cl | CHF₂ | CF₃ |
| 3589. | Cl | Me | CF₃ | Me | OMe |
| 3590. | Cl | Me | CF₃ | Me | CN |
| 3591. | Cl | Me | CHF₂ | Me | Cl |
| 3592. | Cl | Me | Me | Me | Me |
| 3593. | Cl | Me | Me | Me | Cl |
| 3594. | Cl | Me | CF₃ | Me | Cl |
| 3595. | Cl | Me | Cl | Me | CF₃ |
| 3596. | Cl | Me | CF₃ | Me | F |
| 3597. | Cl | Me | OMe | Me | CF₃ |
| 3598. | Cl | Me | CF₃ | Me | OCHF₂ |
| 3599. | Cl | Me | OCHF₂ | Me | CF₃ |
| 3600. | Cl | Me | CF₃ | Me | OCH₂CHF₂ |
| 3601. | Cl | Me | CF₃ | Me | OCH₂CF₃ |
| 3602. | Cl | Me | CF₃ | Me | SO₂Me |
| 3603. | Cl | Me | CF₃ | Me | SEt |
| 3604. | Cl | Me | CF₃ | Et | Cl |
| 3605. | Cl | Me | CF₃ | iPr | Cl |
| 3606. | Cl | Me | CF₃ | tBu | Cl |
| 3607. | Cl | Me | Cl | tBu | CF₃ |
| 3608. | Cl | Me | CF₃ | cPen | Cl |
| 3609. | Cl | Me | CF₃ | CHF₂ | OMe |
| 3610. | Cl | Me | CF₃ | CH₂CF₃ | Cl |
| 3611. | Cl | Me | CF₃ | Ph | OCHF₂ |
| 3612. | Cl | Me | CF₃ | Ph | Cl |
| 3613. | Cl | Me | Me | Me | OCH₂CF₃ |
| 3614. | Cl | Me | CF₃ | Me | 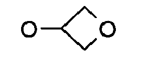 |
| 3615. | Cl | Me | CF₃ | Me | H |
| 3616. | Cl | Me | CF₃ | Me | OCH₂CH₂OMe |
| 3617. | Cl | Me | CF₃ | Me | SMe |
| 3618. | Cl | Me | CF₃ | Me | OCH₂CH₂CH₂F |
| 3619. | Cl | Me | CF₃ | Me | OCH(CH₂F)₂ |
| 3620. | Cl | Me | CF₃ | Me | OCH₂CF₂CHF₂ |
| 3621. | Cl | Me | CF₃ | Me | OCH₂CF=CH₂ |
| 3622. | Cl | Me | CF₃ | Me | OCH(Me)CF₃ |
| 3623. | Cl | Me | CF₃ | Me | OCH(Me)CH₂F |
| 3624. | Cl | Me | OCH₂CF₃ | Me | CF₃ |
| 3625. | Cl | Me | OCH₂CF₃ | Me | CHF₂ |
| 3626. | Cl | Me | CHF₂ | Me | CHF₂ |
| 3627. | Cl | Me | CF₃ | Me | CHF₂ |
| 3628. | Cl | Me | Cl | Me | OCHF₂ |
| 3629. | Cl | Me | Br | Me | OCHF₂ |
| 3630. | Cl | Me | Br | Me | CF₃ |

NMR-Daten wurden bei 400 MHz und in CDCl₃ als Lösemittel gemessen. Die chemische Verschiebung δ ist in ppm angegeben (TMS Standard).

### B. Formulierungsbeispiele

a) Ein Stäubemittel wird erhalten, indem man 10 Gew. Teile einer Verbindung der Formel (I) und/oder deren Salze und 90 Gew. Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew. Teile kaolinhaltigen Quarz als Inertstoff, 10 Gewichtsteile ligninsulfonsaures Kalium und 1 Gew. Teil oleoylmethyltaurinsaures Natrium als Netz und Dispergiermittel mischt und in einer Stiftmühle mahlt.
c) Ein in Wasser leicht dispergierbares Dispersionskonzentrat wird erhalten, indem man 20 Gew. Teile einer Verbindung der Formel (I) und/oder deren Salze mit 6 Gew. Teilen Alkylphenolpolyglykolether (®Triton X 207), 3 Gew. Teilen Isotridecanolpolyglykolether (8 EO) und 71 Gew. Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew. Teilen einer Verbindung der Formel (I) und/oder deren Salze, 75 Gew. Teilen Cyclohexanon als Lösungsmittel und 10 Gew. Teilen oxethyliertes Nonylphenol als Emulgator.
e) Ein in Wasser dispergierbares Granulat wird erhalten indem man
   75 Gew. Teile einer Verbindung der Formel (I) und/oder deren Salze,
   10 Gew. Teile ligninsulfonsaures Calcium,
   5 Gew. Teile Natriumlaurylsulfat,
   3 Gew. Teile Polyvinylalkohol und
   7 Gew. Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew. Teile einer Verbindung der Formel (I) und/oder deren Salze,
   5 Gew. Teile 2,2' dinaphthylmethan 6,6' disulfonsaures Natrium
   2 Gew. Teile oleoylmethyltaurinsaures Natrium,
   1 Gew. Teil Polyvinylalkohol,
   17 Gew. Teile Calciumcarbonat und
   50 Gew. Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

### 1. Herbizide Wirkung bzw. Kulturpflanzenverträglichkeit im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten erfindungsgemäßen Verbindungen werden dann als wäßrige Suspension mit einer Wasseraufwandmenge von umgerechnet 600 l/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Nach ca. 3 Wochen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Wie die Ergebnisse zeigen, weisen erfindungsgemäße Verbindungen eine gute herbizide Vorauflaufwirksamkeit gegen ein breites Spektrum von Ungräsern und Unkräutern auf. Beispielsweise haben die Verbindungen Nr. 50, 51, 506, 734, 735 und andere Verbindungen aus der Tabellen 1 - 3 sehr gute herbizide Wirkung gegen Schadpflanzen wie beispielsweise Avena fatua, Stellaria media, Echinochloa crus galli, Lolium multiflorum, Setaria viridis, Amaranthus retroflexus, Veronica persica, Matricaria inodora, Fallopia convolvulus und Alopecurus myosuroides im Vorauflaufverfahren bei einer Aufwandmenge von 0.32 kg und weniger Aktivsubstanz pro Hektar.

Gleichzeitig lassen erfindungsgemäße Verbindungen zweikeimblättrige Kulturen wie Raps im Vorauflaufverfahren selbst bei hohen Wirkstoffdosierungen ungeschädigt. Einige Substanzen schonen darüber hinaus auch Gramineen Kulturen wie Mais. Die erfindungsgemäßen Verbindungen zeigen teilweise eine hohe Selektivität und eignen sich deshalb im Vorauflaufverfahren zur Bekämpfung von unerwünschtem Pflanzenwuchs in landwirtschaftlichen Kulturen.

### 2. Herbizide Wirkung bzw. Kulturpflanzenverträglichkeit im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) formulierten erfindungsgemäßen Verbindungen werden dann als wäßrige Suspension mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Wie die Ergebnisse zeigen, weisen erfindungsgemäße Verbindungen eine gute herbizide Nachauflaufwirksamkeit gegen mehrere Ungräser bzw. Unkräuter auf. Beispielsweise haben die Verbindungen Nr. 50, 51, 506 und andere Verbindungen aus der Tabellen 1 - 3 sehr gute herbizide Wirkung gegen Schadpflanzen wie beispielsweise Echinochloa crus galli, Lolium multiflorum und Veronica persica im Nachauflaufverfahren bei einer Aufwandmenge von 0.32 kg und weniger Aktivsubstanz pro Hektar.

Gleichzeitig lassen erfindungsgemäße Verbindungen zweikeimblättrige Kulturen wie Raps im Nachauflaufverfahren selbst bei hohen Wirkstoffdosierungen ungeschädigt. Einige Substanzen schonen darüber hinaus auch Gramineen Kulturen wie Weizen und Mais. Die erfindungsgemäßen Verbindungen zeigen teilweise eine hohe Selektivität und eignen sich deshalb im Nachauflaufverfahren zur Bekämpfung von unerwünschtem Pflanzenwuchs in landwirtschaftlichen Kulturen.

## Patentansprüche

1. Verbindungen der Formel (I) und deren agrochemisch verträglichen Salze 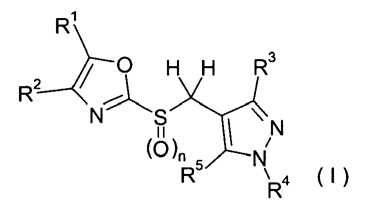 worin die Reste und Indizes die folgende Bedeutung aufweisen:
- n ist gleich 0, 1 oder 2;
- die Substituenten R¹ und R² sind, jeweils unabhängig voneinander, ausgewählt aus der Gruppe, bestehend aus
- Wasserstoff, Halogen, Nitro, Cyano, Formyl, C(O)OH, Hydroxy, Amino,
- (C₁-C₆)-Alkyl, (C₁-C₆)-Alkylcarbonyl, (C₁-C₆)-Alkylcarbonyl-(C₁-C₄)-alkyl, (C₁-C₆)-Alkylcarbonyloxy,
- (C₁-C₆)-Alkoxy, (C₁-C₆)-Alkoxycarbonyl, (C₁-C₆)-Alkoxycarbonyl-(C₁-C₆)-alkyl, (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy,
- (C₂-C₆)-Alkenyl, (C₂-C₆)-Alkenyloxy, (C₂-C₆)-Alkinyl, (C₂-C₆)-Alkinyloxy,
- (C₁-C₆)-Alkylthio, (C₁-C₆)-Alkylsulfinyl, (C₁-C₆)-Alkylsulfonyl, (C₁-C₆)-Alkylsulfonyloxy, (C₁-C₆)-Alkylsulfonyl-(C₁-C₆)-alkyl, (C₁-C₆)-Alkylsulfinyl-(C₁-C₆)-alkyl, C₁-C₆)-Alkylthio-(C₁-C₆)-alkyl, (C₁-C₆)-Alkylthio-(C₁-C₆)-alkoxy,
- Mono-((C₁-C₆)-alkyl)-amino, Di-((C₁-C₆)-alkyl)-amino, N-((C₁-C₆)-Alkanoyl)-amino, Aminocarbonyl-(C₁-C₆)-alkyl, Mono-((C₁-C₆)-alkyl)-aminocarbonyl, Di-((C₁-C₆)-alkyl)-aminocarbonyl, Mono-((C₁-C₆)-alkyl)-aminosulfonyl, Di-((C₁-C₆)-alkyl)-aminosulfonyl,
- (C₃-C₈)-Cycloalkyl, (C₃-C₈)-Cycloalkoxy, (C₃-C₈)-Cycloalkyl-(C₁-C₆)-alkyl, (C₃-C₈)-Cycloalkyl-(C₁-C₆)-alkoxy, (C₃-C₈)-Cycloalkylcarbonyl, (C₃-C₈)-Cycloalkoxycarbonyl,
- (C₃-C₈)-Cycloalkenyl, (C₃-C₈)-Cycloalkenyloxy, (C₃-C₈)-Cycloalkylthio, (C₃-C₈)-Cycloalkylsulfinyl, (C₃-C₈)-Cycloalkylsulfonyl, (C₃-C₈)-Cycloalkylsulfonyloxy,
- Cyano-(C₁-C₆)-alkoxy, Cyano-(C₁-C₆)-alkyl,
- -CONH-SO₂-(C₁-C₆)-Alkyl, -NHCHO, -NHCO-(C₁-C₆)-Alkyl, -NHCO₂-(C₁-C₆)-Alkyl, -NHCONH-(C₁-C₆)-Alkyl, -NHSO₂-(C₁-C₆)-Alkyl,
-OCONH-(C₁-C₆)-Alkyl, (C₁-C₆)-Alkylaminosulfonyl-(C₁-C₂)-alkyl, Di-(C₁-C₆)-alkylaminosulfonyl-(C₁-C₂)-alkyl,
- -C(O)NHR⁹, -C(O)NR⁹R¹⁰,
- wobei R⁹ und R¹⁰ unabhängig voneinander Wasserstoff, (C₁-C₆)-Alkyl, (C₃-C₆)-Cycloalkyl, (C₁-C₆)-Haloalkyl sind, oder
- wobei R⁹ und R¹⁰ zusammen eine (C₁-C₆)-Alkylen-Gruppe bilden, die ein Sauerstoff-, oder Schwefel-Atom oder ein oder zwei Amino oder (C₁-C₆)-Alkylamino-Gruppen enthalten kann,
wobei die zuvor genannten Reste R¹ und R² einfach oder mehrfach und unabhängig voneinander substituiert sein können;
- die Substituenten R³ bis R⁵ sind, jeweils unabhängig voneinander, ausgewählt aus der Gruppe, bestehend aus
- Wasserstoff, Halogen, Hydroxy, Cyano, Nitro, Amino, C(O)OH, Formyl,
- (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl, (C₁-C₆)-Haloalkylcarbonyl-(C₁-C₄)-alkyl, (C₁-C₆)-Alkylcarbonyl-(C₁-C₄)-haloalkyl, (C₁-C₆)-Haloalkylcarbonyl-(C₁-C₄)-haloalkyl,
- (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl,
- (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl,
- (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl,
- (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy,
- (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy,
- (C₆-C₁₄)-Aryl, (C₆-C₁₄)-Aryloxy, (C₆-C₁₄)-Arylcarbonyl, (C₆-C₁₄)-Aryloxycarbonyl,
- (C₆-C₁₄)-Aryl-(C₁-C₆)-alkyl, (C₆-C₁₄)-Aryl-(C₁-C₆)-alkoxy, (C₆-C₁₄)-Aryloxy-(C₁-C₆)-alkyl, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy,
- (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy,
- (C₄-C₁₄)-Arylsulfonyl, (C₆-C₁₄)-Arylthio, (C₆-C₁₄)-Arylsulfinyl,
- Mono-((C₁-C₆)-alkyl)-amino, Mono-((C₁-C₆)-haloalkyl)-amino, Di-((C₁-C₆)-alkyl)-amino, Di-((C₁-C₆)-haloalkyl)-amino, ((C₁-C₆)-Alkyl-(C₁-C₆)-haloalkyl)-amino, N-((C₁-C₆)-Alkanoyl)-amino, N-((C₁-C₆)-Haloalkanoyl)-amino, Aminocarbonyl-(C₁-C₆)-alkyl, Mono-(C₁-C₆)-Alkylaminocarbonyl-(C₁-C₆)-alkyl, Di-(C₁-C₆)-Alkylaminocarbonyl-(C₁-C₆)-alkyl, Mono-((C₁-C₆)-alkyl)-aminocarbonyl,
- (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy,
- (C₃-C₈)-Cycloalkyl, (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cy cloalkyl-(C₁-C₆)-alkoxycarbonyl, (C₃-C₈)-Cyclloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy,
- (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cyclloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy,
- (C₃-C₈)-Cycloalkylthio, (C₃-C₈)-Alkenylthio, (C₃-C₈)-Cycloalkenylthio, (C₃-C₆)-Alkinylthio,
- Hydroxy-(C₁-C₆)-alkyl, Hydroxy-(C₁-C₆)-alkoxy, Cyano-(C₁-C₆)-alkoxy, Cyano-(C₁-C₆)-alkyl,
- 3-Oxetanyloxy-,
- C(O)NR⁹R^{10,}
- wobei R⁹ und R¹⁰ unabhängig voneinander Wasserstoff, (C₁-C₆)-Alkyl, (C₃-C₆)-Cycloalkyl oder (C₁-C₆)-Haloalkyl bedeuten, oder
- wobei R⁹ und R¹⁰ zusammen eine (C₁-C₆)-Alkylen-Gruppe bilden, die ein Sauerstoff-, oder Schwefel-Atom oder ein oder zwei Amino oder (C₁-C₆)-Alkylamino-Gruppen enthalten kann,
wobei die zuvor genannten Reste R³ bis R⁵ einfach oder mehrfach und unabhängig voneinander substituiert sein können, und/oder zueinander benachbarte Reste R⁴ und R⁵ untereinander zyklisch verknüpft sein können und/oder eine (C₁-C₆)-Alkylengruppe bilden, die ein oder mehrere Sauerstoff- und/oder Schwefelatome enthalten können, wobei die (C₁-C₆)-Alkylengruppe durch Halogen einfach oder mehrfach substituiert sein kann und die jeweilgen Halogensubstituenten gleich oder verschieden sein können.

2. Verbindungen nach Anspruch 1, **dadurch gekennzeichnet, dass** R¹ ausgewählt wird aus der Gruppe, bestehend aus H, Halogen, Nitro, Cyano, Carboxyl, (C₁-C₆)-Alkyl, (C₃-C₆)-Cycloalkyl, (C₃-C₆)-Cycloalkoxy, (C₁-C₆)-Alkoxy, (C₁-C₆)-Alkylcarbonyl, (C₃-C₆)-Cycloalkylcarbonyl, (C₁-C₆)-Alkoxycarbonyl, (C₃-C₆)-Cycloalkoxycarbonyl, Mono-((C₁-C₄)-alkyl)-aminocarbonyl, Di-((C₁-C₄)-alkyl)-aminocarbonyl, Mono-((C₁-C₄)-alkyl)-aminosulfonyl, Di-((C₁-C₄)-alkyl)-aminosulfonyl, (C₁-C₄)-Alkylthio, (C₃-C₆)-Cycloalkylthio, (C₁-C₄)-Alkylsulfinyl, (C₃-C₆)-Cycloalkylsulfinyl, (C₁-C₄)-Alkylsulfonyl, (C₃-C₆)-Cycloalkylsulfonyl, (C₁-C₄)-Alkylsulfonyloxy, (C₃-C₆)-Cycloalkylsulfonyloxy, (C₂-C₃)-Alkenyl, (C₂-C₃)-Alkinyl, (C₂-C₃)-Alkenyloxy, (C₂-C₃)-Alkinyloxy, -NHCO-(C₁-C₃)-Alkyl, -NHCO₂-(C₁-C₃)-Alkyl, - NHCONH-(C₁-C₃)-Alkyl, -NHSO₂-(C₁-C₃)-Alkyl, -OCONH-(C₁-C₃)-Alkyl, - CONHR⁹, -CONR⁹R¹⁰, wobei R⁹ und R¹⁰ unabhängig voneinander Wasserstoff, (C₁-C₆)-Alkyl, (C₃-C₆)-Cycloalkyl, (C₁-C₆)-Haloalkyl ist,
wobei der zuvor genannte Rest R¹ einfach oder mehrfach und unabhängig voneinander durch Reste substituiert sein können, welche ausgewählt sind aus der Gruppe, bestehend aus Halogen und (C₁-C₆)-Alkyl.

3. Verbindungen nach Anspruch 1 oder 2, **dadurch gekennzeichnet, dass** R² ausgewählt wird aus der Gruppe, bestehend aus H, Halogen, Nitro, Cyano, Carboxyl, (C₁-C₆)-Alkyl, (C₃-C₆)-Cycloalkyl, (C₃-C₆)-Cycloalkoxy, (C₁-C₆)-Alkoxy, (C₁-C₆)-Alkylcarbonyl, (C₃-C₆)-Cycloalkylcarbonyl, (C₁-C₆)-Alkoxycarbonyl, (C₃-C₆)-Cycloalkoxycarbonyl, Mono-((C₁-C₄)-alkyl)-aminocarbonyl, Di-((C₁-C₄)-alkyl)-aminocarbonyl, Mono-((C₁-C₄)-alkyl)-aminosulfonyl, Di-((C₁-C₄)-alkyl)-aminosulfonyl, (C₁-C₄)-Alkylthio, (C₃-C₆)-Cycloalkylthio, (C₁-C₄)-Alkylsulfinyl, (C₃-C₆)-Cycloalkylsulfinyl, (C₁-C₄)-Alkylsulfonyl, (C₃-C₆)-Cycloalkylsulfonyl, (C₁-C₄)-Alkylsulfonyloxy, (C₃-C₆)-Cycloalkylsulfonyloxy, (C₂-C₃)-Alkenyl, (C₂-C₃)-Alkinyl, (C₂-C₃)-Alkenyloxy, (C₂-C₃)-Alkinyloxy, -NHCO-(C₁-C₃)-Alkyl, -NHCO₂-(C₁-C₃)-Alkyl, - NHCONH-(C₁-C₃)-Alkyl, -NHSO₂-(C₁-C₃)-Alkyl, -OCONH-(C₁-C₃)-Alkyl, - CONHR⁹, -CONR⁹R¹⁰, wobei R⁹ und R¹⁰ unabhängig voneinander Wasserstoff, (C₁-C₆)-Alkyl, (C₃-C₆)-Cycloalkyl, (C₁-C₆)-Haloalkyl ist, wobei der zuvor genannte Reste R² einfach oder mehrfach und unabhängig voneinander durch Reste substituiert sein können, welche ausgewählt sind aus der Gruppe, bestehend aus Halogen und (C₁-C₆)-Alkyl.

4. Verbindungen nach einem der Ansprüche 1 bis 3, **dadurch gekennzeichnet, dass** R³ ausgewählt ist aus der Gruppe, bestehend aus Wasserstoff, Halogen, Hydroxy, Cyano, Nitro, Amino, (C₁-C₄)-Alkyl, (C₃-C₆)-Cycloalkyl, (C₁-C₄)-Haloalkyl, (C₁-C₄)-Alkoxy, (C₁-C₄)-Alkoxy-(C₁-C₂)-alkyl, (C₃-C₆)-Cycloalkoxy, (C₁-C₄)-Haloalkoxy, (C₁-C₄)-Alkylthio, (C₁-C₄)-Alkylthio-(C₁-C₂)-alkyl, (C₁-C₄)-Alkylsulfinyl, (C₁-C₄)-Alkylsulfinyl-(C₁-C₂)-alkyl, (C₁-C₄)-Alkylsulfonyl, (C₁-C₄)-Alkylsulfonyl-(C₁-C₂)-alkyl, Di-(C₁-C₄)-Alkylamino, (C₂-C₄)-Alkenyl, (C₂-C₄)-Alkinyl, (C₃-C₄)-Alkenyloxy, (C₃-C₄)-Alkinyloxy, (C₃-C₆)-Cycloalkyl-(C₁-C₂)-alkoxy, Hydroxy-(C₁-C₂)-alkyl, Hydroxy-(C₁-C₂)-alkoxy, Cyano-(C₁-C₂)-alkoxy, Cyano-(C₁-C₂)-alkyl, Phenyl, Phenyl-(C₁-C₂)-alkyl, Phenyl-(C₁-C₂)-alkoxy, Phenoxy, (C₁-C₄)-Alkylcarbonyloxy, (C₃-C₆)-Cycloalkyl-(C₁-C₂)-alkyl, (C₁-C₄)-Alkylcarbonyl-(C₁-C₂)-alkyl, (C₁-C₄)-Alkoxycarbonyl-(C₁-C₂)-alkyl, Aminocarbonyl-(C₁-C₂)-alkyl und 3-Oxetanyloxy-, - C(O)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann.

5. Verbindungen nach einem der Ansprüche 1 bis 4, **dadurch gekennzeichnet, dass** R⁴ ausgewählt ist aus der Gruppe, bestehend aus Wasserstoff, (C₁-C₄)-Alkyl, (C₁-C₄)-Haloalkyl, Phenyl, Phenyl-(C₁-C₂)-alkyl, (C₃-C₆)-Cycloalkyl; (C₃-C₆)-Cycloalkyl-(C₁-C₂)-alkyl, wobei der Cycloalkylrest gegebenenfalls mit (C₁-C₄)-Alkyl substituiert ist; (C₂-C₄)-Alkenyl, (C₂-C₄)-Alkinyl, (C₁-C₄)-Alkoxy-(C₁-C₂)-alkyl, (C₁-C₄)-Alkylthio-(C₁-C₂)-alkyl, (C₁-C₄)-Alkylsulfinyl-(C₁-C₂)-alkyl, Cyano-(C₁-C₂)-alkyl, (C₁-C₄)-Alkylsulfonyl-(C₁-C₂)-alkyl, (C₁-C₄)-Alkoxycarbonyl-(C₁-C₂)-alkyl, Aminocarbonyl-(C₁-C₂)-alkyl, Mono-(C₁-C₄)-Alkylaminocarbonyl-(C₁-C₂)-alkyl, Di-(C₁-C₄)-Alkylaminocarbonyl-(C₁-C₂)-alkyl, Hydroxy-(C₁-C₄)-alkyl, (C₁-C₄)-Alkylcarbonyl-(C₁-C₄)-alkyl, (C₁-C₄)-Alkoxycarbonyl-(C₁-C₂)-alkyl, (C₁-C₄)-Alkylsulfonyl, Phenylsulfonyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; (C₁-C₄)-Alkylcarbonyl; Phenylcarbonyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; und (C₁-C₄)-Alkoxycarbonyl.

6. Verbindungen nach einem der Ansprüche 1 bis 5, **dadurch gekennzeichnet, dass** R⁵ ausgewählt ist aus der Gruppe, bestehend aus Wasserstoff, Halogen, Hydroxy, Cyano, Nitro, Amino, (C₁-C₄)-Alkyl, (C₁-C₄)-Haloalkyl, (C₃-C₆)-Cycloalkyl, (C₁-C₄)-Alkoxy, (C₁-C₄)-Alkoxy-(C₁-C₄)-alkoxy, (C₁-C₄)-Alkoxy-(C₁-C₂)-alkyl, (C₃-C₆)-Cycloalkoxy, (C₁-C₄)-Haloalkoxy, (C₁-C₄)-Alkylthio, (C₁-C₄)-Alkylthio-(C₁-C₂)-alkyl, (C₁-₄)-Alkylsulfinyl, (C₁-C₄)-Alkylsulfinyl-(C₁-C₂)-alkyl, (C₁-C₄)-Alkylsulfonyl, (C₁-C₄)-Alkylsulfonyl-(C₁-C₂)-alkyl, Di-(C₁-C₄)-Alkylamino, (C₂-C₄)-Alkenyl, (C₂-C₄)-Haloalkenyl, Cyano-(C₁-C₄)-alkyl, (C₂-C₄)-Alkinyl, (C₃-C₄)-Alkenyloxy, (C₃-C₄)-Alkinyloxy, (C₃-C₆)-Cycloalkyl-(C₁-C₂)-alkoxy, Hydroxy-(C₁-C₂)-alkyl, Hydroxy-(C₁-C₂)-alkoxy, Cyano-(C₁-C₂)-alkoxy, Cyano-(C₁-C₂)-alkyl; Phenyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; Phenyl-(C₁-C₂)-alkyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; Phenyl-(C₁-C₂)-alkoxy; Phenoxy, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; (C₁-C₄)-Alkylcarbonyloxy, (C₃-C₆)-Cycloalkyl-(C₁-C₂)-alkyl, (C₁-C₄)-Alkylcarbonyl-(C₁-C₂)-alkyl, (C₁-C₄)-Alkoxycarbonyl-(C₁-C₂)-alkyl, Aminocarbonyl-(C₁-C₂)-alkyl und 3-Oxetanyloxy-, -C(O)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann

7. Verbindungen nach einem der Ansprüche 1 bis 6, **dadurch gekennzeichnet, dass** in der allgemeinen Formel n gleich 1 ist.

8. Verbindungen nach Anspruch 7, **dadurch gekennzeichnet, dass** die Verbindung der allgemeinen Formel (I) mit n = 1 mit einer (R)- oder (S)-Konfiguration mit einer stereochemischen Reinheit von über 50 % bis 100 % vorliegt.

9. Verfahren zur Herstellung einer Verbindung der allgemeinen Formel (III) oder einer Verbindung der allgemeinen Formel (IV), worin R¹, R², R³, R⁴, R⁵ die gemäß einem der Ansprüche 1 bis 6 aufweisende Bedeutung haben 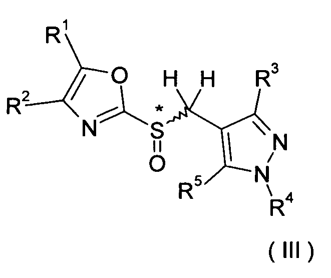 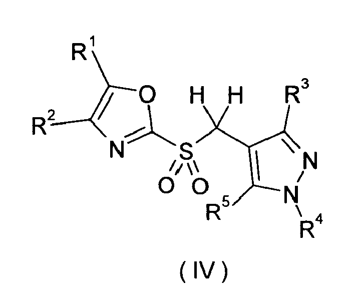 durch Oxidation eines Thioethers der allgemeinen Formel (II) mittels eines Oxidationsmittels, 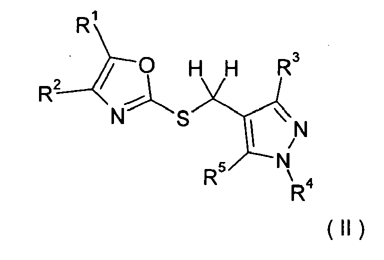 worin R¹, R², R³, R⁴, R⁵ die gemäß einem der Ansprüche 1 bis 5 aufweisende Bedeutung haben, wobei mit einem Äquivalent des Oxidationsmittels die Sulfoxide der allgemeinen Formel (III) oder mit zwei Äquivalenten des Oxidationsmittels die Sulfone der allgemeinen Formel (IV) erhalten werden.

10. Verfahren zur Herstellung eins Thioethers der allgemeinen Formel (II) 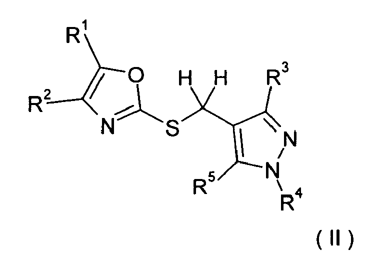 worin R¹, R², R³, R⁴, R⁵ die gemäß einem der Ansprüche 1 bis 5 aufweisenden Bedeutungen haben, gemäß einem der folgenden Verfahren:
(a) Umsetzung von 2-Mercaptooxazol oder einem Oxazol-2(3H)-thion oder einem Salz davon 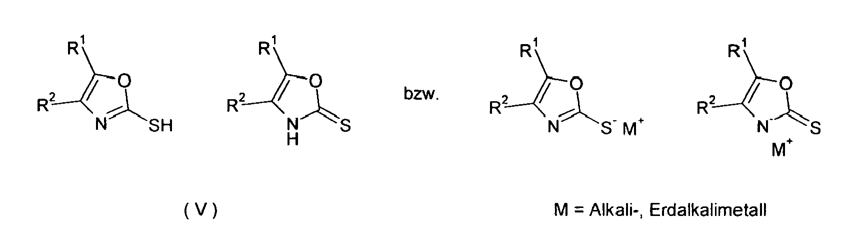 worin R¹, R² die gemäß einem der Ansprüche 1 bis 5 aufweisenden Bedeutungen haben, mit einem (1 H-Pyrazol-4-ylmethyl)-Derivat der allgemeinen Formel (VI) 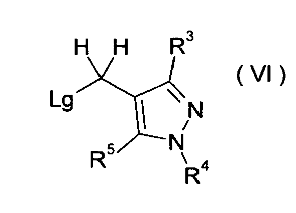 worin R³, R⁴ und R⁵ die gemäß einem der Ansprüche 1 bis 5 aufweisende Bedeutung haben und Lg eine Abgangsgruppe bedeutet, in Gegenwart einer Alkali- oder Erdalkalimetall-Base oder einer organischen Base in einem Lösemittel;
(b) Umsetzung eines Oxazol-Derivates der allgemeinen Formel (VII), 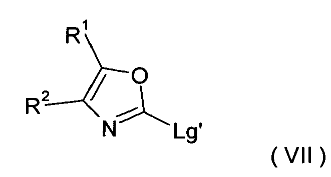 worin R¹, R² die gemäß einem der Ansprüche 1 bis 5 aufweisenden Bedeutungen haben und Lg' eine Abgangsgruppe bedeutet mit einem [(1 H-Pyrazol-4-yl-methyl)]-imidothiocarbamat-Salz der allgemeinen Formel (VIII) 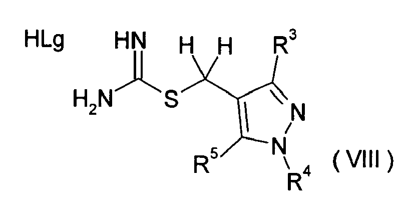 worin R³, R⁴, R⁵ die gemäß einem der Ansprüche 1 bis 5 aufweisende Bedeutung haben und Lg eine Abgangsgruppe bedeutet in einem Eintopfverfahren in Gegenwart einer wäßrigen Alkali- oder Erdalkalimetall-Base oder einer Alkalimetall- oder Erdalkalimetall-Carbonatbase und eines Lösemittels;
(c) Umsetzung eines Oxazol-Derivats der allgemeinen Formel (VII), 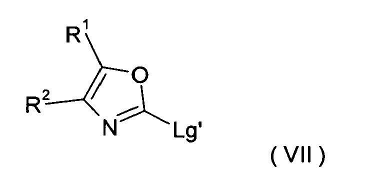 worin R¹, R² die gemäß einem der Ansprüche 1 bis 5 aufweisenden Bedeutungen haben und Lg' eine Abgangsgruppe bedeutet mit einem (1 H-Pyrazol-4-ylmethyl)-Mercaptan der allgemeinen Formel (IX), 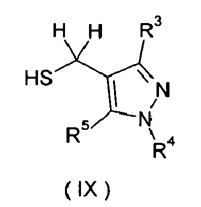 worin R³, R⁴, R⁵ die gemäß einem der Ansprüche 1 bis 5 aufweisende Bedeutung haben in Gegenwart einer Alkali- oder Erdalkalimetall-Base;
(d) Umsetzung eines Oxazol-Derivats der allgemeinen Formel (X), 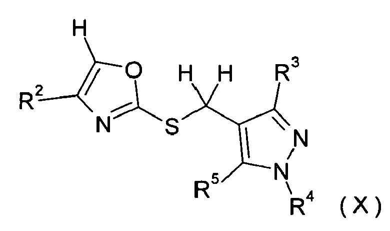 worin R², R³, R⁴, R⁵ die gemäß einem der Ansprüche 1 bis 5 aufweisenden Bedeutungen haben und R¹ einem Halogen oder NO₂ entspricht gemäß dem nachfolgenden Reaktionsschema: 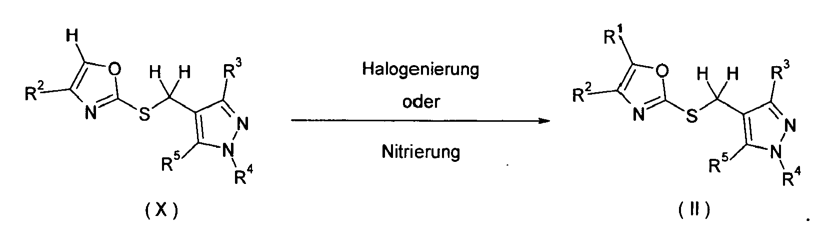
(e) Umsetzung eines (1 H-Pyrazol-4-ylmethyl)disulfid-Derivats der allgemeinen Formel (XV) mit 2-Amino-oxazolen der Formel (XIV) und einem Diazotierungsmittel wie in folgendem Schema dargestellt: 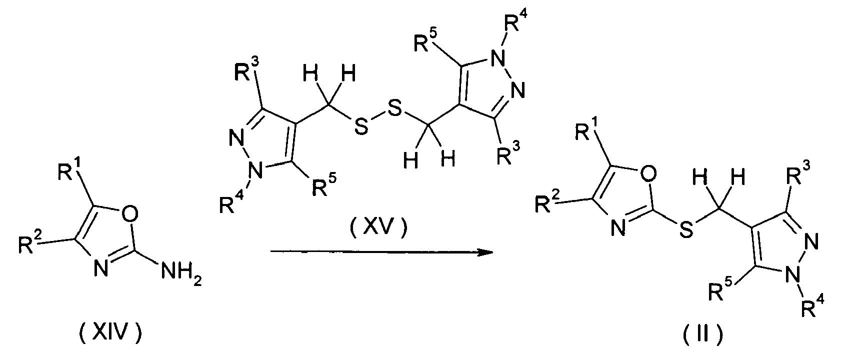 und worin R¹, R², R³, R⁴, R⁵ die gemäß einem der Ansprüche 1 bis 5 aufweisenden Bedeutungen haben.

11. Verfahren nach Anspruch 9, **dadurch gekennzeichnet, dass** die Verbindung der allgemeinen Formel (II), welche gemäß dem Verfahren nach Anspruch 10 erhalten wird, als Ausgangsverbindung in dem Verfahren nach Anspruch 9 eingesetzt wird.

12. Zusammensetzungen, enthaltend mindestens eine Verbindung der allgemeinen Formel (I) gemäß einem der Ansprüche 1 bis 8.

13. Zusammensetzung nach Anspruch 12, **dadurch gekennzeichnet, dass** die Zusammensetzung mindestens einen weiteren Wirkstoff umfasst, welcher ausgewählt ist aus der Gruppe, bestehend aus mindestens einem weiteren Herbizid und mindestens einem Safener.

14. Verwendung der Verbindungen der allgemeinen Formel (I) gemäß einem der Ansprüche 1 bis 8 als Pflanzenwachstumsregulatoren.

15. Verwendung der Zusammensetzungen gemäß Anspruch 12 oder 13 als Pflanzenwachstumsregulatoren.

16. Verwendung nach Anspruch 14 oder 15 zur Pflanzenbekämpfung in speziellen Pflanzenkulturen oder als Pflanzenschutzregulator.
